# EUROPEAN PATENT SPECIFICATION

(11) **EP 3 906 970 B1**
(45) Date of publication and mention of the grant of the patent: **06.07.2022**
(21) Application number: 20173670.9
(22) Date of filing: 08.05.2020
(51) Int. Cl.: A61K 39/12, A61K 39/00, A61P 31/14, A61K 45/06

(54) **PARAPOXVIRUS FOR CONDITIONING AND TREATMENT OF CORONAVIRUS INFECTIONS**
PARAPOXVIRUS ZUR KONDITIONIERUNG UND BEHANDLUNG VON CORONAVIRUS-INFEKTIONEN
PARAPOXVIRUS POUR LE CONDITIONNEMENT ET LE TRAITEMENT D'INFECTIONS À CORONAVIRUS

(43) Date of publication of application: 10.11.2021
(73) Proprietor: AiCuris GmbH & Co. KG, 42117 Wuppertal (DE)
(72) Inventor: PAULSEN, Daniela, 1272 GN Huizen (NL); BIRKMANN, Alexander, 42327 Wuppertal (DE); LISCHKA, Peter, 40477 Düsseldorf (DE); PFAFF, Tamara, 40468 Düsseldorf (DE); ZIMMERMANN, Holger, 42113 Wuppertal (DE)
(74) Representative: Gaentzsch, Peer Christian

(56) References cited:
- WO-A1-2005/017133
- WO-A1-2006/005529
- WO-A1-2019/048640
- WO-A1-2020/058327
- WANG XIAOHUAN ET AL: "Development of small-molecule viral inhibitors targeting various stages of the life cycle of emerging and re-emerging viruses", FRONTIERS OF MEDICINE, HIGHER EDUCATION PRESS, HEIDELBERG, vol. 11, no. 4, 23 November 2017 (2017-11-23), pages 449-461, XP037068530, ISSN: 2095-0217, DOI: 10.1007/S11684-017-0589-5 [retrieved on 2017-11-23]

### SEQUENCE LISTING

<110> AiCuris GmbH & Co.KG
<120> PARAPOXVIRUS FOR CONDITIONING FOR AND TREATMENT OF CORONAVIRUS INFECTIONS
<130> 1092-2 EP
<160> 3
<170> PatentIn version 3.5
<210> 1
   <211> 29903
   <212> RNA
   <213> Artificial Sequence
<220>
   <223> SARS-CoV-2
<400> 1
   auuaaagguu uauaccuucc cagguaacaa accaaccaac uuucgaucuc uuguagaucu 60
   guucucuaaa cgaacuuuaa aaucugugug gcugucacuc ggcugcaugc uuagugcacu 120
   cacgcaguau aauuaauaac uaauuacugu cguugacagg acacgaguaa cucgucuauc 180
   uucugcaggc ugcuuacggu uucguccgug uugcagccga ucaucagcac aucuagguuu 240
   cguccgggug ugaccgaaag guaagaugga gagccuuguc ccugguuuca acgagaaaac 300
   acacguccaa cucaguuugc cuguuuuaca gguucgcgac gugcucguac guggcuuugg 360
   agacuccgug gaggaggucu uaucagaggc acgucaacau cuuaaagaug gcacuugugg 420
   cuuaguagaa guugaaaaag gcguuuugcc ucaacuugaa cagcccuaug uguucaucaa 480
   acguucggau gcucgaacug caccucaugg ucauguuaug guugagcugg uagcagaacu 540
   cgaaggcauu caguacgguc guagugguga gacacuuggu guccuugucc cucauguggg 600
   cgaaauacca guggcuuacc gcaagguucu ucuucguaag aacgguaaua aaggagcugg 660
   uggccauagu uacggcgccg aucuaaaguc auuugacuua ggcgacgagc uuggcacuga 720
   uccuuaugaa gauuuucaag aaaacuggaa cacuaaacau agcaguggug uuacccguga 780
   acucaugcgu gagcuuaacg gaggggcaua cacucgcuau gucgauaaca acuucugugg 840
   cccugauggc uacccucuug agugcauuaa agaccuucua gcacgugcug guaaagcuuc 900
   augcacuuug uccgaacaac uggacuuuau ugacacuaag agggguguau acugcugccg 960
   ugaacaugag caugaaauug cuugguacac ggaacguucu gaaaagagcu augaauugca 1020
   gacaccuuuu gaaauuaaau uggcaaagaa auuugacacc uucaaugggg aauguccaaa 1080
   uuuuguauuu cccuuaaauu ccauaaucaa gacuauucaa ccaagguuug aaaagaaaaa 1140
   gcuugauggc uuuaugggua gaauucgauc ugucuaucca guugcgucac caaaugaaug 1200
   caaccaaaug ugccuuucaa cucucaugaa gugugaucau uguggugaaa cuucauggca 1260
   gacgggcgau uuuguuaaag ccacuugcga auuuuguggc acugagaauu ugacuaaaga 1320
   aggugccacu acuugugguu acuuacccca aaaugcuguu guuaaaauuu auuguccagc    1380
   augucacaau ucagaaguag gaccugagca uagucuugcc gaauaccaua augaaucugg 1440
   cuugaaaacc auucuucgua aggguggucg cacuauugcc uuuggaggcu guguguucuc 1500
   uuauguuggu ugccauaaca agugugccua uuggguucca cgugcuagcg cuaacauagg 1560
   uuguaaccau acagguguug uuggagaagg uuccgaaggu cuuaaugaca accuucuuga 1620
   aauacuccaa aaagagaaag ucaacaucaa uauuguuggu gacuuuaaac uuaaugaaga 1680
   gaucgccauu auuuuggcau cuuuuucugc uuccacaagu gcuuuugugg aaacugugaa 1740
   agguuuggau uauaaagcau ucaaacaaau uguugaaucc ugugguaauu uuaaaguuac 1800
   aaaaggaaaa gcuaaaaaag gugccuggaa uauuggugaa cagaaaucaa uacugagucc 1860
   ucuuuaugca uuugcaucag aggcugcucg uguuguacga ucaauuuucu cccgcacucu 1920
   ugaaacugcu caaaauucug ugcguguuuu acagaaggcc gcuauaacaa uacuagaugg 1980
   aauuucacag uauucacuga gacucauuga ugcuaugaug uucacaucug auuuggcuac 2040
   uaacaaucua guuguaaugg ccuacauuac aggugguguu guucaguuga cuucgcagug    2100
   gcuaacuaac aucuuuggca cuguuuauga aaaacucaaa cccguccuug auuggcuuga 2160
   agagaaguuu aaggaaggug uagaguuucu uagagacggu ugggaaauug uuaaauuuau 2220
   cucaaccugu gcuugugaaa uugucggugg acaaauuguc accugugcaa aggaaauuaa 2280
   ggagaguguu cagacauucu uuaagcuugu aaauaaauuu uuggcuuugu gugcugacuc 2340
   uaucauuauu gguggagcua aacuuaaagc cuugaauuua ggugaaacau uugucacgca 2400
   cucaaaggga uuguacagaa aguguguuaa auccagagaa gaaacuggcc uacucaugcc 2460
   ucuaaaagcc ccaaaagaaa uuaucuucuu agagggagaa acacuuccca cagaaguguu 2520
   aacagaggaa guugucuuga aaacugguga uuuacaacca uuagaacaac cuacuaguga 2580
   agcuguugaa gcuccauugg uugguacacc aguuuguauu aacgggcuua uguugcucga 2640
   aaucaaagac acagaaaagu acugugcccu ugcaccuaau augaugguaa caaacaauac 2700
   cuucacacuc aaaggcggug caccaacaaa gguuacuuuu ggugaugaca cugugauaga 2760
   agugcaaggu uacaagagug ugaauaucac uuuugaacuu gaugaaagga uugauaaagu 2820
   acuuaaugag aagugcucug ccuauacagu ugaacucggu acagaaguaa augaguucgc 2880
   cuguguugug gcagaugcug ucauaaaaac uuugcaacca guaucugaau uacuuacacc 2940
   acugggcauu gauuuagaug aguggaguau ggcuacauac uacuuauuug augagucugg 3000
   ugaguuuaaa uuggcuucac auauguauug uucuuucuac ccuccagaug aggaugaaga 3060
   agaaggugau ugugaagaag aagaguuuga gccaucaacu caauaugagu augguacuga 3120
   agaugauuac caagguaaac cuuuggaauu uggugccacu ucugcugcuc uucaaccuga 3180
   agaagagcaa gaagaagauu gguuagauga ugauagucaa caaacuguug gucaacaaga 3240
   cggcagugag gacaaucaga caacuacuau ucaaacaauu guugagguuc aaccucaauu 3300
   agagauggaa cuuacaccag uuguucagac uauugaagug aauaguuuua gugguuauuu 3360
   aaaacuuacu gacaauguau acauuaaaaa ugcagacauu guggaagaag cuaaaaaggu 3420
   aaaaccaaca gugguuguua augcagccaa uguuuaccuu aaacauggag gagguguugc 3480
   aggagccuua aauaaggcua cuaacaaugc caugcaaguu gaaucugaug auuacauagc 3540
   uacuaaugga ccacuuaaag ugggugguag uuguguuuua agcggacaca aucuugcuaa 3600
   acacugucuu cauguugucg gcccaaaugu uaacaaaggu gaagacauuc aacuucuuaa 3660
   gagugcuuau gaaaauuuua aucagcacga aguucuacuu gcaccauuau uaucagcugg 3720
   uauuuuuggu gcugacccua uacauucuuu aagaguuugu guagauacug uucgcacaaa 3780
   ugucuacuua gcugucuuug auaaaaaucu cuaugacaaa cuuguuucaa gcuuuuugga 3840
   aaugaagagu gaaaagcaag uugaacaaaa gaucgcugag auuccuaaag aggaaguuaa 3900
   gccauuuaua acugaaagua aaccuucagu ugaacagaga aaacaagaug auaagaaaau 3960
   caaagcuugu guugaagaag uuacaacaac ucuggaagaa acuaaguucc ucacagaaaa 4020
   cuuguuacuu uauauugaca uuaauggcaa ucuucaucca gauucugcca cucuuguuag 4080
   ugacauugac aucacuuucu uaaagaaaga ugcuccauau auagugggug auguuguuca 4140
   agaggguguu uuaacugcug ugguuauacc uacuaaaaag gcugguggca cuacugaaau 4200
   gcuagcgaaa gcuuugagaa aagugccaac agacaauuau auaaccacuu acccggguca 4260
   ggguuuaaau gguuacacug uagaggaggc aaagacagug cuuaaaaagu guaaaagugc 4320
   cuuuuacauu cuaccaucua uuaucucuaa ugagaagcaa gaaauucuug gaacuguuuc 4380
   uuggaauuug cgagaaaugc uugcacaugc agaagaaaca cgcaaauuaa ugccugucug 4440
   uguggaaacu aaagccauag uuucaacuau acagcguaaa uauaagggua uuaaaauaca 4500
   agagggugug guugauuaug gugcuagauu uuacuuuuac accaguaaaa caacuguagc 4560
   gucacuuauc aacacacuua acgaucuaaa ugaaacucuu guuacaaugc cacuuggcua 4620
   uguaacacau ggcuuaaauu uggaagaagc ugcucgguau augagaucuc ucaaagugcc 4680
   agcuacaguu ucuguuucuu caccugaugc uguuacagcg uauaaugguu aucuuacuuc 4740
   uucuucuaaa acaccugaag aacauuuuau ugaaaccauc ucacuugcug guuccuauaa 4800
   agauuggucc uauucuggac aaucuacaca acuagguaua gaauuucuua agagagguga 4860
   uaaaagugua uauuacacua guaauccuac cacauuccac cuagauggug aaguuaucac 4920
   cuuugacaau cuuaagacac uucuuucuuu gagagaagug aggacuauua agguguuuac 4980
   aacaguagac aacauuaacc uccacacgca aguuguggac augucaauga cauauggaca 5040
   acaguuuggu ccaacuuauu uggauggagc ugauguuacu aaaauaaaac cucauaauuc 5100
   acaugaaggu aaaacauuuu auguuuuacc uaaugaugac acucuacgug uugaggcuuu 5160
   ugaguacuac cacacaacug auccuaguuu ucuggguagg uacaugucag cauuaaauca 5220
   cacuaaaaag uggaaauacc cacaaguuaa ugguuuaacu ucuauuaaau gggcagauaa 5280
   caacuguuau cuugccacug cauuguuaac acuccaacaa auagaguuga aguuuaaucc 5340
   accugcucua caagaugcuu auuacagagc aagggcuggu gaagcugcua acuuuugugc 5400
   acuuaucuua gccuacugua auaagacagu aggugaguua ggugauguua gagaaacaau 5460
   gaguuacuug uuucaacaug ccaauuuaga uucuugcaaa agagucuuga acguggugug 5520
   uaaaacuugu ggacaacagc agacaacccu uaagggugua gaagcuguua uguacauggg 5580
   cacacuuucu uaugaacaau uuaagaaagg uguucagaua ccuuguacgu gugguaaaca 5640
   agcuacaaaa uaucuaguac aacaggaguc accuuuuguu augaugucag caccaccugc 5700
   ucaguaugaa cuuaagcaug guacauuuac uugugcuagu gaguacacug guaauuacca 5760
   gugugguuac uauaaacaua uaacuucuaa agaaacuuug uauugcauag acggugcuuu 5820
   acuuacaaag uccucagaau acaaaggucc uauuacggau guuuucuaca aagaaaacag 5880
   uuacacaaca accauaaaac caguuacuua uaaauuggau gguguuguuu guacagaaau 5940
   ugacccuaag uuggacaauu auuauaagaa agacaauucu uauuucacag agcaaccaau 6000
   ugaucuugua ccaaaccaac cauauccaaa cgcaagcuuc gauaauuuua aguuuguaug 6060
   ugauaauauc aaauuugcug augauuuaaa ccaguuaacu gguuauaaga aaccugcuuc 6120
   aagagagcuu aaaguuacau uuuucccuga cuuaaauggu gaugugguggcuauugauua 6180
   uaaacacuac acacccucuu uuaagaaagg agcuaaauug uuacauaaac cuauuguuug 6240
   gcauguuaac aaugcaacua auaaagccac guauaaacca aauaccuggu guauacguug 6300
   ucuuuggagc acaaaaccag uugaaacauc aaauucguuu gauguacuga agucagagga 6360
   cgcgcaggga auggauaauc uugccugcga agaucuaaaa ccagucucug aagaaguagu 6420
   ggaaaauccu accauacaga aagacguucu ugaguguaau gugaaaacua ccgaaguugu 6480
   aggagacauu auacuuaaac cagcaaauaa uaguuuaaaa auuacagaag agguuggcca 6540
   cacagaucua auggcugcuu auguagacaa uucuagucuu acuauuaaga aaccuaauga 6600
   auuaucuaga guauuagguu ugaaaacccu ugcuacucau gguuuagcug cuguuaauag 6660
   ugucccuugg gauacuauag cuaauuaugc uaagccuuuu cuuaacaaag uuguuaguac 6720
   aacuacuaac auaguuacac gguguuuaaa ccguguuugu acuaauuaua ugccuuauuu 6780
   cuuuacuuua uugcuacaau uguguacuuu uacuagaagu acaaauucua gaauuaaagc 6840
   aucuaugccg acuacuauag caaagaauac uguuaagagu gucgguaaau uuugucuaga 6900
   ggcuucauuu aauuauuuga agucaccuaa uuuuucuaaa cugauaaaua uuauaauuug 6960
   guuuuuacua uuaaguguuu gccuagguuc uuuaaucuac ucaaccgcug cuuuaggugu 7020
   uuuaaugucu aauuuaggca ugccuucuua cuguacuggu uacagagaag gcuauuugaa 7080
   cucuacuaau gucacuauug caaccuacug uacugguucu auaccuugua guguuugucu 7140
   uagugguuua gauucuuuag acaccuaucc uucuuuagaa acuauacaaa uuaccauuuc 7200
   aucuuuuaaa ugggauuuaa cugcuuuugg cuuaguugca gagugguuuu uggcauauau 7260
   ucuuuucacu agguuuuucu auguacuugg auuggcugca aucaugcaau uguuuuucag 7320
   cuauuuugca guacauuuua uuaguaauuc uuggcuuaug ugguuaauaa uuaaucuugu 7380
   acaaauggcc ccgauuucag cuaugguuag aauguacauc uucuuugcau cauuuuauua 7440
   uguauggaaa aguuaugugc auguuguaga cgguuguaau ucaucaacuu guaugaugug 7500
   uuacaaacgu aauagagcaa caagagucga auguacaacu auuguuaaug guguuagaag 7560
   guccuuuuau gucuaugcua auggagguaa aggcuuuugc aaacuacaca auuggaauug 7620
   uguuaauugu gauacauucu gugcugguag uacauuuauu agugaugaag uugcgagaga 7680
   cuugucacua caguuuaaaa gaccaauaaa uccuacugac cagucuucuu acaucguuga 7740
   uaguguuaca gugaagaaug guuccaucca ucuuuacuuu gauaaagcug gucaaaagac 7800
   uuaugaaaga cauucucucu cucauuuugu uaacuuagac aaccugagag cuaauaacac 7860
   uaaagguuca uugccuauua auguuauagu uuuugauggu aaaucaaaau gugaagaauc 7920
   aucugcaaaa ucagcgucug uuuacuacag ucagcuuaug ugucaaccua uacuguuacu 7980
   agaucaggca uuagugucug auguugguga uagugcggaa guugcaguua aaauguuuga 8040
   ugcuuacguu aauacguuuu caucaacuuu uaacguacca auggaaaaac ucaaaacacu 8100
   aguugcaacu gcagaagcug aacuugcaaa gaaugugucc uuagacaaug ucuuaucuac 8160
   uuuuauuuca gcagcucggc aaggguuugu ugauucagau guagaaacua aagauguugu 8220
   ugaaugucuu aaauugucac aucaaucuga cauagaaguu acuggcgaua guuguaauaa 8280
   cuauaugcuc accuauaaca aaguugaaaa caugacaccc cgugaccuug gugcuuguau 8340
   ugacuguagu gcgcgucaua uuaaugcgca gguagcaaaa agucacaaca uugcuuugau 8400
   auggaacguu aaagauuuca ugucauuguc ugaacaacua cgaaaacaaa uacguagugc 8460
   ugcuaaaaag aauaacuuac cuuuuaaguu gacaugugca acuacuagac aaguuguuaa 8520
   uguuguaaca acaaagauag cacuuaaggg ugguaaaauu guuaauaauu gguugaagca 8580
   guuaauuaaa guuacacuug uguuccuuuu uguugcugcu auuuucuauu uaauaacacc 8640
   uguucauguc augucuaaac auacugacuu uucaagugaa aucauaggau acaaggcuau 8700
   ugaugguggu gucacucgug acauagcauc uacagauacu uguuuugcua acaaacaugc 8760
   ugauuuugac acaugguuua gccagcgugg ugguaguuau acuaaugaca aagcuugccc 8820
   auugauugcu gcagucauaa caagagaagu ggguuuuguc gugccugguu ugccuggcac 8880
   gauauuacgc acaacuaaug gugacuuuuu gcauuucuua ccuagaguuu uuagugcagu 8940
   ugguaacauc uguuacacac caucaaaacu uauagaguac acugacuuug caacaucagc 9000
   uuguguuuug gcugcugaau guacaauuuu uaaagaugcu ucugguaagc caguaccaua 9060
   uuguuaugau accaauguac uagaagguuc uguugcuuau gaaaguuuac gcccugacac 9120
   acguuaugug cucauggaug gcucuauuau ucaauuuccu aacaccuacc uugaagguuc 9180
   uguuagagug guaacaacuu uugauucuga guacuguagg cacggcacuu gugaaagauc 9240
   agaagcuggu guuuguguau cuacuagugg uagaugggua cuuaacaaug auuauuacag 9300
   aucuuuacca ggaguuuucu gugguguaga ugcuguaaau uuacuuacua auauguuuac 9360
   accacuaauu caaccuauug gugcuuugga cauaucagca ucuauaguag cugguggauu 9420
   uguagcuauc guaguaacau gccuugccua cuauuuuaug agguuuagaa gagcuuuugg 9480
   ugaauacagu cauguaguug ccuuuaauac uuuacuauuc cuuaugucau ucacuguacu 9540
   cuguuuaaca ccaguuuacu cauucuuacc ugguguuuau ucuguuauuu acuuguacuu 9600
   gacauuuuau cuuacuaaug auguuucuuu uuuagcacau auucagugga ugguuauguu 9660
   cacaccuuua guaccuuucu ggauaacaau ugcuuauauc auuuguauuu ccacaaagca 9720
   uuucuauugg uucuuuagua auuaccuaaa gagacgugua gucuuuaaug guguuuccuu 9780
   uaguacuuuu gaagaagcug cgcugugcac cuuuuuguua aauaaagaaa uguaucuaaa 9840
   guugcguagu gaugugcuau uaccucuuac gcaauauaau agauacuuag cucuuuauaa 9900
   uaaguacaag uauuuuagug gagcaaugga uacaacuagc uacagagaag cugcuuguug 9960
   ucaucucgca aaggcucuca augacuucag uaacucaggu ucugauguuc uuuaccaacc 10020
   accacaaacc ucuaucaccu cagcuguuuu gcagaguggu uuuagaaaaa uggcauuccc 10080
   aucugguaaa guugaggguu guaugguaca aguaacuugu gguacaacua cacuuaacgg 10140
   ucuuuggcuu gaugacguag uuuacugucc aagacaugug aucugcaccu cugaagacau 10200
   gcuuaacccu aauuaugaag auuuacucau ucguaagucu aaucauaauu ucuugguaca 10260
   ggcugguaau guucaacuca ggguuauugg acauucuaug caaaauugug uacuuaagcu 10320
   uaagguugau acagccaauc cuaagacacc uaaguauaag uuuguucgca uucaaccagg 10380
   acagacuuuu ucaguguuag cuuguuacaa ugguucacca ucugguguuu accaaugugc 10440
   uaugaggccc aauuucacua uuaaggguuc auuccuuaau gguucaugug guaguguugg 10500
   uuuuaacaua gauuaugacu gugucucuuu uuguuacaug caccauaugg aauuaccaac 10560
   uggaguucau gcuggcacag acuuagaagg uaacuuuuau ggaccuuuug uugacaggca 10620
   aacagcacaa gcagcuggua cggacacaac uauuacaguu aauguuuuag cuugguugua 10680
   cgcugcuguu auaaauggag acagguggUU ucucaaucga uuuaccacaa cucuuaauga 10740
   cuuuaaccuu guggcuauga aguacaauua ugaaccucua acacaagacc auguugacau 10800
   acuaggaccu cuuucugcuc aaacuggaau ugccguuuua gauaugugug cuucauuaaa 10860
   agaauuacug caaaauggua ugaauggacg uaccauauug gguagugcuu uauuagaaga 10920
   ugaauuuaca ccuuuugaug uuguuagaca augcucaggu guuacuuucc aaagugcagu 10980
   gaaaagaaca aucaagggua cacaccacug guuguuacuc acaauuuuga cuucacuuuu 11040
   aguuuuaguc cagaguacuc aauggucuuu guucuuuuuu uuguaugaaa augccuuuuu 11100
   accuuuugcu auggguauua uugcuauguc ugcuuuugca augauguuug ucaaacauaa 11160
   gcaugcauuu cucuguuugu uuuuguuacc uucucuugcc acuguagcuu auuuuaauau 11220
   ggucuauaug ccugcuaguu gggugaugcg uauuaugaca ugguuggaua ugguugauac 11280
   uaguuugucu gguuuuaagc uaaaagacug uguuauguau gcaucagcug uaguguuacu 11340
   aauccuuaug acagcaagaa cuguguauga ugauggugcu aggagagugu ggacacuuau 11400
   gaaugucuug acacucguuu auaaaguuua uuaugguaau gcuuuagauc aagccauuuc 11460
   caugugggcu cuuauaaucu cuguuacuuc uaacuacuca gguguaguua caacugucau 11520
   guuuuuggcc agagguauug uuuuuaugug uguugaguau ugcccuauuu ucuucauaac 11580
   ugguaauaca cuucagugua uaaugcuagu uuauuguuuc uuaggcuauu uuuguacuug 11640
   uuacuuuggc cucuuuuguu uacucaaccg cuacuuuaga cugacucuug guguuuauga 11700
   uuacuuaguu ucuacacagg aguuuagaua uaugaauuca cagggacuac ucccacccaa 11760
   gaauagcaua gaugccuuca aacucaacau uaaauuguug gguguuggug gcaaaccuug 11820
   uaucaaagua gccacuguac agucuaaaau gucagaugua aagugcacau caguagucuu 11880
   acucucaguu uugcaacaac ucagaguaga aucaucaucu aaauuguggg cucaaugugu 11940
   ccaguuacac aaugacauuc ucuuagcuaa agauacuacu gaagccuuug aaaaaauggu 12000
   uucacuacuu ucuguuuugc uuuccaugca gggugcugua gacauaaaca agcuuuguga 12060
   agaaaugcug gacaacaggg caaccuuaca agcuauagcc ucagaguuua guucccuucc 12120
   aucauaugca gcuuuugcua cugcucaaga agcuuaugag caggcuguug cuaaugguga 12180
   uucugaaguu guucuuaaaa aguugaagaa gucuuugaau guggcuaaau cugaauuuga 12240
   ccgugaugca gccaugcaac guaaguugga aaagauggcu gaucaagcua ugacccaaau 12300
   guauaaacag gcuagaucug aggacaagag ggcaaaaguu acuagugcua ugcagacaau 12360
   gcuuuucacu augcuuagaa aguuggauaa ugaugcacuc aacaacauua ucaacaaugc 12420
   aagagauggu uguguucccu ugaacauaau accucuuaca acagcagcca aacuaauggu 12480
   ugucauacca gacuauaaca cauauaaaaa uacgugugau gguacaacau uuacuuaugc 12540
   aucagcauug ugggaaaucc aacagguugu agaugcagau aguaaaauug uucaacuuag 12600
   ugaaauuagu auggacaauu caccuaauuu agcauggccu cuuauuguaa cagcuuuaag 12660
   ggccaauucu gcugucaaau uacagaauaa ugagcuuagu ccuguugcac uacgacagau 12720
   gucuugugcu gccgguacua cacaaacugc uugcacugau gacaaugcgu uagcuuacua 12780
   caacacaaca aagggaggua gguuuguacu ugcacuguua uccgauuuac aggauuugaa 12840
   augggcuaga uucccuaaga gugauggaac ugguacuauc uauacagaac uggaaccacc 12900
   uuguagguuu guuacagaca caccuaaagg uccuaaagug aaguauuuau acuuuauuaa 12960
   aggauuaaac aaccuaaaua gagguauggu acuugguagu uuagcugcca caguacgucu 13020
   acaagcuggu aaugcaacag aagugccugc caauucaacu guauuaucuu ucugugcuuu 13080
   ugcuguagau gcugcuaaag cuuacaaaga uuaucuagcu agugggggac aaccaaucac 13140
   uaauuguguu aagauguugu guacacacac ugguacuggu caggcaauaa caguuacacc 13200
   ggaagccaau auggaucaag aauccuuugg uggugcaucg uguugucugu acugccguug 13260
   ccacauagau cauccaaauc cuaaaggauu uugugacuua aaagguaagu auguacaaau 13320
   accuacaacu ugugcuaaug acccuguggg uuuuacacuu aaaaacacag ucuguaccgu 13380
   cugcgguaug uggaaagguu auggcuguag uugugaucaa cuccgcgaac ccaugcuuca 13440
   gucagcugau gcacaaucgu uuuuaaacgg guuugcggug uaagugcagc ccgucuuaca 13500
   ccgugcggca caggcacuag uacugauguc guauacaggg cuuuugacau cuacaaugau 13560
   aaaguagcug guuuugcuaa auuccuaaaa acuaauuguu gucgcuucca agaaaaggac 13620
   gaagaugaca auuuaauuga uucuuacuuu guaguuaaga gacacacuuu cucuaacuac 13680
   caacaugaag aaacaauuua uaauuuacuu aaggauuguc cagcuguugc uaaacaugac 13740
   uucuuuaagu uuagaauaga cggugacaug guaccacaua uaucacguca acgucuuacu 13800
   aaauacacaa uggcagaccu cgucuaugcu uuaaggcauu uugaugaagg uaauugugac 13860
   acauuaaaag aaauacuugu cacauacaau uguugugaug augauuauuu caauaaaaag 13920
   gacugguaug auuuuguaga aaacccagau auauuacgcg uauacgccaa cuuaggugaa 13980
   cguguacgcc aagcuuuguu aaaaacagua caauucugug augccaugcg aaaugcuggu 14040
   auuguuggug uacugacauu agauaaucaa gaucucaaug guaacuggua ugauuucggu 14100
   gauuucauac aaaccacgcc agguagugga guuccuguug uagauucuua uuauucauug 14160
   uuaaugccua uauuaaccuu gaccagggcu uuaacugcag agucacaugu ugacacugac 14220
   uuaacaaagc cuuacauuaa gugggauuug uuaaaauaug acuucacgga agagagguua 14280
   aaacucuuug accguuauuu uaaauauugg gaucagacau accacccaaa uuguguuaac 14340
   uguuuggaug acagaugcau ucugcauugu gcaaacuuua auguuuuauu cucuacagug 14400
   uucccaccua caaguuuugg accacuagug agaaaaauau uuguugaugg uguuccauuu 14460
   guaguuucaa cuggauacca cuucagagag cuagguguug uacauaauca ggauguaaac 14520
   uuacauagcu cuagacuuag uuuuaaggaa uuacuugugu augcugcuga cccugcuaug 14580
   cacgcugcuu cugguaaucu auuacuagau aaacgcacua cgugcuuuuc aguagcugca 14640
   cuuacuaaca auguugcuuu ucaaacuguc aaacccggua auuuuaacaa agacuucuau 14700
   gacuuugcug ugucuaaggg uuucuuuaag gaaggaaguu cuguugaauu aaaacacuuc 14760
   uucuuugcuc aggaugguaa ugcugcuauc agcgauuaug acuacuaucg uuauaaucua 14820
   ccaacaaugu gugauaucag acaacuacua uuuguaguug aaguuguuga uaaguacuuu 14880
   gauuguuacg augguggcug uauuaaugcu aaccaaguca ucgucaacaa ccuagacaaa 14940
   ucagcugguu uuccauuuaa uaaaaugggu aaggcuagac uuuauuauga uucaaugagu 15000
   uaugaggauc aagaugcacu uuucgcauau acaaaacgua augucauccc uacuauaacu 15060
   caaaugaauc uuaaguaugc cauuagugca aagaauagag cucgcaccgu agcuggugug 15120
   ucuaucugua guacuaugac caauagacag uuucaucaaa aauuauugaa aucaauagcc 15180
   gccacuagag gagcuacugu aguaauugga acaagcaaau ucuauggugg uuggcacaac 15240
   auguuaaaaa cuguuuauag ugauguagaa aacccucacc uuaugguugg ggauuauccu 15300
   aaaugugaua gagccaugcc uaacaugcuu agaauuaugg ccucacuugu ucuugcucgc 15360
   aaacauacaa cguguuguag cuugucacac cguuucuaua gauuagcuaa ugagugugcu 15420
   caaguauuga gugaaauggu cauguguggc gguucacuau auguuaaacc agguggaacc 15480
   ucaucaggag augccacaac ugcuuaugcu aauaguguuu uuaacauuug ucaagcuguc 15540
   acggccaaug uuaaugcacu uuuaucuacu gaugguaaca aaauugccga uaaguauguc 15600
   cgcaauuuac aacacagacu uuaugagugu cucuauagaa auagagaugu ugacacagac 15660
   uuugugaaug aguuuuacgc auauuugcgu aaacauuucu caaugaugau acucucugac 15720
   gaugcuguug uguguuucaa uagcacuuau gcaucucaag gucuaguggc uagcauaaag 15780
   aacuuuaagu caguucuuua uuaucaaaac aauguuuuua ugucugaagc aaaauguugg 15840
   acugagacug accuuacuaa aggaccucau gaauuuugcu cucaacauac aaugcuaguu 15900
   aaacagggug augauuaugu guaccuuccu uacccagauc caucaagaau ccuaggggcc 15960
   ggcuguuuug uagaugauau cguaaaaaca gaugguacac uuaugauuga acgguucgug 16020
   ucuuuagcua uagaugcuua cccacuuacu aaacauccua aucaggagua ugcugauguc 16080
   uuucauuugu acuuacaaua cauaagaaag cuacaugaug aguuaacagg acacauguua 16140
   gacauguauu cuguuaugcu uacuaaugau aacacuucaa gguauuggga accugaguuu 16200
   uaugaggcua uguacacacc gcauacaguc uuacaggcug uuggggcuug uguucuuugc 16260
   aauucacaga cuucauuaag auguggugcu ugcauacgua gaccauucuu auguuguaaa 16320
   ugcuguuacg accaugucau aucaacauca cauaaauuag ucuugucugu uaauccguau 16380
   guuugcaaug cuccagguug ugaugucaca gaugugacuc aacuuuacuu aggagguaug 16440
   agcuauuauu guaaaucaca uaaaccaccc auuaguuuuc cauugugugc uaauggacaa 16500
   guuuuugguu uauauaaaaa uacauguguu gguagcgaua auguuacuga cuuuaaugca 16560
   auugcaacau gugacuggac aaaugcuggu gauuacauuu uagcuaacac cuguacugaa 16620
   agacucaagc uuuuugcagc agaaacgcuc aaagcuacug aggagacauu uaaacugucu 16680
   uaugguauug cuacuguacg ugaagugcug ucugacagag aauuacaucu uucaugggaa 16740
   guugguaaac cuagaccacc acuuaaccga aauuaugucu uuacugguua ucguguaacu 16800
   aaaaacagua aaguacaaau aggagaguac accuuugaaa aaggugacua uggugaugcu 16860
   guuguuuacc gagguacaac aacuuacaaa uuaaauguug gugauuauuu ugugcugaca 16920
   ucacauacag uaaugccauu aagugcaccu acacuagugc cacaagagca cuauguuaga 16980
   auuacuggcu uauacccaac acucaauauc ucagaugagu uuucuagcaa uguugcaaau 17040
   uaucaaaagg uugguaugca aaaguauucu acacuccagg gaccaccugg uacugguaag 17100
   agucauuuug cuauuggccu agcucucuac uacccuucug cucgcauagu guauacagcu 17160
   ugcucucaug ccgcuguuga ugcacuaugu gagaaggcau uaaaauauuu gccuauagau 17220
   aaauguagua gaauuauacc ugcacgugcu cguguagagu guuuugauaa auucaaagug 17280
   aauucaacau uagaacagua ugucuuuugu acuguaaaug cauugccuga gacgacagca 17340
   gauauaguug ucuuugauga aauuucaaug gccacaaauu augauuugag uguugucaau 17400
   gccagauuac gugcuaagca cuauguguac auuggcgacc cugcucaauu accugcacca 17460
   cgcacauugc uaacuaaggg cacacuagaa ccagaauauu ucaauucagu guguagacuu 17520
   augaaaacua uagguccaga cauguuccuc ggaacuuguc ggcguugucc ugcugaaauu 17580
   guugacacug ugagugcuuu gguuuaugau aauaagcuua aagcacauaa agacaaauca 17640
   gcucaaugcu uuaaaauguu uuauaagggu guuaucacgc augauguuuc aucugcaauu 17700
   aacaggccac aaauaggcgu gguaagagaa uuccuuacac guaacccugc uuggagaaaa 17760
   gcugucuuua uuucaccuua uaauucacag aaugcuguag ccucaaagau uuugggacua 17820
   ccaacucaaa cuguugauuc aucacagggc ucagaauaug acuaugucau auucacucaa 17880
   accacugaaa cagcucacuc uuguaaugua aacagauuua auguugcuau uaccagagca 17940
   aaaguaggca uacuuugcau aaugucugau agagaccuuu augacaaguu gcaauuuaca 18000
   agucuugaaa uuccacguag gaauguggca acuuuacaag cugaaaaugu aacaggacuc 18060
   uuuaaagauu guaguaaggu aaucacuggg uuacauccua cacaggcacc uacacaccuc 18120
   aguguugaca cuaaauucaa aacugaaggu uuauguguug acauaccugg cauaccuaag 18180
   gacaugaccu auagaagacu caucucuaug augggguuua aaaugaauua ucaaguuaau 18240
   gguuacccua acauguuuau cacccgcgaa gaagcuauaa gacauguacg ugcauggauu 18300
   ggcuucgaug ucgaggggug ucaugcuacu agagaagcug uugguaccaa uuuaccuuua 18360
   cagcuagguu uuucuacagg uguuaaccua guugcuguac cuacagguua uguugauaca 18420
   ccuaauaaua cagauuuuuc cagaguuagu gcuaaaccac cgccuggaga ucaauuuaaa 18480
   caccucauac cacuuaugua caaaggacuu ccuuggaaug uagugcguau aaagauugua 18540
   caaauguuaa gugacacacu uaaaaaucuc ucugacagag ucguauuugu cuuaugggca 18600
   cauggcuuug aguugacauc uaugaaguau uuugugaaaa uaggaccuga gcgcaccugu 18660
   ugucuaugug auagacgugc cacaugcuuu uccacugcuu cagacacuua ugccuguugg 18720
   caucauucua uuggauuuga uuacgucuau aauccguuua ugauugaugu ucaacaaugg 18780
   gguuuuacag guaaccuaca aagcaaccau gaucuguauu gucaagucca ugguaaugca 18840
   cauguagcua guugugaugc aaucaugacu aggugucuag cuguccacga gugcuuuguu 18900
   aagcguguug acuggacuau ugaauauccu auaauuggug augaacugaa gauuaaugcg 18960
   gcuuguagaa agguucaaca caugguuguu aaagcugcau uauuagcaga caaauuccca 19020
   guucuucacg acauugguaa cccuaaagcu auuaagugug uaccucaagc ugauguagaa 19080
   uggaaguucu augaugcaca gccuuguagu gacaaagcuu auaaaauaga agaauuauuc 19140
   uauucuuaug ccacacauuc ugacaaauuc acagauggug uaugccuauu uuggaauugc 19200
   aaugucgaua gauauccugc uaauuccauu guuuguagau uugacacuag agugcuaucu 19260
   aaccuuaacu ugccugguug ugaugguggc aguuuguaug uaaauaaaca ugcauuccac 19320
   acaccagcuu uugauaaaag ugcuuuuguu aauuuaaaac aauuaccauu uuucuauuac 19380
   ucugacaguc caugugaguc ucauggaaaa caaguagugu cagauauaga uuauguacca 19440
   cuaaagucug cuacguguau aacacguugc aauuuaggug gugcugucug uagacaucau 19500
   gcuaaugagu acagauugua ucucgaugcu uauaacauga ugaucucagc uggcuuuagc 19560
   uuguggguuu acaaacaauu ugauacuuau aaccucugga acacuuuuac aagacuucag 19620
   aguuuagaaa auguggcuuu uaauguugua aauaagggac acuuugaugg acaacagggu 19680
   gaaguaccag uuucuaucau uaauaacacu guuuacacaa aaguugaugg uguugaugua 19740
   gaauuguuug aaaauaaaac aacauuaccu guuaauguag cauuugagcu uugggcuaag 19800
   cgcaacauua aaccaguacc agaggugaaa auacucaaua auuugggugu ggacauugcu 19860
   gcuaauacug ugaucuggga cuacaaaaga gaugcuccag cacauauauc uacuauuggu 19920
   guuuguucua ugacugacau agccaagaaa ccaacugaaa cgauuugugc accacucacu 19980
   gucuuuuuug augguagagu ugauggucaa guagacuuau uuagaaaugc ccguaauggu 20040
   guucuuauua cagaagguag uguuaaaggu uuacaaccau cuguaggucc caaacaagcu 20100
   agucuuaaug gagucacauu aauuggagaa gccguaaaaa cacaguucaa uuauuauaag 20160
   aaaguugaug guguugucca acaauuaccu gaaacuuacu uuacucagag uagaaauuua 20220
   caagaauuua aacccaggag ucaaauggaa auugauuucu uagaauuagc uauggaugaa 20280
   uucauugaac gguauaaauu agaaggcuau gccuucgaac auaucguuua uggagauuuu 20340
   agucauaguc aguuaggugg uuuacaucua cugauuggac uagcuaaacg uuuuaaggaa 20400
   ucaccuuuug aauuagaaga uuuuauuccu auggacagua caguuaaaaa cuauuucaua 20460
   acagaugcgc aaacagguuc aucuaagugu guguguucug uuauugauuu auuacuugau 20520
   gauuuuguug aaauaauaaa aucccaagau uuaucuguag uuucuaaggu ugucaaagug 20580
   acuauugacu auacagaaau uucauuuaug cuuuggugua aagauggcca uguagaaaca 20640
   uuuuacccaa aauuacaauc uagucaagcg uggcaaccgg guguugcuau gccuaaucuu 20700
   uacaaaaugc aaagaaugcu auuagaaaag ugugaccuuc aaaauuaugg ugauagugca 20760
   acauuaccua aaggcauaau gaugaauguc gcaaaauaua cucaacugug ucaauauuua 20820
   aacacauuaa cauuagcugu acccuauaau augagaguua uacauuuugg ugcugguucu 20880
   gauaaaggag uugcaccagg uacagcuguu uuaagacagu gguugccuac ggguacgcug 20940
   cuugucgauu cagaucuuaa ugacuuuguc ucugaugcag auucaacuuu gauuggugau 21000
   ugugcaacug uacauacagc uaauaaaugg gaucucauua uuagugauau guacgacccu 21060
   aagacuaaaa auguuacaaa agaaaaugac ucuaaagagg guuuuuucac uuacauuugu 21120
   ggguuuauac aacaaaagcu agcucuugga gguuccgugg cuauaaagau aacagaacau 21180
   ucuuggaaug cugaucuuua uaagcucaug ggacacuucg caugguggac agccuuuguu 21240
   acuaauguga augcgucauc aucugaagca uuuuuaauug gauguaauua ucuuggcaaa 21300
   ccacgcgaac aaauagaugg uuaugucaug caugcaaauu acauauuuug gaggaauaca 21360
   aauccaauuc aguugucuuc cuauucuuua uuugacauga guaaauuucc ccuuaaauua 21420
   agggguacug cuguuauguc uuuaaaagaa ggucaaauca augauaugau uuuaucucuu 21480
   cuuaguaaag guagacuuau aauuagagaa aacaacagag uuguuauuuc uagugauguu 21540
   cuuguuaaca acuaaacgaa caauguuugu uuuucuuguu uuauugccac uagucucuag 21600
   ucaguguguu aaucuuacaa ccagaacuca auuacccccu gcauacacua auucuuucac 21660
   acgugguguu uauuacccug acaaaguuuu cagauccuca guuuuacauu caacucagga 21720
   cuuguucuua ccuuucuuuu ccaauguuac uugguuccau gcuauacaug ucucugggac 21780
   caaugguacu aagagguuug auaacccugu ccuaccauuu aaugauggug uuuauuuugc 21840
   uuccacugag aagucuaaca uaauaagagg cuggauuuuu gguacuacuu uagauucgaa 21900
   gacccagucc cuacuuauug uuaauaacgc uacuaauguu guuauuaaag ucugugaauu 21960
   ucaauuuugu aaugauccau uuuugggugu uuauuaccac aaaaacaaca aaaguuggau 22020
   ggaaagugag uucagaguuu auucuagugc gaauaauugc acuuuugaau augucucuca 22080
   gccuuuucuu auggaccuug aaggaaaaca ggguaauuuc aaaaaucuua gggaauuugu 22140
   guuuaagaau auugaugguu auuuuaaaau auauucuaag cacacgccua uuaauuuagu 22200
   gcgugaucuc ccucaggguu uuucggcuuu agaaccauug guagauuugc caauagguau 22260
   uaacaucacu agguuucaaa cuuuacuugc uuuacauaga aguuauuuga cuccugguga 22320
   uucuucuuca gguuggacag cuggugcugc agcuuauuau guggguuauc uucaaccuag 22380
   gacuuuucua uuaaaauaua augaaaaugg aaccauuaca gaugcuguag acugugcacu 22440
   ugacccucuc ucagaaacaa aguguacguu gaaauccuuc acuguagaaa aaggaaucua 22500
   ucaaacuucu aacuuuagag uccaaccaac agaaucuauu guuagauuuc cuaauauuac 22560
   aaacuugugc ccuuuuggug aaguuuuuaa cgccaccaga uuugcaucug uuuaugcuug 22620
   gaacaggaag agaaucagca acuguguugc ugauuauucu guccuauaua auuccgcauc 22680
   auuuuccacu uuuaaguguu auggaguguc uccuacuaaa uuaaaugauc ucugcuuuac 22740
   uaaugucuau gcagauucau uuguaauuag aggugaugaa gucagacaaa ucgcuccagg 22800
   gcaaacugga aagauugcug auuauaauua uaaauuacca gaugauuuua caggcugcgu 22860
   uauagcuugg aauucuaaca aucuugauuc uaagguuggu gguaauuaua auuaccugua 22920
   uagauuguuu aggaagucua aucucaaacc uuuugagaga gauauuucaa cugaaaucua 22980
   ucaggccggu agcacaccuu guaauggugu ugaagguuuu aauuguuacu uuccuuuaca 23040
   aucauauggu uuccaaccca cuaauggugu ugguuaccaa ccauacagag uaguaguacu 23100
   uucuuuugaa cuucuacaug caccagcaac uguuugugga ccuaaaaagu cuacuaauuu 23160
   gguuaaaaac aaauguguca auuucaacuu caaugguuua acaggcacag guguucuuac 23220
   ugagucuaac aaaaaguuuc ugccuuucca acaauuuggc agagacauug cugacacuac 23280
   ugaugcuguc cgugauccac agacacuuga gauucuugac auuacaccau guucuuuugg 23340
   uggugucagu guuauaacac caggaacaaa uacuucuaac cagguugcug uucuuuauca 23400
   ggauguuaac ugcacagaag ucccuguugc uauucaugca gaucaacuua cuccuacuug 23460
   gcguguuuau ucuacagguu cuaauguuuu ucaaacacgu gcaggcuguu uaauaggggc 23520
   ugaacauguc aacaacucau augaguguga cauacccauu ggugcaggua uaugcgcuag 23580
   uuaucagacu cagacuaauu cuccucggcg ggcacguagu guagcuaguc aauccaucau 23640
   ugccuacacu augucacuug gugcagaaaa uucaguugcu uacucuaaua acucuauugc 23700
   cauacccaca aauuuuacua uuaguguuac cacagaaauu cuaccagugu cuaugaccaa 23760
   gacaucagua gauuguacaa uguacauuug uggugauuca acugaaugca gcaaucuuuu 23820
   guugcaauau ggcaguuuuu guacacaauu aaaccgugcu uuaacuggaa uagcuguuga 23880
   acaagacaaa aacacccaag aaguuuuugc acaagucaaa caaauuuaca aaacaccacc 23940
   aauuaaagau uuuggugguu uuaauuuuuc acaaauauua ccagauccau caaaaccaag 24000
   caagagguca uuuauugaag aucuacuuuu caacaaagug acacuugcag augcuggcuu 24060
   caucaaacaa uauggugauu gccuugguga uauugcugcu agagaccuca uuugugcaca 24120
   aaaguuuaac ggccuuacug uuuugccacc uuugcucaca gaugaaauga uugcucaaua 24180
   cacuucugca cuguuagcgg guacaaucac uucugguugg accuuuggug caggugcugc 24240
   auuacaaaua ccauuugcua ugcaaauggc uuauagguuu aaugguauug gaguuacaca 24300
   gaauguucuc uaugagaacc aaaaauugau ugccaaccaa uuuaauagug cuauuggcaa 24360
   aauucaagac ucacuuucuu ccacagcaag ugcacuugga aaacuucaag auguggucaa 24420
   ccaaaaugca caagcuuuaa acacgcuugu uaaacaacuu agcuccaauu uuggugcaau 24480
   uucaaguguu uuaaaugaua uccuuucacg ucuugacaaa guugaggcug aagugcaaau 24540
   ugauagguug aucacaggca gacuucaaag uuugcagaca uaugugacuc aacaauuaau 24600
   uagagcugca gaaaucagag cuucugcuaa ucuugcugcu acuaaaaugu cagagugugu 24660
   acuuggacaa ucaaaaagag uugauuuuug uggaaagggc uaucaucuua uguccuuccc 24720
   ucagucagca ccucauggug uagucuucuu gcaugugacu uaugucccug cacaagaaaa 24780
   gaacuucaca acugcuccug ccauuuguca ugauggaaaa gcacacuuuc cucgugaagg 24840
   ugucuuuguu ucaaauggca cacacugguu uguaacacaa aggaauuuuu augaaccaca 24900
   aaucauuacu acagacaaca cauuuguguc ugguaacugu gauguuguaa uaggaauugu 24960
   caacaacaca guuuaugauc cuuugcaacc ugaauuagac ucauucaagg aggaguuaga 25020
   uaaauauuuu aagaaucaua caucaccaga uguugauuua ggugacaucu cuggcauuaa 25080
   ugcuucaguu guaaacauuc aaaaagaaau ugaccgccuc aaugagguug ccaagaauuu 25140
   aaaugaaucu cucaucgauc uccaagaacu uggaaaguau gagcaguaua uaaaauggcc 25200
   augguacauu uggcuagguu uuauagcugg cuugauugcc auaguaaugg ugacaauuau 25260
   gcuuugcugu augaccaguu gcuguaguug ucucaagggc uguuguucuu guggauccug 25320
   cugcaaauuu gaugaagacg acucugagcc agugcucaaa ggagucaaau uacauuacac 25380
   auaaacgaac uuauggauuu guuuaugaga aucuucacaa uuggaacugu aacuuugaag 25440
   caaggugaaa ucaaggaugc uacuccuuca gauuuuguuc gcgcuacugc aacgauaccg 25500
   auacaagccu cacucccuuu cggauggcuu auuguuggcg uugcacuucu ugcuguuuuu 25560
   cagagcgcuu ccaaaaucau aacccucaaa aagagauggc aacuagcacu cuccaagggu 25620
   guucacuuug uuugcaacuu gcuguuguug uuuguaacag uuuacucaca ccuuuugcuc 25680
   guugcugcug gccuugaagc cccuuuucuc uaucuuuaug cuuuagucua cuucuugcag 25740
   aguauaaacu uuguaagaau aauaaugagg cuuuggcuuu gcuggaaaug ccguuccaaa 25800
   aacccauuac uuuaugaugc caacuauuuu cuuugcuggc auacuaauug uuacgacuau 25860
   uguauaccuu acaauagugu aacuucuuca auugucauua cuucagguga uggcacaaca 25920
   aguccuauuu cugaacauga cuaccagauu ggugguuaua cugaaaaaug ggaaucugga 25980
   guaaaagacu guguuguauu acacaguuac uucacuucag acuauuacca gcuguacuca 26040
   acucaauuga guacagacac ugguguugaa cauguuaccu ucuucaucua caauaaaauu 26100
   guugaugagc cugaagaaca uguccaaauu cacacaaucg acgguucauc cggaguuguu 26160
   aauccaguaa uggaaccaau uuaugaugaa ccgacgacga cuacuagcgu gccuuuguaa 26220
   gcacaagcug augaguacga acuuauguac ucauucguuu cggaagagac agguacguua 26280
   auaguuaaua gcguacuucu uuuucuugcu uucgugguau ucuugcuagu uacacuagcc 26340
   auccuuacug cgcuucgauu gugugcguac ugcugcaaua uuguuaacgu gagucuugua 26400
   aaaccuucuu uuuacguuua cucucguguu aaaaaucuga auucuucuag aguuccugau 26460
   cuucuggucu aaacgaacua aauauuauau uaguuuuucu guuuggaacu uuaauuuuag 26520
   ccauggcaga uuccaacggu acuauuaccg uugaagagcu uaaaaagcuc cuugaacaau 26580
   ggaaccuagu aauagguuuc cuauuccuua cauggauuug ucuucuacaa uuugccuaug 26640
   ccaacaggaa uagguuuuug uauauaauua aguuaauuuu ccucuggcug uuauggccag 26700
   uaacuuuagc uuguuuugug cuugcugcug uuuacagaau aaauuggauc accgguggaa 26760
   uugcuaucgc aauggcuugu cuuguaggcu ugauguggcu cagcuacuuc auugcuucuu 26820
   ucagacuguu ugcgcguacg cguuccaugu ggucauucaa uccagaaacu aacauucuuc 26880
   ucaacgugcc acuccauggc acuauucuga ccagaccgcu ucuagaaagu gaacucguaa 26940
   ucggagcugu gauccuucgu ggacaucuuc guauugcugg acaccaucua ggacgcugug 27000
   acaucaagga ccugccuaaa gaaaucacug uugcuacauc acgaacgcuu ucuuauuaca 27060
   aauugggagc uucgcagcgu guagcaggug acucagguuu ugcugcauac agucgcuaca 27120
   ggauuggcaa cuauaaauua aacacagacc auuccaguag cagugacaau auugcuuugc 27180
   uuguacagua agugacaaca gauguuucau cucguugacu uucagguuac uauagcagag 27240
   auauuacuaa uuauuaugag gacuuuuaaa guuuccauuu ggaaucuuga uuacaucaua 27300
   aaccucauaa uuaaaaauuu aucuaaguca cuaacugaga auaaauauuc ucaauuagau 27360
   gaagagcaac caauggagau ugauuaaacg aacaugaaaa uuauucuuuu cuuggcacug 27420
   auaacacucg cuacuuguga gcuuuaucac uaccaagagu guguuagagg uacaacagua 27480
   cuuuuaaaag aaccuugcuc uucuggaaca uacgagggca auucaccauu ucauccucua 27540
   gcugauaaca aauuugcacu gacuugcuuu agcacucaau uugcuuuugc uuguccugac 27600
   ggcguaaaac acgucuauca guuacgugcc agaucaguuu caccuaaacu guucaucaga 27660
   caagaggaag uucaagaacu uuacucucca auuuuucuua uuguugcggc aauaguguuu 27720
   auaacacuuu gcuucacacu caaaagaaag acagaaugau ugaacuuuca uuaauugacu 27780
   ucuauuugug cuuuuuagcc uuucugcuau uccuuguuuu aauuaugcuu auuaucuuuu 27840
   gguucucacu ugaacugcaa gaucauaaug aaacuuguca cgccuaaacg aacaugaaau 27900
   uucuuguuuu cuuaggaauc aucacaacug uagcugcauu ucaccaagaa uguaguuuac 27960
   agucauguac ucaacaucaa ccauauguag uugaugaccc guguccuauu cacuucuauu 28020
   cuaaauggua uauuagagua ggagcuagaa aaucagcacc uuuaauugaa uugugcgugg 28080
   augaggcugg uucuaaauca cccauucagu acaucgauau cgguaauuau acaguuuccu 28140
   guuuaccuuu uacaauuaau ugccaggaac cuaaauuggg uagucuugua gugcguuguu 28200
   cguucuauga agacuuuuua gaguaucaug acguucgugu uguuuuagau uucaucuaaa 28260
   cgaacaaacu aaaaugucug auaauggacc ccaaaaucag cgaaaugcac cccgcauuac 28320
   guuuggugga cccucagauu caacuggcag uaaccagaau ggagaacgca guggggcgcg 28380
   aucaaaacaa cgucggcccc aagguuuacc caauaauacu gcgucuuggu ucaccgcucu 28440
   cacucaacau ggcaaggaag accuuaaauu cccucgagga caaggcguuc caauuaacac 28500
   caauagcagu ccagaugacc aaauuggcua cuaccgaaga gcuaccagac gaauucgugg 28560
   uggugacggu aaaaugaaag aucucagucc aagaugguau uucuacuacc uaggaacugg 28620
   gccagaagcu ggacuucccu auggugcuaa caaagacggc aucauauggg uugcaacuga 28680
   gggagccuug aauacaccaa aagaucacau uggcacccgc aauccugcua acaaugcugc 28740
   aaucgugcua caacuuccuc aaggaacaac auugccaaaa ggcuucuacg cagaagggag 28800
   cagaggcggc agucaagccu cuucucguuc cucaucacgu agucgcaaca guucaagaaa 28860
   uucaacucca ggcagcagua ggggaacuuc uccugcuaga auggcuggca auggcgguga 28920
   ugcugcucuu gcuuugcugc ugcuugacag auugaaccag cuugagagca aaaugucugg 28980
   uaaaggccaa caacaacaag gccaaacugu cacuaagaaa ucugcugcug aggcuucuaa 29040
   gaagccucgg caaaaacgua cugccacuaa agcauacaau guaacacaag cuuucggcag 29100
   acgugguccc gaacaaaccc aaggaaauuu uggggaccag gaacuaauca gacaaggaac 29160
   ugauuacaaa cauuggccgc aaauugcaca auuugccccc agcgcuucag cguucuucgg 29220
   aaugucgcgc auuggcaugg aagucacacc uucgggaacg ugguugaccu acacaggugc 29280
   caucaaauug gaugacaaag auccaaauuu caaagaucaa gucauuuugc ugaauaagca 29340
   uauugacgca uacaaaacau ucccaccaac agagccuaaa aaggacaaaa agaagaaggc 29400
   ugaugaaacu caagccuuac cgcagagaca gaagaaacag caaacuguga cucuucuucc 29460
   ugcugcagau uuggaugauu ucuccaaaca auugcaacaa uccaugagca gugcugacuc 29520
   aacucaggcc uaaacucaug cagaccacac aaggcagaug ggcuauauaa acguuuucgc 29580
   uuuuccguuu acgauauaua gucuacucuu gugcagaaug aauucucgua acuacauagc     29640
   acaaguagau guaguuaacu uuaaucucac auagcaaucu uuaaucagug uguaacauua     29700
   gggaggacuu gaaagagcca ccacauuuuc accgaggcca cgcggaguac gaucgagugu     29760
   acagugaaca augcuaggga gagcugccua uauggaagag cccuaaugug uaaaauuaau     29820
   uuuaguagug cuauccccau gugauuuuaa uagcuucuua ggagaaugac aaaaaaaaaa     29880
   aaaaaaaaaa aaaaaaaaaa aaa                                             29903
<210> 2
   <211> 30005
   <212> RNA
   <213> Artificial Sequence
<220>
   <223> SARS-CoV-2 strain consensus, n can be any nucleotide
<220>
   <221> misc_feature
   <222> (1)..(132)
   <223> n is a, c, g, or u
<220>
   <221> misc_feature
   <222> (161)..(161)
   <223> n is a, c, g, or u
<220>
   <221> misc_feature
   <222> (168)..(169)
   <223> n is a, c, g, or u
<220>
   <221> misc_feature
   <222> (176)..(177)
   <223> n is a, c, g, or u
<220>
   <221> misc_feature
   <222> (181)..(181)
   <223> n is a, c, g, or u
<220>
   <221> misc_feature
   <222> (197)..(197)
   <223> n is a, c, g, or u
<220>
   <221> misc_feature
   <222> (243)..(243)
   <223> n is a, c, g, or u
<220>
   <221> misc_feature
   <222> (254)..(255)
   <223> n is a, c, g, or u
<220>
   <221> misc_feature
   <222> (257)..(259)
   <223> n is a, c, g, or u
<220>
   <221> misc_feature
   <222> (261)..(263)
   <223> n is a, c, g, or u
<220>
   <221> misc_feature
   <222> (265)..(265)
   <223> n is a, c, g, or u
<220>
   <221> misc_feature
   <222> (267)..(267)
   <223> n is a, c, g, or u
<220>
   <221> misc_feature
   <222> (278)..(278)
   <223> n is a, c, g, or u
<220>
   <221> misc_feature
   <222> (298)..(298)
   <223> n is a, c, g, or u
<220>
   <221> misc_feature
   <222> (311)..(311)
   <223> n is a, c, g, or u
<220>
   <221> misc_feature
   <222> (389)..(389)
   <223> n is a, c, g, or u
<220>
   <221> misc_feature
   <222> (411)..(411)
   <223> n is a, c, g, or u
<220>
   <221> misc_feature
   <222> (547)..(547)
   <223> n is a, c, g, or u
<220>
   <221> misc_feature
   <222> (565)..(579)
   <223> n is a, c, g, or u
<220>
   <221> misc_feature
   <222> (671)..(671)
   <223> n is a, c, g, or u
<220>
   <221> misc_feature
   <222> (711)..(711)
   <223> n is a, c, g, or u
<220>
   <221> misc_feature
   <222> (743)..(751)
   <223> n is a, c, g, or u
<220>
   <221> misc_feature
   <222> (890)..(890)
   <223> n is a, c, g, or u
<220>
   <221> misc_feature
   <222> (941)..(941)
   <223> n is a, c, g, or u
<220>
   <221> misc_feature
   <222> (1116)..(1116)
   <223> n is a, c, g, or u
<220>
   <221> misc_feature
   <222> (1120)..(1120)
   <223> n is a, c, g, or u
<220>
   <221> misc_feature
   <222> (1159)..(1159)
   <223> n is a, c, g, or u
<220>
   <221> misc_feature
   <222> (1405)..(1405)
   <223> n is a, c, g, or u
<220>
   <221> misc_feature
   <222> (1442)..(1442)
   <223> n is a, c, g, or u
<220>
   <221> misc_feature
   <222> (1454)..(1454)
   <223> n is a, c, g, or u
<220>
   <221> misc_feature
   <222> (1487)..(1487)
   <223> n is a, c, g, or u
<220>
   <221> misc_feature
   <222> (1526)..(1526)
   <223> n is a, c, g, or u
<220>
   <221> misc_feature
   <222> (1605)..(1605)
   <223> n is a, c, g, or u
<220>
   <221> misc_feature
   <222> (1748)..(1748)
   <223> n is a, c, g, or u
<220>
   <221> misc_feature
   <222> (1791)..(1791)
   <223> n is a, c, g, or u
<220>
   <221> misc_feature
   <222> (1968)..(1968)
   <223> n is a, c, g, or u
<220>
   <221> misc_feature
   <222> (2148)..(2148)
   <223> n is a, c, g, or u
<220>
   <221> misc_feature
   <222> (2238)..(2239)
   <223> n is a, c, g, or u
<220>
   <221> misc_feature
   <222> (2326)..(2326)
   <223> n is a, c, g, or u
<220>
   <221> misc_feature
   <222> (2334)..(2334)
   <223> n is a, c, g, or u
<220>
   <221> misc_feature
   <222> (2503)..(2503)
   <223> n is a, c, g, or u
<220>
   <221> misc_feature
   <222> (2689)..(2689)
   <223> n is a, c, g, or u
<220>
   <221> misc_feature
   <222> (2774)..(2774)
   <223> n is a, c, g, or u
<220>
   <221> misc_feature
   <222> (3028)..(3028)
   <223> n is a, c, g, or u
<220>
   <221> misc_feature
   <222> (3094)..(3094)
   <223> n is a, c, g, or u
<220>
   <221> misc_feature
   <222> (3156)..(3156)
   <223> n is a, c, g, or u
<220>
   <221> misc_feature
   <222> (3234)..(3234)
   <223> n is a, c, g, or u
<220>
   <221> misc_feature
   <222> (3316)..(3316)
   <223> n is a, c, g, or u
<220>
   <221> misc_feature
   <222> (3468)..(3468)
   <223> n is a, c, g, or u
<220>
   <221> misc_feature
   <222> (3575)..(3575)
   <223> n is a, c, g, or u
<220>
   <221> misc_feature
   <222> (3795)..(3795)
   <223> n is a, c, g, or u
<220>
   <221> misc_feature
   <222> (3835)..(3835)
   <223> n is a, c, g, or u
<220>
   <221> misc_feature
   <222> (4049)..(4049)
   <223> n is a, c, g, or u
<220>
   <221> misc_feature
   <222> (4345)..(4345)
   <223> n is a, c, g, or u
<220>
   <221> misc_feature
   <222> (4364)..(4364)
   <223> n is a, c, g, or u
<220>
   <221> misc_feature
   <222> (4459)..(4459)
   <223> n is a, c, g, or u
<220>
   <221> misc_feature
   <222> (4936)..(4938)
   <223> n is a, c, g, or u
<220>
   <221> misc_feature
   <222> (5109)..(5109)
   <223> n is a, c, g, or u
<220>
   <221> misc_feature
   <222> (5119)..(5119)
   <223> n is a, c, g, or u
<220>
   <221> misc_feature
   <222> (5141)..(5141)
   <223> n is a, c, g, or u
<220>
   <221> misc_feature
   <222> (5537)..(5537)
   <223> n is a, c, g, or u
<220>
   <221> misc_feature
   <222> (5629)..(5629)
   <223> n is a, c, g, or u
<220>
   <221> misc_feature
   <222> (5773)..(5773)
   <223> n is a, c, g, or u
<220>
   <221> misc_feature
   <222> (5841)..(5841)
   <223> n is a, c, g, or u
<220>
   <221> misc_feature
   <222> (5902)..(5902)
   <223> n is a, c, g, or u
<220>
   <221> misc_feature
   <222> (6083)..(6083)
   <223> n is a, c, g, or u
<220>
   <221> misc_feature
   <222> (6088)..(6088)
   <223> n is a, c, g, or u
<220>
   <221> misc_feature
   <222> (6092)..(6092)
   <223> n is a, c, g, or u
<220>
   <221> misc_feature
   <222> (6367)..(6367)
   <223> n is a, c, g, or u
<220>
   <221> misc_feature
   <222> (6558)..(6558)
   <223> n is a, c, g, or u
<220>
   <221> misc_feature
   <222> (6693)..(6693)
   <223> n is a, c, g, or u
<220>
   <221> misc_feature
   <222> (6752)..(6752)
   <223> n is a, c, g, or u
<220>
   <221> misc_feature
   <222> (6876)..(6876)
   <223> n is a, c, g, or u
<220>
   <221> misc_feature
   <222> (6953)..(6953)
   <223> n is a, c, g, or u
<220>
   <221> misc_feature
   <222> (7016)..(7016)
   <223> n is a, c, g, or u
<220>
   <221> misc_feature
   <222> (7053)..(7053)
   <223> n is a, c, g, or u
<220>
   <221> misc_feature
   <222> (7073)..(7073)
   <223> n is a, c, g, or u
<220>
   <221> misc_feature
   <222> (7536)..(7536)
   <223> n is a, c, g, or u
<220>
   <221> misc_feature
   <222> (7923)..(7923)
   <223> n is a, c, g, or u
<220>
   <221> misc_feature
   <222> (8058)..(8058)
   <223> n is a, c, g, or u
<220>
   <221> misc_feature
   <222> (8369)..(8369)
   <223> n is a, c, g, or u
<220>
   <221> misc_feature
   <222> (8445)..(8445)
   <223> n is a, c, g, or u
<220>
   <221> misc_feature
   <222> (8825)..(8825)
   <223> n is a, c, g, or u
<220>
   <221> misc_feature
   <222> (8839)..(8839)
   <223> n is a, c, g, or u
<220>
   <221> misc_feature
   <222> (9044)..(9044)
   <223> n is a, c, g, or u
<220>
   <221> misc_feature
   <222> (9091)..(9091)
   <223> n is a, c, g, or u
<220>
   <221> misc_feature
   <222> (9214)..(9214)
   <223> n is a, c, g, or u
<220>
   <221> misc_feature
   <222> (9216)..(9216)
   <223> n is a, c, g, or u
<220>
   <221> misc_feature
   <222> (9237)..(9237)
   <223> n is a, c, g, or u
<220>
   <221> misc_feature
   <222> (9331)..(9331)
   <223> n is a, c, g, or u
<220>
   <221> misc_feature
   <222> (9531)..(9531)
   <223> n is a, c, g, or u
<220>
   <221> misc_feature
   <222> (9534)..(9534)
   <223> n is a, c, g, or u
<220>
   <221> misc_feature
   <222> (9548)..(9548)
   <223> n is a, c, g, or u
<220>
   <221> misc_feature
   <222> (9568)..(9568)
   <223> n is a, c, g, or u
<220>
   <221> misc_feature
   <222> (9591)..(9591)
   <223> n is a, c, g, or u
<220>
   <221> misc_feature
   <222> (9618)..(9618)
   <223> n is a, c, g, or u
<220>
   <221> misc_feature
   <222> (9981)..(9981)
   <223> n is a, c, g, or u
<220>
   <221> misc_feature
   <222> (10093)..(10093)
   <223> n is a, c, g, or u
<220>
   <221> misc_feature
   <222> (10140)..(10140)
   <223> n is a, c, g, or u
<220>
   <221> misc_feature
   <222> (10289)..(10289)
   <223> n is a, c, g, or u
<220>
   <221> misc_feature
   <222> (10564)..(10564)
   <223> n is a, c, g, or u
<220>
   <221> misc_feature
   <222> (11140)..(11140)
   <223> n is a, c, g, or u
<220>
   <221> misc_feature
   <222> (11264)..(11264)
   <223> n is a, c, g, or u
<220>
   <221> misc_feature
   <222> (11290)..(11290)
   <223> n is a, c, g, or u
<220>
   <221> misc_feature
   <222> (11467)..(11467)
   <223> n is a, c, g, or u
<220>
   <221> misc_feature
   <222> (11807)..(11807)
   <223> n is a, c, g, or u
<220>
   <221> misc_feature
   <222> (12013)..(12013)
   <223> n is a, c, g, or u
<220>
   <221> misc_feature
   <222> (12098)..(12098)
   <223> n is a, c, g, or u
<220>
   <221> misc_feature
   <222> (12172)..(12172)
   <223> n is a, c, g, or u
<220>
   <221> misc_feature
   <222> (12217)..(12217)
   <223> n is a, c, g, or u
<220>
   <221> misc_feature
   <222> (12259)..(12259)
   <223> n is a, c, g, or u
<220>
   <221> misc_feature
   <222> (12265)..(12265)
   <223> n is a, c, g, or u
<220>
   <221> misc_feature
   <222> (12270)..(12270)
   <223> n is a, c, g, or u
<220>
   <221> misc_feature
   <222> (12412)..(12412)
   <223> n is a, c, g, or u
<220>
   <221> misc_feature
   <222> (12435)..(12435)
   <223> n is a, c, g, or u
<220>
   <221> misc_feature
   <222> (12521)..(12521)
   <223> n is a, c, g, or u
<220>
   <221> misc_feature
   <222> (12524)..(12524)
   <223> n is a, c, g, or u
<220>
   <221> misc_feature
   <222> (12530)..(12530)
   <223> n is a, c, g, or u
<220>
   <221> misc_feature
   <222> (12548)..(12548)
   <223> n is a, c, g, or u
<220>
   <221> misc_feature
   <222> (12570)..(12571)
   <223> n is a, c, g, or u
<220>
   <221> misc_feature
   <222> (12591)..(12591)
   <223> n is a, c, g, or u
<220>
   <221> misc_feature
   <222> (12629)..(12629)
   <223> n is a, c, g, or u
<220>
   <221> misc_feature
   <222> (12635)..(12635)
   <223> n is a, c, g, or u
<220>
   <221> misc_feature
   <222> (12639)..(12639)
   <223> n is a, c, g, or u
<220>
   <221> misc_feature
   <222> (12657)..(12657)
   <223> n is a, c, g, or u
<220>
   <221> misc_feature
   <222> (12717)..(12717)
   <223> n is a, c, g, or u
<220>
   <221> misc_feature
   <222> (12742)..(12742)
   <223> n is a, c, g, or u
<220>
   <221> misc_feature
   <222> (12830)..(12830)
   <223> n is a, c, g, or u
<220>
   <221> misc_feature
   <222> (12850)..(12850)
   <223> n is a, c, g, or u
<220>
   <221> misc_feature
   <222> (13129)..(13129)
   <223> n is a, c, g, or u
<220>
   <221> misc_feature
   <222> (13282)..(13283)
   <223> n is a, c, g, or u
<220>
   <221> misc_feature
   <222> (14286)..(14286)
   <223> n is a, c, g, or u
<220>
   <221> misc_feature
   <222> (14465)..(14465)
   <223> n is a, c, g, or u
<220>
   <221> misc_feature
   <222> (14714)..(14714)
   <223> n is a, c, g, or u
<220>
   <221> misc_feature
   <222> (14862)..(14862)
   <223> n is a, c, g, or u
<220>
   <221> misc_feature
   <222> (15250)..(15250)
   <223> n is a, c, g, or u
<220>
   <221> misc_feature
   <222> (15381)..(15381)
   <223> n is a, c, g, or u
<220>
   <221> misc_feature
   <222> (15643)..(15643)
   <223> n is a, c, g, or u
<220>
   <221> misc_feature
   <222> (15654)..(15654)
   <223> n is a, c, g, or u
<220>
   <221> misc_feature
   <222> (15664)..(15664)
   <223> n is a, c, g, or u
<220>
   <221> misc_feature
   <222> (15867)..(15867)
   <223> n is a, c, g, or u
<220>
   <221> misc_feature
   <222> (16304)..(16304)
   <223> n is a, c, g, or u
<220>
   <221> misc_feature
   <222> (16524)..(16524)
   <223> n is a, c, g, or u
<220>
   <221> misc_feature
   <222> (16592)..(16592)
   <223> n is a, c, g, or u
<220>
   <221> misc_feature
   <222> (16934)..(16934)
   <223> n is a, c, g, or u
<220>
   <221> misc_feature
   <222> (16944)..(16944)
   <223> n is a, c, g, or u
<220>
   <221> misc_feature
   <222> (16969)..(16969)
   <223> n is a, c, g, or u
<220>
   <221> misc_feature
   <222> (17057)..(17057)
   <223> n is a, c, g, or u
<220>
   <221> misc_feature
   <222> (17304)..(17304)
   <223> n is a, c, g, or u
<220>
   <221> misc_feature
   <222> (17430)..(17430)
   <223> n is a, c, g, or u
<220>
   <221> misc_feature
   <222> (17433)..(17433)
   <223> n is a, c, g, or u
<220>
   <221> misc_feature
   <222> (17467)..(17467)
   <223> n is a, c, g, or u
<220>
   <221> misc_feature
   <222> (17480)..(17480)
   <223> n is a, c, g, or u
<220>
   <221> misc_feature
   <222> (17531)..(17531)
   <223> n is a, c, g, or u
<220>
   <221> misc_feature
   <222> (17804)..(17804)
   <223> n is a, c, g, or u
<220>
   <221> misc_feature
   <222> (17915)..(17915)
   <223> n is a, c, g, or u
<220>
   <221> misc_feature
   <222> (18117)..(18117)
   <223> n is a, c, g, or u
<220>
   <221> misc_feature
   <222> (18625)..(18625)
   <223> n is a, c, g, or u
<220>
   <221> misc_feature
   <222> (18660)..(18660)
   <223> n is a, c, g, or u
<220>
   <221> misc_feature
   <222> (18871)..(18871)
   <223> n is a, c, g, or u
<220>
   <221> misc_feature
   <222> (18934)..(18934)
   <223> n is a, c, g, or u
<220>
   <221> misc_feature
   <222> (19032)..(19032)
   <223> n is a, c, g, or u
<220>
   <221> misc_feature
   <222> (19122)..(19122)
   <223> n is a, c, g, or u
<220>
   <221> misc_feature
   <222> (19232)..(19232)
   <223> n is a, c, g, or u
<220>
   <221> misc_feature
   <222> (19667)..(19667)
   <223> n is a, c, g, or u
<220>
   <221> misc_feature
   <222> (20338)..(20338)
   <223> n is a, c, g, or u
<220>
   <221> misc_feature
   <222> (20355)..(20357)
   <223> n is a, c, g, or u
<220>
   <221> misc_feature
   <222> (20880)..(20880)
   <223> n is a, c, g, or u
<220>
   <221> misc_feature
   <222> (20993)..(20993)
   <223> n is a, c, g, or u
<220>
   <221> misc_feature
   <222> (21037)..(21037)
   <223> n is a, c, g, or u
<220>
   <221> misc_feature
   <222> (21045)..(21045)
   <223> n is a, c, g, or u
<220>
   <221> misc_feature
   <222> (21194)..(21194)
   <223> n is a, c, g, or u
<220>
   <221> misc_feature
   <222> (21204)..(21204)
   <223> n is a, c, g, or u
<220>
   <221> misc_feature
   <222> (21373)..(21373)
   <223> n is a, c, g, or u
<220>
   <221> misc_feature
   <222> (21442)..(21444)
   <223> n is a, c, g, or u
<220>
   <221> misc_feature
   <222> (21446) .. (21446)
   <223> n is a, c, g, or u
<220>
   <221> misc_feature
   <222> (21635)..(21635)
   <223> n is a, c, g, or u
<220>
   <221> misc_feature
   <222> (21704)..(21704)
   <223> n is a, c, g, or u
<220>
   <221> misc_feature
   <222> (21767)..(21767)
   <223> n is a, c, g, or u
<220>
   <221> misc_feature
   <222> (21844)..(21844)
   <223> n is a, c, g, or u
<220>
   <221> misc_feature
   <222> (22051)..(22053)
   <223> n is a, c, g, or u
<220>
   <221> misc_feature
   <222> (22093)..(22093)
   <223> n is a, c, g, or u
<220>
   <221> misc_feature
   <222> (22164)..(22164)
   <223> n is a, c, g, or u
<220>
   <221> misc_feature
   <222> (22284)..(22284)
   <223> n is a, c, g, or u
<220>
   <221> misc_feature
   <222> (22363)..(22363)
   <223> n is a, c, g, or u
<220>
   <221> misc_feature
   <222> (22492)..(22492)
   <223> n is a, c, g, or u
<220>
   <221> misc_feature
   <222> (22845)..(22845)
   <223> n is a, c, g, or u
<220>
   <221> misc_feature
   <222> (23044)..(23044)
   <223> n is a, c, g, or u
<220>
   <221> misc_feature
   <222> (23048)..(23048)
   <223> n is a, c, g, or u
<220>
   <221> misc_feature
   <222> (23245)..(23245)
   <223> n is a, c, g, or u
<220>
   <221> misc_feature
   <222> (23463)..(23463)
   <223> n is a, c, g, or u
<220>
   <221> misc_feature
   <222> (24012)..(24012)
   <223> n is a, c, g, or u
<220>
   <221> misc_feature
   <222> (24094)..(24094)
   <223> n is a, c, g, or u
<220>
   <221> misc_feature
   <222> (24385)..(24385)
   <223> n is a, c, g, or u
<220>
   <221> misc_feature
   <222> (24411)..(24411)
   <223> n is a, c, g, or u
<220>
   <221> misc_feature
   <222> (25623)..(25623)
   <223> n is a, c, g, or u
<220>
   <221> misc_feature
   <222> (25647)..(25647)
   <223> n is a, c, g, or u
<220>
   <221> misc_feature
   <222> (25764)..(25764)
   <223> n is a, c, g, or u
<220>
   <221> misc_feature
   <222> (25835)..(25835)
   <223> n is a, c, g, or u
<220>
   <221> misc_feature
   <222> (25858)..(25858)
   <223> n is a, c, g, or u
<220>
   <221> misc_feature
   <222> (26039)..(26039)
   <223> n is a, c, g, or u
<220>
   <221> misc_feature
   <222> (26204)..(26204)
   <223> n is a, c, g, or u
<220>
   <221> misc_feature
   <222> (26386)..(26386)
   <223> n is a, c, g, or u
<220>
   <221> misc_feature
   <222> (26414)..(26414)
   <223> n is a, c, g, or u
<220>
   <221> misc_feature
   <222> (26518)..(26518)
   <223> n is a, c, g, or u
<220>
   <221> misc_feature
   <222> (26789)..(26789)
   <223> n is a, c, g, or u
<220>
   <221> misc_feature
   <222> (27286) .. (27286)
   <223> n is a, c, g, or u
<220>
   <221> misc_feature
   <222> (27336) .. (27336)
   <223> n is a, c, g, or u
<220>
   <221> misc_feature
   <222> (27444)..(27444)
   <223> n is a, c, g, or u
<220>
   <221> misc_feature
   <222> (27585)..(27585)
   <223> n is a, c, g, or u
<220>
   <221> misc_feature
   <222> (27866)..(27866)
   <223> n is a, c, g, or u
<220>
   <221> misc_feature
   <222> (27985)..(27985)
   <223> n is a, c, g, or u
<220>
   <221> misc_feature
   <222> (28024)..(28024)
   <223> n is a, c, g, or u
<220>
   <221> misc_feature
   <222> (28137)..(28137)
   <223> n is a, c, g, or u
<220>
   <221> misc_feature
   <222> (28204)..(28204)
   <223> n is a, c, g, or u
<220>
   <221> misc_feature
   <222> (28313)..(28313)
   <223> n is a, c, g, or u
<220>
   <221> misc_feature
   <222> (28427)..(28427)
   <223> n is a, c, g, or u
<220>
   <221> misc_feature
   <222> (28438)..(28438)
   <223> n is a, c, g, or u
<220>
   <221> misc_feature
   <222> (28469)..(28469)
   <223> n is a, c, g, or u
<220>
   <221> misc_feature
   <222> (28717)..(28717)
   <223> n is a, c, g, or u
<220>
   <221> misc_feature
   <222> (28852)..(28852)
   <223> n is a, c, g, or u
<220>
   <221> misc_feature
   <222> (28914)..(28914)
   <223> n is a, c, g, or u
<220>
   <221> misc_feature
   <222> (28923)..(28923)
   <223> n is a, c, g, or u
<220>
   <221> misc_feature
   <222> (28938)..(28938)
   <223> n is a, c, g, or u
<220>
   <221> misc_feature
   <222> (28941)..(28943)
   <223> n is a, c, g, or u
<220>
   <221> misc_feature
   <222> (28976)..(28976)
   <223> n is a, c, g, or u
<220>
   <221> misc_feature
   <222> (29155)..(29155)
   <223> n is a, c, g, or u
<220>
   <221> misc_feature
   <222> (29290)..(29290)
   <223> n is a, c, g, or u
<220>
   <221> misc_feature
   <222> (29314)..(29314)
   <223> n is a, c, g, or u
<220>
   <221> misc_feature
   <222> (29361)..(29361)
   <223> n is a, c, g, or u
<220>
   <221> misc_feature
   <222> (29363)..(29363)
   <223> n is a, c, g, or u
<220>
   <221> misc_feature
   <222> (29587)..(29587)
   <223> n is a, c, g, or u
<220>
   <221> misc_feature
   <222> (29613)..(29613)
   <223> n is a, c, g, or u
<220>
   <221> misc_feature
   <222> (29656)..(29656)
   <223> n is a, c, g, or u
<220>
   <221> misc_feature
   <222> (29695)..(29695)
   <223> n is a, c, g, or u
<220>
   <221> misc_feature
   <222> (29796)..(29796)
   <223> n is a, c, g, or u
<220>
   <221> misc_feature
   <222> (29802)..(29802)
   <223> n is a, c, g, or u
<220>
   <221> misc_feature
   <222> (29810)..(29819)
   <223> n is a, c, g, or u
<220>
   <221> misc_feature
   <222> (29834)..(29834)
   <223> n is a, c, g, or u
<220>
   <221> misc_feature
   <222> (29887)..(30005)
   <223> n is a, c, g, or u
<400> 2
   nnnnnnnnnn nnnnnnnnnn nnnnnnnnnn nnnnnnnnnn nnnnnnnnnn nnnnnnnnnn 60
   nnnnnnnnnn nnnnnnnnnn nnnnnnnnnn nnnnnnnnnn nnnnnnnnnn nnnnnnnnnn 120
   nnnnnnnnnn nnuuuaaaau cuguguggcu gucacucggc ngcaugcnna gugcanncac 180
   ncaguauaau uaauaanuaa uuacugucgu ugacaggaca cgaguaacuc gucuaucuuc 240
   ugnaggcugc uuanngnnnc nnncnunuug cagccganca ucagcacauc uagguuungu 300
   ccggguguga ncgaaaggua agauggagag ccuugucccu gguuucaacg agaaaacaca 360
   cguccaacuc aguuugccug uuuuacagnu ucgcgacgug cucguacgug ncuuuggaga 420
   cuccguggag gaggucuuau cagaggcacg ucaacaucuu aaagauggca cuuguggcuu 480
   aguagaaguu gaaaaaggcg uuuugccuca acuugaacag cccuaugugu ucaucaaacg 540
   uucggangcu cgaacugcac cucannnnnn nnnnnnnnnu gagcugguag cagaacucga 600
   aggcauucag uacggucgua guggugagac acuugguguc cuugucccuc augugggcga 660
   aauaccagug ncuuaccgca agguucuucu ucguaagaac gguaauaaag nagcuggugg 720
   ccauaguuac ggcgccgauc uannnnnnnn ngacuuaggc gacgagcuug gcacugaucc 780
   uuaugaagau uuucaagaaa acuggaacac uaaacauagc agugguguua cccgugaacu 840
   caugcgugag cuuaacggag gggcauacac ucgcuauguc gauaacaacn ucuguggccc 900
   ugauggcuac ccucuugagu gcauuaaaga ccuucuagca ngugcuggua aagcuucaug 960
   cacuuugucc gaacaacugg acuuuauuga cacuaagagg gguguauacu gcugccguga 1020
   acaugagcau gaaauugcuu gguacacgga acguucugaa aagagcuaug aauugcagac 1080
   accuuuugaa auuaaauugg caaagaaauu ugacancuun aauggggaau guccaaauuu 1140
   uguauuuccc uuaaauucna uaaucaagac uauucaacca aggguugaaa agaaaaagcu 1200
   ugauggcuuu auggguagaa uucgaucugu cuauccaguu gcgucaccaa augaaugcaa 1260
   ccaaaugugc cuuucaacuc ucaugaagug ugaucauugu ggugaaacuu cauggcagac 1320
   gggcgauuuu guuaaagcca cuugcgaauu uuguggcacu gagaauuuga cuaaagaagg 1380
   ugccacuacu ugugguuacu uaccncaaaa ugcuguuguu aaaauuuauu guccagcaug 1440
   unacaauuca gaanuaggac cugagcauag ucuugccgaa uaccaunaug aaucuggcuu 1500
   gaaaaccauu cuucguaagg guggungcac uauugccuuu ggaggcugug uguucucuua 1560
   uguugguugc cauaacaagu gugccuauug gguuccacgu gcuancgcua acauagguug 1620
   uaaccauaca gguguuguug gagaagguuc cgaaggucuu aaugacaacc uucuugaaau 1680
   acuccaaaaa gagaaaguca acaucaauau uguuggugac uuuaaacuua augaagagau 1740
   cgccauunuu uuggcaucuu uuucugcuuc cacaagugcu uuuguggaaa nugugaaagg 1800
   uuuggauuau aaagcauuca aacaaauugu ugaauccugu gguaauuuua aaguuacaaa 1860
   aggaaaagcu aaaaaaggug ccuggaauau uggugaacag aaaucaauac ugaguccucu 1920
   uuaugcauuu gcaucagagg cugcucgugu uguacgauca auuuucuncc gcacucuuga 1980
   aacugcucaa aauucugugc guguuuuaca gaaggccgcu auaacaauac uagauggaau 2040
   uucacaguau ucacugagac ucauugaugc uaugauguuc acaucugauu uggcuacuaa 2100
   caaucuaguu guaauggccu acauuacagg ugguguuguu caguuganuu cgcaguggcu 2160
   aacuaacauc uuuggcacug uuuaugaaaa acucaaaccc guccuugauu ggcuugaaga 2220
   gaaguuuaag gaaggugnng aguuucuuag agacgguugg gaaauuguua aauuuaucuc 2280
   aaccugugcu ugugaaauug ucgguggaca aauugucacc ugugcnaagg aaanuaagga 2340
   gaguguucag acauucuuua agcuuguaaa uaaauuuuug gcuuugugug cugacucuau 2400
   cauuauuggu ggagcuaaac uuaaagccuu gaauuuaggu gaaacauuug ucacgcacuc 2460
   aaagggauug uacagaaagu guguuaaauc cagagaagaa acnggccuac ucaugccucu 2520
   aaaagcccca aaagaaauua ucuucuuaga gggagaaaca cuucccacag aaguguuaac 2580
   agaggaaguu gucuugaaaa cuggugauuu acaaccauua gaacaaccua cuagugaagc 2640
   uguugaagcu ccauugguug guacaccagu uuguauuaac gggcuuaunu ugcucgaaau 2700
   caaagacaca gaaaaguacu gugcccuugc accuaauaug augguaacaa acaauaccuu 2760
   cacacucaaa ggcngugcac caacaaaggu uacuuuuggu gaugacacug ugauagaagu 2820
   gcaagguuac aagaguguga auaucacuuu ugaacuugau gaaaggauug auaaaguacu 2880
   uaaugagaag ugcucugccu auacaguuga acucgguaca gaaguaaaug aguucgccug 2940
   uguuguggca gaugcuguca uaaaaacuuu gcaaccagua ucugaauuac uuacaccacu 3000
   gggcauugau uuagaugagu ggaguaungc uacauacuac uuauuugaug agucugguga 3060
   guuuaaauug gcuucacaua uguauuguuc uuunuacccu ccagaugagg augaagaaga 3120
   aggugauugu gaagaagaag aguuugagcc aucaanucaa uaugaguaug guacugaaga 3180
   ugauuaccaa gguaaaccuu uggaauuugg ugccacuucu gcugcucuuc aacnugaaga 3240
   agagcaagaa gaagauuggu uagaugauga uagucaacaa acuguugguc aacaagacgg 3300
   cagugaggac aaucanacaa cuacuauuca aacaauuguu gagguucaac cucaauuaga 3360
   gauggaacuu acaccaguug uucagacuau ugaagugaau aguuuuagug guuauuuaaa 3420
   acuuacugac aauguauaca uuaaaaaugc agacauugug gaagaagnua aaaagguaaa 3480
   accaacagug guuguuaaug cagccaaugu uuaccuuaaa cauggaggag guguugcagg 3540
   agccuuaaau aaggcuacua acaaugccau gcaanuugaa ucugaugauu acauagcuac 3600
   uaauggacca cuuaaagugg gugguaguug uguuuuaagc ggacacaauc uugcuaaaca 3660
   cugucuucau guugucggcc caaauguuaa caaaggugaa gacauucaac uucuuaagag 3720
   ugcuuaugaa aauuuuaauc agcacgaagu ucuacuugca ccauuauuau cagcugguau 3780
   uuuuggugcu gaccnuauac auucuuuaag aguuugugua gauacuguuc gcacnaaugu 3840
   cuacuuagcu gucuuugaua aaaaucucua ugacaaacuu guuucaagcu uuuuggaaau 3900
   gaagagugaa aagcaaguug aacaaaagau cgcugagauu ccuaaagagg aaguuaagcc 3960
   auuuauaacu gaaaguaaac cuucaguuga acagagaaaa caagaugaua agaaaaucaa 4020
   agcuuguguu gaagaaguua caacaacunu ggaagaaacu aaguuccuca cagaaaacuu 4080
   guuacuuuau auugacauua auggcaaucu ucauccagau ucugccacuc uuguuaguga 4140
   cauugacauc acuuucuuaa agaaagaugc uccauauaua gugggugaug uuguucaaga 4200
   gggguguuua acugcugugg uuauaccuac uaaaaaggcu gguggcacua cugaaaugcu 4260
   agcgaaagcu uugagaaaag ugccaacaga caauuauaua accacuuacc cgggucaggg 4320
   uuuaaauggu uacacuguag aggangcaaa gacagugcuu aaanagugua aaagugccuu 4380
   uuacauucua ccaucuauua ucucuaauga gaagcaagaa auucuuggaa cuguuucuug 4440
   gaauuugcga gaaaugcung cacaugcaga agaaacacgc aaauuaaugc cugucugugu 4500
   ggaaacuaaa gccauaguuu caacuauaca gcguaaauau aaggguauua aaauacaaga 4560
   gggugugguu gauuauggug cuagauuuua cuuuuacacc aguaaaacaa cuguagcguc 4620
   acuuaucaac acacuuaacg aucuaaauga aacucuuguu acaaugccac uuggcuaugu 4680
   aacacauggc uuaaauuugg aagaagcugc ucgguauaug agaucucuca aagugccagc 4740
   uacaguuucu guuucuucac cugaugcugu uacagcguau aaugguuauc uuacuucuuc 4800
   uucuaaaaca ccugaagaac auuuuauuga aaccaucuca cuugcugguu ccuauaaaga 4860
   uugguccuau ucuggacaau cuacacaacu agguauagaa uuucuuaaga gaggugauaa 4920
   aaguguauau uacacnnnua auccuaccac auuccaccua gauggugaag uuaucaccuu 4980
   ugacaaucuu aagacacuuc uuucuuugag agaagugagg acuauuaagg uguuuacaac 5040
   aguagacaac auuaaccucc acacgcaagu uguggacaug ucaaugacau auggacaaca 5100
   guuuggucna acuuauuung auggagcuga uguuacuaaa nuaaaaccuc auaauucaca 5160
   ugaagguaaa acauuuuaug uuuuaccuaa ugaugacacu cuacguguug aggcuuuuga 5220
   guacuaccac acaacugauc cuaguuuucu ggguagguac augucagcau uaaaucacac 5280
   uaaaaagugg aaauacccac aaguuaaugg uuuaacuucu auuaaauggg cagauaacaa 5340
   cuguuaucuu gccacugcau uguuaacacu ccaacaaaua gaguugaagu uuaauccacc 5400
   ugcucuacaa gaugcuuauu acagagcaag ggcuggugaa gcugcuaacu uuugugcacu 5460
   uaucuuagcc uacuguaaua agacaguagg ugaguuaggu gauguuagag aaacaaugag 5520
   uuacuuguuu caacauncca auuuagauuc uugcaaaaga gucuugaacg ugguguguaa 5580
   aacuugugga caacagcaga caacccuuaa ggguguagaa gcuguuaunu acaugggcac 5640
   acuuucuuau gaacaauuua agaaaggugu ucagauaccu uguacgugug guaaacaagc 5700
   uacaaaauau cuaguacaac aggagucacc uuuuguuaug augucagcac caccugcuca 5760
   guaugaacuu aancauggua cauuuacuug ugcuagugag uacacuggua auuaccagug 5820
   uggucacuau aaacauauaa nuucuaaaga aacuuuguau ugcauagacg gugcuuuacu 5880
   uacaaagucc ucagaauaca angguccuau uacggauguu uucuacaaag aaaacaguua 5940
   cacaacaacc auaaaaccag uuacuuauaa auuggauggu guuguuugua cagaaauuga 6000
   cccuaaguug gacaauuauu auaagaaaga caauucuuau uucacagagc aaccaauuga 6060
   ucuuguacca aaccaaccau auncaaangc angcuucgau aauuuuaagu uuguauguga 6120
   uaauaucaaa uuugcugaug auuuaaacca guuaacuggu uauaagaaac cugcuucaag 6180
   agagcuuaaa guuacauuuu ucccugacuu aaauggugau gugguggcua uugauuauaa 6240
   acacuacaca cccucuuuua agaaaggagc uaaauuguua cauaaaccua uuguuuggca 6300
   uguuaacaau gcaacuaaua aagccacgua uaaaccaaau accuggugua uacguugucu 6360
   uuggagnaca aaaccaguug aaacaucaaa uucguuugau guacugaagu cagaggacgc 6420
   gcagggaaug gauaaucuug ccugcgaaga ucuaaaacca gucucugaag aaguagugga 6480
   aaauccuacc auacagaaag acguucuuga guguaaugug aaaacuaccg aaguuguagg 6540
   agacauuaua cuuaaacnag caaauaauag uuuaaaaauu acagaagagg uuggccacac 6600
   agaucuaaug gcugcuuaug uagacaauuc uagucuuacu auuaagaaac cuaaugaauu 6660
   aucuagagua uuagguuuga aaacccuugc uanucauggu uuagcugcug uuaauagugu 6720
   cccuugggau acuauagcua auuaugcuaa gncuuuucuu aacaaaguug uuaguacaac 6780
   uacuaacaua guuacacggu guuuaaaccg uguuuguacu aauuauaugc cuuauuucuu 6840
   uacuuuauug cuacaauugu guacuuuuac uagaanuaca aauucuagaa uuaaagcauc 6900
   uaugccgacu acuauagcaa agaauacugu uaagaguguc gguaaauuuu gunuagaggc 6960
   uucauuuaau uauuugaagu caccuaauuu uucuaaacug auaaauauua uaauunggau 7020
   uuuacuauua aguguuugcc uagguucuuu aancuacuca accgcugcuu uanguguuuu 7080
   aaugucuaau uuaggcaugc cuucuuacug uacugguuac agagaaggcu auuugaacuc 7140
   uacuaauguc acuauugcaa ccuacuguac ugguucuaua ccuuguagug uuugucuuag 7200
   ugguuuagau ucuuuagaca ccuauccuuc uuuagaaacu auacaaauua ccauuucauc 7260
   uuuuaaaugg gauuuaacug cuuuuggcuu aguugcagag ugguuuuugg cauauauucu 7320
   uuucacuagg uuuuucuaug uacuuggauu ggcugcaauc augcaauugu uuuucagcua 7380
   uuuugcagua cauuuuauua guaauucuug gcuuaugugg uuaauaauua aucuuguaca 7440
   aauggccccg auuucagcua ugguuagaau guacaucuuc uuugcaucau uuuauuaugu 7500
   auggaaaagu uaugugcaug uuguagacgg uuguanuuca ucaacuugua ugauguguua 7560
   caaacguaau agagcaacaa gagucgaaug uacaacuauu guuaauggug uuagaagguc 7620
   cuuuuauguc uaugcuaaug gagguaaagg cuuuugcaaa cuacacaauu ggaauugugu 7680
   uaauugugau acauucugug cugguaguac auuuauuagu gaugaaguug cgagagacuu 7740
   gucacuacag uuuaaaagac caauaaaucc uacugaccag ucuucuuaca ucguugauag 7800
   uguuacagug aagaaugguu ccauccaucu uuacuuugau aaagcugguc aaaagacuua 7860
   ugaaagacau ucucucucuc auuuuguuaa cuuagacaac cugagagcua auaacacuaa 7920
   agnuucauug ccuauuaaug uuauaguuuu ugaugguaaa ucaaaaugug aagaaucauc 7980
   ugcaaaauca gcgucuguuu acuacaguca gcuuaugugu caaccuauac uguuacuaga 8040
   ucaggcauua gugucugnug uuggugauag ugcggaaguu gcaguuaaaa uguuugaugc 8100
   uuacguuaau acguuuucau caacuuuuaa cguaccaaug gaaaaacuca aaacacuagu 8160
   ugcaacugca gaagcugaac uugcaaagaa ugugucauua gacaaugucu uaucuacuuu 8220
   uauuucagca gcucggcaag gguuuguuga uucagaugua gaaacuaaag auguuguuga 8280
   augucuuaaa uugucacauc aaucugacau agaaguuacu ggcgauaguu guaauaacua 8340
   uaugcucacc uauaacaaag uugaaaacnu gacaccccgu gaccuuggug cuuguauuga 8400
   cuguagugcg cgucauauua augcgcaggu agcaaaaagu cacancauug cuuugauaug 8460
   gaacguuaaa gauuucaugu cauugucuga acaacuacga aaacaauauc guagugcugc 8520
   uaaaaagaau aacuuaccuu uuaaguugac augugcaacu acuagacaag uuguuaaugu 8580
   uguaacaaca aagauagcac uuaagggugg uaaaauuguu aauaauuggu ugaagcaguu 8640
   aauuaaaguu acacuugugu uccuuuuugu ugcugcuauu uucuauuuaa uaacaccugu 8700
   ucaugucaug ucuaaacaua cugacuuuuc aagugaaauc auaggauaca aggcuauuga 8760
   uggugguguc acucgugaca uagcaucuac agauacuugu uuugcuaaca aacaugcuga 8820
   uuuunacaca ugguuuagnc agcguggugg uaguuauacu aaugacaaag cuugcccauu 8880
   gauugcugca gucauaacaa gagaaguggg uuuugucgug ccugguuugc cuggcacgau 8940
   auuacgcaca acuaauggug acuuuuugca uuucuuaccu agaguuuuua gugcaguugg 9000
   uaacaucugu uacacaccau caaaacuuau agaguacacu gacnuugcaa caucagcuug 9060
   uguuuuggcu gcugaaugua caauuuuuaa ngaugcuucu gguaagccag uaccauauug 9120
   uuaugauacc aauguacuag aagguucugu ugcuuaugaa aguuuacgcc cugacacacg 9180
   uuaugugcuc auggauggcu cuauuauuca auuncnuaac accuaccuug aagguunugu 9240
   uagaguggua acaacuuuug auucugagua cuguaggcac ggcacuugug aaagaucaga 9300
   agcugguguu uguguaucua cuagugguag nuggguacuu aacaaugauu auuacagauc 9360
   uuuaccagga guuuucugug guguagaugc uguaaauuua cuuacuaaua uguuuacacc 9420
   acuaauucaa ccuauuggug cuuuggacau aucagcaucu auaguagcug gugguauugu 9480
   agcuaucgua guaacaugcc uugccuacua uuuuaugagg uuuagaagag nuunugguga 9540
   auacagunau guaguugccu uuaauacnuu acuauuccuu augucauuca nuguacucug 9600
   uuuaacacca guuuacunau ucuuaccugg uguuuauucu guuauuuacu uguacuugac 9660
   auuuuaucuu acuaaugaug uuucuuuuuu agcacauauu caguggaugg uuauguucac 9720
   accuuuagua ccuuucugga uaacaauugc uuauaucauu uguauuucca caaagcauuu 9780
   cuauugguuc uuuaguaauu accuaaagag acguguaguc uuuaauggug uuuccuuuag 9840
   uacuuuugaa gaagcugcgc ugugcaccuu uuuguuaaau aaagaaaugu aucuaaaguu 9900
   gcguagugau gugcuauuac cucuuacgca auauaauaga uacuuagcuc uuuauaauaa 9960
   guacaaguau uuuaguggag naauggauac aacuagcuac agagaagcug cuuguuguca 10020
   ucucgcaaag gcucucaaug acuucaguaa cucagguucu gauguucuuu accaaccacc 10080
   acaaaccucu aunaccucag cuguuuugca gagugguuuu agaaaaaugg cauucccaun 10140
   ugguaaaguu gaggguugua ugguacaagu aacuuguggu acaacuacac uuaacggucu 10200
   uuggcuugau gacguaguuu acuguccaag acaugugauc ugcaccucug aagacaugcu 10260
   uaacccuaau uaugaagauu uacucauung uaagucuaau cauaauuucu ugguacaggc 10320
   ugguaauguu caacucaggg uuauuggaca uucuaugcaa aauuguguac uuaagcuuaa 10380
   gguugauaca gccaauccua agacaccuaa guauaaguuu guucgcauuc aaccaggaca 10440
   gacuuuuuca guguuagcuu guuacaaugg uucaccaucu gguguuuacc aaugugcuau 10500
   gaggcccaau uucacuauua aggguucauu ccuuaauggu ucauguggua guguugguuu 10560
   uaanauagau uaugacugug ucucuuuuug uuacaugcac cauauggaau uaccaacugg 10620
   aguucaugcu ggcacagacu uagaagguaa cuuuuaugga ccuuuuguug acaggcaaac 10680
   agcacaagca gcugguacgg acacaacuau uacaguuaau guuuuagcuu gguuguacgc 10740
   ugcuguuaua aauggagaca ggugguuucu caaucgauuu accacaacuc uuaaugacuu 10800
   uaaccuugug gcuaugaagu acaauuauga accucuaaca caagaccaug uugacauacu 10860
   aggaccucuu ucugcucaaa cuggaauugc cguuuuagau augugugcuu cauuaaaaga 10920
   auuacugcaa aaugguauga auggacguac cauauugggu agugcuuuau uagaagauga 10980
   auuuacaccu uuugauguug uuagacaaug cucagguguu acuuuccaaa gugcagugaa 11040
   aagaacaauc aaggguacac accacugguu guuacucaca auuuugacuu cacuuuuagu 11100
   uuuaguccag aguacucaau ggucuuuguu cuuuuuuuun uaugaaaaug ccuuuuuacc 11160
   uuuugcuaug gguauuauug cuaugucugc uuuugcaaug auguuuguca aacauaagca 11220
   ugcauuucuc uguuuguuuu uguuaccuuc ucuugccacu guancuuauu uuaauauggu 11280
   cuauaugccn gcuaguuggg ugaugcguau uaugacaugg uuggauaugg uugauacuag 11340
   uuugucuggu uuuaagcuaa aagacugugu uauguaugca ucagcuguag uguuacuaau 11400
   ccuuaugaca gcaagaacug uguaugauga uggugcuagg agagugugga cacuuaugaa 11460
   ugucuunaca cucguuuaua aaguuuauua ugguaaugcu uuagaucaag ccauuuccau 11520
   gugggcucuu auaaucucug uuacuucuaa cuacucaggu guaguuacaa cugucauguu 11580
   uuuggccaga gguauuguuu uuaugugugu ugaguauugc ccuauuuucu ucauaacugg 11640
   uaauacacuu caguguauaa ugcuaguuua uuguuucuua ggcuauuuuu guacuuguua 11700
   cuuuggccuc uuuuguuuac ucaaccgcua cuuuagacug acucuuggug uuuaugauua 11760
   cuuaguuucu acacaggagu uuagauauau gaauucacag ggacuanucc cacccaagaa 11820
   uagcauagau gccuucaaac ucaacauuaa auuguugggu guugguggca aaccuuguau 11880
   caaaguagcc acuguacagu cuaaaauguc agauguaaag ugcacaucag uagucuuacu 11940
   cucaguuuug caacaacuca gaguagaauc aucaucuaaa uugugggcuc aaugugucca 12000
   guuacacaau ganauucucu uagcuaaaga uacuacugaa gccuuugaaa aaaugguuuc 12060
   acuacuuucu guuuugcuuu ccaugcaggg ugcuguanac auaaacaagc uuugugaaga 12120
   aaugcuggac aacagggcaa ccuuacaagc uauagccuca gaguuuaguu cncuuccauc 12180
   auaugcagcu uuugcuacug cucaagaagc uuaugancag gcuguugcua auggugauuc 12240
   ugaaguuguu cuuaaaaanu ugaanaagun uuugaaugug gcuaaaucug aauuugaccg 12300
   ugaugcagcc augcaacgua aguuggaaaa gauggcugau caagcuauga cccaaaugua 12360
   uaaacaggcu agaucugagg acaagagggc aaaaguuacu agugcuaugc anacaaugcu 12420
   uuucacuaug cuuanaaagu uggauaauga ugcacucaac aacauuauca acaaugcaag 12480
   agaugguugu guucccuuga acauaauacc ucuuacaaca ncanccaaan uaaugguugu 12540
   cauaccanac uauaacacau auaaaaauan nugugauggu acaacauuua nuuaugcauc 12600
   agcauugugg gaaauccaac agguuguana ugcanauanu aaaauuguuc aacuuanuga 12660
   aauuaguaug gacaauucac cuaauuuagc auggccucuu auuguaacag cuuuaanggc 12720
   caauucugcu gucaaauuac anaauaauga gcuuaguccu guugcacuac gacagauguc 12780
   uugugcugcc gguacuacac aaacugcuug cacugaugac aaugcguuan cuuacuacaa 12840
   cacaacaaan ggagguaggu uuguacuugc acuguuaucc gauuuacagg auuugaaaug 12900
   ggcuagauuc ccuaagagug auggaacugg uacuaucuau acagaacugg aaccaccuug 12960
   uagguuuguu acagacacac cuaaagguccuaaagugaag uauuuauacu uuauuaaagg     13020
   auuaaacaac cuaaauagag guaugguacu ugguaguuua gcugccacag uacgucuaca 13080
   agcugguaau gcaacagaag ugccugccaa uucaacugua uuaucuuunu gugcuuuugc 13140
   uguagaugcu gcuaaagcuu acaaagauua ucuagcuagu gggggacaac caaucacuaa 13200
   uuguguuaag auguugugua cacacacugg uacuggucag gcaauaacag uuacaccgga 13260
   agccaauaug gaucaagaau cnnuuggugg ugcaucgugu ugucuguacu gccguugcca 13320
   cauagaucau ccaaauccua aaggauuuug ugacuuaaaa gguaaguaug uacaaauacc 13380
   uacaacuugu gcuaaugacc cuguggguuu uacacuuaaa aacacagucu guaccgucug 13440
   cgguaugugg aaagguuaug gcuguaguug ugaucaacuc cgcgaaccca ugcuucaguc 13500
   agcugaugca caaucguuuu uaaacggguu ugcgguguaa gugcagcccg ucuuacaccg 13560
   ugcggcacag gcacuaguac ugaugucgua uacagggcuu uugacaucua caaugauaaa 13620
   guagcugguu uugcuaaauu ccuaaaaacu aauuguuguc gcuuccaaga aaaggacgaa 13680
   gaugacaauu uaauugauuc uuacuuugua guuaagagac acacuuucuc uaacuaccaa 13740
   caugaagaaa caauuuauaa uuuacuuaag gauuguccag cuguugcuaa acaugacuuc 13800
   uuuaaguuua gaauagacgg ugacauggua ccacauauau cacgucaacg ucuuacuaaa 13860
   uacacaaugg cagaccucgu cuaugcuuua aggcauuuug augaagguaa uugugacaca 13920
   uuaaaagaaa uacuugucac auacaauugu ugugaugaug auuauuucaa uaaaaaggac 13980
   ugguaugauu uuguagaaaa cccagauaua uuacgcguau acgccaacuu aggugaacgu 14040
   guacgccaag cuuuguuaaa aacaguacaa uucugugaug ccaugcgaaa ugcugguauu 14100
   guugguguac ugacauuaga uaaucaagau cucaauggua acugguauga uuucggugau     14160
   uucauacaaa ccacgccagg uaguggaguu ccuguuguag auucuuauua uucauuguua 14220
   augccuauau uaaccuugac cagggcuuua acugcagagu cacauguuga cacugacuua 14280
   acaaanccuu acauuaagug ggauuuguua aaauaugacu ucacggaaga gagguuaaaa 14340
   cucuuugacc guuauuuuaa auauugggau cagacauacc acccaaauug uguuaacugu 14400
   uuggaugaca gaugcauucu gcauugugca aacuuuaaug uuuuauucuc uacaguguuc 14460
   ccacnuacaa guuuuggacc acuagugaga aaaauauuug uugauggugu uccauuugua 14520
   guuucaacug gauaccacuu cagagagcua gguguuguac auaaucagga uguaaacuua 14580
   cauagcucua gacuuaguuu uaaggaauua cuuguguaug cugcugaccc ugcuaugcac 14640
   gcugcuucug guaaucuauu acuagauaaa cgcacuacgu gcuuuucagu agcugcacuu 14700
   acuaacaaug uugnuuuuca aacugucaaa cccgguaauu uuaacaaaga cuucuaugac 14760
   uuugcugugu cuaaggguuu cuuuaaggaa ggaaguucug uugaauuaaa acacuucuuc 14820
   uuugcucagg augguaaugc ugcuaucagc gauuaugacu anuaucguua uaaucuacca 14880
   acaaugugug auaucagaca acuacuauuu guaguugaag uuguugauaa guacuuugau 14940
   uguuacgaug guggcuguau uaaugcuaac caagucaucg ucaacaaccu agacaaauca 15000
   gcugguuuuc cauuuaauaa augggguaag gcuagacuuu auuaugauuc aaugaguuau 15060
   gaggaucaag augcacuuuu cgcauauaca aaacguaaug ucaucccuac uauaacucaa 15120
   augaaucuua aguaugccau uagugcaaag aauagagcuc gcaccguagc uggugucucu 15180
   aucuguagua cuaugaccaa uagacaguuu caucaaaaau uauugaaauc aauagccgcc 15240
   acuagaggan cuacuguagu aauuggaaca agcaaauucu augguggaug gcacaacaug 15300
   uuaaaaacug uuuauaguga uguagaaaac ccucaccuua uggguuggga uuauccuaaa 15360
   ugugauagag ccaugccuaa naugcuuaga auuauggccu cacuuguucu ugcucgcaaa 15420
   cauacaacgu guuguagcuu gucacaccgu uucuauagau uagcuaauga gugugcucaa 15480
   guauugagug aaauggucau guguggcggu ucacuauaug uuaaaccagg uggaaccuca 15540
   ucaggagaug ccacaacugc uuaugcuaau aguguuuuua acauuuguca agcugucacg 15600
   gccaauguua augcacuuuu aucuacugau gguaacaaaa uunccgauaa guanguccgc 15660
   aaunuacaac acagacuuua ugagugucuc uauagaaaua gagauguuga cacagacuuu 15720
   gugaaugagu uuuacgcaua uuugcguaaa cauuucucaa ugaugauacu cucugacgau 15780
   gcuguugugu guuucaauag cacuuaugca ucucaagguc uaguggcuag cauaaagaac 15840
   uuuaagucag uucuuuauua ucaaaanaau guuuuuaugu cugaagcaaa auguuggacu 15900
   gagacugacc uuacuaaagg accucaugaa uuuugcucuc aacauacaau gcuaguuaaa 15960
   cagggugaug auuaugugua ccuuccuuac ccagauccau caagaauccu aggggccggc 16020
   uguuuuguag augauaucgu aaaaacagau gguacacuua ugauugaacg guucgugucu 16080
   uuagcuauag augcuuaccc acuuacuaaa cauccuaauc aggaguaugc ugaugucuuu 16140
   cauuuguacu uacaauacau aagaaagcua caugaugagu uaacaggaca cauguuagac 16200
   auguauucug uuaugcuuac uaaugauaac acuucaaggu auugggaacc ugaguuuuau 16260
   gaggcuaugu acacaccgca uacagucuua caggcuguug gggnuugugu ucuuugcaau 16320
   ucacagacuu cauuaagaug uggugcuugc auacguagac cauucuuaug uuguaaaugc 16380
   uguuacgacc augucauauc aacaucacau aaauuagucu ugucuguuaa uccguauguu 16440
   ugcaaugcuc cagguuguga ugucacagau gugacucaac uuuacuuagg agguaugagc 16500
   uauuauugua aaucacauaa accncccauu aguuuuccau ugugugcuaa uggacaaguu 16560
   uuugguuuau auaaaaauac auguguuggu ancgauaaug uuacugacuu uaaugcaauu 16620
   gcaacaugug acuggacaaa ugcuggugau uacauuuuag cuaacaccug uacugaaaga 16680
   cucaagcuuu uugcagcaga aacgcucaaa gcuacugagg agacauuuaa acugucuuau 16740
   gguauugcua cuguacguga agugcugucu gacagagaau uacaucuuuc augggaaguu 16800
   gguaaaccua gaccaccacu uaaccgaaau uaugucuuua cugguuaucg uguaacuaaa 16860
   aacaguaaag uacaaauagg agaguacacc uuugaaaaag gugacuaugg ugaugcuguu 16920
   guuuaccgag guanaacaac uuanaaauua aauguuggug auuauuuunu gcugacauca 16980
   cauacaguaa ugccauuaag ugcaccuaca cuagugccac aagagcacua uguuagaauu 17040
   acuggcuuau acccaanacu caauaucuca gaugaguuuu cuagcaaugu ugcaaauuau 17100
   caaaagguug guaugcaaaa guauucuaca cuccagggac caccugguac ugguaagagu 17160
   cauuuugcua uuggccuagc ucucuacuac ccuucugcuc gcauagugua uacagcuugc 17220
   ucucaugccg cuguugaugc acuaugugag aaggcauuaa aauauuugcc uauagauaaa 17280
   uguaguagaa uuauaccugc acgngcucgu guagaguguu uugauaaauu caaagugaau 17340
   ucaacauuag aacaguaugu cuuuuguacu guaaaugcau ugccugagac gacagcagau 17400
   auaguugucu uugaugaaau uucaauggcn acnaauuaug auuugagugu ugucaaugcc 17460
   agauuangug cuaagcacun uguguacauu ggcgacccug cucaauuacc ugcaccacgc 17520
   acauugcuaa nuaagggcac acuagaacca gaauauuuca auucagugug uagacuuaug 17580
   aaaacuauag guccagacau guuccucgga acuugucggc guuguccugc ugaaauuguu 17640
   gacacuguga gugcuuuggu uuaugauaau aagcuuaaag cacauaaaga caaaucagcu 17700
   caaugcuuua aaauguuuua uaaggguguu aucacgcaug auguuucauc ugcaauuaac 17760
   aggccacaaa uaggcguggu aagagaauuc cuuacacgua accnugcuug gagaaaagcu 17820
   gucuuuauuu caccuuauaa uucacagaau gcuguagccu caaagauuuu gggacuacca 17880
   acucaaacug uugauucauc acagggcuca gaaunugacu augucauauu cacucaaacc 17940
   acugaaacag cucacucuug uaauguaaac agauuuaaug uugcuauuac cagagcaaaa 18000
   guaggcauac uuugcauaau gucugauaga gaccuuuaug acaaguugca auuuacaagu 18060
   cuugaaauuc cacguaggaa uguggcaacu uuacaagcug aaaauguaac aggacunuuu 18120
   aaagauugua guaagguaau cacuggguua cauccuacac aggcaccuac acaccucagu 18180
   guugacacua aauucaaaac ugaagguuua uguguugaca uaccuggcau accuaaggac 18240
   augaccuaua gaagacucau cucuaugaug gguuuuaaaa ugaauuauca aguuaauggu 18300
   uacccuaaca uguuuaucac ccgcgaagaa gcuauaagac auguacgugc auggauuggc 18360
   uucgaugucg agggguguca ugcuacuaga gaagcuguug guaccaauuu accuuuacag 18420
   cuagguuuuu cuacaggugu uaaccuaguu gcuguaccua cagguuaugu ugauacaccu 18480
   aauaauacag auuuuuccag aguuagugcu aaaccaccgc cuggagauca auuuaaacac 18540
   cucauaccac uuauguacaa aggacuuccu uggaauguag ugcguauaaa gauuguacaa 18600
   auguuaagug acacacuuaa aaaunucucu gacagagucg uauuugucuu augggcacan 18660
   ggcuuugagu ugacaucuau gaaguauuuu gugaaaauag gaccugagcg caccuguugu 18720
   cuaugugaua gacgugccac augcuuuucc acugcuucag acacuuaugc cuguuggcau 18780
   cauucuauug gauuugauua cgucuauaau ccguuuauga uugauguuca acaauggggu 18840
   uuuacaggua accuacaaag caaccaugau nuguauuguc aaguccaugg uaaugcacau 18900
   guagcuaguu gugaugcaau caugacuagg ugunuagcug uccacgagug cuuuguuaag 18960
   cguguugacu ggacuauuga auauccuaua auuggugaug aacugaagau uaaugcggcu 19020
   uguagaaagg uncaacacau gguuguuaaa gcugcauuau uagcagacaa auucccaguu 19080
   cuucacgaca uugguaaccc uaaagcuauu aaguguguac cncaagcuga uguagaaugg    19140
   aaguucuaug augcacagcc uuguagugac aaagcuuaua aaauagaaga auuauucuau 19200
   ucuuaugcca cacauucuga caaauucaca gnugguguau gccuauuuug gaauugcaau 19260
   gucgauagau auccugcuaa uuccauuguu uguagauuug acacuagagu gcuaucuaac 19320
   cuuaacuugc cugguuguga ugguggcagu uuguauguaa auaaacaugc auuccacaca 19380
   ccagcuuuug auaaaagugc uuuuguuaau uuaaaacaau uaccauuuuu cuauuacucu 19440
   gacaguccau gugagucuca uggaaaacaa guagugucag auauagauua uguaccacua 19500
   aagucugcua cguguauaac acguugcaau uuagguggug cugucuguag acaucaugcu 19560
   aaugaguaca gauuguaucu cgaugcuuau aacaugauga ucucagcugg cuuuagcuug 19620
   uggguuuaca aacaauuuga uacuuauaac cucuggaaca cuuuuanaag acuucagagu 19680
   uuagaaaaug uggcuuuuaa uguuguaaau aagggacacu uugauggaca acagggugaa 19740
   guaccaguuu cuaucauuaa uaacacuguu uacacaaaag uugauggugu ugauguagaa 19800
   uuguuugaaa auaaaacaac auuaccuguu aauguagcau uugagcuuug ggcuaagcgc 19860
   aacauuaaac caguaccaga ggugaaaaua cucaauaauu ugggugugga cauugcugcu 19920
   aauacuguga ucugggacua caaaagagau gcuccagcac auauaucuac uauugguguu 19980
   uguucuauga cugacauagc caagaaacca acugaaacga uuugugcacc acucacuguc 20040
   uuuuuugaug guagaguuga uggucaagua gacuuauuua gaaaugcccg uaaugguguu 20100
   cuuauuacag aagguagugu uaaagguuua caaccaucug uaggucccaa acaagcuagu 20160
   cuuaauggag ucacauuaau uggagaagcc guaaaaacac aguucaauua uuauaagaaa 20220
   guugauggug uuguccaaca auuaccugaa acuuacuuua cucagaguag aaauuuacaa 20280
   gaauuuaaac ccaggaguca aauggaaauu gauuucuuag aauuagcuau ggaugaanuc 20340
   auugaacggu auaannnaga aggcuaugcc uucgaacaua ucguuuaugg agauuuuagu 20400
   cauagucagu uaggugguuu acaucuacug auuggacuag cuaaacguuu uaaggaauca 20460
   ccuuuugaau uagaagauuu uauuccuaug gacaguacag uuaaaaacua uuucauaaca 20520
   gaugcgcaaa cagguucauc uaagugugug uguucuguua uugauuuauu acuugaugau 20580
   uuuguugaaa uaauaaaauc ccaagauuua ucuguaguuu cuaagguugu caaagugacu 20640
   auugacuaua cagaaauuuc auuuaugcuu ugguguaaag auggccaugu agaaacauuu 20700
   uacccaaaau uacaaucuag ucaagcgugg caaccgggug uugcuaugcc uaaucuuuac    20760
   aaaaugcaaa gaaugcuauu agaaaagugu gaccuucaaa auuaugguga uagugcaaca    20820
   uuaccuaaag gcauaaugau gaaugucgca aaauauacuc aacuguguca auauuuaaan 20880
   acauuaacau uagcuguacc cuauaauaug agaguuauac auuuuggugc ugguucugau 20940
   aaaggaguug caccagguac agcuguuuua agacaguggu ugccuacggg uangcugcuu 21000
   gucgauucag aucuuaauga cuuugucucu gaugcanauu caacnuugau uggugauugu 21060
   gcaacuguac auacagcuaa uaaaugggau cucauuauua gugauaugua cgacccuaag 21120
   acuaaaaaug uuacaaaaga aaaugacucu aaagaggguu uuuucacuua cauuuguggg 21180
   uuuauacaac aaangcuagc ucunggaggu uccguggcua uaaagauaac agaacauucu 21240
   uggaaugcug aucuuuauaa gcucauggga cacuucgcau gguggacagc cuuuguuacu 21300
   aaugugaaug cgucaucauc ugaagcauuu uuaauuggau guaauuaucu uggcaaacca 21360
   cgcgaacaaa uanaugguua ugucaugcau gcaaauuaca uauuuuggag gaauacaaau 21420
   ccaauucagu ugucuuccua unnnunuuua uuugacauga guaaauuucc ccuuaaauua 21480
   agggguacug cuguuauguc uuuaaaagaa ggucaaauca augauaugau uuuaucucuu 21540
   cuuaguaaag guagacuuau aauuagagaa aacaacagag uuguuauuuc uagugauguu 21600
   cuuguuaaca acuaaacgaa caauguuugu uuuunuuguu uuauugccac uagucucuag 21660
   ucaguguguu aaucuuacaa ccagaacuca auuacccccu gcanacacua auucuuucac 21720
   acgugguguu uauuacccug acaaaguuuu cagauccuca guuuuanauu caacucagga 21780
   cuuguucuua ccuuucuuuu ccaauguuac uugguuccau gcuauacaug ucucugggac 21840
   caangguacu aagagguuug auaacccugu ccuaccauuu aaugauggug uuuauuuugc 21900
   uuccacugag aagucuaaca uaauaagagg cuggauuuuu gguacuacuu uagauucgaa 21960
   gacccagucc cuacuuauug uuaauaacgc uacuaauguu guuauuaaag ucugugaauu 22020
   ucaauuuugu aaugauccau uuuugggugu nnnuuaccac aaaaacaaca aaaguuggau 22080
   ggaaagugag uunagaguuu auucuagugc gaauaauugc acuuuugaau augucucuca 22140
   gccuuuucuu auggaccuug aagnaaaaca ggguaauuuc aaaaaucuua gggaauuugu 22200
   guuuaagaau auugaugguu auuuuaaaau auauucuaag cacacgccua uuaauuuagu 22260
   gcgugaucuc ccucaggguu uuunggcuuu agaaccauug guagauuugc caauagguau 22320
   uaacaucacu agguuucaaa cuuuacuugc uuuacauaga agnuauuuga cuccugguga 22380
   uucuucuuca gguuggacag cuggugcugc agcuuauuau guggguuauc uucaaccuag 22440
   gacuuuucua uuaaaauaua augaaaaugg aaccauuaca gaugcuguag anugugcacu 22500
   ugacccucuc ucagaaacaa aguguacguu gaaauccuuc acuguagaaa aaggaaucua 22560
   ucaaacuucu aacuuuagag uccaaccaac agaaucuauu guuagauuuc cuaauauuac 22620
   aaacuugugc ccuuuuggug aaguuuuuaa cgccaccaga uuugcaucug uuuaugcuug 22680
   gaacaggaag agaaucagca acuguguugc ugauuauucu guccuauaua auuccgcauc 22740
   auuuuccacu uuuaaguguu auggaguguc uccuacuaaa uuaaaugauc ucugcuuuac 22800
   uaaugucuau gcagauucau uuguaauuag aggugaugaa gucanacaaa ucgcuccagg 22860
   gcaaacugga aagauugcug auuauaauua uaaauuacca gaugauuuua caggcugcgu 22920
   uauagcuugg aauucuaaca aucuugauuc uaagguuggu gguaauuaua auuaccugua 22980
   uagauuguuu aggaagucua aucucaaacc uuuugagaga gauauuucaa cugaaaucua 23040
   ucangccngu agcacaccuu guaauggugu ugaagguuuu aauuguuacu uuccuuuaca 23100
   aucauauggu uuccaaccca cuaauggugu ugguuaccaa ccauacagag uaguaguacu 23160
   uucuuuugaa cuucuacaug caccagcaac uguuugugga ccuaaaaagu cuacuaauuu 23220
   gguuaaaaac aaauguguca auuunaacuu caaugguuua acaggcacag guguucuuac 23280
   ugagucuaac aaaaaguuuc ugccuuucca acaauuuggc agagacauug cugacacuac 23340
   ugaugcuguc cgugauccac agacacuuga gauucuugac auuacaccau guucuuuugg 23400
   uggugucagu guuauaacac caggaacaaa uacuucuaac cagguugcug uucuuuauca 23460
   ggnuguuaac ugcacagaag ucccuguugc uauucaugca gaucaacuua cuccuacuug 23520
   gcguguuuau ucuacagguu cuaauguuuu ucaaacacgu gcaggcuguu uaauaggggc 23580
   ugaacauguc aacaacucau augaguguga cauacccauu ggugcaggua uaugcgcuag 23640
   uuaucagacu cagacuaauu cuccucggcg ggcacguagu guagcuaguc aauccaucau 23700
   ugccuacacu augucacuug gugcagaaaa uucaguugcu uacucuaaua acucuauugc 23760
   cauacccaca aauuuuacua uuaguguuac cacagaaauu cuaccagugu cuaugaccaa 23820
   gacaucagua gauuguacaa uguacauuug uggugauuca acugaaugca gcaaucuuuu 23880
   guugcaauau ggcaguuuuu guacacaauu aaaccgugcu uuaacuggaa uagcuguuga 23940
   acaagacaaa aacacccaag aaguuuuugc acaagucaaa caaauuuaca aaacaccacc 24000
   aauuaaagau unuggugguu uuaauuuuuc acaaauauua ccagauccau caaaaccaag 24060
   caagagguca uuuauugaag aucuacuuuu caanaaagug acacuugcag augcuggcuu 24120
   caucaaacaa uauggugauu gccuugguga uauugcugcu agagaccuca uuugugcaca 24180
   aaaguuuaac ggccuuacug uuuugccacc uuugcucaca gaugaaauga uugcucaaua 24240
   cacuucugca cuguuagcgg guacaaucac uucugguugg accuuuggug caggugcugc 24300
   auuacaaaua ccauuugcua ugcaaauggc uuauagguuu aaugguauug gaguuacaca 24360
   gaauguucuc uaugagaacc aaaanuugau ugccaaccaa uuuaauagug nuauuggcaa 24420
   aauucaagac ucacuuucuu ccacagcaag ugcacuugga aaacuucaag auguggucaa 24480
   ccaaaaugca caagcuuuaa acacgcuugu uaaacaacuu agcuccaauu uuggugcaau 24540
   uucaaguguu uuaaaugaua uccuuucacg ucuugacaaa guugaggcug aagugcaaau 24600
   ugauagguug aucacaggca gacuucaaag uuugcagaca uaugugacuc aacaauuaau 24660
   uagagcugca gaaaucagag cuucugcuaa ucuugcugcu acuaaaaugu cagagugugu 24720
   acuuggacaa ucaaaaagag uugauuuuug uggaaagggc uaucaucuua uguccuuccc 24780
   ucagucagca ccucauggug uagucuucuu gcaugugacu uaugucccug cacaagaaaa 24840
   gaacuucaca acugcuccug ccauuuguca ugauggaaaa gcacacuuuc cucgugaagg 24900
   ugucuuuguu ucaaauggca cacacugguu uguaacacaa aggaauuuuu augaaccaca 24960
   aaucauuacu acagacaaca cauuuguguc ugguaacugu gauguuguaa uaggaauugu 25020
   caacaacaca guuuaugauc cuuugcaacc ugaauuagac ucauucaagg aggaguuaga 25080
   uaaauauuuu aagaaucaua caucaccaga uguugauuua ggugacaucu cuggcauuaa 25140
   ugcuucaguu guaaacauuc aaaaagaaau ugaccgccuc aaugagguug ccaagaauuu 25200
   aaaugaaucu cucaucgauc uccaagaacu uggaaaguau gagcaguaua uaaaauggcc 25260
   augguacauu uggcuagguu uuauagcugg cuugauugcc auaguaaugg ugacaauuau 25320
   gcuuugcugu augaccaguu gcuguaguug ucucaagggc uguuguucuu guggauccug 25380
   cugcaaauuu gaugaagacg acucugagcc agugcucaaa ggagucaaau uacauuacac 25440
   auaaacgaac uuauggauuu guuuaugaga aucuucacaa uuggaacugu aacuuugaag 25500
   caaggugaaa ucaaggaugc uacuccuuca gauuuuguuc gcgcuacugc aacgauaccg 25560
   auacaagccu cacucccuuu cggauggcuu auuguuggcg uugcacuucu ugcuguuuuu 25620
   canagcgcuu ccaaaaucau aacccunaaa aagagauggc aacuagcacu cuccaagggu 25680
   guucacuuug uuugcaacuu gcuguuguug uuuguaacag uuuacucaca ccuuuugcuc 25740
   guugcugcug gccuugaagc cccnuuucuc uaucuuuaug cuuuagucua cuucuugcag 25800
   aguauaaacu uuguaagaau aauaaugagg cuuungcuuu gcuggaaaug ccguuccnaa 25860
   aacccauuac uuuaugaugc caacuauuuu cuuugcuggc auacuaauug uuacgacuau 25920
   uguauaccuu acaauagugu aacuucuuca auugucauua cuucagguga uggcacaaca 25980
   aguccuauuu cugaacauga cuaccagauu ggugguuaua cugaaaaaug ggaaucugna 26040
   guaaaagacu guguuguauu acacaguuac uucacuucag acuauuacca gcuguacuca 26100
   acucaauuga guacagacac ugguguugaa cauguuaccu ucuucaucua caauaaaauu 26160
   guugaugagc cugaagaaca uguccaaauu cacacaaucg acgnuucauc cggaguuguu 26220
   aauccaguaa uggaaccaau uuaugaugaa ccgacgacga cuacuagcgu gccuuuguaa 26280
   gcacaagcug augaguacga acuuauguac ucauucguuu cggaagagac agguacguua 26340
   auaguuaaua gcguacuucu uuuucuugcu uucgugguau ucuugnuagu uacacuagcc 26400
   auccuuacug cgcnucgauu gugugcguac ugcugcaaua uuguuaacgu gagucuugua 26460
   aaaccuucuu uuuacguuua cucucguguu aaaaaucuga auucuucuag aguuccunau 26520
   cuucuggucu aaacgaacua aauauuauau uaguuuuucu guuuggaacu uuaauuuuag 26580
   ccauggcaga uuccaacggu acuauuaccg uugaagagcu uaaaaagcuc cuugaacaau 26640
   ggaaccuagu aauagguuuc cuauuccuua cauggauuug ucuucuacaa uuugccuaug 26700
   ccaacaggaa uagguuuuug uauauaauua aguuaauuuu ccucuggcug uuauggccag 26760
   uaacuuuagc uuguuuugug cuugcugcng uuuacagaau aaauuggauc accgguggaa 26820
   uugcuaucgc aauggcuugu cuuguaggcu ugauguggcu cagcuacuuc auugcuucuu 26880
   ucagacuguu ugcgcguacg cguuccaugu ggucauucaa uccagaaacu aacauucuuc 26940
   ucaacgugcc acuccauggc acuauucuga ccagaccgcu ucuagaaagu gaacucguaa 27000
   ucggagcugu gauccuucgu ggacaucuuc guauugcugg acaccaucua ggacgcugug 27060
   acaucaagga ccugccuaaa gaaaucacug uugcuacauc acgaacgcuu ucuuauuaca 27120
   aauugggagc uucgcagcgu guagcaggug acucagguuu ugcugcauac agucgcuaca 27180
   ggauuggcaa cuauaaauua aacacagacc auuccaguag cagugacaau auugcuuugc 27240
   uuguacagua agugacaaca gauguuucau cucguugacu uucagnuuac uauagcagag 27300
   auauuacuaa uuauuaugag gacuuuuaaa guuucnauuu ggaaucuuga uuacaucaua 27360
   aaccucauaa uuaaaaauuu aucuaaguca cuaacugaga auaaauauuc ucaauuagau 27420
   gaagagcaac caauggagau uganuaaacg aacaugaaaa uuauucuuuu cuuggcacug 27480
   auaacacucg cuacuuguga gcuuuaucac uaccaagagu guguuagagg uacaacagua 27540
   cuuuuaaaag aaccuugcuc uucuggaaca uacgagggca auucnccauu ucauccucua 27600
   gcugauaaca aauuugcacu gacuugcuuu agcacucaau uugcuuuugc uuguccugac 27660
   ggcguaaaac acgucuauca guuacgugcc agaucaguuu caccuaaacu guucaucaga 27720
   caagaggaag uucaagaacu uuacucucca auuuuucuua uuguugcggc aauaguguuu 27780
   auaacacuuu gcuucacacu caaaagaaag acagaaugau ugaacuuuca uuaauugacu 27840
   ucuauuugug cuuuuuagcc uuucuncuau uccuuguuuu aauuaugcuu auuaucuuuu 27900
   gguucucacu ugaacugcaa gaucauaaug aaacuuguca cgccuaaacg aacaugaaau 27960
   uucuuguuuu cuuaggaauc aucanaacug uagcugcauu ucaccaagaa uguaguuuac 28020
   agunauguac ucaacaucaa ccauauguag uugaugaccc guguccuauu cacuucuauu 28080
   cuaaauggua uauuagagua ggagcuagaa aaucagcacc uuuaauugaa uugugcnugg 28140
   augaggcugg uucuaaauca cccauucagu acaucgauau cgguaauuau acaguuuccu 28200
   guunaccuuu uacaauuaau ugccaggaac cuaaauuggg uagucuugua gugcguuguu 28260
   cguucuauga agacuuuuua gaguaucaug acguucgugu uguuuuagau uunaucuaaa 28320
   cgaacaaacu aaaaugucug auaauggacc ccaaaaucag cgaaaugcac cccgcauuac 28380
   guuuggugga cccucagauu caacuggcag uaaccagaau ggagaangca guggggcncg 28440
   aucaaaacaa cgucggcccc aagguuuanc caauaauacu gcgucuuggu ucaccgcucu 28500
   cacucaacau ggcaaggaag accuuaaauu cccucgagga caaggcguuc caauuaacac 28560
   caauagcagu ccagaugacc aaauuggcua cuaccgaaga gcuaccagac gaauucgugg 28620
   uggugacggu aaaaugaaag aucucagucc aagaugguau uucuacuacc uaggaacugg 28680
   gccagaagcu ggacuucccu auggugcuaa caaaganggc aucauauggg uugcaacuga 28740
   gggagccuug aauacaccaa aagaucacau uggcacccgc aauccugcua acaaugcugc 28800
   aaucgugcua caacuuccuc aaggaacaac auugccaaaa ggcuucuacg cngaagggag 28860
   cagaggcggc agucaagccu cuucucguuc cucaucacgu agucgcaaca guunaagaaa 28920
   uunaacucca ggcagcanua nnngaacuuc uccugcuaga auggcuggca auggcnguga 28980
   ugcugcucuu gcuuugcugc ugcuugacag auugaaccag cuugagagca aaaugucugg 29040
   uaaaggccaa caacaacaag gccaaacugu cacuaagaaa ucugcugcug aggcuucuaa 29100
   gaagccucgg caaaaacgua cugccacuaa agcauacaau guaacacaag cuuunggcag 29160
   acguggucca gaacaaaccc aaggaaauuu uggggaccag gaacuaauca gacaaggaac 29220
   ugauuacaaa cauuggccgc aaauugcaca auuugccccc agcgcuucag cguucuucgg 29280
   aaugucgcgn auuggcaugg aagucacacc uucnggaacg ugguugaccu acacaggugc 29340
   caucaaauug gaugacaaag nuncaaauuu caaagaucaa gucauuuugc ugaauaagca 29400
   uauugacgca uacaaaacau ucccaccaac agagccuaaa aaggacaaaa agaagaaggc 29460
   ugaugaaacu caagccuuac cgcagagaca gaagaaacag caaacuguga cucuucuucc     29520
   ugcugcagau uuggaugauu ucuccaaaca auugcaacaa uccaugagca gugcugacuc     29580
   aacucangcc uaaacucaug cagaccacac aangcagaug ggcuauauaa acguuuucgc     29640
   uuuuccguuu acgaunuaua gucuacucuu gugcagaaug aauucucgua acuanauagc     29700
   acaaguagau guaguuaacu uuaaucucac auagcaaucu uuaaucagug uguaacauua     29760
   gggaggacuu gaaagagcca ccacauuuuc accgangcca cncggaguan nnnnnnnnnu     29820
   acagugaaca augnuaggga gagcugccua uauggaagag cccuaaugug uaaaauuaau     29880
   uuuagunnnn nnnnnnnnnn nnnnnnnnnn nnnnnnnnnn nnnnnnnnnn nnnnnnnnnn     29940
   nnnnnnnnnn nnnnnnnnnn nnnnnnnnnn nnnnnnnnnn nnnnnnnnnn nnnnnnnnnn     30000
   nnnnn                                                                 30005
<210> 3
   <211> 137560
   <212> DNA
   <213> Orf virus
<400> 3
   ggatccgcgg cttcgcgggc ggcggccggc tcccgcggcg gctgagccgc ggtgccgcga      60
   cgaacgcgga ccaggagttc ctgcgggagg agctacggca gaggctggaa ctgctgaatg     120
   ctttcgagga cgggcgtccg cgggaacgcg actccgcgga ggcggcaccc cgcagccgcg     180
   agacctcgct ctggagtcag ggagtcaggg agtcgggagt cagggagtcg ggagtcaggg     240
   agtcgggagt cagggagtcg ggagtcaggg agtcgggagt cagggagtcg ggagtcaggg     300
   agtcgggagt cagggagtcg ggagtcaggg agtcgggagt cagggagtcg ggagtcaggg     360
   agtcgggagt cagggagtcg ggagtcaggg agtcgggagt cagggagtcg ggagtcaggg     420
   agtcgggagg tcagggagtc gggagtcagg gaaacagaag tccaagtagt acgggtgaga     480
   caggagtcag gtggtcgggt gaccgcgccg tccgagtccc gcaaaaagtt tttagactct     540
   gagagaggcc gacctgcctc caacggttcc gcggaaaagt ttttataaaa agttttcggg     600
   agaggccgac tgccttccaa cggttcctcc tctcgcgtgc cgcgggcggc cgctctcccg     660
   cgacggtccc gccaacgcgt ttacaaagtt ttaaaagttt tcgagagagg ccgacctgtc     720
   ttccaacggt tgcgcgaaaa ggttctgcgg aggtttggaa gagccgcccg ccctccgaca     780
   tcctccgaaa agttttcgca aaaagttttt aaaggtttcg cgaggagttt tcgagggagg     840
   cgacctgcct ccgaggttcc gcgtaaacgt ttttacaaag cgtcggaggt gggggcgacc     900
   tgcccttcct ctcctccgaa aagctcttga gagtgtggga gaggcggccg gccttcgcgg     960
   tcgcgcgaaa aggttctgcg ggagttcgcg ggagctgacc cgccctccgc gcccctccga    1020
   aaagtttttg cacgaagtct tttggcgttc tcgagaggac gcctaccccg acggtaacgc    1080
   ggaacgtctc gggaggtcgg cctctccgct cccgcggtcg cggcggcggc ccgtctccgg 1140
   aggttccgcg aaaagtttta taaaaagttt tgagggaggt accgacctcc taaagtttta 1200
   cagagagttc tcgcaaaagt tttgggaggt cgacctgacc tcctaaagtt atcgaagagt 1260
   tctcgcgaag agttctcgca aagagtttga gaggccgacc tgcctccaac ggttccgcgg 1320
   aaaagtttta taagagtttt gagagaggtc gacctacctt ccaacggttc cgcggaaaag 1380
   ttttataaga gttttgagag aggtcgacct accttccaac ggttccgcgg aaaagtttta 1440
   taagagtttt gagagaggtc gacctactct ccgaggttcc gcggaaagtt ttataaaaag 1500
   tttttacaaa gtttttgagg gaggtcgaca cctcggatcc tccgcctcgc gtgccgcggg 1560
   cggcctccaa gagttttacg gaacgttctg gagaggagag gccgacctcc caaagatttt 1620
   gcggaacgtt ttggagagga ggactggcct cgaaaagatt ttacaaagag tttggaggga 1680
   ggactggcct ccaacggttc cgcgaaaaag ttttgcaaga gtttggagag aggtcgacca 1740
   ccctccgagg ttccgcgaaa gtgtttgcgg agagtccgag agaggcagac actgcctgcg 1800
   aaaagttttc gcggacggtt gagcgggtca ccgacctccg acagtttaca aacgttttac 1860
   ggagagttag agaggcagca gaccgacctc cgaacagttt agttttacaa agttttggag 1920
   ggtgaactgt catcgggaaa gttttacgaa gagttttggg agaggccgac ggttagccga 1980
   gcgagcgcgc gagcgagttt acgttctctc gctcgtgtgc gcgttaactc gcactggttc 2040
   cctctctaac tcggagtggg cgagcgagtg gttgactcgt cctccgctct cactctgagt 2100
   ggtgataact gttaaccatt aactcgtcct ccactctctc actctctcac tctgagtgag 2160
   gattgacttg ttaactcgtc ctccactctc tcgctctctc gctctgagtg agtgaggatt 2220
   aactgttcaa ctcgtcaact cgtcctctgc ctctcatcca agactgagtg ggtagttgac 2280
   tgctcttgtt ctcttactcc gaggggtgat tgagttagca acagctactc gttctcttgt 2340
   tctctcacac cgagtgagcg agtgagcaac gttacttgtt ctctcacacc gagtgagcga 2400
   gtgagcaacg gttaactcgt tctctcgttc tctcgttcta ttattccgag gagtggcgag 2460
   tgaggtaacg gttactgtta cttgttctct gttcccttcc tcggtgagcg gtgcgtcaac 2520
   ttgttctcgt gagtgagtac ggtcacttgt tctctcgttc tctacctcga ggagtgagtc 2580
   aacttgttct cgtgagtgag ttgaccgtaa ccgttccctt acctcaagtg agtggcggtc 2640
   gcttgttctc gtgagtgggt taaccgcgtt cccttaccgg agtgagcggg cgggcgataa 2700
   aaataatcaa ttgactgatt cgctcgtgag cgagcgaagg gcggcggaca agggcgcggg 2760
   atgctggtct aatctactaa ggccgattac aaaaacggat gggagaccgg gagggagagg 2820
   gtcacagctc cgagcggtgc atccgcgcca gctggcggcg ccactcggcc gcgggccgcg 2880
   gccgcccagg ccgcgttgta gccgcccgcc accgcgacgc aggtcagctc gaactccggc 2940
   ccgagcgcgc gcacgtcgta gatgtgcacg gtcgcgacgt tcagcagcag cgcgcccttg 3000
   cgcagcgagg cgaaggtcgc gtgcatgccg gcggcgagcg ggtacaccgg cgagagcgtc 3060
   ccgccgccgt gcgcgaccac cgccatgtgc cgcccgtcgc cgccgacggt caggcaggtc 3120
   accgaggcgg agccgttcgc gcggacgggg cccgcctcca cgagggcggc cggcagcggc 3180
   ggcgccgcgg ggcggaagag cccgccgagg aggaagccca gcgccaccag cgcgagcgcg 3240
   ccgagcagcc tgcgcggcga ggggtgcatg cttgttggct gcggtgttgg cgtctggcgg 3300
   gtggaaggcg ggtgtggggt gtccgcggct gcggcgggag ggtctcgact gctaggcggt 3360
   cctttttcac tttgctccgt ggcgcctggt ccggggcaag ggctgcggcg ggcgcccggg 3420
   cgggcgggcc gctaccccgc cgcgcggccc gcggccgcca gcgccgcggc cagccgccgc 3480
   caccgcgggc ccgccgcgcg cagcagcccc gccgccgagc gccccgcgcc gccgcgggcg 3540
   cgcgccgagg ccgcggcccc gcgccgcgcc agcagcagcg gcagccgcgc gtccagcggg 3600
   ccgccgcggc gcagcgccgc gcgcagcagc gccgccagcg gcagccgccc cgccgcgtcc 3660
   gggcccgccg cgcgcgcgcc cgccgccagc agcgcgcgca ccagcgccgg cgagggcgcc 3720
   gcgcgcggac caggtgctcc acgagcaggg tggtgagcaa ggattctcga gaagtaggag 3780
   tcatgtgtga cgacaaggag agacgttata ttaggcgcgt cctacttcac tttgaagatg 3840
   gtgtaaagtg ttaaaacttg aacaccgttc actccaccac tgccgttacc gtgtcctgcc 3900
   ccaaaagcaa ccacagtgct ttttccacca cctgttccaa atccgttcca aaagctccca 3960
   tccattgttg ttagaacttt cagatgtttc tctaggttgt ttagttccac tgcaagtttc 4020
   gaccattatc gttactggac atgctgttgg taatgagttt aataaccaat cataaaaata 4080
   gttataattt gttataaagc taataaagta gcaaacactt taatgttata ttttgcctaa 4140
   ccctccgtta acaccaccat taacaccacc acttaagctt ttactaccac cactaccacc 4200
   tccaacacac attcttttct ctaaaggtcc ccaaattcca cctcctgaac ttggacgttt 4260
   tacagcacct ccgggtgtac ttgcgtaccc tttagaagtt ccactgtgac tgtagatatg 4320
   atactgtcct tctccaggca tgattaaagt gtgttgtaat tagtgttatc tacgcaactg 4380
   tgcgagactc tcgaataaaa agaagctaca ttttacaatt ttgattagct gatgtaccac 4440
   gctgtatcgc ggccaccaca agcacccgat ccagtagaac caaatccaga gtcgccgcgg 4500
   tcagtgttgt ccaagcagtt aacctcttga actgctgggc acgatatgcg ttcgcatatt 4560
   agctgagcta tcctgtctcc cttcttaacc tcaaagtcac tgtttccaaa gttaaacagc 4620
   accactccga cgttgcctcg gtagtcttcg tcgatcacgc cagcgcccac gtcgataaag 4680
   tgtttgactg caaggccaga acgtggtgct atgcgtccgt agcaaccaga agggggcttt 4740
   atcagaaggt cagtaaatac tacgcgactg caatgcgaag ggatgacaca gtcgtatgca 4800
   ctacataggt ctaatcctgc ggcaccagga gatcctctgg ctggtatagt ggcgttttgg 4860
   ctgaggcgaa caacctgaag agtttccgtg tggcagaact ccatggctag ggtggcgagc 4920
   ggccgatcga ctacggggtg tacaatttac actttctcca gaaaaatcag gggcgggtca 4980
   gcatggcgcg gcgcaagtcc agcagcgagt cgtacgacag gaagcacagg atggaggtca 5040
   cgatctccgg cggcagggcg cacgggcaca tgaggccggc gatctgctcg gccagcgaga 5100
   cgcgcagccg catcatgcag atcttgccga agagcgccgt cccgtagatg gggaactcgg 5160
   ccgcgcgctc caaaaaggcg ttctgcacga agagcgcctt cgcgtcgtcc gacgcgcgca 5220
   gcacgtccaa cagcgtcgcg tccgtgtggc agcgcaccgc gcgcatgctt gcgatctcct 5280
   gctcgcacgc gcggatcacg gtcgcgtagt ccgccagcgc gcgctccgcc agcatgcgcg 5340
   cgccctcgcc gcgcagcgcc agctcctgca cgcacagcag cgcggcctcc gagcgtcgga 5400
   acacgtggcc ccattgctcg gatgtgatca gcgcgcgcgc gagcagctcc gtcggcggcc 5460
   ggcgcgcgag cacggccgcc gtcgcgcgca cgttgttgcg gcgcagcatc tccgaaaccg 5520
   cgcataggcc cgaggccgac atgtgctcga gctccgcgcc catgcgcacc agccggcagc 5580
   aggcgccgtg gctgaacacc gccgcgcggt gcagcgcggt ctgcaggttg ttgttgcgca 5640
   ggttcaggtc cagcccgcgc tcgagcacga agtccacgac gccgcgctcg cagctcccgt 5700
   aggtcgccat gtagtgcagc atggtgttcc cacacgcgtc tacggcggcc gggtccacgc 5760
   ctaggcccgt gagcgtgcgc accatgccct cggagatctt ggccgtgcgc gcgaggtggt 5820
   gcagcgttgt gcgcccgtac gcgtccacga cgcacgcgtc cgcgcccgcg cgcagcatca 5880
   tgtccacgag cgcggcggag acgccgccgg agcacagcag cgccgccagc ggcgtcaagc 5940
   cgttgcagtc gcaggcgttt gggttcgcgc cgcgctcgag cagcagccgc agcacgtcct 6000
   cgcggatcca ctggttcttg gcgtacacgt gcagcggcgt tacgccgtag gtgttgccct 6060
   cgttcacgcg cgcgcccgcg tccagcagca gccgcgcgac ctcgagctcg gcgccgtcgg 6120
   ggccgcagaa agccaggaag gaggagagca cgctgtcgca gacaacgacg ctggcgtcgc 6180
   agaccacgtc cgcgcccgcc tccagcatga gcgcgaccac ctccggccgc acgccgtcgt 6240
   actgcacgta ggcgtgcagc ggcgtgcggc cgcaggagtc cttggctttc acgtccgcac 6300
   cggcctccag cagcacgcgc acgatctccg cgcactgctc gtgccgcgcg aagtgcacgc 6360
   agaggtgcag cggcgtgcgc ccgtgctcgc cgcggaagtt cacgtctgcg tcggtggcta 6420
   cgagcgcgcg gaccgtttcg aggtccacct gcccggactc caggtagcgg aagagcaggt 6480
   ccgcgtgcgg gaccacgacg gactcccgcg agagcatggc ggcgtttaca aatattgaaa 6540
   tcttttttca ctcatcttta tggcgctgaa cgcgcaataa gggtgagagt aaaaaacttc 6600
   tacaaaaagc gtacaaaagg tacaaaaggt aaaaaaggcg gggcggggac gggctggggt 6660
   gctgcgagct gaattggcct ctacacaggg acgccctcgc cggagccggt gagccggtag 6720
   ccggcgccgg cgatcatggt caagcgctgc acgagctcgt tgcgcttgac gccggcctct 6780
   gaaacgcaca ccatgtggtg gatgtaccgc tcgatgcact cgcagcgcgg gagagtggag 6840
   tcaagatcgg atgcgagttg cagaatgtca tcccagagct cggagaactt gctgtacagt 6900
   tctcggaggt ctctctccat tcgagccgta agagagtcag gatgcggtgt tccttcggga 6960
   gtctgagcga acaccgcgaa caggctggtt atgccgtgtt ctagaataga gtggttccgc 7020
   gttaatgccg cagacaaggg tcgtcgtccg cgcaacgact ggcggcagag cgctgtttgt 7080
   gccgcaccgc ccattcctct ggcgatcgca tccaccgacg cagtgatcat ctgcgcgccg 7140
   acgtcattgt agcgcgcgtt aaactcagta atcatgatta cgagattgca gatttcatag 7200
   tagcactttt ccaagtcgac gcgcagtttc acgatctggt tgacaatctt gcacgccttt 7260
   cgccgcgtct ccgccacgtt ggcgactcgg acttgcgctt cctggtcgat ggacggcgga 7320
   aacacttcaa acccaaggtc gcacagttca gcggtgggga ctagcgtcac gatgatgtac 7380
   tccgcatcgc cacccacttg cggcaggaag aacaccgacc gcgcggcggg aacgaccaga 7440
   acgtcgcctt cctgcatgtt ggtttttaga aacttagtgt tgttcacgga gatgccggcc 7500
   atgccctcgt tttgacacaa tattatggtg acgtacgcgg cgaccgtggg ggccatgtgg 7560
   tggcgcatgt accactcgtc gtgcttgagt ttcagaccgt gagattcgcc aacctcgaag 7620
   tgcatgttgg cgtctctgac gtagcgcgag aactcgctgc gacagattcg cgcgggcgcc 7680
   cggtggaacg tcgactcgaa gagactgatg gctgtccatt cgcccacatg agtgaccacc 7740
   gaagaagtgt tttcgatccg agtctcgaac accgagtcca cgagcaccgg acagttggtt 7800
   ccgggcaccg tcagcaccaa gggccgcgcc tccacggggg cgacggacga agccacggag 7860
   tcggtgtccc cgtacccgta gtcgtcgtcg gagtcgccgc ctccgtcggc cccgtcgcgc 7920
   ggcctccgca gcggcatgca gccggcggtg ggaacgcact ggtttcggcc acggccgaag 7980
   cggccaaaca gtctcgccag ggctgacatc cttggacggc cacaccaaaa ccaaaaaaac 8040
   atattttatc agttatttgt cgattttcac cggctcaccg agggcaggac ctcctggatc 8100
   ccggacaccc ccgccaggca gcgggccgcg cgctcgcgca cccaaaagcg gtcgtagccg 8160
   tgccggagca cgaaggccgc cgtggcgtgg cagtccacgc gctcgatgaa gccgtggacg 8220
   gcgcggcgcg cgtagctcgc cgcgaaggcg cggaccaccg ccgagcagcg ccccgagggc 8280
   gagtcgtccg tctccagcgc cagcggcatg ctcgcgatgc gcgacatcag gttggaggtc 8340
   tgcgggatgt tgagctcgcg cgtggcggtc atctgcgcct cgagcccggc cttgagcacc 8400
   tcgtcgcagc ggccccactc cagcgcgcag accacgcgga tctcgtaccc cttgagccgc 8460
   agcgcggtct cgatgtccac ggaggtgagc accgcgctga agcgcaggcg ctcttcgtcc 8520
   gcggggtcga agagcacggg gatcttaacc tccgcgctgc gcgtgacctc gcagagcgcg 8580
   atcgcgagca gcccgcgcgt gagcttgctc accatgcgcg gcttgcccac ggggtacagc 8640
   tggctcgcga cctcgcgcag cgggtacgcc agtcggaagc agcgcgcgtc cgcgggcacc 8700
   gggctcgcgc ccgtctcctc gaggaagagc gcggcctcaa ccatgttcag cgcggagaag 8760
   tgcaccgggc aggcggcgca gccgcgcgcg gcgttcgcga gcaccatctc gcgcagcccg 8820
   cggaaggccg ccatgtcgca ggaggggaag atgcgcgcga gcgcggcctg gtgcgcgagc 8880
   gccgcgtccg agagcgcctg cgcgccgcgg cggcgctcct cggcggcggc cgcgctctcg 8940
   tccgcggaga ccacgtcttc gggcacgtcc acgcagacgc cgccccagaa ctcgcagtac 9000
   tcggagaaga gcgtcgcggg cgcaaagcgc gcgaggtcca cgaaggcgac gcggttgccg 9060
   agcctggaga gcagcgtgtt ctccgagatg cgcgtccagc ccttgccggc gagctccatg 9120
   acctgccgcg tgtcgaagag ggagctgtag aagccgtaca cggtgatgtt ttccttgcac 9180
   gtcgtcagcc acatgaggaa gtcgcgcacc accagcttcg cgcagtctcc ggagaacacg 9240
   gggccggcgt tcgtcgcgat ggagttcagg cgcacggtgc cgtcactgcc gaagcggtac 9300
   acgaaccagg cggccacgct gttgccagag ggcgtgtgaa cgtgtggctg cgcccaggag 9360
   tcggcgctcg cggcggtgcg cacgtcgtgc gagagcacct cggtgtcggg gcgcgagtag 9420
   gtgctggggt ctttgatcca gatggcgtag ctgcccacgc agcacacgtt catgaggtcg 9480
   agcagcgtct gccggcgcag cggcgtgccg agccggcgca cggcgtcgtg cgagaccatg 9540
   cgcaggtcgt agaggcccac gtccgagagc cactggttga gctcgtccat ggacagggcg 9600
   tcgcgggggg gcgggctgtc ttcgaaggcg gcgcggagct cgggctccgt ctccgcgcgc 9660
   tgccgcagga tgtccaggaa ggggctggag gagtcgggga tgtagcagtc ggggtcgtgc 9720
   ctggacacta tagcgaaccg ctgcgtcgcg ggcggcgggg ctagcgcgtc ggcgcgtgcg 9780
   tcgatgaagg tgcacgatat acgcacggac ttgagcgagg ggaggacgac tgcggcggcg 9840
   cgcgcgccct ccgcgtcgaa gatcatcgtc tttccgtccc tcgcctttgc gagcgcgtat 9900
   tctccaggca cgaggtccgt cggcggcggc tcgtcccagg cctgccggtc agggacgccg 9960
   ccgcacacct ttccccagaa ccccagcatc ctccaaaata cctataagga cggccaatag 10020
   cggggcttgc gggcgttcgg accttccgcg ctttaatttt aatttattgg cttgcagaac 10080
   tccgagcgcc agtcccgctc gaagaccgcg gacaggtcct tgacgatgtc gcccttctcg 10140
   gcgttcacgc tcacgaaggc gtggtagcgg tagtgcgtgc cgtcgaggtt ggcgaccgtg 10200
   aggtgcgcga aggtgtcgtc cacgatgagc agcttagtgt tgttcgcggc gtcgtcccgg 10260
   ccgggtacca cgaacttgcg cacggacatg tccacgctgc cgacgccaaa gtcgtcgagg 10320
   ctgcgcgcgg ccgagaccga aagcgggtcc gcgttcttcc actcggtaat gatcacgcgc 10380
   acgcgcacgc cgcggttgat ggccgcgcgc agcagcgcgt caatgatctg cggccagtac 10440
   tccacggcgc tggcgtgctt gatcaccggc accatcgaga gcagcgagag gtcgatgctg 10500
   ttcttggcgt tctcgatgcg gtgcagcacg aggtcctcgt cgagcgtgcg gtagaagcct 10560
   aggaagcgct ccggcgagtc cgagaagaat acgccgcccc cggagtggtc gaggtggaag 10620
   ttcgtggccg tgggcgtgac gatggcgcag cagagccgcg tgaacggcac cttcggctcc 10680
   acgatcatgg agtagaaggt gttgtagcgg ttcatgaggt cccaggccag gtgcttgttg 10740
   gtggagtaga gcccgaggtt cttgatggtg gacacggacc cgcccgtgag cgaggcgctt 10800
   cccacgtacc agtgcccggc gtccgagagc cagaagctgc cgagaaggtt gccgacgccc 10860
   tccttggtgg acaccttgac cttgtagtag ttgacgcccg cctcgcgcag ctcgtccgcg 10920
   tccttgtcct tgctctgcac gtccacgagc agcgtgacgt ctacgccctc cttggcgagc 10980
   gtgcagagct tgtccttgac gtcgacgccc tccttggtgg agctcaggtt gcagcagaag 11040
   ctgcagatgt acaagaactt cttcgcggac tcggcgatag cggtgaagca gtcgagggtg 11100
   ctcatgttgc cctgcgccaa agacgccacc tctgcgggca gcgtctccac gacgcggcag 11160
   tcggcgccca gggggatgga ggagaacggc cacatttatt tatctcacaa aaataatagg 11220
   gcttcaggga aagtctttta gcaggcgggc gagttcttcg agttcgctta ggagttcttc 11280
   catttcttcg gaagtcagca actggagctc ggacttgatt tgaatatctt cgaggaaacc 11340
   gtctagcatg ttcgccatgt cttccgggga gcactgcgcc acatcttcgg ggacaggatc 11400
   gggtgtgggc attaggtctc cgcttacttg aacgtcgtcc atcatcctgt cgatgaggtc 11460
   ttcgacttct agacggggtc cgtagatcag catatttggt gatggaggta gtttaaggtg 11520
   cgagagttag tgttatacga ccgccaacgt gtgtttatcg cgcgtacatt ttcaataatt 11580
   aacaaactcc ccttcctgcg cctgctcgag aagcagctcg tccagctcct cctgtcggcg 11640
   cgcggccacg cgtctttccg cgaagagtac catcagctcc agccccacgc cgcacagacc 11700
   caggacgccg aacaccaccg ccgccgagat cgacagaccc agcagcaccg acatcctcac 11760
   gcgggcatcc ggctatttaa tcgttctgga aacgtattaa tatgggcgtc gtcatgtgcg 11820
   ggtgtctgtt tgtgtgggcg ggctggatcg cgcgccgcgt gcgcggcctc tgcgcggcgc 11880
   tgcgccagag ggtgtcgcgc gacaagggct acgtggccgt catccagacc tgcgacgacg 11940
   actacttcac agaggaggag ttcgacgacg gcaagcaggt ggtcgcgctc ctgcgcgacg 12000
   tctcgcgcgt ggttgccgcg cccgcgggcg tgacggaata agttaggata aggagtcgag 12060
   gggagaaaaa cagcggtcac actataaact cgcgcgaggc cgattttgac gtgctcatgt 12120
   ctggaagctc cgctttctgc agcgcggagc ggcacacgaa gcacacttcc gtgttggtgg 12180
   gagttatgca gtggacgtgg tagccgtgcc cgcacaccat gacttggaac ggacacgcgc 12240
   cgggacaggc cgcgtttatg catccttccg gcgagcgctt gttgcagatg tagcacacgt 12300
   ctgagcacgc cagcgtgcag gacacggcca gcttccactg cttaacctta acgggcatgg 12360
   ctagttgaac acgaccatgg gcgagtcgcg agcctcgagt cgggggttca gggcaaaccg 12420
   tttcacgccg tcaacggttc ttctctttgc aattttctct ctgcacaggc tcgtcagcgt 12480
   catctcggcc aggcgcgcgt cgttgcctag gtgccgcgcg gcgtcctcga ccgtcacgcc 12540
   cgtcttgccg gcctcgtcca tgagcacaat gcataccagg tgcgcgctag agcatatgac 12600
   ctcctgctcg cgcccgccgg cagcggggat ggttagctcc gcgcgcccga aggccgccag 12660
   cggcgccacg tcgtaggcag tgtctgctcg ggcgagcgcc gactccacgg caccgcggag 12720
   cgactccggc ggcgtcagcg cggccagcgg caccggcgtg ggcacggtgt acacgttcac 12780
   gggcatgagc accatctccg ggtcgtggtg gccgctctct tcgccgtcgt gctccatggg 12840
   ctgcggcggc ggcagcagcg ggagcagcag ccgtccggac atgagccggc gcacaaggtc 12900
   gttgagcgcg gacgaggcca tcggcgggta cagctccatg gccagcttca gcgataggtg 12960
   cttctcgagg ttgacgccgg tgtagacgct cttcacgatg cgcgcgaagg ccacgcgcgc 13020
   gaaggccgcc agctcctcgc gcggcaggcg ctcgatgtag gagagcagca tgtcggtgtc 13080
   gcacggcggc gccgcgacca ccgcgccgta gagcgccttg cccgagagct tttccagcgc 13140
   ccttgcgtgc aggccgtggg tcttgagcac gtccacgtag ttcacgtaca ggcagagcgc 13200
   gcgatcgagg ttgctctccg cgacgtgcgt ctcgatgcac tccacgatga gcgggcccat 13260
   gcggtccttg atgaggtcta tgagcccgcc gtaggcgact cgcgcgctca tgaggcacgt 13320
   gcggcagtac gccatcaggc cctcgaggtc cgcggctatc acgtcctcga ccacgttcgc 13380
   cacgacgcgc tgccagagcc gcaccttgct cacgttctgg tgccgcacca tgtccacgag 13440
   ctcgtcgtac gagccgccgg gctcgtgcgc gcgatcgacg atgcacctcg ccatggtgcg 13500
   gctctggcgc atgagctcgt tcgtgaagcg cacgcacgcg tcctcggaga agagcgcgct 13560
   caggcaggag tagcagcggt ccgcgacgag gtgcgggaag cggcactcca cgacgccgcg 13620
   gccgatccgc agcacgcact cgccgtacat ctcgtccatg gcctcgcgca gacagtcgtc 13680
   cagcacgtcc gcgttgtgcg cccactggat cacgcagagg tagggctcga tgttctcgcg 13740
   cgcgttttcc acctcctgca ccatgtactc gagcacggtc atgtcctcgt ggatgtcggt 13800
   gcccagcatg cgcccgggcg gcagccagct cttgcgcgcg atcgcctctc gcaggcacgc 13860
   caccgccgtg aaggtgttga cgcggagctt ggtcagcagc cgccgcagtc gggagatgtg 13920
   tgccacggag aggtccatct ccatggcctg ggcgatgagg cgcgtgagtt cctcctccat 13980
   ggcggcggct ccgcgggcag atatacgcga acaacggtaa gccgtgctat ttcatttttg 14040
   gacaaaaagc tagtcgtcga cgcgcatgtt gtcgaggttc cggcacagcg agagcacgtc 14100
   gtcgcgcgcg cgcctccggc gcagttgatt gttcgcgcgc cgcgcgtccg cgagcgcctg 14160
   tctgtacatc gcggagtccg cgtacccgtg cagcggcgag cgccgagcgc cgggcctcgg 14220
   gctcgcgcgg cgcgggagcg gcgttggcgc gcgcctcgag cgccgcgcga agtgcgcctg 14280
   catggccagc aggcaaccga acggcaccat gtatcggtcc atgaggcact ggctggccgc 14340
   ggacggctcg cgcgggtgca tcacgccgcc gcccacgtcc tccatgacgt cgcgcagcac 14400
   gcagcgcagc atggtctcca tgctgcgctg cgtgaaccgc accggggagg cgtcgacgta 14460
   gaagccgtcg gccacgaagt agagcgcgtc cagcccgccg agtttctcgc cgagaccgac 14520
   gaagagctcg tccacgtgcc agtccaccac cgaggccttg aagagcacca cgtgccggat 14580
   gtcgtgcgag cgcgcgagct cccaggtgtc ctcgccgatg ttgctggcgt cgatgcggcc 14640
   tgtcatgcgc acgctcacgc acggcgtcat cccgttcttg tagcagaact ggcgcgcgag 14700
   ctcctcctgg cgtacgatgt cgaccatgct ctccatgaag gaggtggaga gcagcatcgc 14760
   gccgcgcgcg gcgcgggtcg cgttttcgtc cacctccact tccatcccgc cgccgatcct 14820
   aatcatctat cgtatttaaa ttttcggcgg agcagacacg cggctgctcg ctgcgcgatc 14880
   gcttcagccg cggcggcgtc acgcacgcgt tgcggcggcc ggcacgcacg gacgaccgcc 14940
   ggggctgttc gctgagcgag cgccgcggcc gcgtgacgcg acagtcgcag gtgggttgcc 15000
   gggagtcgct cgcgcgcctt cttcgcattt cgccggaacg ccgcgtttat gtaggggatt 15060
   atattttcaa cgtaactaaa tggacggggg cgtgcacaaa cggcctttca tcgtgaacgt 15120
   ggatggcatg ggcaaggtgc tcgtgctccg gtacttgcgg atgtgcgagg tgcccgaggc 15180
   caagtgcgag gggtcgcgcg cgtcctgcgt gctcaagatg gaccctcccc gctcacccag 15240
   ctgcgagcgc aggccgtctc tcccgccgtc ccccccatgc cccatgcgca cgcctcccgg 15300
   gtcgccgctc caggctccct tgatgcgcac gcagatgctc caggggctgt tcgacgctgc 15360
   caaaaacaac ggcgagcaga tgtgccgccg ccagtaacct aggctgcgca gtacgaaagt 15420
   tagtgcgtga tcacgttttt tgcaatgtcg atcacgccgt gcgtgcccgt cttgcgctcg 15480
   cgctccacca cgctagtcac gggccgcgcg tccgagacta gcgaccccag catcgagcgc 15540
   acggcgccct ccgcggcggg gtggcgcgtc agcagcagga acatcacgat gtgcgcggag 15600
   acgccgcgcc ggctcagatc gtgcacggcg tcgccgtcca tgagcacggt gttcgagaag 15660
   tacgtgaaca gagtgttgtc tcgcaccagg aaggccgagt tcgagacgct ctcgaagtcc 15720
   acgatctcgt cgtcctgcac gcccatgtcc aacagcgtct gcacgagcgc gggctcgtcc 15780
   aggaacacca ctgcgcgcgc gaacccgcag tccagcgcgc gcgcgtccgc ctccaacacg 15840
   cgcgaggcgc cgccctccgg cggcaggaag gcgcagggca gcggcgtgcg tccgtccgcc 15900
   ggcgcctccc cgagctcctc gagcgcgaag gccagcagcg tctccatgcg cgcgcgcgcc 15960
   ttgtcgaagt tgtccgcgag gtcgcggatg cggtctgtct gcgagaacat cttcagcatc 16020
   gccatgagct gcacgaaggg gtgcagcacg tatatgttgt ccacgagcag cgtgggcagc 16080
   gcgcgcagcg tggcctgccg cacgttgaag ctgtccagga tgtgcccgcc ctcctcgtcc 16140
   tgcagcacca cgtagttctt caggtagggc acgcgcagca gcacggtctg ccgccccgtg 16200
   acgaagtata tcaggaaggc gaggttgatc aggaacgggc gcgcgttcgt ctgcaccatg 16260
   tcgatgtcgc cgtactctat ctcggggttc agcaggtgca gggcgtacga gccgtagcac 16320
   acgcaccgct tgttgtgtcg gcgcaggtgc tccttcacga gccgcttgac cacctccacc 16380
   aggtccgagt gcttgtgccg cgccatcggc gcggcctcct cgggcggcgg cagcactgcg 16440
   tacgagttga gcgcgcggct ggcgagcgcg cgcgcgcggg gcgcgtccac gcgccgcacc 16500
   gccgcgttga ttgcaggcgt cggcgtcgtg agagagccca gcgtgcgcgt gaactcgctc 16560
   acgatcacgc tctgcagctc cagcaccgtc aggatctggc ccagcttctc caggcgccgc 16620
   tgcctcgaga agtactcctc gatgcgcgcg gcgatctcct tctcggagcc gcctagcttc 16680
   ttgaagaagc gacgacgact ctttacaaca agagagagaa aaagcttcct atcgaagttg 16740
   aggacgcggg tcatgttgcg gcgctgcgcg cgcaggagca cgcagcgctc catggagggg 16800
   cgcgagccga ggtactcttc gatcacgggt ggagccatga cagctatttt ctgaacccgc 16860
   gattattgta cagcgcaagc cgcgcgcaga cctgctggca cagcagcgtc gtgtttcgca 16920
   tgcacacgcg cgaggactcg atcgtgcgcg cgtccggtgc ccaggcgcgc agctccatca 16980
   gttcctgctc gacgaagtcc acgggctcca cgaagcgctc tgcgcagagt ccgtccgtga 17040
   acgcgttgac gatctgccgc acgagcacta ccacgtccac ctgctccacg aggcgcacgc 17100
   ccatggcgac gtgcacgaag aggcagcgga agagcgcgtc catggccatc tggtggtccg 17160
   agcagggccc gaccgcggtc ttgcagccca gcgcgaagcg cccgatgccg cggtactgca 17220
   ccatctccga gggcgagaag gagagccgct ccatcttgag cacgggcggc gggcccgccg 17280
   gcagtccgcg cgcgaggtcc agcaccggcg tccacccggg cgtgaacatg tccgggatca 17340
   ggaagagccc gtagctggcc atgcgcgcga tgtcgaaggc gtggtccacg accttgttca 17400
   cggcgctgtc cgcgcggttt acgcgcagcg cctgcaggat cacgtttccg gaagcgtgcc 17460
   gcgtgatcgc gaggtccgcg gtcgcgtacc cgcgcaggcc cggcaccgcg tacgcggtca 17520
   gacacacggc caggcgcgcg ctgtgctccg aggagaagat ctcctgctcg tagccctcct 17580
   cgggctcctc gcactcgcga gggcgccgca cgtcctcgac cgagcgcagc cgcatctctc 17640
   cctccgacac cagacagcca agcgactccc tcaccgccgg cgcgagcacc tccgtggcgc 17700
   agagcgcgtc gtgcacgcgc ttgagtgtgt tcggcttcag cgcgtagccg aagagcagcc 17760
   gcgtcatccg cgagcccgag aacgcgaagc ggcgcacgta ctcctccgcg agctcggggc 17820
   ggtcgttgat ccacgaggta gagaagacgt ggtcggaggc gaaggggtct gcaccaaccg 17880
   cgagcagtgt ggacagaggt acggtgtcga ggaagtccac gacgtcgggg aagttctggc 17940
   gcacgcaggc ctcggcgacg cgtctggtgt gcacgcacat gtcggtgacg ggcacccggt 18000
   ggccggactc cacgacggac acgcagacgt cctcggtgac ggcgtccacg ggcatggtcc 18060
   gcagcagctt gccgagcacg tcgccgaacc cgccctcgag cgccttgcgc cacacgaacc 18120
   ccgcgtcgaa cttcccgggg aagtccgcga tcaccgaaag ctccgcgtgc gagaggttgt 18180
   ccacgttgag gtaggtggcg gcgtccacga agatgggccc gaaggcgccg gtgtcggaga 18240
   cgcggtctct gaggtagtcc ctggcgtagt ggaggtactc ccgcacctgg ccggcgcgga 18300
   tgcgctcgag cgcgaaggcc ttcatggtct cggagcagag cacggagtgc cgcagcccgt 18360
   ccagtgtgcg ccgcacgtcg tagacgccgc gcatctgcgc gagcatctcg acggcgtccg 18420
   ccggcgtcgc cgccgcgagc cccgggttca ctggaggtat cctgtgttct gcgagcatgc 18480
   gcttgaggaa acagaggtcc agcggccgcg tggtgtacag cgcggaggcc atctcggggc 18540
   gcgtctcgac gatgtcctcg atcatctcgt ccgtgaatgc cgcgttgatg ttgtgcacgc 18600
   tgcgcgcgtt cacgtgcagg aggatgtcgc ccacgttgtc ggggaatcgc tcctcgatga 18660
   gccggacgtc gtcctccgtg atgttcatgt agggaataca gcggcagagc agcgcgtagt 18720
   ccgcgaactg cgcgatgtag ggcgtgtgga actcgatgtg tctggcgaag agcgcgccgc 18780
   agcgccgccg cgagagctct tcgagcaggt cctcgggcgt gacgtgctgc gggcggaaga 18840
   ggtgcaggtg cgtggggtgc tcggcggcca cgcgcgcgta cagccgccgc gggaggtgtc 18900
   tggggtgcac gccggcgagc acgagatcca tggcctctga ggtagacagt gcggcgaacg 18960
   cgcgctcggt gccgcccgcg gcgacagcgg cgccgacaaa tctcttgagc agctgcagca 19020
   tcgcgtgttt gggctttcgc ggaaggcgct tattttaatg ttattggcgg tggccggtgc 19080
   gagataaaaa ttagaactga tgccgcagtt gttgatgata tgatgattgc gctggccggc 19140
   gcgagataaa aattagaagc tgatgccgca gttgttgatg aggatggtga gtgcgctgga 19200
   gcaagcggtg tggcgcgcta gcttcttgct ggccccgtcg gccacggcaa cgacctttcc 19260
   ggatatcgtg atggtgcagg tgaagcgcgg acagtgatcc tctccgccag cacgcgtctc 19320
   gcagaactcc agaggtctgt gtgtcatcat gcagaactcg ttgaccgcgc tgaccgggtt 19380
   aagacttttg aggcgtatca cggcagactg agtcatgatg tcgatgtcgc cgccgaaaag 19440
   cgtatcgcac ccagcctcgg tctccatggg ctcggtgtcg gagttttcgt cctcctcggt 19500
   gggcgcggag ggcgcgcact ctacgaacca gcggggcggg tttccgtcct cacagcaaac 19560
   ctcgtccgag tccagcaggc ggtacagctg gcggtttgcc tcgtgtttgg atatgccgag 19620
   ctccttcgcg atttgcttgg ccggcagctt gtcgtcggat tttctgagaa gctcgaggat 19680
   cagagacgcg cactcgcagg ccattgtggc gatttacggg gcgtgcgttt ttttaggatt 19740
   ttggcttgcc tttcttttcg cagaacttgg gaggattgaa actcttttgg caatttttgc 19800
   aggcgtactt gatcaagggc ggctcgtccg ccgagcgcgt ctggatcatc atcggcatgg 19860
   tgttcttgct ctggcacgag gggcagggca ggttgaactt ctcgtcgagc acgttgaagt 19920
   acccgctgta gtcgtggtcc ggcacctcct cgatgtcgta gggcacgcgc gcggccacgc 19980
   acttgaccgc gaagagcagg taccgcagcg cgtcgtgctc cgcgccgctg gtcgcgcgga 20040
   tctgcgcgca caggtccgcg tagtcctcgt tcgcgtccac ctccaggctg cgcttgttct 20100
   tgtacgagag ccggttcttg gcgtccttcg agtactcgat gccgatgttg tgcgcggggt 20160
   caaagttggt ctcgtcggtg ttcgaggtct tggtgttcac gatgttcttc agcgcgaagc 20220
   gctgcgcgca gtccagcgcc catcgcgcga tgcgcgcggc ctccgccgcg tcggtgtgct 20280
   tcgccgcgag gtcgcgcagc cggtcttcgt ccatcgcccg attttaggtt gggtatatta 20340
   tctcaattcc gctcttccgc gggccgcggg cgcgcgcccg cggcaaatta ggcgttacaa 20400
   atggacttcg tgcggcggaa gtacatgata cacgccatcg accgcaacct cgacttcatg 20460
   aaggccgagg tccagcagaa ggtctccatc ttctccctcg ggcacgtgct cgcgctccac 20520
   tacctggtca ccgcctttcc gcaggcggtc atcaccaagg acgtgctcgc gagcacaaac 20580
   ttcttcgtgt tcgtgcacat gtcgcagcgg cacgaggtct tcgacgccgt gctcaaggcg 20640
   gccttcgacg cgcctcagct ctttgtgcgg gcgctctcgc ggcacttcga ggccttcgtt 20700
   gccgccatcc gggcctaccg cgcgacctgc gcggagctgc tggccgacgc gcgcttcatg 20760
   gaggtggctg cgcgcgcggc cgagctcgcg gaggtcattg gcgtgaacca cgacatcgcc 20820
   gcgaacccgc tcttcgcgga cggcgagccc gtgcgcgacg cggagctcat tttcgcaaag 20880
   accttccgca agaccgagtt ccgcgccgtc aagcgcctcg ccgtgctgcg gctgctggtc 20940
   tgggccttcc tcgtgaagaa ggaccttggc ggcgagtacg cggacaacga ccgccaggac 21000
   ctgtttacgc tgctgcagaa ggccgcgggg cccgtgcgcc acagcgcgct cacagagagc 21060
   atccgcgagt acctcttccc cggagacagg cccagccact gggtctggct gaacgcgcgc 21120
   gtggccgacg acgcagaggt gtaccgcgac cggcccgcgc gcacgctcta cgagcgcgtg 21180
   ctcagctacg cgtactcaga ggtcaagcag gggcgcgtga acgccaacac gctcaagctc 21240
   gtgtaccggc tcgaggacga ccccgacatc aagggtctgc tgctgcagct catctacgac 21300
   gtgcccgcgg acatcgtcgg cgtcgtggac tccgcgaacg aggagtggcg gagctacttc 21360
   gtgagtctgt accgcgagaa cttcgtcgac ggacgcacct tcacctcgga cgcgcgcttc 21420
   cgcgacgacc tcttccgcgt ggtcgccgcc gtcgatcccg acttcttcga gcccgagcgc 21480
   atccgcgagg ccttcagcgc agacgcgcgg ctgcgagagc gcttcacgga catggacctc 21540
   aacaacgcct tcatgtcgca cctcatctac gactccgtgg accccgacgt cgccgccgcc 21600
   gagcgcgggc tcgcactgcg cgtgcacaac gaggactccg actacttcat ccgggagtac 21660
   aacacctacc tcttcctcag cgagaaggac ccgctggtgc tggaccgcgg ggcgctcacg 21720
   cggctctcgg acgtccccgc cgagcgcttc cgcgacctct tcagcgacag tgtgctgcgg 21780
   tacttcctgg acgcgaagct gggcacgctc gggctggtgc tcgaggacta ccgcgaggac 21840
   gtggtcgccg ccatgcttcg gcacctgcgc cgcgtcgagg acgtgtcttc cttcgtgacg 21900
   tacgccgcgc gcaagaaccc cgcctgcgtt cccggcgtcg tgcgcgcggt cgtgagcaac 21960
   ttcaaccccg cggtggtcgc ggccatgcgc cccttcctgc gcgagcacat gacgcgcgtg 22020
   gacgcgctgc tggacggaat gccgcacctc tcggaggccg accgtcggta catccgccgc 22080
   gtggtgctgc agggccgcgc ctgattcgcc gtcaataaat cgcgatggtg gacagcggca 22140
   cgcacgacgt ggactccgcc gcgcaggagc gcacgcccaa ccagcagacc ttcttcacca 22200
   aggggctcag tccgctgatg cgccacacct acatctacaa caactacgcc tacggctgga 22260
   ttcccgagac cgcgctctgg agcagccgtc tgggcgacta ccgcgtcacg gacttctacc 22320
   cgatctcgct gggcatgctc aagaagttcg agttcatgtt ctcgctgctg gcggaccccg 22380
   gcggcgcctg ccccgcgtac gagcccaagc tcaacaccga gttcctgaac cgcggctcct 22440
   tctcgggacg gtacgtgaac cccttccacc gcttcgcggc gctgcccgag cgcgagtaca 22500
   tatccttcct gctgctgagc tcggtgccca tcttcaacat cctcttctgg tttaagggcg 22560
   agaccttcga cactgccaag cacagcctgc tcggcgccgt gtacaccacg cccgagcggc 22620
   acatcgagct cgcgcggtac ctgcggcgca cgggcgacta caagccgctg ttcagccgcc 22680
   tgggcaacga cgacacctac tcgaagccct tctctgggtt cacgcgcatc agcaacccca 22740
   cgcccatcgg gcggctgccg ccctcggact tcgagacgct ggccaacctg agcaccattc 22800
   tctactacac gcgctacgac ccggtgctct gtttcctggt cttctacgtg ccggggctct 22860
   ccgcgaccac gaagatcacg cccggcgtgg agttcctcat ggagaagctc tcgctcgcgc 22920
   ccgagaacgt ggtgctgctg tagcctcaaa cataaaatat aggcgccttt gatcgcactg 22980
   cttcagttca gacagagcta agatggcttc ctacatcagc ggcgctagcg ccagcgcgaa 23040
   caccgcccag ggcggcgatt ctcagtaccc acagtactat tatcacacac gcacctccca 23100
   aggcgacatc cgcgacgaaa gcgaaggttg cttccacacc acggacgacg agcacttgga 23160
   tctgtccgac gactacctcg gcgatggcgc accacactgc ggacacagcc acaaccacag 23220
   tcgcagagat ggagatcggc accgccagcg cgcaccgcgg ctctatgagg acccggtgcc 23280
   cgcgaacatc atggtgccca cgctcagtct agagcagctg ctggaggaaa cctcggtcgc 23340
   ggggggcctt ctcggcggca ggacagagag ggacgtggaa cagctcctgg aggagttctc 23400
   cgcactctgt cccggggacc agatcaccgc gctgcgctgc atggcggcct ccttttaccg 23460
   cgacgcgctg ttcgcgccgt acgcctgcat gcacctcatc gccagtcgga tgcgcgtgca 23520
   ctacgcgcgc gaggtcgtgc acgtggccga ggacctcgcg gacgcgatgt ctgcgaacag 23580
   cggcgtctgc ttccggcggt accgaaagcg cgtgctagag gacatgctcg cggaggagat 23640
   gggcgtgtac aattacctcg cgcgcgccaa cgcggacatc tgcgaggaca acctgctatc 23700
   ggccgtggag acgctgctgc ggcgcttccg tcggatgggc tgctaccgct ctctgtgcat 23760
   gctcaagatc ctcgcgctgc agcacgagga cctggccggc ttcatccgcc gcagcataag 23820
   aaaaacctgc aacttggcac acgcgcgcac gcacacggtt tacgtgtagt taccctgtaa 23880
   agacgggctt gctcccgaac aagcgctcga agaagagcgt gcacatagcc ttattgtcca 23940
   gcaagttgac tatctctgta cacagcctct tgaagtacac ctcgtacatg atccgctcgt 24000
   ttttatccag tctgaaggtc ttgtcgacca cgcgctcgta ggacttcacg ttcgcgatcc 24060
   ggcgccgcca ggggccctcc tcgcacacgt acgcgaagaa gtagcgctcg ccgatctcga 24120
   tggcctccgc gttcgccgcg ttgtaccgcg tcactagcgc cacgttgggg ttgtcggggg 24180
   acttgaagtt cttgtggtgc gttcggctca gcaggaacca gtccagcggc atgctgcgcg 24240
   cctcgaactc gaaggtgagc tcgtcctcca gcgagcgcag gatctccacg cccacgttcc 24300
   cggagccctc ctccgccagc gcgcggcaga gcatgtcctt gtacttgcgg atcatgagct 24360
   tgtggaaggg cgccacgtcg cggcgcgtct cgctggtgcc cttgctcacg cgctcgctgc 24420
   cgccgccgtc gctcaccgca aacttgatcg tggtgtactt cttcttggac tgcatgatca 24480
   ggttgcagta caccgcttcg aactccacct tgaagttcgc gaagagcacg tgctcgttga 24540
   tcacgcgctc cagacagcgc cccacgcgcc gcgagaacgc gatgtcggag gcgcccacct 24600
   ccaggaacac ggagtcggtg tcgccgtaca cgctgcggaa gcccacgcgc tccgtgcgct 24660
   cgccggccac cgccgcgtcg atctctagct ccgcggcgcg gcccgcgaag gcctcgtcgc 24720
   gcagcagcgg gttgtccggc gccgccgcca gcgacagccg cgtgccgcac accgacgcgc 24780
   cgtctagcgt gcgctccagg tacgcgatca tggtgcgccc gatggccgtg cagctcttgg 24840
   ccgaggcgta cgagaagagc gcgctgttgc ggaagcccat gagcccgtac acggagttgg 24900
   ccgtgatctt gtacgtgtac tgcatcgagt tgtagatctc gcggtccacc gcggtctccg 24960
   cggccttcat cagcttcttg tacttggcgc gcgcgtccag gaaggagcgc agcagcatcg 25020
   ggatgatgcc cttggcctcg cggtcgaaga tggccacctc ggcgacgagc tccggcgagc 25080
   gcggctcgca gggcaccgcg atgtaccgcg gcgccgggaa catccgccgg acgtccacgg 25140
   ccgcgacctc cgcgtcgagc cggttgtccg agacgaccac gccgacaagc gtctccggcg 25200
   acaggttcgc gtagatgcac acgttcgggt acaggctgtt gtagtcgaag atgagcacgt 25260
   gcttgttgtg catcttctgc ttgggcgcca tcacgcggcc gccctcgtag aagaacttgg 25320
   acttcgtgtc cgcgcgcacc atcaccgtgc ggttctccag cagcagcttc atcagcgggc 25380
   ccttgatgca ggtgctcgcg cggtactcga agaccacgct ctgcggcagc aggtacgtgg 25440
   acgcggcggc cgcgatcttg gtctccacgc cgtagtgcga ccagaggtag aggcagaggc 25500
   aggcgtcgtg caggcagtac cgcgccatgt ccagacacac gtccagcgag tagttcgcgt 25560
   acatgtccgc gaggctgacg tcgtccttgc cgaaggccag cgtgacgcgg tcgccgggcg 25620
   cgcgcgccgc ggggtccgcg aggtccacgg tgaagccgtc ctcgtcgacg cgtttgtgca 25680
   gcacgcggca cacgcgctcg tccacggtca cgtagttgcc ggtgctgagc acgcgagcga 25740
   acacggccgc gttcccgtcg gcgtcggtgc tgcggtcgcc gcgaaagcgg accgcgtccg 25800
   gccgcgcgtc ctccacgacc gcggtgcagt ggaaggcgtt cttggatatg gcgtccagct 25860
   tgtaggagtc cagcttctcg gtgcgctgga tgaaggcgta caggtcgaag tagatggtcc 25920
   cgttgttgtt gttgatgtgg aaggtggtgc tcgagacgcc gccgacgccc ttgtggctgg 25980
   acttcgtgcg ctcgtacacg cagaagttga ctgtctcggt cccgtccggc agccggaagc 26040
   ggatgtgctc gcccgtgagc agcgacagcc gcgagtccag gtaccgcagg tcgaagttgt 26100
   ggccgttgaa ggtgaccacg aagtccagcg gcatctcgag caggcgcttg gccacgcgca 26160
   gcagcgtcac ctcgggacac agcgtgacct ccgcgtcgaa cttcacgtcc gccgggtcca 26220
   ggcagaccgg gatctcgcgc cgtgccgcct cctcgaggtc cgcgtcggag agcatatcgg 26280
   agttcgtaag cgtgaatcgc cgctccgcgc cgtccttgtc caccacgcag aagctgatgt 26340
   gcgagacggc gttcttgaag acggaaggaa actttttctc gaagtggcac tctatgtcga 26400
   ggaagagccc cgagcgcgtc acgttgaagc gcgggatctt ctccgcgaag cacgcgccgg 26460
   ggtcgtcgca gtggaagcag ttgctcccca ggtcgcgcag cagcgcgggg tccacgcggt 26520
   agcacccgtc agggtcgatg tcgtgcgcca cgaagaacca ggacacgttc agaaagtcgg 26580
   acatgaagac ctctggcggc gcgagcttgc gctcgctggc caccagacac agctctatct 26640
   ccgagcgctg gcgctccgga atcttcgccg agcgcgctac gatctcgtcg atgctgacca 26700
   cggacatggg cccgagcgcg cgcgtccacg ccagcggctg ggcgatgtcg gccaccgcgt 26760
   ccgcgcgcac cacgtagtaa aagtgctgca cgaagcgcag gtacacgacg gcgttgtcgg 26820
   cgcggcgcgc cttgaggaag aggaaccggc tgtcattgcc gcggttctcg aaccagttca 26880
   aacatttcag ctccatttca aagagcataa taacatttca tttaaatgga gcctcgcttc 26940
   tggggccgcg ccatgtgggc ggtgatcttc atcgtgctgc ggcgcttcga ggagcaccgc 27000
   gacctcgagc gctgcaagcg gcagctgtac gtgatctgct ccacgctgcc ctgcatcgcg 27060
   tgccgacgac acgccaccgc cgccatcgag aaaaacaacg tcctctccag cgaggacccc 27120
   aactacgtgc tcttcttctt catcaagctc ttcaacaacc tcgccttcga cgacagatac 27180
   aagatcgacc ccgcgaaggt gcgcccgctc gtctagagca tgccctcgta cgcgcgcgag 27240
   ttgtccgagt acacggtcac cgcgatgccc tcgcgcggcg tcacgtgcac gtgcatggag 27300
   tccgtgatca cgaagcccac gcccgtgacc ggctcctcgc ccgcgtcgtc cgtgaagagc 27360
   agcccgtggt tgaagaggta gaactcgttc tctgcgagcg aaagccgccc gcggtcgcag 27420
   aggtagtaga agatgtcgta gtcgcgcacg gccagcgtga acgtggactc gctcaccacg 27480
   atgtacttgt ccagctcctc cagcacggac gcggccgcgc ggcgcgcggt ggcgcggcac 27540
   tgcgtcgggc actcgcacgt gtctgcagag tacaggatgc gcgttcgccc cgaggcggtc 27600
   tcgagaaaaa cgttaatcgc ctccatcgcc cagaagcgac tcgaggatcg cgagcaccgt 27660
   gcgcagcacg agcccgatgg tgcagaaggg aacctctccc gactccgagc actcgcggat 27720
   ctccgtctcc acgcggtcgt gcacttttat ggagggagcc gtcgttccag tggcctccat 27780
   cgcgacggac accaccttgg ccacgaactc gcggatcttg ctcatgcgcc ggagcacggt 27840
   cacgcggaag aagacggccg cgagcaggta ctcggccacg cagctcacgg cgatgagcgc 27900
   ctgcgcgtgc ctggtgttga gcacgtgcgc gtcgggcacg aagtccgaga tcttcaggtg 27960
   cgagagccgc acgagcgcgt tggtgtcctt gacgccgtcg caggaaatgt tcgcgaagat 28020
   gagcttctcg tagtcgagcg cctcgaccac gcgctgcgcg tccatgtgcc gctcgcccga 28080
   gagcgcgcgg ctcaaagggc cccagcacac cgagcccgcg aagcaggggt ccaccacgcc 28140
   gtgcatcgcc agcagcgtca cgtcggccac cgccgcgagg atggccgcgt cgtcgagctc 28200
   gttcgcgggc gtcgcgcccg tcagggacgc gctgcgcagc gcggacccgc ccggcgccgc 28260
   gtgccgcgcg cagaactcgc acccgcagcc cggcggcagc ggcggcttcg agagcagact 28320
   tatgagcccg cccacgtgcc cgtgctcggt gatcagaagc gccaggatgc tgtcggtgct 28380
   gccctctatg gcggctgtgg ccgcgctaga aggctctccc gtgacctgcc atccgcagac 28440
   aaccttgagc atcttgcgct tgagctcgtt gggcgcctcc gaggccagca tcgtgcgcgg 28500
   gaacgtcgcg ttgaggcgga agtcctgcag cagcttttcg agcgtcgcgg tcttggcgtg 28560
   ccgccctttg cgccactcct cccccaggtg ccacatgagc tcctcggccg tgtccagcgt 28620
   cggcgagacg cgggccttgg gcacgcgcgc cgcgctgcgc atcagcggct ccgaggcgcg 28680
   gaagctgccg cgcgcgtgca ggcgcatgga cgcagacgag gaccgcatcg agggtctctg 28740
   gtggaagctc gtcatcgtga agcgccgcgt gagcggacct acctcgtcgc gcgactcgtc 28800
   gaagtccggg tcagggtccg tcgtgtcggt ctcggcgctg tgcgtgctgg gcgcgctgct 28860
   gcgggcgctg cgcgtgctgc gcgtgctgcg ggtgctctgc gtgctgaagc tgcgcgagca 28920
   ggagtccgcc gtgtccgctt cctcgtagtg gaagtccacg tgctcctccg gcagccggcg 28980
   cgagcgcgac ttggagatgc cgaccatctt gtccgcgccg accgggcgca cgcagagcgc 29040
   catcgcgccc tcgcgcaccg cctgcgcgaa ctcgcggctg gcaaccatgc tggggttgag 29100
   gaacttgatg atgttgaagt atggccactc gcagacgagc cgcgcgcagg tcgcgacgtc 29160
   gtcgacgctt ctgagggccc tgttcagcgg ggggatgttg ctgccgtcgg ccagtgcgac 29220
   gaggtcctcg ggggagatct cgagcttggg aatcatctcg ttcacgagcg cggggtcgat 29280
   ggcgtggcag acggtctcta cctcggtgcg cgagagcttg agctgcgcgc aggccgccca 29340
   cggcgcgcac gtgagcgcga acaccgcgga gactcggccc ttgccgacca tcgccacaac 29400
   ctcgggctcg tagaccaggc cggccttgag cagagcctcg agcacctcta tgtcgtcggc 29460
   ggcgagcagc gcgcgcctgt ggaagcacac cgcgggcatc atcttcttgc agatgccgcg 29520
   ggtgctcttg agggccgtga taaggtcggg gagcctgacg tgctgcggct ggaagaacat 29580
   gacgttggcg gggctcgcgg ccaccgcctc gtcgtacacc gacatcggca ggtcgccgtg 29640
   gagcccgcac ggcagcaggc tcagcagctg cgcgcgcgag agcttctcgg cgcagaagtt 29700
   cttcttggcc agggcggccg ccgcgtcgcg ggccttcttg gggtataaca acatggcggg 29760
   ctttaaacac gaaacaaaaa tccgggttgt aacatttcaa ttttgcatgt tctgggcctc 29820
   ctcgcagagt ttctccaggc cgccggccac gatggcgtcg acgaagaggt cggcctcggt 29880
   gaagcggtgg ttgccgcgca ccgccaccag cgcgttctcg gtgaccacca ccgacagctg 29940
   ctgcgcagcc gtgcgcaggt cgaagtgtcg gcacgcgagc ccgtggccca gagtctggtc 30000
   cagcgccgcg agcacctcct ccaggctctc ctcgcggtgg ttgtagaacc acatcagcac 30060
   gaagtaggcc acgtaggtgt agaggtagtg cgcgaccgcg cgggcgcgca ccagcggctg 30120
   gttgcagcgc gcgaaggcca ttccgctggc gatgaggtcg tcgtccagcg tggcgtagtc 30180
   gccccagttg agcgcgctga actgcacgct gtagaccgcg cgcacgaacc cggactcgag 30240
   gatgtcgatc tgcgacggcg cgccccaggt caccagccgg tacacgatgc cgcgctgcat 30300
   cagccgcatg aggtcgccgc cgacctcgcg caggcggtgc gtcagcgagg cgaaggccag 30360
   cttccgctgc gtgtactgca ggcccacggc cacgcacccg tccgactcca ccagcacgag 30420
   cttgtggtcc gtgcagccgt cgctgcgcag gccgccctcg cgcgccatca tgtcggtgac 30480
   gaacatgtac ttgcgcgcgc gcggcccgac caggatgcgc ttcttgcctt ccatgcgcag 30540
   caccacgtcc tctagcagcc gcgtgctgtc cacgcgctcc acctgcgggt ggatggtggg 30600
   ctggtagttg tacagcagcc gcggggacca gttgtaggcg aacgcgaaga ggtgcgtgtc 30660
   caggagagag atggggaaga cgccgccttc ggtggcgcgc cggaggatgg cgagcttggt 30720
   ctccgcgggc agcaggctcc cggtcaccgc gccgaagaac atggggtggt tgcggaactt 30780
   gagcgcgtac gcgctcagca cctcctctcg cgagaatatc gagtccgcgg ggttggagct 30840
   cgcgggcagg agcacgtcct ccacgctcag cacctcgtcg atgaagggca gcaccatgtc 30900
   cacgttggag gcggtgaacc actccatgtc cacggcgttg cgctccacga tcacccggat 30960
   cgtctcctcg gatatgttct cggcgaaggc gctctgcagc tcgatcaggt tctcggtggc 31020
   gtcgatgctg agcccgaggc gcatgccctg ttgcagcacg tcgttgatgg ggttcacgta 31080
   catggccacc gccatgcacc cggccggctg cacgcgccag aggttggagt acgcggacac 31140
   gtccgtgatg cgcagcgtct ccagcacgtt gtacatcgcc gcgcgcattt ccagcgtgtc 31200
   cacgtacacc tccgcgtgct cgaagatgat gtcgagctcg aacgcggaca gcggcagact 31260
   cacgtagatc tggcaggcgt caaagagttc ctgcgcgtac tcagagaagc acgcgtacgc 31320
   gatcacgccc gcgcggttga cgatgtagtc cgccttgaac atcttcgtga ggtaggcgta 31380
   cagcgaccgg caggccacgt gcggccttag ctcgtcgagc acggcgtcca gcacctcgcg 31440
   gtgctgcagc acggaggggt gcaggaactg cgtgaagtcc accgcggtct catccatgaa 31500
   gtcggggatg gtctcgacca ccaagcggtg gttccgcacc gcgcggaagc cggtgttcat 31560
   cggcgcgatg ctgtgctggt cgtggacctc cagcaggatc tcgtagccga tctctcggca 31620
   gctcagcgcc gcgcgcgcct ccgtcacgct cgcgttccgg aagaactgcc gcaggtgctc 31680
   gtccgtgcgg cagagcgtga cggattgggg gatgcgggaa aggtcgcaaa gagggctgtg 31740
   gacggagatg cgccgcagcg cgtggtctac gaactcaaaa ccgcgcctcg acatcgtgaa 31800
   gtcgcggagg gcgtacatgt tgtaggaaca gaggcggaag aggcagtcta tgtctagcat 31860
   gttggaaacg cagtacgcgt gtctgtggag gtgcgggagc atggcgggca cgacctcggt 31920
   cgtgttctgg agcatggtgc atacgaggtc gggcgctgtg aggtcggggg tgacgatgag 31980
   gatgcaggag ctggagaact tgctgagctc ggaggccagc cggtgaaggt tgtagtcgtg 32040
   gcgctggctg aagttgctgt ttatcgtgcc cgtgaagccc atgagcgccg ccagcgtcag 32100
   gtcctcgcgc tcgatggccc agatgtctag gccggaggct atcatgcagc gcaccagcgc 32160
   ggcggcgcgg tcgctcgtgg ggtagctcat ggtgctgtcg gtcgtgcgaa tcagcatggg 32220
   ataatgcttc atttttacgg tcgggggttg cggactgtgg ggcgcacagg gcctgcgggc 32280
   ggctcgtgcc ggtccgcggc gttcgccgaa cgcaggaacg ggcccatgcg cgcccaggcc 32340
   atccacagcc ccgccgtcag cgccagcagc cagacgaata ccacgatcat cttttatgta 32400
   gctggaactc gcgctcactc cccgccgcac ggcgacgggg agagcccaga gccgagctcc 32460
   atgcgcgtgc tctgcacggt gagcgactcc acgagcttgg acacgttcat gcgcgtgttg 32520
   tcgggcacca ggtgcgcgag gcgcgcgtac acgtcatcgt gcatgcgctt gctgcagcgg 32580
   tccaggctcg cggagagcgc ggagactagc gcggtgtgct tcgcgtacac gaagtcgccg 32640
   agacacacgg tctcgagccc gagcacgtcg gcgctctccg cgtccttgag cgccacgaat 32700
   atcttctgct cctcgcgcgt catcgagcgc atgaggtagt cgtgcagccg cgagcgcgag 32760
   atgagcccct gagagatctg cgggctgcgc atgaagcgcc ggcgcatcgc gcacagcagc 32820
   tcctcgtcga cgacgtacat gctgtccttg atggagctct tctcgtcgat cacgagcaga 32880
   ccgtcgttgg cgaccacgtt gatgaaatcg tccacgtgcc gcgcgtctat gtcgtagcgc 32940
   gtgccgccgc actcgatgtg cgagggcggc gacttgagcc ggttcgcgag cgccttcacg 33000
   tcggagacgt cgatgtacag cgaggactcg cgcgggacgc agccgaggat gcgcgtctcg 33060
   agcggcgtga ggatgagcac gcgctcggcg ccgtcgacga gcttgccgtc gtcgggggaa 33120
   aagaagttgt tctccacgat gctcgagacg aggctggcga gcacgccgtc gcggtaggcg 33180
   ccgagcgcaa actcctgcac aaagggcgcg tgcagcaagt ccacgggaat gcgcatctcc 33240
   acgcggcgcg cgaccgactt cttcttctgc aggtgccgcc ggtccaccat ctcgtccacg 33300
   atgtccgata tgcgggagca gaggtacgcc ttgaggacgt tggcgtttac cttgttgaag 33360
   atgagccgtt cgtcctccat ttaagctgct cagacgagct ttaaatagtg gaaacacagc 33420
   agcacgccga tcgccgccgc tatcaggccg attagaaaaa cggtggtcca ggggacgccc 33480
   ttgggcctat cgcacgccgg cttttcggtc attacggtgc gcacgatgtt taggaactcc 33540
   tcgaagtcct cgtccgagtt ggagaggaag gagccgaaga cgccggtgta cagtttgtcc 33600
   atttactact agatattaaa cggcgcttcc aactcctcgt cctcgaagcc cgcgccaggc 33660
   tcgacgacgc ccaggccgcg cacgtcctgc tcctcgttga acgtggtctg agtctcgctc 33720
   atgcgcacac acgtctgctc gccctcgaga ccgagcacgg tcagcgagca ctcgcgcggc 33780
   atggtgatct tcttaaccgc gaaggtgact ttgccctcgc cgcccgagcg gtagaacacc 33840
   accggcgcca ggatgagcgt cgccatctgc gcgtcgcggg ttgcgaggtt ttctatctct 33900
   cgcgtcagcg gacatatctg cggctgggtg tcgtcgtcgc tggtgaactc caggagagcg 33960
   ccggtcaggc ggttgagata cagacatccg gacttaaagg tgttgtcgat ggccgtttcg 34020
   gtgttgaagt tgcgcagcga cggcggaacg cgagcgccgg ttttgatgtt gtcgtatatg 34080
   ttctccagca gctggtagag cagcggactg gcgctcacgg gcttgaggcg accgaagtac 34140
   ccgctgtcgt tctgccgctg catgtcggtc ttcttgctcg ggtagatctt aaactcgccc 34200
   ttcacgacga tgagcggcga gacgagcttg gatgctagac tttcgacgag acacacgttg 34260
   atgttggagc agctcgggta ctgcgacgga gttagagtca cggcctcgat gaccttggtt 34320
   tggctcgacg agagcgactt agcgaagttg atcgcgtcgg tgcacgacat cgcctggttc 34380
   tcgccgaacc gcctggcgga cgctgcatcc tcctgctgag gagcgcggtt agacgcgacg 34440
   gtggttttgg atacagcgcg tttcattatt gcggcgattt taaagtacgt gtatactttc 34500
   agttttgtcg ccgagcgttc agcgcctgca tgcagaggaa gtacaggatg atggtgcacg 34560
   ggatcgtggt cagcagcgat acgaagtcca tcactgtgag gacgcgcagc gccccgcgcg 34620
   agcggatgcc cagcgagggc gcgccgcggc gcgcgatggt ggccccgttc gtcaccacta 34680
   ccagcagcat taggatggtc gcgcccacgg cgacgcccag gtcccgcgac tccatttata 34740
   gtacagtata gagcgaccgc gtcacgaact ctcggctggc caacacgcgt ccgtcgggcg 34800
   ggtgtccgcc ggccttcccg cggaactccg ggacctcgaa gctggacttc gtcacgcggt 34860
   acgtgtactt gccgcgccag accaggtttt ccttctggaa gacgccgtcc atggtcacgc 34920
   ccgccatgaa ggcgtccttg acgatgacca gcaccgcgtc tagcttgcgc ccgttgatgt 34980
   gcgtgacgaa gtccgtgccg ctgcggctcg cgcagcggat gtccacgccc gagggcaggt 35040
   ccaccacgaa cacgaagcgc ttcggcgcgt agagcaccag gtccgaggac ggcgacgccg 35100
   aaggcgccga ggggaactgc cggtggtcaa aagggtgcac cacgcccacg atggacgtga 35160
   cgcggtcgtc cgggaactgc gtcgcggcgc cgccgccgcg gtgccgcgtg accgtgcttc 35220
   tgcccacgtc gtcgcagacc acgtgcagct ccgacacgat cggcagcagc gtggccagca 35280
   tgcggtcggt ctctgtgcgc gtcgcgcagc ggtacgcgat cccgcagtgc gcgtcctgcg 35340
   tgcgcccgaa gaagagcacc agcacgctcg cgtcctggtc gaagggacac acggccatca 35400
   cgcccaccgg cggcggcccg tggcctgcgt acgcggagga gaactcctgc acctcgacca 35460
   cggcgtcctc gcgcgcctcg ccgggcacca tcgccgccgc cggccgcagc gcccgcacgg 35520
   tctgcttaac cgcacgcgcg gcggaggccg cgctcggcgc gactacgcgc acggccgcgt 35580
   gcgcgcccgg cggcggcgcg gttccggcca tccagcccac cggcgagaag aacacgtcgc 35640
   agacgtgcac gcccgcggcc tgcagcgcgc gcgcgagcgc gcgcacggcc tcccactcct 35700
   cgcgaaaggc gctcgcgacc gcgagcgcct tcagcaccgt gtccacggag ttgacgggct 35760
   tctggaagag gttctcgttg ttgtagatga actcggggag ctccacgggc actgtgaaca 35820
   gcctaatctc gtgcgcgccg ctgggcgtga gccgcgtcgc gggcttgcgc acgccggcgc 35880
   cgatctgctt gaagaagtgg ttcatggcgc cgccggcttc tcgggctccg gcgggagcag 35940
   actatttatt cgggaggtta tcctttccga aagcacctgc acggacttcc gcgtccagcg 36000
   ctccatcttc atgtactcct tcatgccgtc gctgagcacc tcgacggcct ccagcttggg 36060
   cgctgtcggg tcgaagagga tgctcttgag cagcgtcatc ttcttgtccg cgaggaagcg 36120
   gaagtaagtg tagatgcagc gcagcgcgcg gaagttctcc gggtgcttga tggtgcacag 36180
   gatcatgaag atgcaggtga acatgccgca ctcggactcc atgagctggt tgacctcgag 36240
   gttgatgcag ccgcgccgcg ccttgaagtt gtccacgaag aagcgcatga gcacgtccac 36300
   gtcgcagttg cggttgtcca ggtccgcggt ctcggcgttc acgttgaagc cgtccgagaa 36360
   ggagtagaag tagaagtact tgcaggggtg gaactccgag gggctgttgc cgccggagtc 36420
   gtagaaggac acgagccgcg agacggtgtc gaagatgcag cacttccagt ggaacatgta 36480
   gcagaagccg aacatcacgt agcgccgccc ggcgcgctcg atcttgtcct tgagcgtgag 36540
   gctgaccatg ttgcagcgga agcggtccgc cttttcgtgg atggccgcgc cgttgaggaa 36600
   gttcaggttg aactggccca ggtacgcgac ctcggtgccg aacgcgaagg gcgccaccag 36660
   actctggatg ctcacgttgc tcatccaggc gctgcggtcg ggctttatgg cgatgggcac 36720
   taccttggtg ttcacgcccg tgctgacgcc cgcgcgcgcg aggtcgtcca cgttcagcgg 36780
   catctgcgag aagtccacgg cctcggacac cttctcgcgc aacgagggct tgaagaagaa 36840
   gcccagcggg accttccact ccagcgcgat cgcctcgcgg aagccgtagc gacccttgag 36900
   gctggccagc aacgcggtct tctgcgcgac ctcgtccttc tcggtgtccg gcggcgcggc 36960
   gtcgatgagc ccgcgcttcg cgaagtccag cagcgccgcc agcgggatgc acgagacgcg 37020
   accggccgtc gcggattcgt cgaagcgccg caccacgtac ccgttgcagt tggtcttgaa 37080
   gttggacacg tccaggtgcg cgctgagccc caccaccgag tagatgtggc acagaaggtt 37140
   ggtgaacccc agctccggga ttttgctcac cactaaatcc gtgtacttgt ccatttatca 37200
   tggagaatca tctgccggac atgctgatgt ttcccaactg cgtttctgtg tttccctttg 37260
   agtactcgct ggaggacgtg ttccgcctcc ccgaggagcg acggcgcgcg ttcgccatgg 37320
   ccgtgttccc gctctccaag caccgctgga ggggcgcgcg gctccagcgc gacgagcgaa 37380
   gcgtgtggct cagcgtcgag gaggaccgcg ggcgcgcgct ggacgagcgg aactgctcct 37440
   ggctctcgga cgtggccgcg cgcatggtcg acgacgaggg ccgcgcggtc acgcccgagg 37500
   cgtacgcctt catgcgcgcc gcgcccggcg cgcgcgtcgc cgagctcgcc gcggacgcgg 37560
   gcgtgctagc gggccttgtc gccggcggca acgcgctgcg cgtcttctcc tcggagtcca 37620
   cgcaggcgcg cgagggctgg aaggcgcgca gcgtgggcgt gctcggcaac gcggcgccgc 37680
   tggcgcccgt gccgctggca tcgctgcgtc cggaagtgca gcgcgagctc ttcgccgcct 37740
   ggatcggccg ccgccccgtg gtgctcacgg gcggcacggg cgtggggaag acctcgcagg 37800
   ttcccaagct gctgatgtgg ttcaactacc tcttcggcgg cttcgagcgc ctggacgccg 37860
   tccgcgagtt cgcggagcgc ccgctcgtgc tctcgctgcc gcgcgtcacg ctggtgcgcg 37920
   cgcacaccgc gacctacctc gcctcgctgg gcttcggctc ggccgacggc tccccggtct 37980
   cgccgcggta cggcgccatc ccggacgccg agcggaacac ggccccgcgc gcctacgggc 38040
   tcgtggtggc cactcaccgg ctcacactga ctgccatccg ccgctacgac acggtcgtag 38100
   tggacgagat ccacgagcac gaccagatgg gcgacatcgt ggtcgcggtc gcgcggaaac 38160
   tgggctcgaa catgcgatcg ctggtgctta tgacggccac gctcgaggac gaccgcgcgc 38220
   gcctggagga gttcctggac cggcccgcct ttgtgcacat agagggcgac acgctcttcc 38280
   ccatccgcga ggtctacgtg aagaacacgc aacagccgcc gctctcgcgc aagtacgcgg 38340
   aggcggagct gcagaacgtg gcgcaggcgc tgggcacctt cgtccccgag cagggaaagt 38400
   gcggcatcct cttcgtagcc acggtggcgc agtgcgcgct cttcgcggag accatcgagg 38460
   ccaagcaccc cgggctgctg gtgcgcgtgg tgcacggaaa ggtgccctcc gtggccgcgg 38520
   tgctcgagga ggtatacgcc gcggaccggc ccgcggtgct ggtttccacg ccgtacctgg 38580
   agtccagcgt gaccgtgcgc accgccacgc acgtctacga cactgggcgc gtgtacgtgc 38640
   ccgagccctt cggcggccgc gagaccttcg tctccaagtc catgtacacg cagcgcaagg 38700
   gccgcgtggg ccgcgtggcg cccggcacct acgtgcgctt cttcgacacg cggctcgcgc 38760
   tgccgctgaa gcgcatcgac tccgagttcc tgcacccgta cgtgctttac gcgcgcatct 38820
   tcgggctaac gctgcccgac gacctgctcg tgcagcccag cgacctcgcg ctgctgcgcc 38880
   gcaccgagga gtacgtcgac ggcttcggca tcagcctctc gcgctggacg cagctgctgg 38940
   accggcacta catgcacatg gtcgagtacg cgaaggtgta cgtgcgcggc gggcgcctcg 39000
   ccgccgcgct ggacgccttc gagcgcaccg gcgtgatgac gcacgaggcc accgaggcca 39060
   tccgcgccgt ggacatgctc gcggccgtcc taaacgtgcg caagtccaag gaccgctacc 39120
   gcgcggagtg caaggtgctc ttcgggccct tcgcgggcaa aaagttcgtg gtcgccgggc 39180
   ggcgtccgcc cggctcgcac gtgctcatgg tcacagaccg cgtcttcatc gaggccgagc 39240
   ccccattctg aggaccacct tcttggagac gcccgagaag tcgtcggcga cgccgcggcg 39300
   cgccaccaca aggcagtacg aggtcacgtg cgggcagcgc gcgatgcagc ggaaggcttc 39360
   ctcctgcgac agcgagaagg cgaacacgta gaaggtgtgc ggggacttca gcggcgtgtg 39420
   gtccatcgag tagatgacac cgagcttctt catgcgccac ataagcgcgt tgatgtggtc 39480
   ggcgcgcagc gcgcggccct tgagcacgcc gcagacgaag ctcgagcagg ccacgacgtc 39540
   gtagcgcgtg ttcctgccga agaccaggtg cggcgcgccg gcggcgcgcc gcgcggccgc 39600
   gcgattctcc acgatgtcct ctatggagcg ctcgctcgca aagaagtcca ggaacatgta 39660
   ctggtaggcc acggccgggc gcgacttgct gaacttcatg aaggcgtccg agtccatgat 39720
   ggcgtccatg tcctcagcgg cgagccggtg ctgcagccgg atgccctcga aggtgtggaa 39780
   gagccgcgcg tccgcgtgca tggacagcgc gagagtgacg aagttgagaa ggtccgcgtc 39840
   gccaaagcgc acgagcacgt taccgggcgt gcgcgtcttg cgcatgagcc gcgcgggcgc 39900
   gccgtcgttg tggctgcggc ggcgcatttt gtcgccgggg gactcgggcg gcaggtcgat 39960
   catgaccagc cggtgccgct gcgcgtcctc ggcgttgaag atcgaggacg tgaagcccgg 40020
   gtacagcacc acgcagtcgc gctccgagat ggcgtgcagc acgtcgcgct tgagcccggc 40080
   caccagccgc tccgcgttct cgacgaagta gttctcgtag tccaggatgt cgtgcgccat 40140
   ccaggggaag ttcaggtacg cgttcatggc gtagtcctcg gcgtcgaagc agatgcgcgt 40200
   gtctggcgtc gccgcgatcg gaaggtcctt gatgccgcgg agcagcccgt cgtagtcgga 40260
   ctcgtccacg aaggagagca ccacaaagag gtcctcgccc acggtttcgt agtcgaagag 40320
   gtggtaaagc tctcttagcg ccagcacggc gagcgcgttg tccagcgagg cgtgcacgcg 40380
   cgccaggatg ctgtagaagg gcgtggccat catcacggcc ttgccgccct cgcaggcaac 40440
   ggcgcgcggg aaaatgacct ccggcgtgcg cggcagccgc ccgaacgtcg cgttcagcag 40500
   cgcgaccgtg gccgcgtcgc tctggcgcag gaacactacc accgaggggc ccgagatgct 40560
   gagcatgcgc tcgcgcatgc gcgctggcag gtccggcgtg gtcacgaggt ccgcgaagcg 40620
   gccgccgttg tagaggtcgc cgccgccgag gaaggtgagc acgtcgaagc agtgcagcac 40680
   ctcgttgcgg aagtagtact cgttctcgag ctccttggcg tacgcgcgta tgtccacgtt 40740
   ctcgaagttt gttcgcagac cgccgccgtc gaagaaccag gacgcgagct cgcggacggc 40800
   gtccgcgggc ctgttgcggc ggctcttgca ccagaagctc atgtagttgc gcgaggtgga 40860
   ggcgttcgcc aggaagaagc ggtggtcgaa ggagatgagc acatgctcga gcaggtgcgc 40920
   gagccccagg accgcgccca cgtcgcgccc aaaaccgaag tttgatatcc ccaggtagac 40980
   gtcccgtttc atagacggcc tcaggaacac cctgacgccg ttttccaaca ctatcattct 41040
   ccggtattta cttacccaaa agtagtatgg ggagaagtgt ttgaacgtcc cctcgccttt 41100
   ttaaatcaaa agtagacttc tcgcgcccgt gcgccaccgt cacgcgcgcg cggcgcgagt 41160
   ccataccggc gatcaccgcg ctgctctgcg gtgcgtccgg ccgcgggaag agcacggtct 41220
   cggagatccc gtccagctgc gcgtcggtgc gctgccgcca cgcgtgcgcg tccgcgagct 41280
   cgcgcacggc cagctgcatc ttgttcgtcg gcaggaacgt gaacacgtac gccgccgcca 41340
   ggaaggctgc gaagagcacg aactcaaccg cccatgacat ttagggagct gattttgttc 41400
   cacgcggcga cgcacgtcgt gacgggcgac cccgaggcgc cgcggcgcgc ggcctcgctg 41460
   tgccgcggct tcggcgtgga cttccgcgcg attcacgcgg agttcgcgcg gcggtacccg 41520
   cgcaccgcgg ccgccgtgga gcgcgcgcag ccgctgcccg aagtcgatgc cgcctttccg 41580
   ccggacgcgc gccggcaagt cgtgcggctg cgcctcgagg ctgcggcgct ggtcgtcaag 41640
   gagtcgcgtg cgctatcggc ctccatgcgc ggcgtggcgg tggtcgacgg ctgctgcgtg 41700
   cgcgtgtgcc gcgccaacga cgagctgcta gagttcctcg cgcggcgcta cgaccccgcg 41760
   gtctaccgct acgcggaggt gccctcgccg agcgtgcgcc cgggctcgaa agtcttcgcg 41820
   tgcgcgggcc gcagcgtcac ctttgcggcc gcgcaccgga gccgcatcac ggccaaccgc 41880
   ccgctgcgcg tggtcgtgac cgaggcctgt gtggacggcg tgctcgcgcg cggcgccgcg 41940
   gaggtcttcg accgcggctc cggcgtgctg ccccgcgcgc tgcgcgagat cttctaccgc 42000
   ctcgacgagg acggctgtcc cacgggccag acgccaggct tcgcggacag tatggcgtcg 42060
   cgcagctgat ctatgtccac ctttttctcg tcgatctgcg ccacgaccac gaaactgcga 42120
   atgtccacag cggccatggt cttggccacc gggtcgtact tgaggagcag cacgtactcg 42180
   ttgccgaagt gctcggtgac ctcggtgatg agccggtaca cgcccatgcc gagcacgttc 42240
   accgcaccgt ccttggcgaa gagcgagagg atgttcacgc acttcagctc catctcgccc 42300
   tcgaggcgcg cgagcatgcg ccgggtgacc tcgcatactg aacaaagagg cttacctagt 42360
   aagataagcg ttagcttagc cgcggtcggt gacgcgtcgg aggccattta tggggatcaa 42420
   aaacttaaag gcgttgctgc tcagccacgg cgcgctgacc ccgcacgagc cgggcggcga 42480
   cgagcgcttc cctgccgtgt tcgtggacgg cttcagcgtc atgatgacca tggcgtactc 42540
   gtgcgcggac gaagacgagt tccgcgcggc cgtcgaggag cgcgtgcagc actggatgag 42600
   cgtgtccgag agcgggcgga tcgtggtctt cctcgaccgc ggcgagattc cgatcaagca 42660
   gccgctgcgc gaccagcgcc gcaaagccac gcgcgaccgc gccgcgcgcc accgcgagtt 42720
   catcgccgcc gcggaggcag acgcggcggc agaggccgtt ggcgcccgcg aggacaaaca 42780
   ggaggacgag cacgcggagt tcgccgagga gatccgcgct gagaagcagc taaagctgca 42840
   gcgcatccgc ttccagctca gcatcgccaa ccacgaggtc gttaagtcgc tgatagagtc 42900
   cacgctcgcg cgcgctggcg atgccgtgga gatcgtcttc tgcgacggcg tcgacgcgga 42960
   gatggtcatg tgcgcgcgcg gacgcgccga ggccgagcgt cgcgggcgct ggccgctgct 43020
   cgtgaccacg gaccaggacg cgcttttgtt cacgtccacc gatcgcgacg agaagatagt 43080
   gagcaccgtc tccgcctgct acgcgttcag gcccaccgag acgaccgagt acctgtgcaa 43140
   acttgcggcg ctggccaacg gctgcgactt cttccccggg ctcggcggca tatgcgtgag 43200
   tgtggagtcg ctgcgccgcg ccacgctttt cccggaattc tccgtgcgca acgccgccgt 43260
   gagtctgtgc acgcggccca tgcggctgtc cacgcaggac gcgctggagc cagaggccgc 43320
   cgccgaggtc gtggaattca tcaggcggta cgccgccggc gacgagcgca tctaccgcga 43380
   ggtgccgccc ggcgcgtgct gcggacgcgc gtttgtgcgc ggagcgctcg cggccgagtg 43440
   ggccgacgcg ctgccggcgg ccacgggtct gagcgtggtc gcggacatga tcgcgtgtct 43500
   gcccgcgcgg cgggaccccg cgcccgagga ggtagagcgg ctgctggcgc tggaggcgcg 43560
   cgcgcgaggc gcgcgcgtca cggatgcgat gctcgcgcag actgcgcagc tgctgggtta 43620
   cggcgcgagt gcgggcgccg acggcgcctc cgccttcgcg gtctcgggcg ccaagggcct 43680
   gatgtgtcgc ctgcgcggca cggccatgtt cttcaacgcg gagtacgtgg aaattgaaag 43740
   cgaacccaga ctgttaaagc tgcggtagca tggtgttccc gatcgtgtgc tcaacgtgcg 43800
   gccgcgacct gtcgcacgag cggtttctgc tcatcgtgcg acagcggccg ctaaaggttg 43860
   ttttgcggac ggtgcgcaac gtctgctgcc gtataaagtt gtctacacaa atagagccgc 43920
   accggaacct gacggtgctg cccatgctcg acataagctg atttttcttt tccgctcgta 43980
   tgcgcgagtt cggactcgcg gcgcgcatgg cccgcgccat cgaggacgtg tgtccgcgcg 44040
   gcgcggtgat attcgtatcc agcgccgcgt ccatgaccga ctgcctgaac ccgtcggtgt 44100
   tcaagcacgc ggcgatatac gcggggcgcg tggaccgcgc gccgctgccg ccgccctcgc 44160
   cggtcccggc ggaggccgtg acggagccct gtgcgataga cgccatagcg ccttacggcg 44220
   cgcgcgtggt cctgctctcg gagctgctgc ggagctgcgt ggccgttcag gcctaccgcc 44280
   tggcagtccc cggcgccctc gcgctcatga acctcgcggc cgacgcggcc ttcgagctcg 44340
   tgggcacgcc ctacggcttt aacagcgacc gaacgtactg cttcaagctc gttgccgact 44400
   gctttgctag cgtgggcgtg acaacgaaga ccaggcgcat catgggtcgc gacgtcgtgc 44460
   tcagccagga cttcctggag agcggcatgt ggaccaaggt gctggactcc gccgcggagc 44520
   cgccgtggct ggtctagaac agcggcggcg cgcgggtccc gagcacgggc cgcgccacct 44580
   gcagccgctg ctgcagcgcg cggcactgcg cctcggcgtc ggccgtctcg gcggggtcga 44640
   cgggcgtcgg agttgcggag gtggtcctga acggctgcgt gttcaccgag acgcggatgc 44700
   gctccttgca ggagcgctgc tcgatgcagt tggccagcat cttcatcacg tgcaggtact 44760
   ccagcaacac gaacttttcg agggtgatgc cgtcgaaggg cgacgacccc accacgccca 44820
   gcgggctgga caccgcgccg tcgagcacct cgccgcggga ctccttgcgc gcgcgctcga 44880
   gcaggtcctc tgtccgagcc accacactgc cgaagtcggc ggccgcgggg gcgggaacag 44940
   gcgcagcagc gctgtccgcg tccgccggca tctcctcgat cttgagaccg gccgcgaact 45000
   ccgaggccgc gtgcacgggc gaggcgccgc gccgcaccat gaagtcgcac agacgcgata 45060
   gcgcggagga gcgcaccggc atgtcgagca ggcgctcggc ctccatctcg gcgaccgagt 45120
   cggcgcacgc gtccggcgcg cccgcccgca cgagctcgtc gcagcacccc gcctccttca 45180
   tgagcgcggg catgagcttg tactgcgcca tgttcaccag cccgtacttg agctcgagca 45240
   ggtccgcgag ctcggaggcc atgggtcggt ttttggtgta gatgacgcgc tccacggcct 45300
   ccgccatgtc cacggcctgc atgagctcgc cgacgagcac gctggccacg agcgtggcca 45360
   gcgtgacgcg cacggtgggc acgcagaccg cgaagaagga ggtggagtgg gtgaagcgca 45420
   tgagcgcgcc gtgcagacgc gcgaggtccg cgctgttgcc cgcgtgcacg aagcgccggc 45480
   gcagccgcgc cagcgcctcc acaaggtcct ctcgcgtggt cacgcgcacg ttcgcgatgc 45540
   acaggtcgtg gatcgcgttg gcgatctgcg cgcggcgctg cggcgagctg ccgggcagca 45600
   gccgcgcctt ggcctcgacg tcgacggtgc tcgagagaca gccgcaggcg gcgccgcgga 45660
   cgacgaactt caacaacgac tcgaacacgc gcgcgcccgc gcggggcgct tgcttggacg 45720
   actccattta ctttaaataa tttacgagat caaaataaaa tgactctgcg catcaaactc 45780
   gagaagctca agcagatcgt aacttacttc tcggagttca gcgaggaggt ctcggtgaac 45840
   gtggacgtcg gcgatggcct catgtacata ttcgcggcgc tgggcgggtc cgtgaacatc 45900
   tggaccatcg tgccgctcag cgcgagcgtg gtatacgacg gcgatgtcag ccgcgtgttc 45960
   aacctgcccg tgctcaaggt gaaggcctgt ctgtgcagct tccaccccga ctcggtggtg 46020
   agcctggagc ccgacctcga ggacaacgtg gtgcggctct cgagccacca cgtggtcagc 46080
   gtggactgcg acaacgagcc cgtggcgcac cgcacgaaca ccgccatctg cctgggcatt 46140
   aaccagcgca agtcctacgt gttcaacttc cggcgctacg aggagaagtg ctgcggccgc 46200
   accatcgtca acctggacct gctgctgggg ttcatcaagt gcatccacca gtaccagtac 46260
   atcacggtct gcttccgcga caagaagatg gtgctgcaca cgcccgggaa ggtggacaac 46320
   ttcttccgcg agtactccat gaccgagtgg gcgcccgacc tcgagcgctt ctcgttcaag 46380
   atccccatct cctccgtgaa caaactccgc ggcttcaaga agcgcgtggt catgttcgag 46440
   tcgcgcgtgg tcatggacgc cgacgacaac atcatcggca tgctcttcac cgaccgcgtg 46500
   ggcatgtacc gcgtgaacgt gttcatgtcc tttcaggacc ggtctctttc atgcgactaa 46560
   atactcatgg gcgggtcggt gagcctgccc tcgcgggacc tgccgccgcc ggtgcgcacg 46620
   ccggagatga acatcgtgcc cgagcgcgac ctcgcggaca cgatggcgcg cctctccacc 46680
   gcagacccgc cgcagccgct gggcgtcggc gacgacgcgc gcatggccgt gctgaagacg 46740
   accttccccg agttcgcgat atcgcggccc gcgacgggca tgctcgccgc gcagcgaatc 46800
   aggtacgacg gcgacccgcg cgtctgctgc ggcgggttcg ggatctcgca ttactgggag 46860
   aagggggcgc gccgatcgaa cgtcgcgttc gagggcgcgg cgctgcgcac ctgcgacccc 46920
   acgcgcttcg acgcgggcgc gtgcgacgcg ctgctcttcc gcgagtgcgc cgccggcggc 46980
   gtcgacgcgg acttctgcgc gcactggatc aacgcggccg tgacgcggcg cacggaccga 47040
   cagtcgcgcg cgcggctgaa cgacatgttc gtgcgcgatt gccaaaacga cgccgcccgg 47100
   cctcactgcg tggcctggat ccgcgcgatg cgaagcgcgc gcgcgacggc ggacgacggt 47160
   ctaatagacg ccgtgctctc ggtgcagagt cccgagttca agggcaagca catgcgctgc 47220
   agctacccct cgccggccac tctcgccatg gccgcgaacg tggacgagcc gcgcgagtgc 47280
   tgggaccccg agtgcgtggc cgggaacgtg gacttcatgc taagcgataa ctacacgaac 47340
   ctgggcttgt gtcggctctc gcgctgctcc atcggcgtca cacacctgcg gattgacgcg 47400
   cgttcgcggc tgcgcatgcg gtgcgccggc gcgcttgccg ggctcacgaa ggcgcccgtg 47460
   aaccagactg tcgtcgtcgg cgacaacctc gcgcgcgcct tcgagccgcg cgtggaaacg 47520
   ctcagcgtgt tggcgctgtg cgtggtgtat ctgctaattg tctggctcta aatgggggcc 47580
   gccgccagca ttcagaccac cgtgaccacc gtcagcgagc gcatccgcaa cgagctcgag 47640
   cagagcgcga gcgctagcgc gaccgccgac tgcgacgtca ccatcgggag tctgattatc 47700
   cgcaagaacc taggatgcag cgtttccgtc cggaacatgt gctcggccaa cgccggcgcg 47760
   cagctggacg ccgtcatgaa ggccgtgagc agcaccttca acgacctctc gtcggaccag 47820
   aaggcctacg tgcccgggct gctcacggcc gcgctcaaca tccagaccac ggtgaacacc 47880
   gccgtcaagg acttcgagac gtacatgaag cagacctgca cggcggacgc ggtcgttcac 47940
   aacaaaatca agatccaaaa catcgtcatg gaagagtgcg cctctctgcc agggagtccg 48000
   gccacgcacc tggatttcgt gaacaccggc acggccgtgg gcaactgcgg cgtgaaggcc 48060
   gtgatggacg tgctcgcgaa ggccagcacc accgtgcgca acgaccagga ggccggcaag 48120
   ggctaccaga ccatcatcat cgcgatcgtg gtcgccatcc tggcggccat cttcgcctgg 48180
   tacgcgcggc acatgctatt catgtccacc tccgacaaaa tcaagctcga gctcgccaag 48240
   aagcccgtgg tgcactggac cacctacctg gacaccttct ttacggaatt tccgccgtcc 48300
   gtctagatac gcgcaacatt gaaacattat atccacctct caaacggcgg tatggtccga 48360
   cgcgtcctcc tcgagcgcgt ggacggcatc gtcgagcact cgcgcgcaga ccgacgctac 48420
   ttggaggcca ttcagcgaca cctcgagggg tctacgcccg ggctgcggca gatgtggcgc 48480
   ttcctctacg acctgctgct gacggtgttc gtcgtcatgt acatcgtctt ccgcctaatc 48540
   gtgcgcaacc ccggcatctg cgccatcctc gcgctcgcgg ccgcggtgta ctacctgttt 48600
   ttgtgtctct ttagcatgga ctgatggcga tcacagacag accatcgccc gcgcgcgcgt 48660
   gaccagctcc ggcgccgcga agacgtcctg caccgggaag tcgtcgatct cgaacacgga 48720
   gccgtccgcg gaccagatca cgcgcacgtt gtcgctcacc gagacctcgg tcagcgtcac 48780
   gcccagcaca accgcgtcgt tggtgctcac cagcaccagc gcgccgggct ccgcgcgccg 48840
   gtgcagcggc ggccccgaga ctgagcgccg ctgcacgcgg aacatgtccg cgaactgctt 48900
   cgagagcaag tccaggtggt tgcggatgat ccactcgaag aagtacgcgc aaccgccgcc 48960
   gccgcacagg aagcgcgaac ccgcgggcat cagcagccgc acaacgtcca tgtagcaggc 49020
   ctgcggcagg ctcgcgcggt acagccgcgt cttcggcgag agcaccacca ggctggaggt 49080
   gctcatctgg aagaccagct ggctaacgga gacggtgagc gtgcacgcgg gcacggaaac 49140
   cacgtccagg cagatgtcgt ccagaaagat gctccgctgg tagaggtggt acaggatggc 49200
   cacgatctga aaggccgtgg cgtcgctgat ggcgcagggg cggtcggcgc agcgcatctg 49260
   cgcgcaggac cagcccccga aggactcgaa gcagacggtg agcatgcccg tgctcggaca 49320
   gtgtggcgag cgccgacaca ccggaaagcc cacggccttg cggcagcgca ccatggtcga 49380
   gagctctatc cagcagcctg cctcctcctc gcccatgccc atggctaccg gcgtgaaggc 49440
   cgtgacgtcg tcgcagatgc gccgctccag aaaccccacg cccgaggagg ggtgcgcggc 49500
   cggcggcgag gtgatgcgcg ccgggacgcg gctcggagcg ggctcgggag gcgagctgcg 49560
   ctcgacccgg gcagtcgccg ccggccgcga tgccctgcgc gcgggcgcgc gctcgcgcaa 49620
   cttgtttgac ttgctggcct cgtcgctagc gtcatcgaag cggtcgttcc tgtcgccgcg 49680
   gacgtccgcc tcgtcgcccg tcggctgcgc ggcgggcgac gtgccgtccc gcgtacggcc 49740
   cgcgttcggc gcgaatgtca cgcgccggtg cacgtacggc tccgtagagc ccgtgggggc 49800
   gccgcgcccg cgcccgcggc ggaaggcctg ccgggacgcg ccgaagcggg cgaactcccc 49860
   cttcgcccgg cccctttttt cttccatgat atttatcaca aaaaaaactt ctctaaatga 49920
   ccaatctgct ttcgttggtc gacccggagg acctggcctt ctgcgccggg ttcccgtcct 49980
   tcgacgagac catgctcgtg atcgcggggg cgcgagtgcg cttcccacgc tcgctgctct 50040
   cgctcttcaa cgtggtgccg cgcaccatga cgcgctacga aaccgagctc gtgggcaccg 50100
   agatggtggt gggcgccgtg ttcaccaccg cgtacaacgt ccgccgcaac ctaggcctcg 50160
   gcgaggagcc cgtgaccatg cgcgacatcg agaagtactt cctggactcc gagaacgagg 50220
   tgctcacgct catcgtgcac aacaccgact tttccgccat gagcggcgtg cgccggcgcg 50280
   gcggccggcg catcgccaac cccgtcatct tccgcagcgg gtccacgccg ctgctcatcg 50340
   tgatggagtc gcgcaagaag accaacatct accgcgagcg caccgcggag caggccaacg 50400
   cctcctacag ggaggtcggc tcctcgctcg cgctggtcac tcggtacgcg ggtctgcagc 50460
   tggttgacgt gcacacgccc agctccgtgc taacggtctc cgccgtctac ggcttcaccg 50520
   aggacaaggg gctcaagaag ctgggctccg acaaggagct cgcggactac cagtccacgc 50580
   cgctcaccga ccccatccgg ctcagcgact tctccaatat attcgacggc gtcaagaaga 50640
   gcatccagct cacgaacgtg cccgtgccct ccaccggcgc cgaggccgcg ccgtaggctt 50700
   tcatgcgcga taaatcggat ggcggcgccg acgacgcccg cggtgcacct cacgccggtg 50760
   ttcgtggagc ctacgatcgc gcactcgctg ctgcgcgcag agtcctacct cgcgatcgcg 50820
   gtccttgagc tcgtgctcgc gctcgcgctc gcgctcgtct tcttccgcga cgagctaggc 50880
   tcgctattcc gctgcgcgcc gcgagcgcct tcgccgctgg acgcgtacct gcaggcgagc 50940
   ctcgtctgcg acggcgacgc gctgctgatc gagctgcccg agggccgggt gccggcgctc 51000
   gcgctggacg ggcggcccgt cgcgttcccg gggtgcgaga gccttttgta ccgcataaat 51060
   ggaccacgaa aagtacgtct tgtcgatgtt cttggaggaa gataactcct tcttctcgtt 51120
   cgtcgccgcg ctgtccgatg acgaggcgct cggcgccgtg cagtccgctg ccgccctcct 51180
   ggacttcctg ctctccgtgg tggtccgcgg caaggagaag ctcgccgccg cggggcacca 51240
   ctacgactcc atcgcggacg gacgcgcgcg cgccgcgttc gagttccgag acctgcgcga 51300
   gctggcgcag ctcttcgacc ggcggccctg cggcgtccag gaccgcgtgc gtgtgcgcga 51360
   cgggcccgcg cgcgccttcg tggacgcggc actggggctc atgcgcgagc gaggcttcga 51420
   cggcacgcag gccgcggagc gcgcgcgcta catcgcgccg aacgatctgc ccgcgctggg 51480
   ggcaatatcg gccacgctct cgccgggtct ataacgtaaa aaatattagt aaaattctga 51540
   aggtccgtgt gtttcgcggg cggccaacaa accagtcgct taaatggagg gggtggaaat 51600
   ggacaagccg ctcctctact tcgacgagat cgcgggcgcg cgcgactacg acgcggcctt 51660
   cgcggagaag cacgagccgc ccaagatccc cggccgcgga cagatgaagc tgctggtctg 51720
   cgagctcgtg tttctcaacc ggctgcacct gcacggcatg ctcgacggca gcgtcatcgt 51780
   gtacgtgggc tccgcgcccg gacggcacat ctgctgcctg cactcgcact tccaggagct 51840
   cggcgtctcg cttaagtggg tgctcattga cgggcgcaag cacgacccct gtctctcggg 51900
   gctgcggaac gtgaccacgg tgacgcgatt cgcggacgag gcctacctcc gcgagctgcg 51960
   cggcgagctg cggcgcgcca agatcgtgct catttcggac atccgctcca accgcgtgga 52020
   cacagagccc accaccgcgg acctgctgcg cgactacgcg ctccagaaca ccatggtgag 52080
   cgtgctcaag cccgtggcct ccagcctgaa gtggcgctgc cccttcccgg actcctggga 52140
   gaaggacttc tacgtgccct gcggcaagga gatgctgcag ccgttcgcgc cgccgttctc 52200
   cgcggagatg cggctgctca ccgtgtactc ggagacgcgc ccgaagctgc gtctgatcac 52260
   gctcagcgac gcggtcaact atgaaaagag gatgttctac ctcaatagcg tggtccgcca 52320
   gcgcgtaatt ctgaactttg actatcccaa ccaggagtac gacttctttc acatgttctg 52380
   tctgctctcg tcggtggtgt gctcgtgcga atttaaatcg cccaaagaga aggtgctgag 52440
   cctgcagaac cgcttcttcc gcttcctgcg catcccgccc tccatcacgc tcgggctgcg 52500
   ccggcacgat gaaccgccac aacacgcggt acctggccaa gatcctctgc ctaaaggccg 52560
   cggtaagaag cgaccccttc gcggtggtaa gtagggacac cgtgcgcatg tacgacatcg 52620
   aggtcgagta cggcgacctc gtgacggtgg tcaccgtcac gcacaaactc gagaccagcc 52680
   gcaccgtctt ccaggtcttc aacgagacct ctgtcgcgta ctcgccgctg ccggacgact 52740
   acggcgagcc catcgtgctc accacgtaca tgcagcgcga gcacaccaag ttcccgctct 52800
   ccatgctcta catcgacgtg gtcgcctcgg acatgttccc cacgtttaag cgccccaccg 52860
   aggaggaggc cgcggtggtc gcggccatgc agcgcgtggg cgggcgccgc gagcccgtgc 52920
   tcaagctccc gcgcatgctg gacaccgagc tcgtgtgcaa gatactgcac ctgcccgagc 52980
   acccgctgcg cgtggtgcgc ttcctgcgcc gaaacatgtt cacgggcgtg gaggtcgccg 53040
   accgctcggt gtccgtggtc ctcgactgac gaagggcagc acggtcagcg aggccgccgc 53100
   caccaagcac agcggcagcc acgcgcgcgg gtccgccacg ggcacgaaga cgtgctggtt 53160
   caggtacttc gcctggaagc gctccgcggt ggagtccacc ttggacccgc aggcgttggt 53220
   gaggcgcacg accgcgtccg cgacgcgcac gtccccgagc gatatcacgc agtcagagac 53280
   gttgcacccg gcgatgtttt tcttcagcgc gcgcggcagc agcgcgtccg cgcgcttgca 53340
   gggcgcgtac cagcagtagt agggcaggcg cgtgtcgcgg ccggtgtcga ccacggcctg 53400
   gctgggcttg aggcacgcgc agcgctcgtc gtccgggtgc gcgtcgcaga aggcgtaaat 53460
   ctcctcgtcg ggcgcgtccg gcccgggcgc ggtcggcggc gccgcgcgac ggcggaagaa 53520
   catctctgaa aaaatacttc gaccagaaaa cgaccaccga tcttatttca aagataaaaa 53580
   tactattaat acgcactcgg agaatcatgt cggtggtggc gcgcgtgtcg tacagcctgt 53640
   actcgcagag cgagataagc gccacggacg tggtcatcag ccagttgaag aacgacgagg 53700
   acctgggcac ggtgaaggac ccgcgcctgg gcgcctcgga cgggtccata tgccgcacct 53760
   gcgggctcac ggagatggag tgtttcgggc actggggcaa ggtgcgcatc tacgagtcct 53820
   acatcgtgcg ccccgagtac atccccgagg tggtgcggct gctcaaccac ctctgcgtgc 53880
   gctgcgggct gctgcgctcg cgcgacccgt acacgacgga cttggccgcg ctcagcgtgc 53940
   acgagatgcg caagatgaag gaccggatga tgtccaagaa gaaggcctgc tggaacagca 54000
   agtgtctgca gccgtaccag aagatcgtct tctccaagaa gaagatctgc ttcgtgaaca 54060
   aggtggacga gatacccgtc cccaacgcgc tcatctacca gaagctgacc tccatccacc 54120
   gcaagttctg gccgctgctg gaggtgttcc aggaccccgc gaacctgttc tacaaggagt 54180
   acatgcccgt cccgccgctg ctcatccggc cggcgatcag cttctggata gacaacatcc 54240
   ccaaggagac caacgagctc acctacctgc tgggcatgat cgtgaagtac tgctccatga 54300
   acgccgagga gcaggtcatc cagcgcgccg tgatcgagta cgacaacatc aagatcatct 54360
   cctcgaactc gagcagcatc aacctctcct acatcatcgc gggcaagagc aacatgctgc 54420
   gcagcttcgt ggtcgcgcgg cgcaaggacc agaccgcgcg ctcggtcatc gggcccgact 54480
   ccgcgctctc ggtgtgcgag gtcggcatcc ccgactacat ccggaacacg ctcacgcaga 54540
   aggtgttcgt gaactacctc accagcaagc gcgtgcgcgc gctgttcgag gaccgcgcgg 54600
   tcaagttcta cttcaacaag cggctgcgcc agctcacgcg catcaaggag ggcaagttca 54660
   tcaaggacaa gatccacctg ctgcccggcg actgggtgga gatccccatg tccgagggca 54720
   cgaacgtgat attcggccgc cagccctcgc tgcaccgaca caacgtcata tcctcgaccg 54780
   cgcgcgcctc gcccggctac accatcaaga tcccgcccgg gatcgcgaac tcgcagaacg 54840
   cggacttcga cggcgacgag gagtgggccg tgctcgagca gaaccccaag tccgtgatcg 54900
   agcagagcgt gctcatgtac ccggtgacta tcttcaagca cgacgcgcac ggcgcgccgg 54960
   tgtacgggtc catccaggac gagatcgtgg ccgcgttctc gctgttccgg caccagaacc 55020
   tctcgctgga cgaggtgctg aacctgctcg ggcgctacgg gcgagacttc gcgccggagc 55080
   ctggccagaa gaccttctcg ggcgccgacg tcttccgatt catgataggc gcggacataa 55140
   acttcaaggg cgtgctcgag aacgggcgcg tggtggcgcc gaacgtcgac agcgacctcg 55200
   tggtggccat gcgcgcaacc tcgctagcgg ggctgatcgc ggactacgcc acgaacgtgg 55260
   agggcgtgcg cttcgtggac atggcctcct acgtgtacaa gcggtacctg gccatctacg 55320
   gcttcggcgt gaccttccgc gacctgcgcc cggacccgag tttggttcgc cggctgcacg 55380
   cgctgaacac cgagaagata gagcagatca aggacgcgta ctcgcggtac ctgcaggacg 55440
   tcgcggacgg gaagctggtg ccgatggcgc ccgcggacga ggccgacgcg ctggactcgc 55500
   tgctggccaa cctgaccaac ctcaacgtgc gcgagatcaa cgagtacatg cgcgagacgc 55560
   tggagcgcaa ccccgataac agcctgctaa agatggcgcg cgccgggtac aaggtcaacc 55620
   ccacagagct catgtacctg ctgggcacct acgggcagca gcgcgtgaac ggcgccgtcg 55680
   ccgagaccaa gatatacggg cgcgtgctcc cgtacgcgtt ccccgactcc gcggacccgg 55740
   aggcgcgcgg ctacatcatc aactcgctca tgaacggtct ctccggctcg cagttctact 55800
   tcgcgatgct ggtggcgcgc tcgcagtcca cggacatcgt ctgcgagacc tcgcgcacgg 55860
   gcacgctcgc gcgcaaggtc atcaagaaga tggaggacac ggtcgtggac gggtacggac 55920
   agatcgtgag cggctcggta ctgctcaagt acgcggccaa ctacgcgaag atcccggggt 55980
   ccaccaccaa gcccgtggag ctgctcttcc cgcacgagag catgacctgg ttcctggaga 56040
   taagcgcgct ctggacgaag atccggcacg ggttcgtgcg catgcaccgg cagcgcctgg 56100
   ccaccaagat cctggcgccg ttcaacttcc tggtcttcgt gaaaccggcg ccctcggagg 56160
   cggaggcgct ctccgcgcgg gacctgtacc acatgatcca gcgcgtgatg aacgacgtgc 56220
   gcgagaagta cttcttctcg ctggcgaacg tggacttcat ggagtacgtc ttcctcacgc 56280
   acctgaaccc ctcgcgcgtg cgcatcacgc gcgcgaccgc cgagctcatc ttccgcaagc 56340
   tgtaccagaa gctgaacgcg ctgctcggcg gcggcacgcc cgtgggcatc atgtccgcgc 56400
   aggtgctctg cgagaagttc acgcagcagg cgctctcgag cttccacacc accgagaaga 56460
   gcggcgccgc gaaggtgaag ctgggcttca acgagttcag caacctcatc agcatgagcc 56520
   gcaaccacac cgagatagtg gcgctgaccg cgccgagcgc ggacaagctg atgccgctga 56580
   aggtaaactt cgagttcgtg tgtctgggcg agctcgtgcc cgagatcgag acccggccct 56640
   cgggacggcc ctccgtgcac cgcgtggaca tcacggtgca ccgcctgcgc atcaagcgcg 56700
   cgcacctgac cgaggtcctg gtggacacca tcatcgagcg cttcgtgtcc ttcaacgtgc 56760
   tcgtgaagga gtggggcagc gacatgaccg tggagggcga ccgcgtcacg tacacgctgc 56820
   tgctgcgctt cgtggagccg gagcagctca acttccacaa gttcatgctg gtgctgcccg 56880
   gcgccgcgaa caagggcaag gtgagcaggt tcaagatccc gatcaccgag accacggtct 56940
   acgacgactt cgacgccgcg cgcaaggcct accgcatgaa catcgagctc atgagtctga 57000
   aggagctggg gatattcgac ctcgaggacg tgaacgtggt ccccggcatg tggaacacct 57060
   tcgacatatt cggcatcgag gccgcgcgcg ggcacctctg cgagagcatg ctggacacct 57120
   acggcacggg cttcgactac ctgtttccct cctgcgacct gctcgcgagc ctgctctgct 57180
   ccgggtacga gcccgagtcc gtaaacaagt tcaagttctg gaacgcgagc gcgctgaaga 57240
   aggccacctt cggcgacggc cgcgcgctgc tgaacgcggc gctgcacaac cgcaccgacg 57300
   cggtcgcgga caacagcagc tgccacttct tcagcaagac gccctgcgtg ggcacgggct 57360
   actacaagta cttcgtgaac gtggagatgt tcatgcgcat ggagcgcgag atccaggcgc 57420
   gcgtggcggc gcgcaagatg gaggagatcg aggaggccgc cgaggaggag ttctaggcgc 57480
   gacggcgcct tactttgcga ccgtgtcacg acgacacgac acggttagga cggcgagtcg 57540
   cagacgaaca tttttatgag ctggtagcgg aagttggcgt tttccaggaa ggcgccgcgg 57600
   aggtcccgga tctcgtagta ggttttgagg aagtacacga agcgcgcggg ctgcgtcata 57660
   gtcgggttct ccgcaagccg cttgtgcatc acgtacccca tggcggcggc gccgctgcgg 57720
   ttgacgccgg ccacgcagtg cacgagcgtg ggcttctgct cggcctcgag gcgcgccagc 57780
   agcttcacga gcgcgggcat gatggaagcg atgttcgtcg tgtcgtcgtc tctcagcgga 57840
   atgtggtacg ccgttatccc cgcgggcgtc gagtacttgg acatggtcat gttaaccaga 57900
   cacttgaagt cgacgccgga gtccccccgc agcacggcgc gcgcgtcctc ggcgctgccc 57960
   aagtacacgt ggtccgtgag ccgcgtcatg cccgagggca gggccagcgg cggccccgcg 58020
   cgcgtgcacc gcagcaggag cctggcgtac cactcgctct tatcgcccat atttatttat 58080
   atgatacaaa tggcagacgt cacaacactg acggccaacg gtctgaccct ggagttcgcg 58140
   cgcgagcgcg ctctgcgcag tctgcgcgcc gcgcgcacct ccacgctggt gttcttcacg 58200
   ctcacgctcg cggcctcgct gttcgtgctc tggctgcagc taaccgagtt tcccgtcttc 58260
   gaggagctcg gcaagtacgc gcgcatcaag agcgcggtgc ggtcctggcg cccgctggtg 58320
   gaggctaaga cagagatcga gtccgacctc ggccggcaga agaccgccga ccggcccgag 58380
   ctcttcgagt tcaggtgcgt ggacttcggc aagttctacc tgccggtgag gtacagcccc 58440
   acgaccttcc tgccgcaagc cgtgcgccgc ggcgcgggcg atggctggat ggtgcacaag 58500
   gcggcggccg tggacctcgc cgcgcagcag ttctgcgagt ccgtgctgcg gcaccgcgcc 58560
   aacaacgtca tcacatgcgg gtcagagatg atgcggctgg tgggctacag cggctacttc 58620
   gaggacgacc actggtgcgc cgcgacgtcc ggcgtgctga cgtgaacgat cacacgatgg 58680
   ccgtgaccag cagcccggcg atgaaccaca gcagccgcga gttcggcagc agcagcacga 58740
   gcaccagcag gtacgccagg atgaagatgt cgaccacgtc cacgtcgaag agccccatga 58800
   aggagaagag cggcgtggtg aggaagtaga tggcgccggg ccagaagcgc gccagccacg 58860
   tggcgagcag cgaccacagg gagggcgcgc cgctgagccg cgtcttcacc tgtatgtagt 58920
   actcggggta gaccacctgc tcggcgccgg agagcaccac gcgcgccaga gagagccgct 58980
   tctccagcgt gaacacctcg gtgagcaggc cgctgcgcag ccctccctcc ttgatgatcg 59040
   cgtcgtagag cttcttcatg ccgccgacgc tgatgatgta ggcgtctagc gagacgtcgt 59100
   acccgccggg gtagaccatg agctcggggt cgccggtgcc ggggacgttg gtggccagcg 59160
   cgccggtcat gtaggtctcc ttgagctgcg tcatgtacca gccgttcgcc ttcatcgcct 59220
   cgatgagcgg cttcaccatc tcgggcttgc ggaaggtcat gtcgttgtcg accaccagga 59280
   tgaagtcatc gtcggagtac ttggtgggga cagtgccggc cgatatgctc tcccagaggt 59340
   tgaggtggtg cgctgcgcgg cgctgcatct ccttcggaca cgtggacttg cacatgtccg 59400
   tgaagaagtg cgggtagtct ttggagtcca cgtctttcca ttccaccgcc ttgagcacgt 59460
   ggtcgccctt ggggtgcggc gcgggaggag atggcttggg tgccggcgcg ggggccggtg 59520
   cgggggctgg ggcaggagag ggagcaggcg cgggttgagg cttgggcggg tcgtcggcga 59580
   ggcccaccag gtacggcagc gtggggaaca cctccttggt cccgcggcct tcggcaaccc 59640
   cgattatgta ggccgtgatt tcgggtggat ccatttagtt attaaaatta atcatataca 59700
   actcttttat ggcggctatg gattcggcta tccagtcctt gaccgagccc acgatgcccg 59760
   ccaggaacag gaagaaggcg aactccaggt ccacgcggtt cagagagtcg ctgaagtaca 59820
   cgaagacgtc gctgtccggg aagaagctgc gccggaacat gttgtacccg ttgaccttgt 59880
   gcgcgacgtg ctccgcgctc agcagcgtct cgtcgaaggg gtacgggtcg ctgaagcgga 59940
   acacgtacat ggccgggttt gcgtagtagt acttcatggt gtttgtgacg aagaggctcg 60000
   ccagcgagat gatgattttt ttcttctcga tctcgatctt gatgtggtcc tcgaagcgct 60060
   tcatgttgta ggcgttggtg tcgtgcacgc ggatgagcac gcgcgagtcc gacatgatgt 60120
   cctggaactc cgcgcgcgcg tcggggctct cggcgggcgt ctccgcgggc cgcgccacct 60180
   ccgcgcacac cgtcggccta gcgcgcggcg gcgtgcgcat gggccgcgcc cccacgcgct 60240
   gcgaagcgaa aaactccacg gcgcgagcct cgcccgcgtc cgcgtacgac tccaccaggt 60300
   agttgcggct gcgcgtggtg cggccgatgg tgttcagccg gtgcagctcc gcgaccagcc 60360
   ggcggtagtg cgcctccagc tcctcgggca tgatggaggt gtacacctcg gtgagcagca 60420
   tcacggtgtc gaagtcctcc ttgccgcaga cgcgcgtctt cacgaggaag tggtgcacag 60480
   ccgtcgcgat agagagccgc agcgtggact cggtgacctc gacgctggcg tccttggtct 60540
   tcttcgcgct ccgcgaggcc atgaacgaga cgaggaagtc cgcgctgctg ttgagcacga 60600
   tgaccagcgc gacgatgaag ttgaggttca gcgtcttcgc ggactggaac agctcggtgg 60660
   ccgacgcgtg cacgtcgagc aggttcgcgg agagccgcag gaagaacacg ccgcgcttga 60720
   tctcggccgc gaagcgacgt tcgtactcct gccggcgcgc gttgatcgcg atgaggaagt 60780
   tcaggatgag ccggttgatg ttgtacttca cggcccaggt ctgcgtcttc atgatggtgt 60840
   cgaaggacat cacgatgttg aagatgaagc gctggctgtg cgagaagtag ctgtagggct 60900
   cgctgaggaa gatggacttg ttggtcgcgg gcaccaccac gcccgcgcgc gcgccggacg 60960
   cgtcggtgtt caggtccggg atgttcatgc cgcagatgcg gcagtaggcc atgccgtcct 61020
   caaagtacac gaactcctcc acgaactcgt tgatcttggc gaagtagtcc acgtccacgc 61080
   gcatcgcgac cgcgagccgg atctggtgct cgcagggcgg cgactcgaag cgcaccccct 61140
   cgccccagcc cggcggctcg cgcacgacca gcgcggtgcg cgaggccggg cggaacttgg 61200
   cgtcgcgcgc gttgagcagc gccgggaaga ggtcgcagag gtgccggctc gagaggaaca 61260
   cgtacttgta cagcagccgg cgcgcgtccg cggccatggc gtccacgaag gcgcggcccc 61320
   actccgcgac cgcgggctgc tcctccgcaa agttgttcgg gtagaccttg tccgtggccg 61380
   cgaggaacac cttcttcacg tcgaggaagt cgcggatcac gatggggacg cgcgcgccgt 61440
   cgagctcgta catgaacacg tagcgcaggt tgagcttgcg ccgcgagacc gggatgccga 61500
   tgtgccgaca caggtacgcg aactcgaggt acttcttcga gaagcggatg cggtccaggt 61560
   tcttggagac gtactgcagc atgttgcgca tgttgaaggg gatctcgcgc acggcgggct 61620
   ccgcggcgtc gtcgaaggcg gtgcgcagat cgctggtgcg ctgtacgacc acggcttcgc 61680
   cggtggcgtc gtcgtgcacc agcacgttaa cgcgccgctg ccggatgacc atgtcgaagg 61740
   tgttgaagaa catctcgtac atgctgtgcc gagtgtcgtc cgcgatgcgc tcgcccaccg 61800
   agaggctcgc ggtggcgtcg tcacgcacct gcttctcgaa cttgtacccg atgtaggaga 61860
   atatcgagat cagcgtggcg tcgtcggcgt cggggttctg ctccatggtc gcgaagagca 61920
   ggcggatgtc gtcctccgtg atcgcgtcca cgttgtacag gttgaccacg aagatggact 61980
   tgttctcggc gatgaagtcc gtgtaggact tggtggccgt gttcgggtcg cgcatgtacg 62040
   cgcggatctt cggcacgatg ctcgcgagga tggactccct ggaatccatt taaggacggc 62100
   aagggcgcgc gagaccgtct caaaactgaa atcgtataaa ctcttaaaaa atcggtattg 62160
   aaagtacgca ccaccaaata aagcgtcgag gtcgggcatg tcttcgtggc gactcaaaat 62220
   gagcaagtgt tcaggttcca gcagcgtcca gactctcgag gatctgcgta atcgtcttcg 62280
   ctccgaggcc ttgggcaacg attgccaaga gccccgcgac gacctcttcc ccagcggcga 62340
   ggagtgtctg gacatcgacg ggccctgccc ttgcgatgag gcggagcagg agatcgacca 62400
   ggagcagttg cccgtgcccg aaaccgtgcc cgaaccgccg gccaagactc ctaagcgccg 62460
   accagtgaag aaggataagg cagataaggc agataaggac aagtcgacca gaggcgcaaa 62520
   gaaaccgtgc ccttcggacg acaaggatga cgagctcaag agcaacgacg tcgacaacaa 62580
   cgaagagtcc ggcgacacag acggcggcgc gagcgcccga agccccagcg acatcgacaa 62640
   cgtggacgaa atggacgact ccgacctcat ggtggcgttc tccaccatcc tcgcagactt 62700
   caaggacctt acccaacgag tgaaagctct ttcgtccgtg ctcacggacg tgcaggcggc 62760
   cggcatacgc aggagcttct cgacgctcgg caaggctctg acggaggcgg cccacatcgc 62820
   caacaccgga tctaagccag tcactgcgcc tcgcaagaag aaggccgccg cctgcaagaa 62880
   gtaggcgcac taaatagcga ggctcggtat gcgggcgctg cacctgtcag acggcaaact 62940
   tttttttgac aaggagctga cgcagccggt ccccgacgac aaccccgcgt acgctgtcct 63000
   tgcgaagatc cggatcccac cgcacctctc ggatgtggtc gtgtacgagc aggacctcga 63060
   gtctgcgcag cagggcctca tcttcgtcgg gcgcgacgcc aagggccgaa agcagtactt 63120
   ctacgggcgc ggacacgtgg agcggcgcac ggccgtccgc aacgccgtgt tcgtgcgcgt 63180
   gcaccgcgtc atgaacaaga taaacgcctt catcgacgac cacctcgcct ccggcagcga 63240
   ggccgaggcg cagatggccg ccttcctgct catggagacg agcttcttca tccgcgtcgg 63300
   caagacgcgc tacgagcgcg agagcggcac cgtgggcatg ctcacgctgc gcaacaagca 63360
   cctcgccgag gccgagggcg gtgaggagat ccgcgtcgcc ttcgtgggca aggaccgagt 63420
   cgcgcacgag tttgccgtgc gcgaggggca gcggctcttc gcggcgctgc gtcggctctg 63480
   ggacccgggc gcgcccgaca ggctgctgtt cgaccggctg agcgagcgcc gcgtgtacac 63540
   cttcatgcga cgcttcggca tccgcgtcaa ggacctgcgc acctacggcg tgaactacac 63600
   cttcctgtac aacttctggt ccaacgtgcg ctcgctggag ccgcgtccct ccgtgaagtc 63660
   gctcatctgc acctccgtgc ggcagaccgc cgagacggtg gggcacacgc cctcgatctc 63720
   gcgcagcgcc tacatggcca ccgcggtgct cgagctcgtc agggacggcg cgttcctgga 63780
   cagagtcgcc gccaccgaca cgctcgacga cttcgtggac atcgtcgtgg actatgtaaa 63840
   taactctgag caggtaaatg gatgaggcgc tgcgcgtggc ggcgcgcgtc gtggacgggc 63900
   tccggccgct ggacgtggcc gtgtgtctcg cgcagctgcg cggagccgcg cccgagcgcc 63960
   gcttcccggc gctcgacgag tgctccggcg aggccttcct ggactttgag ttcgccggcg 64020
   gggacgtggc gtcgcggtac ctctccgcgc acacgcgcga gctccgtgcg gcggagcggc 64080
   gcgagcacat ggccgcgatc gcgcgctgcg tcaccgaggc cgacctggcg ctcgcagacc 64140
   gcccccgggg caaggcgcgc gcggcgctgc gcgtgtgccg caaccgcgag aaagtcgcgc 64200
   gcttggcgag gctgctgcgc gacgccgaga gcagcggcgc ggacttcgcc ttcatacgcg 64260
   cggccgtggc gtagcaaaac gtaaaaacaa cacattccct aaatcgccat ggacgcgcca 64320
   agtctcgact gcatgctcgc cgcactcgcg gcgaaggcgg cctcggtgga ccgaggcgct 64380
   cccgaggacg aggtgcacca cgaagtggag ctcgtgctcg tggacccgcc gctgtccacc 64440
   ctggccgcca cgctgcgcct ggcctcggag acggagtcct tcatcctctt cacggtgacc 64500
   gcgctcgcca aggaggaggg caagctgcgc gcgcgcgtgc ccatgtcgcg cgtcgtcggc 64560
   ctggacgtga agaacgtgca gctggtcaac gccatcgaca gcatcgtctg ggagcgcaag 64620
   gcgctcgtgg aggagaccgc gctgcaggaa ggctgtctgc tgcgccactc caccgagcgg 64680
   cggcacctct tcgtggacta caagaagtac ctctcggcca tccgcgtgga gctggtaaac 64740
   cgcgtgcgcg tgcgctccaa agaagtcgtc gcggacttca agttcaagta ctttctgggg 64800
   tccggcgcgc aggccaagag ctcgctgctg cacgcactca accaccccaa ggtgcggccc 64860
   tcgcccacgc tggagttcga ggtcgtcccc gcgggcgagg ccgtggacga ggccgccgtg 64920
   ctcgcggagc tgcgcgccgt ggcgaaggcg ctcttcatgg cgcccaccga cgccgtcttc 64980
   ctggcgccgc cggccgagat gccggtgcgc acgctcatgc tgcagaagca ggagatcccc 65040
   gcgctagacc tcgacggcct tttcgcggtc tccaagacgg acggcgtctc cgcgagcgtg 65100
   tgcgtggacg aggacggcgt cttctgcgcg ttctcgcacc tcgcgtacac catccggtac 65160
   ccgctcgcgc gcgaagtgca gggccggtac cggctctggt gcgaggccgt gcggcccgtg 65220
   ggcgagcgcg tgtggtccat gttcgtgctg gtcgtggagg agcctgcggg cgatgaccgc 65280
   gtcgcggccg tggccggcgc cgtggaggcg ctgcgcggcg tgtgtgcacg cgtcgagttc 65340
   aaacctaagc gcgtggacgg gcccttctcg gcgacctccg agctggtgga gcacatcaag 65400
   agcgcgctgc agacggagcc agagggcgtg gtgctcttct acgcgcgcgg agagaagtcc 65460
   aagcgcgacc tcaaggtcaa gcgcgacaac acggtggacc agaccacgaa cgtgatgttc 65520
   cggtacatgt ccagcgagcc catcgtcttc ggcgagggct ccaccttcct ggagttcaag 65580
   cggtacagca acgaccgcgg gttccccaag gagtacggcg cggggcgcat cttcctgcgc 65640
   gaggacgtgg tctaccacaa caacatctac tgcatcgagt tcacgaagac gcacctggag 65700
   gtgggcctcc gcagcgtggt cgtgcccgtg aagttcatcg gcgagttctc gcaggagggg 65760
   tacctgctgc ggccgcgcct ggccaaaacg gagtgctact tccgcaaccc ctcattctac 65820
   gggaaccagc actcggtggt gctcgagcac actcgcgacc agctgctctc ggtgggggac 65880
   gtgttcgacg agagccgcat ggccgccgtc gggcagacgc tggccaacga cgccttccgc 65940
   ctgaacccgg acacgcccta cttcaccaac cgacgcacgc gcgggccgct gggcgtgctc 66000
   tccaactacg tgaagacgct catgatatcg ctgtactgct cgaagacctt cctgaacaac 66060
   gccgagcgac gcaaggtgct ggccgtggac ttcggcaacg gcgcggacct ggagaagtac 66120
   ttcttcggcg agatcgcgtc catggtggcc acggacccgg acgcgcgcgc gatcgagcgc 66180
   gccatggagc gctacaaccg cctcaacgcg gggctgaagt cgcgctacta caagtttaac 66240
   tacatccagg agaccatccg atccgagacc tacgtggaga gcatccgcca ggtcatgtac 66300
   ttcgggcgct tcaacatcgt ggactggcag atggccatcc actactcctt ccacccgcgg 66360
   cacttcgcca cggtgatgcg caacctgcgc gagctcaccg cgcccggctg caaggtgctc 66420
   atcaccacca tggacgggga cttcctgtcg acgctctccg agaagaccag cttcgtgatc 66480
   aaccgcaacc tgcaggagag cgaaaacttc atgtcgatcg agcgcgtggc cgatgaccag 66540
   gtcatggtct acgcgccctc gaccatggcg cagcccatga cggagtacat cgtgcgccgc 66600
   gcggacatcg tcaagctctt cgcggacaac ggcttcgacc tcgtggacca cgcgaacttc 66660
   gagaccgtga tccggcgcag ccgccgcttc gtcgagggcg tctcgcggct ggagacgcgg 66720
   ccctccacca agaacttctt cgagctcaac cgcaacgcgc tcacggagat ggacagcacc 66780
   gacgtggccg cgctgctaaa gatctacgtg ctgtacgtct tcagcaagcg gtaggcagaa 66840
   ccagggcgtc gattccgcgc ccgcgccggc gcggaaggcg ttgaacagct ccgccagcca 66900
   ggctgcggtc tcgcgcgcgt cgatcgggcc gccgtcgtcc ggcggcggct cgcgcgccgc 66960
   gcgcaacacc agcgtctccg cgggcggcag aggctccaga gcctcgaaga ccgcgcggct 67020
   cgggaacagc gcgcgcatca tgcgcgcgcg gtggccgaac accgccttga ccgcgcgcag 67080
   tgccgagcgg ttgtccagcc gcagcgctcg gtcaaaacga tgcacgcgcg cgggcgcgcc 67140
   gcggtggtcg cgctccacga gcacgtgccg ccacgccagc gccgcgccga cgcggtccag 67200
   gctgggcgcg agcgccacca ggcttttcag cgcatgtaaa tctccgcgca tggccgacgg 67260
   ctccatttac tactgcggag gaacgcacgt ggtcgcggcc gcgccgggcg ccgcgcttgt 67320
   ggtgctggac gcgcccggtg cggcggcggc ggccgcgccc gcggggcagc gcgtcttctt 67380
   cgccgagtac ggcctcgaga agcgggccgg cggcccgatc acggcgcggc tgcgccgctc 67440
   cgggttccgc ggcgccgcga acgcctgggc ctccgtggcg gacttcgagg ccggcggccg 67500
   tccctccgcg tggacgctgc gcgcggagga ggcttcgcgc gtgccgctgc cgacggacgc 67560
   ggcgctggtc ctggcctggg gcgcgcgcga ggagccgctg cgggcgtgcg tgctggcgcg 67620
   cgcggcagac gcggaggcgc cggtgggcgc cgcgctcaaa gaagccgcct tcgacgcgcg 67680
   ggcgccggcg gccgcgctgt tcgcggcgct gggcgcgccc gcgctcgcgc ccccgctgcg 67740
   ggcgcggcta gtggcgccgc cgggcgcgcc gccgcggacg cggctctgcg agaacccggc 67800
   catgctgcgc gcgttcgcgg tgggctggtt cggcgcgcag ctgggcgagg cctccgaaaa 67860
   tgaaaaggta tttgccgcct ttgataaggc gaggtcgtgt ttggacgacc gctgatggcg 67920
   acgcccgcga acgcgcccgc gctgctcgtc gcggcgctgc gacaccgccc gtaccgcgtg 67980
   gagtaccacc cggactggga gccggtcatc gagacgctgg tggacgagta cgacgcggtc 68040
   gcgccctggc tgctgcgcga cgcgacgagc cccgagcccg agcgcttctt cgcgcagctg 68100
   gcgaagccgc tggcggacaa gcgagtgtgc gtgtgcggca tcgacccgta cccgcgcggc 68160
   ggcaccggcg tgcccttcca gtccccggac ttcagcaaga agaccatccg cgcgatcgcg 68220
   agctcggtcg cgcgcacgac cggcacgcag ggctacgcga actacgacct ggacgcggtt 68280
   ccgggcgtgc tgccctggaa ctactacctc tcctgccgcg agggcgagac caagagccac 68340
   gcgatgtact gggagcgcat ctcgcggctg ctgctgcagc acgtggccaa gcacgtgagc 68400
   gtgctctact gcatggggcg cacggacttc cagaacgtgc gcgcgcgcct ggacgtgccg 68460
   gtgacgctgg tggtgggctt ccaccccgcg gcgcgcgacg ggcagttcgc gcgcgagcgg 68520
   gccttcgagg tcatcaacgc cttattggag ctcaacggga agtctcaagt ggactgggcg 68580
   cgaggatttt ctttttatag tgaaaattaa tccgtggtcc taaatggcgg cgcccatatg 68640
   cgataactct cacgtgttcc tcctcaagcg cctgggcgtg ccgtcttcct gccggcgctc 68700
   ggaggacccg cgcttcgtgg agatcctgac tcccttcgag ctctcaaact acatcgagcg 68760
   gcacccggga tgctgcctct tcgagacgct gcgcgacgag gaggactgct ccgtcgtgcg 68820
   cgtcttcgcg gacgtggaca tggacagcgt gctcgaggag gaggacttcg tcgcggcgct 68880
   ggaggacctc atcgtggagc tcgcggcctt cttcgaccgc ttcgcgagcg gctcctgcgg 68940
   caccgtgccc ggcgaggtca agcgcgccat gctcgcgaac ttctcggtca cgcgatccac 69000
   ggccgagcac aagaccagct tccacctgat cttcacggag acgtacacca cgctggacac 69060
   gctggtggcg gcgaagcgcc cgctgctgga cctgtgccgg cgctcggaca acgtgctgct 69120
   gcgcgcgctg gacacggccg tgtaccgccg cggcgcgacg ctgcgcgtgg tgggcacgcg 69180
   caagacgccg gagtcgagcg cggtccaccg catgcagtcg cccgacgacg acatcaagga 69240
   ctacctgttc acgttcgtgg agctctcgga cgcgagcgtg tacttcgagc tcgcggagcg 69300
   cgagcagcac acgctgagca ccgtctgctg ggagacctcc tacatcccct tcggcgacgc 69360
   gatgcggcgc gtgtgccagg cggtggtcaa cgacatcgtg aacctccgcg acatcaccga 69420
   ggacaacttc ctcgacacgc cgctggtcat cgactacgcg acgcgctgcg cgctgtgcaa 69480
   gaagcccaag cacaagcacg cgcaccacat caccatgggc aacggctgcc tgcgcctggt 69540
   caagggcggg aacgcgcaca gctgcaaggt caagatcatc cagctcgagg gcaaccggct 69600
   cttcacggcc gcgcagatca tcatcgcgtc cgaggtcgtg aagctcaccg agcgcaacga 69660
   ctacatcgtg tggctgaaca actcctggcg cttcagcgcg gaggagtcgc tcatcaccaa 69720
   gctcatcctg gacgtgcggc actcgctgcc cgcggactac gccaacgaca tgctgtgtcc 69780
   gcgcaagcgc aaggtcgtgg agaccaacat ccgcgacatg ctcgtggaca tctccgagac 69840
   ggacacgcag tacgacaagc tgcccttcac gaacggcgtg ctggacctgg ccacgggcga 69900
   gttcctcacc ggcgaccgcg cgaaggcctg cgtgtgcacg gtctccaccg ggtacgcctt 69960
   ctcgcgcgag gagttcgcgg ccgcggcgga ctcggaggcc atgcgccggc tggtcggcgt 70020
   catcgacgac atccagccgg acacgcccga gaacgccgat aaccgcgcgc tgtacgagcg 70080
   cgccatgtcc agcgcgctct gcggcgccac gaagacggtc atcgtcttct tctacggcga 70140
   caccatgacc ggcaagtcca cgagcaagcg tctgctcatg tccgcgctcg gcggactctt 70200
   catcgagacc gggcagaccg tgctcacgga cgtgctcgac aagggcccga accccttcgt 70260
   ggccaacatg cacctgcggc gcgcggtctt ctgcagcgag ctcccggact tcgcctgcaa 70320
   caacgcgcgc aagctgcgct ccgacaactt caagaagctg accgagccct gcatcgtggg 70380
   ccggccctgc ttctccaaca agatccacaa ccgcaaccac gccaccttca tcatcgacac 70440
   caactaccgc ccggtcttcg accgcgtgga caacgcgctc atgcgccgcg tggcgctggt 70500
   gcgcttccgc acgcacttct cctcgtcggc cactcgcgcg gccgccgcgc acaacgtcga 70560
   gtacagcgcg gtcaaggaga tggacgagag cctggacacc aagatccagc gcaactactt 70620
   ccgctacgcc ttcctgcgcc tgctcgtgca gtggttcggc aagtaccacg tcccgcaggt 70680
   ctcgctggcg cccacgcccg acgcggtccc cgacttcgcc ttccaccgcc gcgtggccga 70740
   gctggtggtg gccagcaacg acgcgcaccg ccgcgcgatg gagtcgctgt ccaagctggg 70800
   gtacgtgctc gtgggcggca acgtggccat gcccgcggac gccttccggc agcggctggc 70860
   cgcgcacttc aacgcgcgcg tgcacggcgg cgacatagac gccttcatgt tcaagcacaa 70920
   gaaggtcgtc aacgtaacgg aggagtacgt ggagtacgta ttcatcgaag atgtcgagaa 70980
   taaatagacg ggtatgaact cggacgtgat aaagctgttc gtcgggcacg acgagtccgt 71040
   gcccggcatc ctgccgcacc agctcgcgac cgtggacttc ctgatacgcc gcgttctaga 71100
   cgacaacgtc agcgtgcttc tcttccacat catgggctct gggaagaccg tcatcgcgct 71160
   gctgttcgcg atggtggcct cgcgcaccaa gaaggtgtac atcctggtgc ccaacgtgaa 71220
   cgtcatgaac atattcaact acagcatggt catggtcgct aacctgttca acgcgccctt 71280
   cgtggccgag aacatattcg tgtactcgac gactagtttt tattcgctaa actgcaacga 71340
   cggcgtcata aactacaacg gcctcggcaa gtacgagaac tcggtcttcg tggtcgacga 71400
   ggcgcacaac atcttcggga acaacaccgg cgagctcatg atggtgatca agaacaagac 71460
   gcgcgtgccc ttcctgctgc tctcggcctc gccgatcacg aacacgccgc tcacgctcag 71520
   cagcatcatc agcctcatgt ccgagaagga cgtggacgtc ggcgacatcg tggtgcaggg 71580
   caagaaggtg ttccagatcc tgctgaacga gcacggcgtg cgcgtgatcc gcgaggtgct 71640
   caaggggcgc atctcctact acgagatgcc ggacacggac atgcccgagg tgctctacca 71700
   cgggcgccgc ttcctggaca cgcgcgtggt ctactgccgc atgtcgcgcc ggcaggagga 71760
   cgactacctc accgtgcgcc ggctctgcaa caacgagatg ttcgagaaga acatgaacaa 71820
   cgtgtccatg gcggtgctgg gcccgctgaa cctggtgaac aacctggacg tgctcttcca 71880
   ggcgcaggac aaggacctgt acccgaacct gcgcatcagc aacggcgtgc tctacgggaa 71940
   cgagctcacc aagctggaca tcagctgcaa gttcaagttc ttcatctcga aggtgggcgc 72000
   catgcgcggg aagcacttca tctacttctc caactcgacc tacggcagcc tggtcatccg 72060
   caacgtgatg ctcagcaacg ggtactcgga gttcggcggc tcgcagagca acaatccgca 72120
   caccacgccc gacgggcgcg ccaagacctt cgcgatcgtg accagcaaga tgaaggcctc 72180
   gctggaggag ctgctcgagg tgtacaactc cgcggagaac aacgacggtg gcaagctcat 72240
   gttcctcttc tcctcgaaca tcatgtccga gtcctacacg ctcaaggagg tgcggcacat 72300
   ctggttcatg accatccccg acaccttctc gcagttcaac cagatcctgg gccgcgccgt 72360
   gcgcaagttc tcctacgcgg acgtggccgc gcccgtgaac gtgtacctca tggcggcggt 72420
   gtactcggac ttcgacgagg acatcgtctc gctggaggac tacagcgtgg aggacatcaa 72480
   cgcgctgccc ttcgacgtga agaagctctt ctacctcaag ttcaaggcca aggaaaccaa 72540
   ccgcgtgtac gccatcctgc aggagctctc ggacgcgtac tccgcgcgcc cgcacccgca 72600
   gctcgtggac gtggtgctgg gggagatcgt gcgccagttc ttcgcacggc actgccgcgt 72660
   gcccgccgag gacgccgcgc tcgtggccgc cgtcgaggcc gttctcggca cgcgcgaggc 72720
   agcggccgag tacatccgcg cgatagtgga cggacacttc ttcgtgacca acaagacctt 72780
   cgggaagtgc ctgctcttcc ggcacgagcg cgacatcgtg accgtgccct tcgagctcga 72840
   gcacgacccc ttcgcgtggg cgatcaactt ccgcaaggag gtcagtgtgg tgaatatata 72900
   acggcaaaca taaatagaaa gactgtcctt ttgcgcgatg tcgaccttcc ggcagacggt 72960
   gtacctggcg gtgacgctgc agccgcacga gctcacgctc gacttccgcg gcaacgtcgc 73020
   ggaggcggtc atgcgcgagt acctctacaa ggagaagggc gggctcatgg ccaccgacat 73080
   cgaggtctgc ctcggaaacg agatgccgct ggggcgcatc gtgaacaacg cggttgtggt 73140
   ctcggtgccc tgcaacgtga ccttcaagta ctaccgcgtc ggcgacaccg tgagcggcac 73200
   gctcaacgtc gaggacgaga ccaacgtctt cgtggactgc ggagacctca tctgccagct 73260
   cggcaagagc tcgggcggcg tgaccttcaa cgagtccaag tactgcctcg tgcgcaacgg 73320
   agtcgtctac gagcacggca gccgggtctc ggctgtgctg cgcgaggcgc gctccggacg 73380
   cgagtccgcg ttcgtgttct ccgcagtgct gctggacggc gtccccgccg aggagaagga 73440
   cgagaagaag gacgagggcg agaaggccgc ggaggaggag acgcccgcga gccccgccgc 73500
   caaaaactag cattattggg ccgcgcgaac cttcgataaa tgcgcacgta cacgtcgctg 73560
   ctctcgaagc tgctcaagag caaccggcgg ctcgggagca cgcgcgtctt ccgcgacccg 73620
   ctgcagcaca tcagcgcgac cgcctttgtg caccggcgca tcgaccggca ccggcgcgtc 73680
   tccatctgcg ccgtgctcac caccaccgac gggctcgtgg tcgcgtgccg gcgccggtac 73740
   tcctttttgt cctccgagct cgcggagacg cgctcgcccg cgcggcgcgt gctgctcgca 73800
   accaagcacg cggacgctct cgcgcgcctc ggcgccgcgc gcccgcgcga cgacgtcatg 73860
   tttccgggcg gcgccccgct gtccggggag tcgccgctgg cgtgcgtgct gcgcgaggtc 73920
   gaggaggaga ccgggctgcg cggcgaccag gtcagcgtgg acgagcggct gttcgtgcac 73980
   gccttcatcg acgacctggt ctcgggccgc gacttcgacg cgatcatctt cacgggcgca 74040
   gtcgcgcttt cgagcgcgga ggtggcgaag cagttccggc ccaacgacga ggtcaagggg 74100
   ctggtcttcc tgcaccccga ggacgcggag ggcgtgggcg tgatggcgcg gctggcggcg 74160
   ttcgcgcgct gcgcggcgcg cctgcgctgc tggggcgcgg ccgtcacgcg atagaggcgg 74220
   ggtccaccac gtacacgagg cgcccgccgc tcacgcgcac ggtgggcggg tcgcccagcg 74280
   cggtcaggaa gttcccgtcg tcgtcgaaga ggcgcccgcc gcgctcgagg aagcccttgc 74340
   gcaccgtgac cagcgccgtg gaggtggagt accacacgct ctgcccgtcc gcgagccgcg 74400
   ccgcgcgcgc gggcccgcgc gcgtccgccg ggcgcgccac cagcgcggac cagccggagt 74460
   cgtcctccag cggcgcgaag tccgtgaagg cctcgcgcac ccactccagc gagcagcgct 74520
   tgagcacgcg gaagagctgc gtgaactgcc gggacttgtc gcggatgagg gccagcaggt 74580
   cctcgtccac ggtggcggcg ccggagtcct ggcgcgcgac cacgaagtgc acgttcacgt 74640
   agcggcggtc gggcggcgtc atctcgtggc tgttcaggcg caccgcgcgg cccacgatct 74700
   ggcgcagcga ggcctcgttc caggtcatgt ccaggatgaa gatgtcgttg atggagagga 74760
   agctgaggcc ctcggagccg ctcagcgaga acacacagac cttgatcttc tcgccgtcgg 74820
   tgttgtcgca ggcgttgaag gcgtccacga gcttggcgcg cgtgtcgcgc gtgcgcgagg 74880
   agaactccac gctggagacg ccgaaggcgc ggaagtagag cagcagcatc tcgatgccgg 74940
   tcacgttgac gaagggctcg aagaccagac acttgcccgg cgaggccagg atgcgcaggc 75000
   agacctcggt gtacttgcag ctgcgctcgc gcagctccgc gagcagcgag acgtccgcgg 75060
   aggtcatgcg gtcgccgctg acgggcgcgc cgctgcggaa gagccgcatg gccgcctccg 75120
   agaagacgcg gtccttgacg gcgcgcgcga agtccaggaa gagcgcggcc acggcctcgt 75180
   cgtactcctg cttggagagc acggacttgt cgggcgcgtc ctcgaaggcg aaggtggccg 75240
   cgatgcgccg gtacacgcgg aagaccgcgg cgccggactt gcgctccatg gcggccgcgc 75300
   ggcggtaggc ctcggtctgc ttcgcggtca tgtccacgta catcatgcgc acgcgcttgc 75360
   gcgcgaaggc ggcggagccg tcgacgtcgt cgaagatgga ggcctcgttg gtgactaagt 75420
   acgagcacag gccgccgagc ttgtccacga ggtcctcggg gttcgcgagc gcgccgccgt 75480
   tgaagagcgg cgtctgcccg accacgccgg ggcgcagcag gttcacggcc atggagaact 75540
   ccttgacgct gttcaccacc ggcgtggccg tgaggcagag cagcttcccg cggcccatgg 75600
   ggatgttctt cgcgaggtag ttgtacaccg tgcgcgcggg ccgctggcgc ccgtcctcct 75660
   tggtcagcga catcgagatg aagttgtgga actcgtcgat gaccacgcag acgcggctgc 75720
   tcgacgaggc ggtcttcatc agcgtgaaga agcggtggtg gaagcgcggg tcgtcgtagt 75780
   tgatgaaggt gcacccgggc acggcctcgg gcgcgaagcg catcatcgtc gaggtccagg 75840
   gctgctccac gagcgccttc ttcacgagca cgaccaccgt ccagtccgtg aagacgtcgc 75900
   gcaggtgctt gagcacgtac accgcggtca cggtcttgcc cacgcccgtc tcgtggaaga 75960
   gcagcagcga gtgcatgctg tccaggccca ggaacacgcg cgccacgaag agctggtagt 76020
   ccttgaggcg cacggactcc tccacgccct gcatctcgga gggcatgtgc gcggtgcgcc 76080
   gcagcgcgta gtcgatgtag gccgcgtgcg cgctggtcat ggcgacggtc ggcgctcctt 76140
   ttacggggtc tgtcgtctat ctattgtcgg cgcgggtctg atttaggggc agtagttaca 76200
   aaaacgtttc cgctgctcgg cgcggcgttt ggaggagcgg ttgcggccgc ggcggcgcag 76260
   gcgcgcgcgg cgcgtcttcg tggtgcggtg gccgaaccag cgccggtgca tgaccgggtg 76320
   cgagaccgcg gccgcgcgat ccgcgctcat gcaggttgcg taggtgcggc acatgctgcg 76380
   cagcacgcgc cgcgtgcgcc gctccacggc gtcgagccgc ctcgcgacga tgggaaagag 76440
   ccggcgccag ccgcgcacgg cgaagagcgg gcgctcgcag accgggcgcg cgagcgcgtg 76500
   gtaggcgccc agcagccgcg ggtccagcga gcgcacgtag gtctccacga agccgttgcc 76560
   gaagacgatg gcctgtgcgc atagcgggtt cgtcatctcc ctcttggagg cgatggcgtc 76620
   gcccacgaag gcgcgcacgc cgcagtgccg cagcaccagg cgccgccgcg ggaagtgcag 76680
   gtgcgggccg agcgccgcgc gggcggcggg gatgtgcagc cgcggagaaa aacgcgcgcg 76740
   tccctccatg gcatctaagc gctccgtctg ttttcagtta tatcgccgcg ggcggctact 76800
   gcagcagcag cttgagcttg cgctggctct cgttctcgat gctcttggac tcggaggtca 76860
   tgctctcgta gagcagcgag tgcgtgacgt agagcgcctc gtacacgcgg ctggcgaagg 76920
   ccacgaagcg gtccacgaac tcgctctcta cggggtcctt gagcacgcgg aagggcacgg 76980
   ccagcgcgtc gcgccaggcg gcggccttgg tgcgctcgcg cacgtgcgtc acgaaggcgg 77040
   cgatggccgc gcgccgcggc tcgctggcga ccatgacggc gctgtccttg agccagctgt 77100
   cgctcacgca cttgaagagg cgcacggtgc cgaagaggct gcagtacacg cgcagcgcgt 77160
   gcaccacgtc ggtgccgaag agcgtgggca gcttgagcac cacgaagcgc tcctgcgtga 77220
   tctcgagcag cgggcgcatc accgcgaagg tgatcgcgtg gtagtcagcc acgtagaggt 77280
   tgttctcggt gaggtggttg ttggagcgga tggcgccgcg ctccttgcgg tagagccggt 77340
   tgcgcgcgct gaggtcgagc acgaccgcgt cggccctgcc gcgcgctctg gagcgcacgc 77400
   tggtgatgcc gtgcgcctcg agcaccttct cgacgtcgcg ctcgtcgatc atgaggtcgt 77460
   gcgtgtacag actcagcatc tccgtgggca tgcggttgat gtcgttcacg cgcgagcact 77520
   gcaggaagta gttggtcccg taggccaggc tgggcaggtg cccgacgccg agctgcaggt 77580
   ccagcgcggg cgtcgagtcg aaggtgggca gcgtcacgct gagcccctcg cggatgctgc 77640
   ggcgcacggc ctccaccgcg tccatggccg atttattgga cgcacagtct gttttcattt 77700
   cgcggctact gcgcagtcac cttctcggcc acgatccccg cgtcgtagct gagccggtac 77760
   acctcgttgc acaccacgac catctgccgc ggcacgtaca tgagcgggtt gtgcgcctcc 77820
   atgtgcgcgg tggtcacgcg caccgccagc ttgtccttgc ccctggagac gttggagttc 77880
   agcgcggtgg gcgagaagaa ggtgctgggc gtaaagttga actgcagcgt gcgcacgccg 77940
   ggcgtcttgc cgaggatctc gccgaagacg cgcgagactg cgctgttctc cgagtacagc 78000
   acctcgttgc cgaagcgcac gtccatgcgc gcgatgacgt cgatcttgtt cttgaagtcc 78060
   acgcccttga ggaaggggtc ggccacgaag aggtccttgg cgcgcgcctc cggcgagcgg 78120
   ttgtcgccgt tgtacacgtt gcgctggcag gtccacacgc ccacgggcac ggaggcgtcg 78180
   ccgatgttca cggagtggat ggcggtcgtg aagcggatgc gggaggtcgc gcggctgtag 78240
   gcccccgtga tggcggagaa cttcttggac atgttgtaca cgacggagtt cttcctggtg 78300
   gcgaacacta ggatgttcgt gtgcaggaac acgcgcatgc ccacgggcac gtcgtcgatg 78360
   cgcacgaaga cgtcggtgtc ctggatggac acgacgcccg acgggggcac ctcgactatc 78420
   tccgcggtct cggggaagcc ctcggggtag cagttcgaga cgatcaccat gtcctccagc 78480
   aggcgctcca cgaaggccat cacgaagtcg ccctcggact gctggaagcc ggggtacgat 78540
   atgaagcggt tgttggcgtc gctgagcacg ggcttcatgt acacggacag ggaggtgcac 78600
   gcgtgcacgt ccgtgatcac cgcggtggtg tggttgatct gctccacgcg ccgccgcggc 78660
   atctcgatga aggccgggcg cgggcacagg ttcttgacca tgtagccgat gaagctcagc 78720
   tccatggagt aggggaactc cttggcgagc ttggcggcgt cgaaggtctc gtcgtagacc 78780
   atgacgcagg cgacggggtt cagcgtgacc gtgaccgtga ccttgctgtc gctgagcttg 78840
   agcgtgctga aggtcttgtc cgcgtcaaag ggcgtcttga tgtaggcgtg cacgcaggcg 78900
   gcctccttga tgacgtcgtt gggcgagctc ccggtggaga ggtcgttgag ctcgcgcgag 78960
   aagcccgaga gctccatcac gcgctcgttg tccaggcagg agtcgaacag ctcctcgccg 79020
   gaggtctccc agatggtgtc cgcggcggag ttcacggcca cgtggcggat gagcttgtac 79080
   gcgatgtagg gcacgtagca catcttgccc acgcccttta tctcgggcag gtccacgctc 79140
   agcacgaagt tgttcatggc cgagatgtac ttgtcgcgga tctcgaaggt cacggtgacc 79200
   gcgtcgctgg tggtgtccac cacgccctgc gtggtgatgt actgcggcat gtacaccgtg 79260
   ggcgcgcggt ggtccgtggc gaacacgctg gcgcgccgca cggcgtcgtc gccgcccacc 79320
   aggctcacca cggagttatt catttattcc ctgggaaaac cagttaaata aggctcttca 79380
   gagccatgcg caccgtccgg ccgtcgggct ccaggtagca gcgcccgtag acgccctccg 79440
   tggcgcgcgt ctcgttgatg agcgcgcgca cgcggtcggg gtccgcgtac atctccagcg 79500
   gcagcagctc gatcttgggc tcctcgcgca gcgcgacgag gtgccggatg gagcccgcga 79560
   aggagtcgcg gcacaaccgc gagcagaact cgcccacggc gccgccgtcg agcgtctcca 79620
   cggccagcgc ggccgtgccc acgcgctgac ggcagaacca gcacgtgccg tccgcggcgc 79680
   gcagcgccag ccgctccgcg gacaccgtgt tgaagtactt cggcagcacg tactcgatgc 79740
   ggcacgccgc cggcggcgcg caggccgacg cccgcggcgc ggatatgtcc acccgcgaga 79800
   gcgcgatgcg cttcatgggc ggcggtggct gctatttatg tcgcccgcgg cttttcaaag 79860
   gtcgagcgag cacgccgcga agcgcgcggg cgagaacacg tactcgtggc cgaactccgg 79920
   gatctgcgcg gcgcgcttgc gcgcgcgcat gtgcgcgagg aagttctccc aggtgagctg 79980
   gttgctgttg ttcttcgcgt agttcttcac ggtctgcgga cgcaggttgc gcgtcacgcc 80040
   cgtgacctcg aagatcttgt ccaggaagaa ggagtagttg atggttttgg tgggcgtgat 80100
   ctcctggcag aagaagacca gctgcttgaa tatctcgatg acctcgttga tcttctcggt 80160
   gctgaggtcc agcttctcgt ttttgacctg gttgatgatc tcgaagacca gcttgtagtc 80220
   cttcttgttg atcatttcgc tgtccttgag gaagctggag acgtagttgg cgtccacgtc 80280
   ctcgggccgg atctggtgcc ggtccatcat cgcgcgcagg tcgcggatga cctcctccga 80340
   gcactgcttg gagagcagcc gccggagcac gttccgcagg tggatgagct tgttcgacac 80400
   gtggaagttg gacctcttct gcacgcggat gcccatggga aacacggtct cgcagaacag 80460
   gcagaactcg tagtccgcgt cggacacgag cccgttgcgg cggcagccgc cgcacatgcg 80520
   caggttcatg cttgctccag ccccagcacg cgcaggatct cgcggtccag cactttagtg 80580
   tccagcgtgc gggttctaca gaactggagg aagcccgcga gcgcgcgcgc gcgccctggc 80640
   tgcgagagca gcagcatgcg cgcgttctcg gggtcctcgt tgatgacgcg cgtgaggttc 80700
   agcgagcacc gcgtgcagcg ccgcggcggg tcgagctcga ccgagtacgc cgcgaaccag 80760
   acgttgtcgc ccatgtatta tttattaaca cagaacgtcg cacatgttgc gcgaggacat 80820
   gtacgggtcg tactcctgcc cgtagatgag gatggtgcag taccgcgaga tcatgagcat 80880
   ggcctcctcc atggtgagca ggtcgtcctc gaacatggcc ttgtgctgca tgtgctggct 80940
   ctgcttggcg gccatcgcgg ccgcgccgtc gccgcgcagc cactcgttca gacactggcc 81000
   gtcgccgccg gcgccgctct cctgcgcgaa ggtgttgcgc atggcgcgca tgagcctggc 81060
   ctccctggcc gagcggctga gcacggtcat ggggtcgtag agccaggggc ctgcgtccgt 81120
   gaacaggatg gtgcagtacc cgttggcgaa ggcgtccgcg ccgctgcagt tgtcgatgcc 81180
   gtcgccgacc ctgtagcaca ccgcggagac cagccggtac atgatgccgt tgagcatcat 81240
   gtcctgcgac acctcgatgg gaatgtcgct gatcacgggc cgcatgttcg tgaagcagtc 81300
   gcccgtgctg gccatgcccc cgcgccggtt caccaggaac accagcacgc cgttcgtgat 81360
   cacgggcgcg cggtcgcgct cgtagaggta gccgctcgcc gcgcacacgg cggacgccac 81420
   gtccgtgcgc gagaccgcgc ccatgttctg ggcgggcatg tacagcactc gcccggcctc 81480
   cgcggtgcac gagaagggct gctccccgcc cacgtggatg ggcgccgtcg atgtcgtgat 81540
   catcttgctg gagtccacca ccaggtaggg caccgtgtgc atggccatgt cgcccatgcc 81600
   cccgatcccc gttccgaacg acggccgcga cacgctcacg agcgtcggct tgaacgagac 81660
   tatggagaag atggaggcca agatctgctc ctcgtcggtc atgatggagg cgcacgaggg 81720
   gtggatgatc ttcattaggg cgttgtcgat ggactcgtcg ctctcgcagt agaagacgcc 81780
   catgcggagg ttcaggatgc accgccgcag gttggtgtgc agcaccgcgc gctggatctc 81840
   catggacacg gagtcgccga cgccgggcat cacgatgggc gtttcctcgg tgagcttgtt 81900
   caccagcagc tggtagttgt tgggtcgcac gcggctgttg tggtggagct gcgccaggag 81960
   cgagaggctg tccccgttga cgaacgcgga ctcgatggcc ggcagcttta cgccgaacag 82020
   cgccatggcg atggggtgca cgaagcccac ggagtcggac gacttgaacg agaagagcag 82080
   gtcgcccgag gagctcatgt ctttgaagtg cacggactgg aactgcgtgg cggagagcaa 82140
   gttctggtag ctggacatgc tctgcaggtc gtcgatctcc ttcatctgct tgtttacgcg 82200
   ggtcgagttc ctcccgtaga tgatcaccag cgggtgcgtc tggctcacgg atagccccga 82260
   gtccgtcatg gcggcgcgca cgctgttcag cagatcgaac agctccgacc ggtctctgtt 82320
   ctggatcccg acctttgcca tcacagacat gagctcctgg atggtcatgt tcttgtggtc 82380
   tcggcgcgtg gaccgcaggt agtcggcgat catctcgccc tccttacgga tcttcatctg 82440
   ccagtcgtgc atggaggtca tgcggtccac aggcatgagc acgctgtcgg aggacgactg 82500
   cgcggcgctg ccggactggc gcgagccagg gcgcgcggac gacgggcgcg cggagctgcc 82560
   gcggctggag gaggacctgg acctccgcga ggatcttcgc tgcgaagagc tgcgcacggg 82620
   ccgctgggcg cgcgccccgg cggaaaccat gtcctcgcgg tttatgctga ggagcgagct 82680
   gcagaccgcg cacgacagcg actgctttgg aatgtggatg tggtcgcact ccagggacat 82740
   gcccgcattg tcgtagcccg ggaccaagtc gaactttgca ttaaaaaaat ctgatgcgca 82800
   cgcgggcgat tccatttata ccggaagttt ttatgaggtg ccggtattat ccacgcgatc 82860
   tcgcagtgtg ctgggagact ctagcgtagc cacggacccc gtgagcagac gacgcaagtc 82920
   gttgatggcc gactgcgtga cggacttcgc cgtctcgatg tcgcgcgtaa ggctgagcga 82980
   ctcggcgttg aggtcgcgca cgctgtccgc gatgtcggcc agctcctttt tgatgaaatc 83040
   cttatcatta tcggcgttga tgactttgtc cggcactcta gactctagaa ccggtgacgc 83100
   ggcgggcgcc ttgatcgtcg ggcagctgga cgccgggtac tggggcggcg gcaatgattg 83160
   ctgtaggaag atgggcttag cggcaggcgc cggttttgtc ggcaagggcg gcgccggcgg 83220
   gcactgtcgt gtagacggcg ggcacgccgg cgcggggcac gccgccggtg gcggacatgt 83280
   cggcgcagtt gcgggtggac acgcgggcgc gggcgccggc gcgggacacg tcgcggcagg 83340
   agccgggcac gcgggagccg gagctggagc cgggcacggc gcggcaggag ccgggcacgt 83400
   cgcggcaggc gcagggcacg cgggagccgg agccgggcac gccggcgcag gaggacatgc 83460
   cggcgcagcg gcaggacaga ccaccgctgt cgcggagcac gcggcaaccg gcgtggagca 83520
   cgcggcgggc attacgttca gaggcgtcga ctggaccttg gcaccgcggg gcagtagcga 83580
   tggcgctagg ggcgactgtc cggtggttgg acgctgcatg caggcagtcg cagatttcag 83640
   acgggactgg taatacctgc ccgcttcctt caccgtgtac ttgtcgacgg agtctacctc 83700
   ctcatctgga ggacatggct gctccggtgc gggaacgact gtttgaggac acttggtgaa 83760
   cagactggag ctggtctccg atagcaccag tttagacttg gccaagtcgg aggcaaacct 83820
   tcttctgaga tccatttaag ccttcaaaat tgaacgtgta cgccgaccgc taaatggaag 83880
   aatcggtggc cgtcgagtac gcggacgagg acgaagatga gattgaggag tacgaggagg 83940
   aggacgatga cgaggaggaa gagtctgccg agggcgccgc cgcctcctcg gtcagcgacg 84000
   tagcgctctc tgccgccgag aagctggtgg cctcggaggt cccggacgac gcggctgccg 84060
   cggacaccaa cgtgcgtcaa cgcgtcaccg cgcgcgtgga ggagcttaag gcgcgctaca 84120
   cacggcggat gagtctattt gagctcaccg gaattgtagc agagagtttc aatcttctgt 84180
   gtcgcgggcg gctgccgctc gtggcggacg ccgcagaccc ggcgctcgac aacgagctaa 84240
   aagtggtggt tcgggagctc gaggagggcg tctgccccat cgtcatcgag aaaaacggcg 84300
   agttcctctc gccgggcgac ttcgaccccg agtgcctgcg ctaccacctg acgtacatga 84360
   ccgacctctg gaagtcccag gggcgcatgt agccgcggct acgccgactc ggcggcctcc 84420
   gcgatttttt cttttatcat gtccagcagc tcgcgcacca cgatggggcg gccgcagtac 84480
   gtgatgccgt tttcggatat cacgctctgt gcgatgtcca ccagcgagcc ctcgcgctcc 84540
   cagtactcgc gcgcgagcac ctccttgtac aacgcgcggt ggttggccac gtaccgcacc 84600
   agcgtctgga tgttcttcac gcccacgctc ttgaggtcct gcggcgagaa cttctcgcgc 84660
   agcgacacga agacgtcccg gacgagcttg ccgatctcca cgttggtctt gaactcgttg 84720
   tacagcacca cgtagagctt gcacacgacc gtagcgaact tcgcgggctt gagatccttg 84780
   ttctggaaga ccagcatgct gctcatcacc ttcttcatga aggccaggta cttcgcgcgg 84840
   tcgccctcga cgctcacgct cccgacctcg aggtcggaca cgcagcggat tccgtgctcc 84900
   gcgctctccg cggagacgcg cagcagctcc tggtactcct tgagcttctg cttgtccgtc 84960
   atcagcgagt tgtcgaatac cgccaccagc ttgagcacgt agttctcgtc cgagaagacc 85020
   ttgttcagac acttcaccag gaagctgtag tggctctgca ggatcttcat gaccgcgttg 85080
   gcgccgctgg ctccgcggac gtgcgatatc atctccatga tcttcttaga gtcgtcgatg 85140
   atctcctcgg tgtcgttgcg catgttgcgg tacattgcgt tcagcgagac cagcgtctgc 85200
   gcggccagga gcacgtcgcg gaacacgcgc gcgaactcgc gcttctcctc cgcgtcggtg 85260
   atgctgttgt acacggactt cgccaccgcg ttcgacttca ggaaccagaa ggagagcgcc 85320
   tggtagttga agtgcttcat cagcgccagc acgtccgcct cgctcatttc cggcgcaatg 85380
   gggcacaccg agctctcgag cacgggcacc atgctgacga gcgtgtccac gtccgtgtcg 85440
   aagtccaggc agtccacgca gagcccggtg ccgcggctca ggtgatcgcg gctgatgtcg 85500
   tagaagcgct cgtagcaggt gcggaggcgg tccatgtcgg ctgcgtttta gagagacaca 85560
   cactcttgaa ttatggctgc gggtagaact cctgcagcag cgccggcgca cgcgcggagt 85620
   ccggctccac tcccagcttc agcgcgcagt tcacggacca ggtcttcatg aagcggtcgg 85680
   gcgcgtccgt gaccacgtgc cggaagagct tcgcgaagtg gcggctcacg gcgttgggca 85740
   cggtcgcgtt gcgcacgaag gccgtgaagc gcgaggtcag cttcggcgcg aagcgcttgc 85800
   cgtccacgaa gaagcccgag gtggtgagcg agagcccgtt ctcctcgcgc accacgcgtc 85860
   gcgcggcctt gtgcggaaac atgctcgcga ggcgcccgct cgcgtcctgg tctaggtgga 85920
   tggcgtccgt ggccgcgtcc ttgcggatgc gcaccacgtc gtgcacgatc tcctggatga 85980
   ggatgcgcgt ggccgcggtc tccgccagcc gcatggggaa gtagaccatg tccccggaga 86040
   tgagcacgtt cccgctagcg tttacgtagc tcactatctc ggacacggtg cgcagacgca 86100
   cgatcgcgcc ttcgcagcag tgcaccacgt agtacccagc ggtggcgcgc aggcgcttgt 86160
   tgtccgcctc gaagtccgcc tccaacccct cgttgaagta cttgtcgaat atgatgggca 86220
   ggaaggatag ttttgactcg gtgaccacct ttccgaagtt gaggatgtac gggttcagcg 86280
   cgctgcggtc gacctcttcg tcgtacacgc aggacttgaa ggtgtcggtg tgcgcctggc 86340
   tgcgcaggaa gcagcacgga atgcagatgc gctgcaggcg gtggaagatg gagaggaagc 86400
   ccacgctgtt gtagcgcccg tcggggtcca tgcacgaaaa catgacgccg tttccgttta 86460
   cgaagacctc gcgcgtctcg gacttgaaga agttgttgct gaccttggcc atgttcgcgt 86520
   ccagcgactg cacgatcacg ggcttgcggt tcttggtctt ggtgttctgg cagatgcgcg 86580
   accagtacac ggtctccacc ttggtgaagt ccgaggactg cttcacgttg ttgaacatca 86640
   cgctgatggc cacgatcaag aacgtgaagt acttctcgat gttcgggatg tagttcttta 86700
   ccttcacgga cacgtgcgac ttcgcgagga tgatcgagat gcgcttgtcc gtggagagca 86760
   ggatgttgtt ggtcgccgtc tccacgaaga tgaagctcgt ttccatgtcc agcttcatct 86820
   tagacgtgat ggtcgtgttc agcgacacct tgtaggtgat gtcgcccttg acgcggtcca 86880
   tctttacgtc catgctctcg atgagcttcg tgaacagact cacgtcgttc accgtgagcg 86940
   tcttcccgtc gctcgagatg accaggtcgc cttccgggcc ccacaccgag aggttcagcg 87000
   gctcgtccac cagcaggaag cgagtccccg tcatcgacac gaagaagtcg tccgtcttcg 87060
   agagcaggat gtcgaagtcg cccacctccg ctcgcttgtc cggcgactgc tgcgcgatcg 87120
   cgcggagccc ggactcgcgc aggttcgtgc ggaagatgtt gttgaacttg gtctccacgt 87180
   tcatgtttag gtcgaggttc gcgaactcgc ggatgagccg ctcctcgaac ttgaggatgg 87240
   agtcgttggg ctcctcgaag gagccgaact ccggcgcgga ggtgtccgcc gcgcgcgcca 87300
   cccagaccac caggaagttg cacgcgtccg cgtacgcgtt gtagaggatg ccgtccgtgc 87360
   ggatgagcgt tttcttttgc gtgggcgaga acgggttgaa gatggtgttg tccacgtagc 87420
   tgtactccag gttgttcttg tgcgagtaca cgatgatctc gtcctgcagg cccagcaggc 87480
   tccccaagta ccccttgagc tgccgcacgc gcatggtcag caagatgtgt ctgcgcacgt 87540
   gctcggggtc cttctggatg tactgcttcg cgaagaagta gatcggcgag gcctcgtcca 87600
   cggagtcgta cagcgatagg tacagcacgc gctcgatctc ctggtggcgc cccaccagca 87660
   ccaccagctg cggcgcgacg gtgtagagca tgttcgcgcg ggcgtattta tagccggcgt 87720
   taaactgaaa taaaatacgc gggtcgcgag gcagcgccat gttccagccg gtgcccgaca 87780
   tggccgccga ggccgacatc gacctcggcg acgtcagcgt ggacgcgacg cgcgcgggcg 87840
   cgcgcgagaa gaccgtcttc ttcgcgcgca acaagcgcat gtacccgcac cgcagcaagg 87900
   acgaggagcg caagctgtcg ctgggcttct tcttgcagcg gctggacttc ctcacgtcgc 87960
   gcgaggtcaa cctgcagttc cggtcgctgg acgcgctgcg caccgagaac gtcatgaaga 88020
   agaacaacgt gctcgtggcg ccgtacatcc tcatcgcgac gctcgcgggg cgcggtttcc 88080
   gcatgacgga gaccatggtc gagctctact tccccgagct gtaccgcgag accagcaagc 88140
   gcttccgctt ctgcgcgcag ataaaggtca tccaggactt cctggggttc gcccacgaca 88200
   gctaccacac ttacgacttc gagacgtact tcgcgttcgt ggcgctggtg ctgcgcggcg 88260
   cggactctgc ggccgaggcc ttcgacgtcc gcgccgagag cgggcttgtg cgcagcctca 88320
   ccgagatcac gtaccggctc tacgtgatgc agctgcgctc cgacgccgcg cagtggagcg 88380
   tgagcaccgg cgccgtagtc tcgcaggcgg tgaacaccgt gctgtcggtc gtcggcgacc 88440
   tcgctgcgcg cgcggaggcc gagcggctca cgcccgtgtg cgacctcgcg cgcgagaacc 88500
   cgctctcgct cgaggacctg cgcaagtacg gcccgcggct gcgctcgctg ctcacgacca 88560
   tggcgcgcgc gcgatccttc aagacgaacc ggcgggacaa ggacgcgctg tcccggttct 88620
   gccgactgac ggcgggccct agcccgtctg cgtgccgcgc gtcgccatag gcgtcggcgc 88680
   gcgctcgccg ccggaacact cggggtcgct gaacatgtag atgagcgcga cgcctagcag 88740
   caggtacatg atcatgctga tcacggtttt gaacacgacg gcggcgaacg tgttggaccg 88800
   cagtcggtgc tcgcagaagt gcatgaacag gtgccgcatg aggtcgatgg ccccgttggc 88860
   cacctggaaa agggcgaggc cgccgatgga cttgatcacc gtcacgtagc acggccgcat 88920
   tccgacgacg ctatttactc actgtcaaaa gaaacggcgc catccgaccg gaggttgagg 88980
   ttgcgcttca tgttgttcca gtacatctca ccgatgctcg agtagtacgc cgtcagccgc 89040
   gatatttttt ctcgcaccag ctcgtaggcc ttctgcatct ccgcaacgcc gatctccgcg 89100
   tcgcccacgt accggccgct gcggcgcacg atcagcagca gcgccttcag gttctccagc 89160
   gcgatcatgt ccatgtacag cgacttcgag agctgcacga agaggttgta ccgctccagg 89220
   atgctgttct tcacctcgtc cgcgatcggg actccgaaga tgcgctccgt ggtgtacacg 89280
   gactgcgtga gctgcttgaa gagcgcggag atgcagcagg tcgcgcgctt gacggcgtcg 89340
   agctgcttct cggagcgcgc gctcgcgatg ctcagcgcgc tgttcacgac gttgctcgtg 89400
   tcgcgcacgt agcgcgtctt cagcgcggcg ttgatggcat ccgcgatctc gttgctgctc 89460
   acgctcgagt cgtccgagct gcccgagacc tcgtccagca gccccgagat cgtgatgtcg 89520
   ggcgagccgc cgacggtcac cagacggtcg agcaggttgc agggcatgga catgaggatg 89580
   ccctcgctcg agaggcagcc ctcgtcgatc atgctctgca ggttgcgctt gaaggccgtg 89640
   ttttcgggca tgaacccgtc cacgctcatg agctcgtcga ccgtgctcgc ggaaaagatg 89700
   cccctcacgt tgatgcggtc cagcatgccc atgtcctgcg agcacagcac caccgactgg 89760
   tcggccgtct cggcggcgtc cttggcgccg ccgtagatga tgcgcggaaa ccgccagctc 89820
   gccggaaaag agaaggaggg aaaccggcac tgcgcgctcg ggcctcggta gccctgcgcg 89880
   tcgcgcacgt tggtggccgt gaccatgaac tgcagcaggt cgtgcgcgga cgccatgatc 89940
   ttctccacct cctccttgct gcagcagacc ttgcccaggc tgcgcgcgat gttcgttttg 90000
   ctcactgagg gcgagaccgt gacggcggtg tgtcggcggc tgccgagcgt gtacgcgctc 90060
   acgctaacgc ggtaccccat ggcgccgaag agcagcttca cgaagtccag gtagctctcc 90120
   ttattgatgt agtgcggcgc gcccttgtct tccatcctca gcccggcgta ggccatgagc 90180
   acttccttca tcgccgtctc ggggtccgag ttgcacacca gccgcagcat ctggaagaac 90240
   tgcatgaagg cgcgctgcga gagcccgatg tggtggttgg gctgcgtcga ccggcgcggg 90300
   aactccctgg gcgtcatggc gttgatgccc gagagcgtct ccatcacgag cgcgcccacg 90360
   gtcttctggc ccatgacgcg cgggtaaaag cacacgcgga ggggctcctt gccggccgcg 90420
   agcgcgtccg agagcagcga gcagtacgtg acgttgtcgt ggtcgaagag cgcgaaggtg 90480
   tagcagacgg agctcatgaa gagcgagtcg gcggtgctca tggacttgaa ctccgtgtac 90540
   gcgattccgt cccagaacag gctctttccg ggcgcgatca gcggggacgc gcggtcggcg 90600
   cgcatcagca tggagagcag cgtcacgtag taacggatgt tggcggaaat gtctacgaac 90660
   tgcatgccgg gcgaggccac gcgcagggtc gcgcccgagg tagtgagcac ctccaggctg 90720
   tccatgagcg tcacgctggg gtgcagctgc gcaaggcgcg ccagctggct ctggtagaag 90780
   atggacacgg cgaggctggc cacgctgccg cgtgccatgc gcaggttttg cccgttgaag 90840
   gtgagctggc gcagcgagaa cacggagtcg aagtactgga agaaggtgag caggtacttg 90900
   agcggcatgg tcgtcagctc ggtatccacc tgcggcgtct gtgcgagcac gattccgttc 90960
   ttggccgcgg cgtcggggat gtcgtacatg gcgtccattc tggcgcggga ggcgtcggtg 91020
   agcagcgcgc gcacgttgag cagcatgagc aggtcccgcg ctagcatggt cccgtcgacc 91080
   agccgggcgc gaaagccgat ctcggcgggg ccggcgatgt tggggtagat caggttcagc 91140
   aggtacgtgt tgtcgaagct cagcgagggg aaggagatgg gagacttcgc cgggaggccg 91200
   gtggggtagc gcacgtagcc gccgcagatg cgcgcgtgcg cctcaaagct ggtcacgcga 91260
   gtcttcagca ggttgcgggt gaagggcggc acgtccttga aggactgcgt gcagatcacg 91320
   gggttggcgg tgtcggtcag cttgaggttg gtgggcttaa gctccgcgaa gttggggccc 91380
   agcagcacgg ggacgaggtg cgagttggcg gcgctgtcga gcaggaagtt gatgccgaac 91440
   tgcttcacgg cgacctcggt ttcctcgtcg ctggcgagct tctccgcgtc ctcgaggaag 91500
   agcgcgtcca gcgggtgcac gtacgtgcgg ttgacgtcgt agctgggctt gaagtccgaa 91560
   cacagcgtgg ggagcacggt ggagacgagc tggaacatgt attccgcgcc ctccacatgg 91620
   tgcaaggcca tgtgcacgtt tggggccgtc atttatttag tattaaatga cggccgtacc 91680
   ggtaaccgat attcctggag actacgggcc gacgtccttt tcggaggaca actacccgct 91740
   gaacaagcac tacgagctca ccaaaggcca gctctcgatc ctgcgcacgg tcaacgacaa 91800
   gctgctcgcg cgcaccgtgc agcactcgga cggggagagc gatgagagcg aaagcgagga 91860
   ggacgacatc tccagtccgc tgccgccgga cgaggaggag ccggactcgt gcgtggcccg 91920
   ggtcatgccg cgggacgcgg acctggcggc gccaaaaaag gccgacggct acatcattgc 91980
   cgccgagcag cagcgccagc agcgcataaa cattctggta tccgatcgag aggccgtcgt 92040
   ggagcgggag ccggttcaga cgtcgttcgc gcgcgtctcg gctatcccga tccacgggga 92100
   cggcgcgcgc cgcaccaccg cctccttctc cgcgaccacg ccgtcgctgg gcgccgtgtt 92160
   cgacgacgcc aagcgcgtgc ggctgctgga ggaggaggtc aaggagctcc gcagaaagtg 92220
   cgcgacctct caggataacg gaaacctgga gaacttcacc aaggtgctgt tcggcaaggc 92280
   gccgcgcgcg agcgagctga acaagcgcgt ggtcatcgtg aactacgcca cgctgaacaa 92340
   cgtgacgctg tccatggagg acctcgagaa gtgctccgac gaggaagtgg accgcatgta 92400
   ctcggtcatc cggcgctaca acgagacgcg gaagaagaag atcctggtca cgaacgtggt 92460
   catcatcggg atcaccgtgc tcgagcacgt gctggtgaag cttggcttct cggaggtgcg 92520
   cgggctcagc gccgacctct cgtcggagct catcgacgtg gagatcggcg aggactgcga 92580
   gcacatcgcg gagcgcctgg ggttcgggaa cagcccggtg ctaaacgtgg cgctcttcgt 92640
   ggtaaagctg ttcgtgcgga agctgaacct gatctgatca acacatgccg ccgtcgaggt 92700
   ccatggcgtt catgaggttg gaggcgcggc ggcgcgcgcc ggtggaagcg gtggaggcgc 92760
   tcgaggtcgt ggagcaggga gtgttgctgg aggaggcgcg gcggcgggag ctagaagcgg 92820
   aactcgaggt tccgctggtg gtgctgcggc gactcgtgcc gctcgtgccg ctcctgctag 92880
   tgccagtgcc agtgccgctg cggcgtgaag taccggtgcc ggacctgccg ctggagcttt 92940
   tcttgcggcc gccgttaacg ctgtcgatgc cgagcaggtc ctcgcacacc tcgccgacgg 93000
   ttccctgcac gtccaacttg ccgttcttga caaccccgta cacgatcttg ccgcagttgg 93060
   acacagcctg gatggtggtc tcgtcgctgt caaaggcgtt cattccgccg cacccgccgt 93120
   cgttgtttct tcgagaaggc gcgccgctgc ggcgactcct ggtgctgccg ctggaccgag 93180
   ttccggagga cctggagccc gtggaccggc tgccggtcga cctggtgccg gtagtgcgct 93240
   ttctggacga agaggaggag gcgcttccgc ggcgggtgga cgaactagcc tccagcgcac 93300
   cggcgccgcc cacacaatcc acgtcggcgg cggcgcctcc gcgaatgacc tgctcgttgt 93360
   tgagctgcgt caggagagat cgcagctgcg gcgcgatctt ctgcaaggtg ctcacgtagt 93420
   cgtcgtagct gctctgcggg cgctgcgcca ttttttcgga cgccatttat tacgcggaat 93480
   atctacgacg acgcagcact gaatcggttt ctcgcgacgg gagattccgc ggtcggcgcc 93540
   ggtgcggggt tgtcaccggg cgacgaggta accagcgcgt ggaaggcgcg cacctggtcg 93600
   tccgtcatct tgtcctcgaa cgaggacgcg cccgggggga gcaggtcctt gttgcgcgga 93660
   acggcgggcg ccgagacgca cgaccggcgg tacatcatga tgacgatgta gcacacgatc 93720
   gagatgacga tcacggtcag cagcgcgtcg aggagcccca tttattacct gtatatgccc 93780
   gcgtttaccg ggcggtgagc tcaatgtcgg tgttgtttag ccgggcgtac gggacgctgc 93840
   cggagcactt cctgtacatg ctgaacacga acagcccgag cagcagcacg gcgcccacta 93900
   tgaagcaggt tacgcacagc gcgcgccaca cgtagtcggt gacgttgttg gtgttcttgc 93960
   tgaaatccac gaaggcgaag acgcaggcgg ccgtcagcag cagcacgccg catatcagca 94020
   ctccggagta gtaagagctc aaggtctcga atatgtccat ttatctgagg agaaatttaa 94080
   attactgaat ggacgaagtg gaatagaaac cacgagaaca cgacggactg cagcacgaag 94140
   atggtgctca gcttcgtctt catgggcatg cagaagttcg cggccagcgc catacagaag 94200
   atgaacacga gcaccgccgg gtcgtagtcg gacaccattt acactacgct aaaaggcata 94260
   tctcggcgcg cgacgtccac gagcaccagc acgcggacgc ccgcgggcgc gccggcggcg 94320
   accgcggcga gctgcccggc cgtggggttc accagcagca gtgcgcgcgc ggttcgcggg 94380
   acggggtctt cgtaggccat ggtcggcgtg gacccgggac gcagcggccg cccctgtctg 94440
   tcgaagaggc cctcgggaaa cgaggtgccc ggaacggcca cgacgacggt gtcgctatct 94500
   agaaacattt atggtcttgg tttccacgga tcgcctcgag tagaccgcca cgaagtagaa 94560
   gatgacgccc gccgcgagcg ccgccaccag gaagggcggc acggcgggca ggttcgcgga 94620
   cgcgttgtcg cgcacgccgg ggtccgggtc tgcgtagccc gcgcccacgg ccttgccgca 94680
   gtcggcgatc atgtgcgcgc gcgagttctg catgaccagg ctgtccacgt cgatgcggca 94740
   gcccacgtag cggcaccgcg agcgctgctc gtcctggctg aagaagagcc acttgcggtc 94800
   gcgcgactgg tccgtgcact cgtgcgcgcg acagacgcgc gggcccaggt acttcccgag 94860
   cgtggtgccc gcgacacacg cgcactccgg cgcggcgcgg tgcgcgtcgc agtagcgccg 94920
   cagcgcggag tcgccgaagg cgaaggaggc gggccgcgcc acgcgcacga actccgagca 94980
   gaagcgcgcg tccatgtgct tggcgcagag cgccgcgtag gtgtccagcg ccgcgtagcg 95040
   gcccgtgcgc agccaggcca tgcactcggg cgcgtcaggc tccaccgcgc agcggctggc 95100
   cataacgccg tcgcagtgcg cggtcttgta cccgttcgcg aacacggacg ggcacccggg 95160
   ctccggattt gtgcagcagc gcgccatggc ggcgtccgtg ggcggcgccg aggcgccgat 95220
   ctcgaacgcg cacatggtgc cctggcgcag gtacggcttc gcgatctcgg gaacgtagtc 95280
   tgcgcgcagc agcgagcccg ggcggaagaa gagcgagtcg cagggcgggc ctcgcacgag 95340
   ccgcgcgcgg ctcgccagct ctggcgagag gaagcgcccg cactgcccgg ggtccatggt 95400
   cggcagcaga cagaaccgcg gccgtacggt cttcagcttc gggtcggaga aggtttctga 95460
   ttcttccgcg aaggcgaagg tgtccgtggc gctcgtgtgt gtgacgcgca gcgcgtactc 95520
   gcctggcgtc ggcgtgtcga gcacctccac cttggatacg gtgtccccca tttgaagacg 95580
   ctatttacgc cgctgcctac tcggcgaaga ataggtcctc cgacttggcg cccgcgtaca 95640
   ccgggcaggc gggcgcggcg gagcgagtgc gcacgatacc gcggccagtg aggcggaagg 95700
   cgtagatggc gaacagcagg ccgagcacga tgtacatgaa agtggtggcg cccacagacc 95760
   cggtcacgtg cgtcacgatg atggtgacga tggacatgat cgtgcacacg atggccatgc 95820
   cggtgttgtt ggccgcgtag gggtgcatga tctgcatggc cgcacagtat ccgatgacca 95880
   ggcacggcag cgggaggata agtgaggcaa tacctatcat tactagagcg agcacggggg 95940
   tggacgtcaa ggccaataca aaaatcacaa tacctgttag tatgcggata tcctcgtact 96000
   ggaggacgct gtaaggcgcg atattccctc cgggcactgg cctggggtta gccgggacta 96060
   ggggggagtc ggcagtgccg gggtccttgg ggagaaaggc attctgctcc tccgggctga 96120
   agagctcggc gtcctgaacg ccgccggcgg tgaactcgtc gttatagtaa ctaaagtagc 96180
   tttccattta tatgttgaaa aatgtttgga ggcgtacagg tggacgacaa actctacgcg 96240
   tacctaaaaa aactcgccgg acgcgggcgg ccgctgtgtc tgttccgcga caacggcgag 96300
   ttcgtcgaag tcttcgcggg gtccgcgttc cgctttgtgc tgcccgtggg cctcttcgcg 96360
   gacctgcgcg tgcgcacgcg cggcgtggcc ttcccgaaac tgcgcgactc cgcgcgcatg 96420
   cgcggcgtgc gggtggacgc gcacacgctg ccctcgctgt accccaacca gcgcatcgtg 96480
   gtggacgagg tgctcgcggc ccgcgaccag ttgctggccg cgggccgcgc cgtgtacgtg 96540
   acgctgcatc tggcttgcgg cttcgggaag acgctgaccg cgtgccacct catcgccacg 96600
   cacggccgcc gcgcggtggt gtgcgtgccc aaccgcatgc tggtgccgca gtggcgcgcg 96660
   gccgtggcgg agctgcgggt gcccttcgcg gtctcctgtg acggcgcggc ctcgctgctg 96720
   cgctcgggcg agctcgaccg cgccatggtg gccatcgtgg tcagccggca cttcgccaac 96780
   gacgacttct gccgcgcggt gagccggcag tttgacgtgc tcgtgctcga cgagtcgcac 96840
   acatacaacc tcatgaacaa caccgcggtc tcgcgcttct tgaccaagta cccgccgccc 96900
   atgtgcttct tcctgaccgc gacgccgcgc acggccaacc gcatctactg caaccgcgtg 96960
   gtgaacgtgt ccgtggtcag ccgcctcacc aaggtagtgc gcgtggtgga cgccttcttc 97020
   gagccgtaca ccacgcccaa gatccgcacg ctcgagcgca gcctcgaggg accccagaac 97080
   aagtaccacg tcttcaccga gaagatcctc ggcgaggacg tgcaccgcaa caagctcatc 97140
   gtggacaccg tggtcgcggc catggccgcg ggcgaggcgc ggcgcgtgct cgtgctcacc 97200
   aagctgcgcg aacacatggt cgggctgcac gccgcgctct gcgagcgcct cggtgcggag 97260
   acggtctttc tcggcgacgc caagaacagg aagacgcccg aggtcacgcg cgcactgcgc 97320
   gacaaggacc gcttcgtgct cgtgtccacg gtcttcttct cgggcacggg cctggacctg 97380
   cccaacctgg acgcgctcgc ggtggccgcg gccgtgctca accgcatggt catggagcag 97440
   atgatcggac gcgtgtgtcg cgagtcgcac gccaacacgc gcacgctgtt cgtgttcccg 97500
   gactcctccg tgcgcgcgat ccgcgacacc gtgtctgcgt ttgcgcagcg gctcgtggcg 97560
   ctggcggtgg acgggctggg cttcgtccgc gagcgcgccg ccgccggcgc gaagaacgag 97620
   ccggcgctgt acagcgccat cagcgggcga gatctcgcag cggtgtaagc gcggacccgc 97680
   acgccgcgca cgagagcgtg ctggagcagg cgagtcccag cgacagtgtg gacagcctgt 97740
   ccacgtcctt gatgctcacc agccgcgagt tgcacgacga gcacacgggg tcgctactat 97800
   catcgaccac cgtggtgacg cggcggcgtc tgcgcttttt gtttccagcg gcgacatcga 97860
   ccacgcctcc cttagagccc cccttcgccc ccgccttagc tttcaccgcg ctcatctttt 97920
   atttatcata aaaacacgtc tgcgtacgcg ttcgcgcaca cgtcccgcaa atccgcgcgc 97980
   gcgccgcagc gcgtgaagcg cgcggcgtcc gcctccgcga tccgcgcgca cggcagcggc 98040
   gcgcccttct cgtccgccat cacgcgcgca gagatcccgg tggcccccag cgcgtacgac 98100
   accaccacgt cgccgacgca gcggtacacg ttgccggagc cggcgaggcg gtcgaacgcg 98160
   gcgccctcct ggcgcagctt gtcgaatatg cgaggaacga ggatgttaaa aatgagaacg 98220
   aaatagcaga tcagcaaaaa cagcgagatc atgacctccg agagcgattt atataccttg 98280
   aaagagctaa tacgacttcg ggactcgctg cacctcgcca ccggcgccgc cgtcgagcgc 98340
   tacaacgcgc tcgtggagtg ggccgcgcgc acgtactgga cggtcgcggt gctgccctcc 98400
   gcgccgtgcg cctccatcga gaagtactac tgcgtgtgca aacccgactg cgcgctcgag 98460
   cccggcgagt actccgtgag ccggctgcac ttcggactca cgcacgcctg ggtgcgcggc 98520
   gccgccttca actcggccag cggcgccgag gtcgagccgc cagaggaggt gcgtagggcc 98580
   tgcgaggcgc tcgacgccgc cttcgcggac ctcaccttcg tgcgcttctc ggtcttcggc 98640
   cgcgagtgga cggtcgacga cgccgtcaca gaccactcct cgcgcgacga ggtgctcgcc 98700
   gcgtgcgccg cctccggcgt gcgcgtcgcg cgcacgctgc gtgtgcgcgt gcgggcggga 98760
   gagtccttcg cgcgcgcaga cttcgacgcg gtgcacgccg cgctgcgcgc ggagggcgac 98820
   gtcgctcgcg gcaccgcggt ctgtctcgcg ctgcgcgggt catcgcgccg ctggatagcg 98880
   gaccgcgcgc ctcgatgctt catgcgcgtg cgccgcgtgg agctcgagcc cgtggacgcg 98940
   cggcaccact gcccggtgct gatctccgcg cgcggcgacc gggtgctctg ccgcggcgtg 99000
   gggcacctcg cggacgcgcg cgcgcgcgag ggcgtcttcg tggccgtgcg caggtacccg 99060
   gagtgtctgg tgctctgcga cgaggcggcc gccggcgcgg cggagtgctc gcgcgaggag 99120
   gcgctgcggc tgctggtgcg ccgcttcggg cgcgacttcg ccgtcagcga ggagggctac 99180
   gtcttccgcg tgcaggacat ggacttgcgc ggcgtgtccg cgcgactggg gctcgcgccc 99240
   tgcgcgagcc tggaggagct gcgccgagcg gtggagcgcg accgcgcgct gatgaggcgg 99300
   ctgcgcgcgg agggcgccgt gcgcctcgcg tgcgagtgcg tgggataccc gcgccagaac 99360
   gcggtggagc tcataaataa tatgcgcttt caaataacgg aagaaggcgc ggtggcgaac 99420
   tttgagctgg cgaacgcgag ctgtctcggc aacccgaccg cggagtccat cttcgcgagc 99480
   ttcgcgcagt tcgtgccggt cttcaacgtg ctctcggcga tcgcgcgcgc gcagccatga 99540
   tcgtggcggc cttcgacctt ggcacgcgca accccgcgcg caccgtgctg gaggtgctcg 99600
   acggcacggt gcgcgtggtg gacgtggcca agctggactg gagccgcgac tgggagaagc 99660
   gcgtgcaccg cgacgtgacc gccttccccg cgaacgtggt gctcgtggag cgccagtgca 99720
   agatgtcgcc tttttctaag ttcatatact tcatacgcgg gctgctctac gacgggcggc 99780
   gccgcacgcg cgtgctcgcg gtgccgccgg ccatgaccgg cagcacctac cggcagcgca 99840
   agcgccgctc ggtgcgcacc ttcctcgcgc tcgcggagag cttcggcatc ctggacgccg 99900
   tgcccgcgcg gaagaagctc gacgacgtcg cggacagctt caacatggcc atcaattacg 99960
   tgctccgaac aaactgaaat acgactggaa cgaataagtc atgctggcgc tgttcgagtt 100020
   cctgcggtcc gtggaggact gctaccggcg caccatcttc aacttccaca tcgcgcacag 100080
   cgccgaggcg ggcgatgtct acggcgtgct gcgcgaccgc attttggcgg ccacgcgctt 100140
   cgaggaggta gcgccgccag ggctcgcgga cgcgctggcc aaggtggtct actgcgacat 100200
   aagcaccacc aagcacctgg tcaaccacgc ggccttcgcg gcgcgcgcgc ggccggcgcg 100260
   gcgcggaggc agcctcgcgc agttcttcga cgtgcacgtg ggcgaggacg cggagagccg 100320
   ccgcaccgcg gagatcttcg accgcgagcg ctcctcgctg gtctcgtacg tgaagaccac 100380
   ggccaagcgc tgcaagatcg actacggcga gatcaagcgc accatccacg gcgggcggca 100440
   gacctacttc tcggggcggc gctcggacga cttcctgagc accaccgtgc gcgcggaccc 100500
   gagcaagccc tggatcaagt ccatctccaa gcagctgcgc gtggacatcc tgcaccacgc 100560
   gatctgcacg cgcggcaaga gctccatcct gcagaccatc gagatcgtgc tcacgaaccg 100620
   cacctgcgtg aagatattca aggactcgac catgcacata atcctctcca aggacgaccg 100680
   cgagcgcggg ctcgcggacc tcgcggacaa gctcttcggg acctacgcga ccaccttccg 100740
   cgtcatcgcg gccatcaccg gcaacgcctg cttcgcggcg gtggcggacg cggccgcgcg 100800
   cgtggtcgcg ctcccggacg cggacgcgaa gctggcggcg gtgcgcgggc tcgcggagtg 100860
   ctacggcgtg cgcaacttca aaatcggcat gttcaacctc accttcacgg gcgccatcga 100920
   gcacacggtc ttcccctcgc tgatccccgc ggagagcaag atcaagttct tcaagggcaa 100980
   gaagcttaac atcgtcgcgg tgcgctccac cgaggagggc cgcgagtgcg tggagcaggc 101040
   gcaggcgctg ctcgcggcca tgcgcgagcg ctccgcgcgg ctcgcggccg cggacgtggc 101100
   caccgcgagc gtggacttcc tcaaggagct gctggggcca tagtgaaata atactgattt 101160
   cttaaatatg gagcaggcgc tcggatacaa gtttttgttg cccgacccca aggacgacgt 101220
   ctactaccgc ccgctccact tccagtatga gtcatacgcc aacttcatca agcaccggct 101280
   taaggacatc ctcacggtgc ggcgcacgct gctcaccttc aagaacggca ccgagtccat 101340
   cgtgctcgag atcgacgacg tgaagatctc ggcgccggag ttctcgccca tcgtggccag 101400
   catcaagggc cacagctacg aggcgctggt caccttcacg gtgaacatct accggcacgt 101460
   gatgaccaag gacggcctca ccgttaccaa gatcaacagc tacgagggca ccgactcgca 101520
   cctcgtcaag ctcccgctgc tcatcggcta cgggaacaag aacgcgctgg acccctccaa 101580
   gttcgtggtc ccgaacgcca tcggcggcgt cttcatcaac aagcagtcca tcgagaagct 101640
   cggcatcaac atgatcgaga agatcaccac ctggcccaag ttccgcgccg tgaaggccaa 101700
   ctccttcacg ctctccttct cctcgatctc gcccgtgcac gtgatgcccg cgcggtaccg 101760
   acactacaag atcctgctcg acgtgaacca gcccgacaac ttcgtgatct cctccgcgaa 101820
   gaccttcatc accgtgaacg tgatcgtgat ggtgcagttc ctcgcggacg tcacgctcga 101880
   gttcgtggcg cgcaacctct gcttcgacat gccgcccgag gccgcgcacc tggccaccgc 101940
   gctcgtggag agcgcgaaga ccgtgcccgc gggcgcggac gtggccgagt acgtgaacgc 102000
   gctcatcgcg gccgagcacg cgaagcagaa gtcgacgctg tccaaggagg agttccgcta 102060
   cgagatgctc agcaacttcc tcccgcacat gcaggacagc gccaaccagc tcaagggcct 102120
   gtacctgctc tcgctggtgc gcaagatggt cttctgcgtg ttcttcccga accggtaccc 102180
   ggaccgcgac tcgctggtct gccaccgcgt gtacacctac gggcgctact tcgaggcgct 102240
   ggccatggac gagctcgaga cctacatcgg gaacatccgc aacgacatcc tcgcgaacca 102300
   caagaaccgc ggcacctgca ccgtgaacat ccacgtgctg accacgcccg gcttcaacca 102360
   cgccttcgcg gcgctgctca gcggcaagtt ccgcaagtcc gacggcagct tccgcacgca 102420
   cccgcactac tcctggatgc agagcatctc catcccgcgc agcgtgggct tctaccccga 102480
   gcaggtcaag atctcgaaga tgttcaaggt gcgcatgtac caccccagcc agtacggctt 102540
   cttctgcgcc tcggacgtgc ccgagcgcgg gccgcaggtc gggctcatct cgcagctctc 102600
   cgtgctcgcc tccatctcga acatccgcac cgcggacttc gtcgagctca ccaagcgcgt 102660
   ctgcgactac gtgcgctcct accccgcgcg cgacatcagc tacttcgaga ccgggttcgc 102720
   ggtcaccgtc gagaacgcgc tcgtggcctc gctgaacccc gcgatcgtgg acgcgttcgt 102780
   gctcgacctg cgccggcgca agcggctcgg cttcttcggg aaccgcgaga tcggcgtcgc 102840
   gctcgtgcgc gaccgcatga acgaggtgcg catcaacttc ggcgcgggcc ggctcatccg 102900
   cccgctgctc gtggtcgaga acggcgtgct cgtcatggac gcggaggcgg agcggctcga 102960
   gcgcgacctc gccgcgatga ccttctcgga cgtgctgcgc gagttcccgc acgtgatcga 103020
   gatcgtggac gtggagcagt tcagcttcag caacgtctgc gactccgtgc agcgcttccg 103080
   cacgctgccg cccgaggagc gcgcgctctt cgacttctgc gacttcccgg ccgagttccg 103140
   cgacgggtac gtggcctcct cgctcgtggg catcaaccac aactccgcgc cgcgcgccat 103200
   cctcggctgc gcgcaggcca agcaggccat ctcctgcctg agcgcggacc tgcgcaacaa 103260
   ggtcgacaac ggcatccacc tcatgttcgc ggagcggccc atcgtggtca gcaaggcgct 103320
   ggagacctcc aagatcgcgg acaactgctt cgggcaccac gtcaccatcg cgctcatgtc 103380
   cttccgcggc atgaaccagg aggacggcat catcctgaag cggcagttcg cggagcgcgg 103440
   cgggctcgac atcctcacct gcaagaagta ccaggtcgag atcccgctcg agaacttcaa 103500
   caaccgcgag cgcgtgcgct ccgcggcgta ctccaagatc gacgtcaacg gcgtggtgcg 103560
   cctgaacgcc ttcctcgagc agggcgacgc catcgcgcgg aacgtgtcct cgcgcacgct 103620
   cgacgacgac ttcgtcgccg acaaccagat cagcttcgac atcgcggagc ggtactcgga 103680
   catctacgcc gcgcgcgtgg agcgcgtgca ggccgacctc accgacaagg tcaaggtgcg 103740
   cgcgctgacc gtgcgcgagc gccgcgccat cctcggggac aagttcacca cgcgcaccag 103800
   ccagaagggc acggtcgcgt acgtggccga cgagactgag ctgccctacg acgagaacgg 103860
   gatcgcgccg gacgtgatca tcaactcgac ctccatcttc tcgcggaaga cgctctccat 103920
   gctcatggag gtcatcctca ccacggccta cgggcacaag cccttcgccg aggacggctc 103980
   caaccgcccg atctgcttcc ccagcaccaa cgagaccgac ttcgagacct acatcgagtt 104040
   cgcgcggcgc tgctacgcgc tctcgcaccc cgaggccgcc gcggacgacc ccgagttcga 104100
   gcaccgcgtc ttctgcgagc gcgtgctctt cgaccccgag accgacgagc ccttcgcggc 104160
   gcgcgtcttc ttcgggccgc tgtactacct gcgtctgcgg cacctcacgc tggacaaggc 104220
   cacggtgcgc tgccgcgggc gcaagaccaa gctcatccgg caggccaacg agggccgccg 104280
   ccgcggcggc ggcatcaaga tcggcgagat ggagcgcgac tgcatgatct cgcacggcgc 104340
   ggccttcacc gtcgccgaga tcctgcgcga ctccgaggag gacgcgcagg aggtgctcgt 104400
   ctgcgagaac tgcggcgaca tcgcggcgcg gctcaacggc acgcacgtct gcatccgctg 104460
   ctccaagatg agcctctcgc cggtgctcac gcgcatggac tccacgcacg tgagcaaggt 104520
   cttcaccacg cagatgaacg cgcgcggcat aaagatccgc gtggagttcg agaagcagga 104580
   cccctgcttc tacgggactc cgaaacggtt cagcctcgcg cccgacgagt cgctgttctc 104640
   gccggaggac tgaacccgcc gtcgcgaccg cgtcgcgacg actagcttat cgttcgactg 104700
   atgcgaaacg cgcggcggcg ccgcgactta gcttatctcg actgatgcga acgcgcgacc 104760
   tctcgcgact ttctagcttc tcagactgat gctaccatat cgcggcgtgc tggccccacc 104820
   accagggctt ctcgccgtgg ctgacgcggg gctggctgcg acgcgcgctg cagtagctgc 104880
   gcgcgcccca gtcgccgcgc acgtgcgccg ggggcaggct cccgtccagc gcgtgccgcg 104940
   tcacctcggc gccgggccgg cggcacgtgt gcacgtccgt cttgttggag acgagcaccg 105000
   cgtactgccg catggtctct atgtgatgct ccaagtgctt gcccgccatc cggttggact 105060
   cgcagcacgt ttttgcttcg gctaaggttt tttctagagg ggatagtagc ttatccacgc 105120
   gctcgggcag gacgcacgcg gagccgtcga accctacttt gaacggggtc accttgatgt 105180
   tcccgtcgta gcggtcccac agcatcctga ggtaggttgt accgtcgggg tctgggtctg 105240
   tccacactct aagcttttcg ctacagcggc cgtcgtacgt aagacggtct ctacgctcgt 105300
   agtagtttct gcttatgttg ttggggtctc catgctcgta gtagtataaa tcgtacgcgc 105360
   ctggcttttt taagtcgttt tcgtcgttgc tgacgtgtat cacgtcggga taataggata 105420
   tcctaactgc actacaatct atagtatttg gtctagtaag ctgttcgaga tcaccttgtt 105480
   catcatgatc tactgatttg tacacggcac cgtcgtgttc cgacggacgt atgaatatgt 105540
   ccatggtaaa cgatgtaccc actttggaaa acgtatccca tgcagtaaag catagtccgt 105600
   ccattataaa ctcaggaaca ctcataacaa atcgaaatct gtgaagtttt tcgaacacca 105660
   cttttacatg gtctttgtca cgaacatcat tgccgtttac ttcagacatg aattgaagga 105720
   acgctaaaga gtttcttgtt tcttcatgaa tctttccatt atacgtccat ccagtttcta 105780
   gaattctata tatgcttttt gcatcgaccc cgtaccacca gtacatggga actccgaaat 105840
   atatagctgg gtttgagtac caatgggcaa gagtgcccat tgcgtttaaa aagtcttgac 105900
   aaaaaaatgc agtttttctg tcgataactt gacttggact acgttcgtgg acatcgtaca 105960
   tgtccataat tggttcattg gtaacggtta catgacccgt cattatcttt ttaacaatca 106020
   taagatacag tttgcctaaa gtcgaaatat gtataacgtt aatttttaca tgttctccta 106080
   acgtaattgc gtttttactt agccattcgt cgtctacaaa aatcttacga tacataggat 106140
   ttctctctac gtatcttcta aagtatagat ttaccggtct accggcgaca ttagcgccat 106200
   ctatagcagg agcaagctgt atgtatcgtc gtataatgtc tcgtataagc tttctgtctt 106260
   ctctgggaat acacgacacg gaacttagag actggtgcca gtgtctttca accaaagact 106320
   tgaacctagc aaccaacgcg ttgtcactct ccatttataa ttaaataatt atcccaactt 106380
   cgtatgttaa tccttattac cagatagcac cgctccttcc tctccaccac gtactatcta 106440
   aaggatacct gtaagggtaa tgtctggata acgggcgtgt gagccaagac gtgttatggt 106500
   gtcttcccca ccaacggtcc acttctctaa ctaccggagt gctagacgtt gtcgatccca 106560
   ctactgttgt ttctccatta cctgtaatct ttgaagcgca acaagtgtta gtctttgcca 106620
   aatcttctaa cggcttaatt aggtcgttaa gtctgtcaat atccatgcac tgaggtgtat 106680
   cgttaccagt ttccccaact ttgggaggaa cttcctgttt agtgtccaaa taacccataa 106740
   acctgtctct aagcattctt ctgtacgtgc ttttgtcatt gtctatgtct cccaaaaacc 106800
   tgtgatttac gcatccgttg tacgtaagtc tgtcccttcg ctctcgctcg ttatttccta 106860
   cttcttctat tgtactgtaa tgttgatagt ccaagtaata gccactgttt tcatgatttc 106920
   ttgtaaatat aatcggtgtt ttattattga catcgtgttg cctactgtac gtatcttcca 106980
   tggatctagg aacttgtcta gaaaattgag gactagaaat acgccttcca aatcctggat 107040
   gataaaccaa acgcaatgca ctacagtcga catcgtcact gtctctagtt atcccatcaa 107100
   caactccttc tttgtgatgc tccactgttt taaagttaac tcctctgtat atgttgagtg 107160
   ttaaaaaaat atccacattg aacgcagttc catattttgt tattttatcc cacgacgtat 107220
   aacacaaacc atccataatg aattccggaa ccgatactac aaaattagta tcgaacttat 107280
   caaacgaaat gtttactcgc ggttgtgctt cgtatttttt gtcatacaca tctgtcatgt 107340
   attgtacaaa atctatagct cctctagcat cgggcatgct aacgtctgga aaatcatttt 107400
   ttaactgttc tagtacgaac tgttgatttt ggtcatctct ccaccagtac attggcaatc 107460
   ctatcacaag agacttattt ttataataat ttgctacaga agctatatgc cacatgaaat 107520
   tatagcaaaa ataatccatc tgtatgttta aaaccggttt actagtctgc tgagagtagc 107580
   tatccatgat agtgtttccc tcgccaatac ttcctgacat tattctgtaa acaaccatta 107640
   acaaaaatct tcctaccgtt gttatgttgt caaaagttct tatgttttga gcactttcga 107700
   gtaaccatat gttattttca aatatacttt tgtaaactgg atttctgtcc acataactct 107760
   ttaaaaatag atttactgga aggccgcttg ggttatctcc ctgtataggc ggcgcttgct 107820
   ttatgtattc gcgtaacaaa tctcttacaa cttttctagt tcttctaggt atacatgacc 107880
   tattattcaa aggctttgtc cagttagcgt ttataaacgt tgtaaagtca ctgactagct 107940
   tctccattta taattaaata attacagacg gcaacacagc ggttatctaa tatctgctgt 108000
   atcctgtctg tacatctatt tttctgttga gatcaagaag agctttacgt agactctcca 108060
   agtgtctttc tagtctgtct aaccggttac ctgtttctct gcagcaatca gttatagttt 108120
   tgtaactgtc taacaagctt acgaggcgct cttccacact ttctttagtt ggagctccag 108180
   ccgcgtacac tccgttggtt gaattgcctg tatcatcagg ctgagtcaat aggttttctc 108240
   cgtcattttc atccatattg agtccaacga acacaaacga gtaagtgttc ctctatttaa 108300
   agtattgatt ttagaaaaag gcaagcctcg ctgccctgat tcggcggcaa acacgggttg 108360
   aacacgcgga agtcgctcgc ggccgtgaag atctcgtccg cgcacgcctc cacgctcgcg 108420
   aagcgcccag gcgagacgcc gtcgtgcgag cggaacccga actccgaggc cgccaccgcc 108480
   gcgcccttga agagcacgca gcgccacttc ttgcgcacgt cgaaggcctc gtcgttgggg 108540
   tcgaacacgc gccggtccac gcgcgggccg cccgccgtgc gcgcgaactc cagcgccgcg 108600
   ttcgccgcgt tgaactcgcg gatgttgtcg tagttctcgt agacggccca gagctgcagc 108660
   gccacgaaca tggcggccgc cgccgcgagg gccacgcaga gcgcggacac cgcgtccatc 108720
   ttttatgtgc agaattattc gttggcgcgg agctcgcgca gctccgcggc gcgcagccgc 108780
   gcgaaggctg ctttgagcgc gcgcagcagc tcctcggtgt ccgcgcgcag catgtcgaag 108840
   cggtggtagc tgtccaggcg cgcgcggcag ccgaagaagc gtgcgacgca cgcggtgacg 108900
   atgtcgttca cgtagagcac gcccgaggcc gtgcagtaca cggagcgcgg ctcgcgcggg 108960
   tccggcggca cgtccacggc gaccgcgtgc gcggccacgt cctcgagcac cttgcgctcg 109020
   agcacggcga ggaagtcgcg cagctggcgg cggttgtcca gccaggcgta ggtggtcgcg 109080
   aagagcgtga gccgcccgcg cggcgcgatc gcggtgtagg gcgcgtaccc gcggaactcc 109140
   cgggggtgca cgaccttgac gttctcgtgc tcgcgacgga aggcctcggt gtcgagcagc 109200
   gccgcgagcg cgtccacgag cttgtcggag acctccacgc ccgcgccgaa ggcgatgagc 109260
   tcgatcttct gctcgctctt ggggcggaag tcgtggaagg tgtgcagcag catctcgcgg 109320
   agctgcggcg gcttctcgac ggcctcgagc gcgtcgccgc ggacgaggaa gtagtcgagg 109380
   tcgtgcagcg agacgtgctg cccggcggcg ctctgcacaa acttgaggaa gacgcagagg 109440
   cccgcgcggc gctcgagcac gtcctcgacg tgcgcgtgga atacgtgccg cgagggcatg 109500
   gcctcgatcg cggagagcca ctcctcgttg acgcaggtgg tggtgttctc cagcaccacg 109560
   ccctgcgtga gcgcgggcca ctgcaggtgg aaggcgaact cgtgcttgat gagcgaggcc 109620
   acggccgggt ccaggtccac ggccagcgcg gcctcgccga cgaggggagc gtccgccatc 109680
   acgcggagga cgcctggccc atctcctttt tcgccttttt attcaggatc attattcttt 109740
   cgttgacgag gtccatgagc atcttgatgg cggcggccgc ggccgccgcg tcgccgccgc 109800
   acatctgcgc gatgcgcgtg agcatgtgca gcagcgcggc ctcgtttagg tcctcctcca 109860
   tttagaggcc gtgagggcgc gcgtcgtcgc gacgagggga cgcctcccgc ggcagcgtgg 109920
   tgcgcacggc gaaggcgagc agcgcgccgg cgcactgcgt gagcacacac tccgcgagcg 109980
   cgacgaggag ctcggagggc gcgagcacca tttagaggcg cgcgcgggtt taattgccgc 110040
   cgtcagagtc ggcatcatcg cccttgtcgc cgccgtcctt gcagtcgccc ttggtctcgg 110100
   cgtcgacgat gtcggcgagc cgcgtcttca tgtgcgagaa ctgcgcgagc aggatgccgg 110160
   ggtcgagaca gcgcttgacg acgctctcgt cggcgaagtc gtagcagatg cgctcctggt 110220
   tctggcagaa caccgagtct tcgatgatca acaccctcct ggtcccggcc gaccgcatga 110280
   tggccatggc ccggatgagc ctcttcttcg atccgcgtat ggacatggac cggagcacgt 110340
   tctccacgtc ggagtcggag acgttgcagc agcagaggtg cgtgatgctg gcgcgcccgt 110400
   tgacggggat gtgcttgtag gtctggcaga gcagcaccag cgacacgttg atgtgccgcc 110460
   cgtagttcat gaggcccaag agtgtgggcg accgcgtctg cgtgtcgccc atatcgtcga 110520
   gaatgatgag gaacttctgc ttcttcgtct gcgcgtgccg ctcgatcttg cgcttggcga 110580
   ccgagaggtt gtactcgagc tcctcgtgcg tggtgacctt gtggatgtgg tccggccaca 110640
   cgaagccgtc gtaggcggcg ttgtagacgg gcgtgaagag caggatgtgc ttgaagcggc 110700
   gcacgagcgt gcggaagagc gagagcaggt aggcggtctt gccggagccg gagccgccga 110760
   cgagcgccat cctgaagggc gcctcgatga gactctcccg cttgaagcgc acctcctgca 110820
   cgacatccat cgtatattta ctgtcactaa attaccggct ccgagaaata tagaaattag 110880
   agcctcctag agcacaccga ggcgcatcgg caagatggca cataacacgt tcgaaaacga 110940
   tagcgagtcg gcagctaaca accagtacgt ggcgtcagtc aagcgccaga aaatgattcg 111000
   gcgatacatt aagatgttct tccggttcgt tacggcgata gctatcattg tcctggctat 111060
   tctagttgtg attctgtcgc tatctctaga cgaatgtctg cacagagaac accctcatga 111120
   ctattcacat gtacaaaatt caacatgcga cggcattact ttaggtggtg aaaagtgtct 111180
   cagacttaat ttgccagcaa cgtgggaaga tgctaataga caatgtggta atcttgggtt 111240
   ttacctacca tctactggcc ttgaaaagaa atttccttgg cttgtgacct atctcgacgg 111300
   aacttgggga aacactcaga actccgtatt tggaccaacc ggtgacttgc agaatgtcat 111360
   aggaccgaaa gaatacaaat atttttgtgt gtccgattag atgattataa tctaataaat 111420
   gggttgctgt aaggtcccta accgccagtc tataaggact ttgaaaaagg cgtcctgtcc 111480
   ggtcgccagt ctcgtcacca ttctctccct agtcaccagc ctcggtgcga tagtcagata 111540
   caccaatttt tttctaaaag aagcatgtga cgaaggatgg atgcccataa aaaacatatg 111600
   cattttaaac acgcactttg aagccaccaa tgacgatgcc cacaggatat gtgaaaacct 111660
   agacggaaag ccgccggcca tccctaaccc tactctgtta aagggtgtca cagttctcac 111720
   cggcgaaaag aaattttgga tgacccatca cgaagactat actactgtgt ttgagcatat 111780
   agacgatagg acgactccta aaaacacaga ctatgacagt aaaaaacaca cttgtttgat 111840
   gagcgaggac ggattgatac accataactg catgatgaac gtgactgtgg tatgcatgaa 111900
   ggagatgcac ggataactga aaatatactg tttgaacgca aagacgccat gtcgcgactt 111960
   caaatactga cctcatttgg acaaatcttc gcacccgacg aagctcggct gcgcgagatc 112020
   gcgcgtgatt tgggaatatg caccataaaa cgcgcattcg gcgacatgct gtacggcttt 112080
   atagacttcg acccggtgcc cctgacccaa gtaaacatgc tcatgtccaa ctgctacttc 112140
   gcggtcaacg gcaacctgct tccgtgcacg gaggacttcc ggctcagact cccggcaacg 112200
   gagatctctg cggcctacct gacgagaacg ggacggacga tcctgtgcgg caaagacttc 112260
   aacatagtag cgccgtcggg gttcaagccg tccatgcggc tgcgcgacct cagtcacgtg 112320
   tctgcgcttg tagagatcct ggaagtctac gacgagtccg gggagtacca attcgtgctc 112380
   ggccccagcg cgcagttcat gctgcggctg atggagaagg agaacgtctg tctgttcggc 112440
   agcgggtggt gcatagtgga cctgcgcaag ctggacgtac ccatataatc agcatccttg 112500
   tttttatcct gtctttttat cagtttttta gctagttaaa acataaatag taaagctaaa 112560
   aagaggagtt ctggagtctt gcaacaacca ggatgaaggc ggtgttgttg ctggcgttac 112620
   tgggagcgtt caccaacgca gcgcctttgt tagaaagcca gcgttctaac agtgaggaaa 112680
   aagcaaattt ctgctcgacg cataatgatg aagtgtacgc caggttcagg cttcagatgc 112740
   gcgtgggtgt acgacacagt ccgctctaca ctcccagcaa catgtgcatg ctggacatag 112800
   aagactccgt tgaggacata gaagagtcca cagaaaaaga atacgcgtct acggccacgg 112860
   gtgaggcggc cggagtgaac gtgtcagtgg cactagtggg agaaggcgtg agcataccgt 112920
   ttagttacat aggccttgga ttcaacccgt cgcttgaaga tagctacctg tacgtcaacg 112980
   tctcgtcacg agctccttgg gttaaacaga cttcggacct atccgcgaac ggcggctggg 113040
   gtatcaaaca ggttctagaa aaagagttac tggccatcca aatagggtgc gacaaccaaa 113100
   aatttcccga agaacccaca actacacccc cctcacctgt cactacaacg ctttcctcaa 113160
   caactccaga tctgaatgaa gaaaacacag aaaatacgcc gacgaccacc ggcgccagtg 113220
   tagacagaaa gcgcaatcca gctgacattg acttctcgct gctcgtggac ccccgatgcg 113280
   tgacctctgt agacctgcac gtcgagctca gggacgcgtg catagactac aaacaagagt 113340
   cgccgttgtc gctgaagggg aaatatggag acggcgaact agtaaaaaag gagattaaag 113400
   acgtgggaaa gaatcacaat atgtgcagtc ttaacctcaa ccctggcaat tgagctgttt 113460
   ttattcggca atataatagg tgattattga acattaaaca aaacttatcc cacaacgccg 113520
   caacaatgga agtgctggtg atcatctcta ttatcgtcgc cgtaatatgc ttgaccggag 113580
   cggtaatgta cctccttatt gaactcggct tagccgccga gcgcgctaac aaacgcgcgc 113640
   gcgtgaagaa aaatatgcgc aaattagcca ctcaattggg aaatggatct gtcgactccg 113700
   gcataggcat aggcccgtgc ataatgtcgc gcaccatgga ctctggaccc agtcgctggg 113760
   acagcgacag tgaggatgac ggggacagcc tgtccacgac gtccaccagc ggagggggga 113820
   ctctcacccg agtgtgggtt gggagcgccg ggcctatgta cgaaaacttc tgcgggaacg 113880
   gcacccgcca ctcccccacc aacgaccctg gctaccactc gcgggagact ctctgcagcg 113940
   gacctccccg tcaggcgccg gcgctaccgc ccaccccgaa gcccgacgag gtaacggtgg 114000
   acgtggggcc cggtcccgac gaccaacacg gtccgtacga ggaacctgat cccattcccc 114060
   cgcaggaacc cgagccgccg gtgcagattg aggtaaccat caacgggccc ggtggagaag 114120
   gcgaggcgga aggagaattt ttctacgacg agtagccgcc aaaactgaat aactatcggg 114180
   cttcgtaaac gcgcagacat gccgctgttc cggaagctca tggtttcgcg ctccctggtc 114240
   aaggaatgtc tgactctgga cttccggcag ggcgagcgtc tccctacgcg atgcttcctc 114300
   ccggtgcccg cggggacgac attccacaga gtctgcgaca cctcgccgct gacggacgaa 114360
   gtctcccggc acgtgcagga gcccgtcatg ggcaccggac gggtccagta ctactacttc 114420
   gagagcggcc agggcatgat cggcgacaac gcgggcatgg cgcgcatgct cgtgtgcacg 114480
   cggtcggcgt acaacggcgg cgacgtcgtc gtgcggtcca cgcggagcag agcagacaag 114540
   accgtggtcg cgccctgcca gggcatggcg ctgctgctga gccccttctg cgccttcgac 114600
   atcacgccgg tcgagagcgg ctccgcgata ttcgcggagg tcatcgtcac cgcgcccagc 114660
   atggactacg tcgaggcggt caccggcacg ggcgaggcgg ccgtgcggat attcaactcg 114720
   caccacccgc tctggccgcg acacggctcg aacgtctgct tcgcgctgcg gttgctgcga 114780
   gacgtgcgca cgggcgagcg cgtggtcgag cagatgttca tggacgggcg ctggcacacc 114840
   gtgctgagga cgtcctgcgg caacaaggtc tgcgtgcccg ccgacctcgt gggccagacg 114900
   aacctcgagg aggtgccctt ctgcgacgtg acgcccgaga tcatgcgccg cgcgctggcg 114960
   atcgacccgc cgtacgaggc cgtggcgcac ccgcaccgct gcgtgtacgg cgccatggac 115020
   gtccggtgcg cgaacgagta cctcgtgtac tgcaccttca agacggagcc gacacggcgc 115080
   agcacgtcct cgccgggccc ggacagcccg ctgtcgcccg cgactccgtc gacctcgcgg 115140
   gccgcggccg cgcgcgtccc cacgacgccg caggaagtgg cctcgccgac cacgaggctc 115200
   ctggagacct gtctgcgcga cgccctcgac ggactctgac ccgaaggacc caccgtccac 115260
   tcacattcca ctgccagaca actcaagctt tttctgcatc tacctcgcta ataattgaat 115320
   tgttatagga caaacaggcg cactcgagca caatggcgtg ttttatcgaa ttgttagact 115380
   ccatcttcaa ccgacgccac cgtaatttcg ggccggagga catgtacagg ccctctgacg 115440
   ccccgccccc caaatcgcac acgcctcgca ctccccgcac cccgcggacc cagtgtcccg 115500
   gacacccgcg gcgacaaagc tcctctccca tctacggcgc ttatgtggac tccctgccga 115560
   ggaacagaaa gcggttccag aatcaacaca gttgtcccgg agattacgag cggtgtcaac 115620
   tctcggacac catcagcctg gatgcgacgc tactcacggt tacagtgacg tccatctcca 115680
   gcatatccag ctctagtagc tcagactcta tctctctggg gcagtgcaga ctgtccatgg 115740
   tgtccgcaac atcaacctcc acctccacaa ccttctcctc ctcggaatga gcgccacact 115800
   tatttttgta taatagtttg tattgaacct tagagacatc cacaaatagt taggaagcat 115860
   gagtagttca agtagcgaga ccacccctaa gcccaagccc atccctgctc ctcccatgac 115920
   tcaggaggag tttaacaaag aagtgaagaa acgaaaagaa cagaaaaagg aaaaatctag 115980
   aaccgttgaa cgtgagtcag aaaccgtaac tgtatcttcc gacggatcag agataaaaaa 116040
   gacttacgag cgcgagtctg agagaacaac cgaaacagaa aagaacaaca cgtcaaccga 116100
   tgataataag cagaacaccc ctgtagagaa accagaggaa accaagcctg cttctactcc 116160
   tgaaggtgtg aagccagccg agactcctgc cccgactact gacccccaac ctactacaca 116220
   accacccgca gaatcaaacc ctggaagtca acccgcacct gcttcagaac caacccccgc 116280
   acctgagcct gcaccggaac ccactcagcc tgcatcagta actcaacccg ctccaacacc 116340
   agagccaagt ccagccccta agcctactcc ggcttctgaa ccaaccccag catctgagcc 116400
   tacttctgct ccagaaccta caccatccgc agaaccaact cctcaaccaa ctgtagaaac 116460
   accaccatct gctccagcac caactcccga ggcccaacca cccgccaaca gcaatcccac 116520
   tactgaaact accactggta ccagcacctc ctaagtgagt acgtaagcat ttcggagtaa 116580
   cgtcgtagca agcgctagtc cgccgcgagc ggttctcgca agttttttcg ggtaaaaagc 116640
   gtacaccgtc gccttgtcgc ggcggtgtac gcttttttca cgcccttttt gcaaaattta 116700
   aattgtaccc gcgccggctc taggaaagat ggcgtgcctc agagtgttcc tggcggtgct 116760
   cgcgctgtgc gggagcgtgc actcggcgca atggatcggc gagcgcgact tctgcacggc 116820
   ccacgcgcag gacgtcttcg cgcggctgca ggtgtggatg cgcattgacc ggaacgtgac 116880
   cgccgcggac aacagctcgg cctgcgcgct ggcgatagag acgccgccga gcaacttcga 116940
   cgcggacgtc tacgtcgccg cggccggcat aaacgtcagc gtgtccgcga tcaactgcgg 117000
   cttcttcaac atgcgccagg tagagacgac gtacaacacg gcacgccggc agatgtacgt 117060
   gtacatggac tcctgggacc cttgggtgat cgacgacccc cagccgctct tcagccagga 117120
   gtacgaaaac gaaacgcttc cgtacctgct ggaggttctg gagctagcga ggctgtacat 117180
   tcgcgtgggc tgcacggtgc ccggagagca gcccttcgag gtgatcccgg ggatcgacta 117240
   cccgcacacc ggcatggagt ttctccagca cgttctacgg ccgaaccgcc ggttcgctcc 117300
   ggcgaagctg cacatggacc tcgaggtgga ccaccggtgc gtgagcgccg tccacgtgaa 117360
   ggcgttcctg caggacgcct gtagcgcccg caaggcgcgg acgccactct acttcgcggg 117420
   gcatggctgc aaccatccag atcgccggcc aaaaaaccca gtaccgcgcc ctcagcacgt 117480
   atcgtcgccg atctccagga agtgcagcat gcagacggcg cgctaagggc gctcaccgcg 117540
   ctgacggcgg ccgtggtgtg cgcgatcgcc atcgcgctcg agcgcggggc ggaggccgac 117600
   gccgtggacc ttatccttat aaaattttca atgatatgct agtttttatg cgaccttcct 117660
   tagaaaattc ggaattcaaa aatgaaataa aacggcgttt agcacgcata ttattaatac 117720
   cgaccaccat ggcaggcgtc cgcagctgcc agaagaaagt cccttctact gcgggctcca 117780
   tgtcatttca acggggcaac cggagcatcc agccggcgat gtccgaggcg ttgcagaatg 117840
   atttcagcta caacccgcga ccgcctccgc cgagcgcaga agagattgac ttcttctgcg 117900
   tggacatgcg caaagtactg atggaaattg aggccaagcc cagcagctcc aagtaccccg 117960
   atttcatcca cccggttgac agcagcccgc cgtgcacgcc ggcgcgcaag cgcaacggct 118020
   tcggccgcaa ggcactgaac aaaacccagg tgccgcagca ggccaagcgt gacggctact 118080
   cccgctaatg cagtccacac acttcacaca ctacatcagc actcaagctt ataatcacta 118140
   cacaatgaat cagcccacca cgtgcgaagc acacacataa aatcacccac ctgtcctgat 118200
   cgttcccaat actcccaatc accgtgcttt acacgcacgt taatcaccct ttccttcctt 118260
   catgcgttcc tgatcgttcc tcctccttaa tcacacacac accccgtaat tttgtacttt 118320
   tgtactttaa tttgtacact ttacacactg actttgtact gcctttgtac tttatttttg 118380
   tactgaaatt ggacgatact tatctttgta ttcacatcca agttttgcaa attccacagc 118440
   cggtagcgaa aagtgaaatc gtaccgtttt aggcttcgat ccccctcccg cgcgaagact 118500
   cgccagcatg gactctcgta ggctcgctct tgccgtcgcc ttcggaggcg tcctcgccag 118560
   catgacacag cgccgccgcc tggcttctct catcgccagc atcggccaac ggctgatggg 118620
   cggcgacggc atgcgtcgcg tcgccgttcg gttgatcgac cagctcatgg ccggaccccc 118680
   ggacatcaac gacgaggcct tccagcgcga gatccgcgtg ggcgagctct tccaggcgct 118740
   ccaccgcgtg gtcgagcagg cacgccgaga gaagtacttc gaggtctgcg gcgccggcaa 118800
   cgacgccgac gcgcccgtcg tcgagatgga caccgcggcc gcacccccgc agccccagcc 118860
   cgcgcccctc gtggtcacgc cgcagaacgc gttcatgttc gtgccgcaag gcagccacgt 118920
   gcacgtggac gagagcgtgg acccgttctt cggcatgagc ccctccatct tcgggcgcga 118980
   cctcccccct cagccgcccg aggagctgct gagcgactac gacccgctca tgagccaggc 119040
   cggcgagccg ccgagcccgc ggtcgccctg cgaggccgac ctctggtgct tcgagacgct 119100
   cggcgacagc gacagcgatt gagaccgcac cacaccctac ctcacccacc ccacactcca 119160
   cctcacctca ccctaacacc cgacacccaa cacttcaacc ggacaatgaa ggagtcccac 119220
   atttcactga aggacgcgga tgaagccgca ctcccccaca tgaaggattg gcaacggtca 119280
   aacatttcac ctgcaatgaa ggacgatgcg cggtcgcatt ggcctgcgac cgacatcgca 119340
   cacatgaagg acacaattgg tttgttaatc cggacaatga aggacaaatt gtttttgtta 119400
   atcaggacaa ttagaacaca atcaaatttt tgtacgatca taaaatcgat atttgatgca 119460
   catatattag taagtatatt agactaaatt ctccggggag gcaagcagtt ggatacggcg 119520
   gggcggggca cgacgtgcac ggagagttcg ggcgggtccc ccttcccccc acccccacgg 119580
   caccacgatg cgcctaatct tagcgctcgt ggcctgcttg ttggcggcgc cgatgccgtt 119640
   atcgggtcgt tcgacaagca ctccaaacac gtccccctcc gcactcggct cgacgagttc 119700
   ggaaccaagc tcggaagacg ctgtggcttc gagcacaacg acaagcacac tcacaagcac 119760
   tacaagcaca ctcactatgt ccacaagtgt ggacaccact actacctcgg gcgctacaac 119820
   gtccgcaaac agcactcctg cagcgagtgt gagctcctcc acacccgcaa ctaccgaggc 119880
   atcgacggca ccaacgacgc cgtcgacgcc gacgacagtg aaggtaacga agggcaagga 119940
   agacacgaag gcgtctgcct acctcgtttt actaatcacg ttcatggtca tgaccacgct 120000
   cgtgatggtc gtggtcgtgg tcgtggtcgt gtacaaacag ggactctgta actgctgctg 120060
   taagtttccc ctgctgcaaa gagctcaagg actacctcga cgaggaggag agcgccgggc 120120
   tgtacgacgc cttgacgtgg agccactcag actccggcct ccggctcgtc gtgcgcgcgg 120180
   accccagatg atgaggatcg gataagatcg gcgtgttttt cccgcccgtc gcgaacatta 120240
   tgcctctaaa tgccgagaat taactgaaat tcaaacacgc tttgggactc aactccgtga 120300
   cccacactca accatggctg gcttcctagg cgcattcaga ggcgtgtgct ccgacttatg 120360
   gcagtcgctc cgtggacacg gacaccactc ttccagctgc ccgcgacgac gcgccaacag 120420
   catggacgac cgcgaccggc gccgacaccg ccaccgcgag atccccaaca gctcggcgtc 120480
   gctgaacagc gacccgatgc cgcaacgcag tgcgggtgcg cgccggcact acgactgccg 120540
   cccctcggaa aagagcagac actcctccga caagcaccac tcggcggacc gacaccactc 120600
   ggcggaccga caccaatcgg cggacaggga cagacaccgt cgcagtcgca agaactacga 120660
   ctcgcacccg tcgcgcagga accgcaacta cgagcgggcg gactaccaga gacacccctc 120720
   acaaacccac ccagacgccc ccgcgcagac ctcgacgctc aaggtgacct ccctaagcac 120780
   cagctgcagc accctgtccc aacatcacta cgagaccccc gaccacatct acgacatccc 120840
   ggaagacagt cgcggggcgt cggctccccc tcgcgcggac ctcgcgctcc ccccgctcgc 120900
   catgcccaaa tccaagccgc gtcgcacgcg cccggcgtcc atgaacgact gcctgatgaa 120960
   gcactgcggc gccggcagac ccaacctcca agacgacata tgcacactat gtactgatat 121020
   agagacacag ctgagcgcac tagagaagtc tctggagtca gagctcaact tctatcgtcg 121080
   ctacatacaa gacactaaga cattgctcgc cacgcgagca gcaaacatcg gcagcaaagc 121140
   tctgatctac accgacgact acaacggcag tggcgacgtc ggcgaaaagg agcactgctc 121200
   ggaggagtgc tgcaaagtgg aggaagttct gtgagaaagt gcgtttttct gtaatgtgaa 121260
   ataagatagc cttatgtgtg cacagacatg gcgaacaggc tcgtgttttt cgaccccgag 121320
   accctagccg aggccgacgg catccccggc tatggggtgt tcgagcccgg caagaagaaa 121380
   tgcatcttca caaagatccg caccagcgtc gcactcgcgt gccggtacgc cgtctcggac 121440
   ggcggcctca tcgacgagtt cgtcatggct acatacggga ccagacgcgc gtgccggctc 121500
   gtccggcacc tgacgatagg cgcggagggc gtgatgaccc ggcccgccag caactgcgcg 121560
   ccgcacatgg tgctcatctg cctcagaggc gtggccgccg tgtccagcga ggacatgggc 121620
   ttcggtcgct gcatcatgga gcgcggcacc atgttcatgg tcaagtccgc gcacagcgcc 121680
   gtcgtctgcg gcaaccccgc ctgcgagctg ctcgtcctct tctacgacta cttcaccccc 121740
   atcccccggc cgctctccgg agacgaggtg ctgttcaccc gcgacctcgc gcacgtggac 121800
   tacgcccccg agtcggcggt cgtcttcaag atggattaca acctcgagac cgacgtggcc 121860
   acgctgtttg tcggggggta catattccgc gccaagggcc tgatgatgga gacgcgcgaa 121920
   caagtgggcg acgagtgcga ctgctgccgc cacagctcgc cggtgctcgt catggatcgc 121980
   gagaagatga tgtcgtcgct gcgcatgatc cccagcatcg tgcccggcca gcgggagatt 122040
   tggcttcgcg agcgcggctg ggccgtcctc gagacggacg cccgcggaca ctgcgagccc 122100
   ggcgtcctga ggctggcgct cgccggcctg cggctgttcg caggatgcct gcgctccgtc 122160
   gtggggcggc gcgagctgtc gctgttctgc tacggcgtcg ctcccaagtt cggcggggag 122220
   ttcgaggacg cgccgcgccc catggagatc gacggttagt tgtttttatc cctgtacata 122280
   cgccgcaaac tgaaacttta gggcaccgcg taatagtgca cgaacgccca gtggaccgct 122340
   tccgcagcca tggaaaacaa cgacggcaac gaacgcaaca acgaacaccc gcacgttcga 122400
   gaattcaagg aggcgtccct gtacgggttt ctggtggcgg ccgcggacgt gaccgtcgaa 122460
   gacgtgcacc ggtaccttca gttcggcgcg gacgtgaact acaggggcgc gtacctgtgc 122520
   acgccgctgc acgcgtacct gcagtccggc tgcgaaaagc gcctagacgt cgtggacgcg 122580
   ctgctggacg ccggcgcaga catcaacgcc aaggagatct gcggtctcac gcccgtgcac 122640
   ctgtacgcga gctacgcgga cgtggacgta gagttcatgc gcgggctcat cgagcgcggc 122700
   gcgagcgtgt gcggcgagag ctcggtcacg ggctgcctgt actcgtacct gtacacacac 122760
   agcgtggacg gcggcgcgcg cctggacgtg gtcgagctgc tcgtgcaggc gggcgcggac 122820
   gtgaacgtcc gcggcgaggc gcgcaagacg ccgctgcacg tgcactgcgc gggcttcgag 122880
   gtggattcgg acatcgtgga tctgctgctg cgcgcgggcg cggaccccga ggcgctcgac 122940
   gaacacgggc tcacgcccgc ggacgtgctc gtgaagtccg tgggcgccaa cgtggagacg 123000
   ctgcggctct tcctcgacgc gggcgtgagc gtggccacgt cgcgcgacgc gcgcggacgc 123060
   acgccgctgc accaccacgc agactccttc cgggcgagtg cgggcatcgt gcgcgaactg 123120
   ctcgccgccg gctgcgacgc ggcggccgcc gacgacctcg gaaacacgcc cctgcacagc 123180
   ctcgccacct tctgctcgtg ccggcgctcg gtgctcgacc agctcatcgc cggcggcgcg 123240
   gacatcaacg cccgcaacca ctacggccac acctgtctgt actacgcgtc catctacaac 123300
   ccctccgtct gctcgcggct catcgccgcg ggtgcggacg tgaccgcgcg cacgccggac 123360
   ggacgcacgc cgctctcggg catgatcatg cgcaagcaca cgcgcgccgt gcgcgccgcc 123420
   ctggcgacgc ggcctcccgc ggacgccgtc gccgcgtcgc tggacgtcgc agtacagccc 123480
   gagcccacgg acgccactcg cgcgtgcgtg cggtacgtgg tgctctgcgg cggcacgctc 123540
   tcggcgcgcg tgcggtcgcg acacgcggac ttcgtgcgag agtgcgaaag cgaggtggtc 123600
   gtgctcagaa ccaccgtggt ggggctgccc ggcacctcgc tgctggacat cgtgcgtgcg 123660
   gcgcagccgc cgccggtact gctctccccg cgcgtgcacc acgtgctgca gaagctgtgc 123720
   gtgtacgcgg agttggtgga cgcgcggctg cgcgagatgc ggcacaagac caacctcgtg 123780
   gacgcggtgt cgcggctcgt gtgtccgtgc gcgctgccgc cggaggtggt gcgcggcatc 123840
   ctcgtgcacg tgccgataga cagcctgcgg cacacgttga ccctcggcgt ggcgcaggcc 123900
   tcgcgtttcc ttccctcgca taaatgaaat attatttttt gtggtagacc ggatctcccc 123960
   gatggacccc gccggacaac gactgcgcgc gccagggccg tggcgcctga acccgccgac 124020
   cgcggccgcg ctggaaagcg cgctgctgcg gcccgcggcg tcggcgggcg ccgaccgctg 124080
   cgcgaacgcg cacgtggacc gccgcaacat gggcgtcggc gagggccgcg aggtgcccgc 124140
   ggacgtcgag gggctcatga ccgagatcca cctgcggtac ggaatgacgc gcgtccaccg 124200
   gaacgttcac ttcgtgcagt tctggcacgg cgagcacgtg cgccggcgcc ccgcgcgaca 124260
   cgtgttcacg gtctggatct gcctcagcgg cgaggtgcgc atctacgcag agtgctgcca 124320
   ggcggggcac ggcttcgtgc tctgccgaca gatggcggca gggtacatgt tcgtgaccga 124380
   acccacggac tcggtcacgg tctcggtgcc gcaccgactg cgcaactcgc ggtcgccggt 124440
   gtggctggcg gcggtcttcg ccacgcggca cttcgagccg ctgccgccgc ccatgtacgc 124500
   cgtgcccggg cacgtggtgc tcgcgcgcag cgcctccatg ctctgcgact gctggccgtc 124560
   ggacccgcgg cgccgcaacg tgatcttcta catgcggctg tcgggcgcga tggtgcgcgt 124620
   ggtcgtgccg ggcgcggagc tcgagatcga gtgcacctcg gggttccggc cggaccactt 124680
   ctccatcaac gacgagtgcg tgtgctgcga gcgtccgcac gtcgcgcgaa ccgcggtgtg 124740
   gacgctggcg gagatttgcc gcggcgccac ggtggtgctc gcgccgccac tgccccgcga 124800
   ccgcgccgcg gggctgctcg cggagatccg cctggcctcg ctgcgatggg tgcgcgtgcg 124860
   tgcggtccgc agcggcagag aaagcgtggg cccgttcccc tcggtggtgt gggcggcggt 124920
   cttctccgcc gttcggctct tcctggacgg aaccgtgcct gccttcccgg cgtgtgtgga 124980
   gaatggacgc gcggcgtacg gcatggtgta cgtgcccccg gaggagccgc ggatggacgg 125040
   gctctgtgtg ttcccgacgc ccgccgagcc ggcggcgctc ttcgtccgcg gagaccaggt 125100
   gcttgaggcc ggcgcggccg ccgccataat cgcggccgct gagaagcgcg tccaggccgc 125160
   caatgggtct cctgctgccg cggaggagga cataggtgcg gcggccgatg ccgccgcaga 125220
   gagcgtggag caggaccagc gcgtcgagtt cgaccttggg cctgggcctg accccagcca 125280
   agaagcgccc gcggacgcgc agcgtgccga ttcggacgac gacaccggct ccgagaccga 125340
   gaccggcgac gagagtgtgg gcggcgagga tgacagcgac tcctcctcct cttactcggt 125400
   gatgtcggac gacgaaaacg acagcggcga cgagggctgg ggcgactcta gcgactccgg 125460
   catcgaggac gacgacggcg gtgtcgccag gccgccgagg aagaagagga ggaagagcgc 125520
   gacgtcctcg gcgcagcggc ccagatgctc ggagactgac cggtggtgaa aacataaaaa 125580
   taaactgttc aacacttgta ctccgggcac caacactact atccataccc accctccctc 125640
   cacacactac aatggcaaac agagaagaga ttgacgcctc cgccgtcatg gctgcctacc 125700
   tcgcgagaga gtacgcggcg gctgtagaag aacagctgac gccgcgcgag cgcgatgcgc 125760
   tcgaagccct tcgcgtttcc ggcgaggagg tccggtcgcc gctgctgcaa gaactctcga 125820
   acgcgggcga gcaccgcgcc aaccccgaaa actcgcacat ccccgccgcc ctcgtctccg 125880
   cgcttctcga agcccccacc tcccccggcc gcatggtcac tgcgattgag ctctgcgcgc 125940
   agatgggccg ggtatggacg cgcggccgcc agctcgtcga attcatgcgg gtcgtgtacg 126000
   tgctcctaga ccgtctgccg cccacggccg acgaggacct cagcacctgg ctgcaggccg 126060
   tcgcgcgcgt gcacggcacg cggcgccgcc tgcaccgcgt tctcggcgtc ggggccgtca 126120
   tggcaggcgt cggtatgctg ctgctcggcg tgcgcgtgtt gcggcgcaca taacttttta 126180
   tctcggctca aactgaaata cgacattgga ctacgaaacc tatgattttg ctcacggccg 126240
   cgcgagatag gataataaat aacctttgag caactaacat ggccgatgag agagaggccg 126300
   acggcgcgct gttccggtac ctggagagcg aggaccgtcc ggacgtggag cacatgcgcc 126360
   ggctgctgga cgagggtgcg gacgtgaact acgcgggccc gcgcgggtac gcgccgctgc 126420
   acatgctcat gcgcggcaac ccgctagacc ccgacgcggt gcgactgctg ctcgccgcgg 126480
   gcgcggacgt gaacgcgaca tcgctctgcg ggttcacgcc gctgcactcc tacatgtgct 126540
   tcgggaccgt gacgccagac acgctgcgtg cgctcatgcg ccacggcgcg agcgtcagcg 126600
   acctcgagcg caacatcaac gcgctgatcg agtacttcaa ccgcgacggc tgcatgggcg 126660
   gcgcggaggc gaccgtgatc gcactgctgg tggagcacgg cgcgcacgtg aacgccaaag 126720
   acgaccttgg acgaacgccg ctgcacatct acctgtccgg cttcttcgtg tcggcaccgg 126780
   tggcgctcgc gctgatcgcg ctcggcgcga acccgaacgc cacggacgcg tacgggcgca 126840
   cgccactgca cgccttcctg cgctcccgcg acgtggaccc cgctgtgctg aagacgctca 126900
   tcgccgcggg cgcagacccg ctcgcgcgcg acatcatccg gcgcacggcg ctgcactacc 126960
   actgcgagtc cttcaagacg cgcgctagtg ttatagagac gctggtggcc gccggctgcg 127020
   accccgcgag cacagacctg ctcgacaaca cggcgctgca cagcatggcc atgggcagct 127080
   cctgccgcgc ctcgctgatc cgcccgctgc tggccgcggg cgtgtccgtg aacgcgcgca 127140
   acgcgcggct gcagacgccg ctgcacctcg cggccgtgtt caacccgccg gcctgcgcgc 127200
   ggctgctggc cgcgggcgcg gaccccgcgc tcgcggacct ggacgagaca acgccgctgc 127260
   tgagcatggt gcggcacaac tgcgcacgcg cgctgcgcac ggcgctgccc ttggcgccgg 127320
   acgcgctggt ggccggcgcg gtgaaccgcg tgaacgcgcg cacgccgagc gcggccacgc 127380
   gagagtgcgt gatggcgctg gcgctgcgcg gcgcgctgga cctgctgagc gcggagagcg 127440
   ttgccaccca cgcggccgcg atccgcgcct gcgaggcgga ggtcgcgctg ctgcggagca 127500
   cgcgcctggg cgcgccgccg acgacgctct tcgcgctgct gacaggacga ccgaacacgc 127560
   tggtttccgc gaaggcggcg cgacgcgcga tggcggacgt gtgtgtctac cgcgcggcgc 127620
   tggccgcgcg cgtggagcgc gtgcgccgca agtcctcgct ggtcgagcgc ctcaccgcca 127680
   tggtgtgtcc gtgcgctctg ccgccagagc tagtgacgcg catcctcgcg ctcctgaccg 127740
   tggaggaact cgcttgcgca atgcgcaaat aataatgaac tataactagg cttattagag 127800
   gcactatttg tgcagagtcg ttagttatag ttagtgtact taccattgga atgtcgaaga 127860
   acaaaattct ggtgtgtttg gtaattattc ttacttatac attatacaca gatgcgtatt 127920
   gtgttgagta tgaggaaagt gaggaagata aacaacagtg cggtagtagt agtaattttc 127980
   ctgcgagttt accgcacatg cttagagaac tcagggcagc gttcggaaag gtaaaaactt 128040
   tcttccagat gaaagaccaa ctgaacagta tgctactcac acagtcgctc ctcgacgact 128100
   tcaaaggcta cctcgggtgt caggcacttt ctgagatgat acagttttac ttggaagagg 128160
   tgatgccgca ggcggaaaat cacgggccgg acatcaaaga gcacgttaac tcgctgggag 128220
   aaaaactcaa aacgctgcgt cttcgactgc gtcgctgcca ccgcttcctg ccgtgtgaga 128280
   acaagagtaa ggccgtggag caagtcaaac gtgtgttcaa catgctgcag gaacgaggtg 128340
   tttacaaggc catgagcgag ttcgacatat tcatcaacta catagaatca tacatgacta 128400
   ctaaaatgta aaaatgtata caacttttag ttatcgttcg gattctcgta tcgttctgca 128460
   tactatgtat ataaaatgta tattaacata gttacagtta cagttacagc tatattttta 128520
   tgctcacaag atgctatata attgaaagga aattgttcac tctctgtcag ggcgccatgg 128580
   actttctagg cgccgcgctt cacgactacg ttgccgatgc gcccaaggtc tgcgccgagg 128640
   aggtgcggcg gctgctggcc gcaggcgcct ctgtggagta cgcgggcgag ttcgggaaga 128700
   ccgcgctgca ccagtacatg ggccgttccg gcgcggaccc cgccgtcgtg cgcgcgctgc 128760
   tggacgccgg cgcgcgcgtg gacctcccgg agacctgctg cggctgcacg cccgtgcacc 128820
   tctgcctcat ggccgccaat atcgacgtgg aggttctccg catgctcgtc cacgagggcc 128880
   gcgtcgagga ctgcggccgc gccgagctcg cctccgtggt gctcaaggag ttcgtggtga 128940
   accgcgcctt cgacgagaac gtcagcgagc gagtgatgcg cgttcttgtg gccgcgggcg 129000
   cggacgtgaa cgccgccagc gtggtcgacc gcacgccgct gcacgtctgc ctcacgggca 129060
   tgtccacgca cccgggcacc atcgccgcgc tgctgcgctt cggcgcggac gtgaacgccg 129120
   tggacctctg cggcatgtcg ccgctggcgg tgctcgtgcg ctcgcgcgcg gcgaccgcag 129180
   agctggtgcg catgctgctc gacgcgggcg cagacgcaca cgctgtcgac agtcgcctgg 129240
   actcgctgct gcaccagcac tttcagtccg cgcgcccgcg gccggaggtg gtgcgcgagc 129300
   tcatccgcca cggctgctcg ccgcgggcgc ggaaccgaat cggcaacacg ccgctgcacg 129360
   aggccgcaaa acactcctcc tgcaaacact cgctggtggg gccgctgctg gctgccggcg 129420
   cgagcgtgga cgcgcgaaat aacacgggca ggacgccgct ccacttggcg gtggcgtcca 129480
   acccgcgcgc gtgccgccgg ctgatcgcgc ttggggcgga cgtggtcgcg cgcagttacg 129540
   cgggcgtcac gccgctggcg cagctgatcg cggacaataa ctccgcgctg gtgaccgcgg 129600
   cgctgaacac gcagcccgag ccgcgggccg tggcagagtc gctgcgagcg accacgcccg 129660
   tcggcgagac agcgtgctcg cggctctgtg tggcgtacgt ggtggcgcgc gcgccgagcg 129720
   aggtcctcgg cgagcccgag cgcgccctgc acgcggcctt cgtggcggag tgcttagcgg 129780
   aggtagcggc catacacgcc gtgcgctgcg gcacacctcc ggtctcgctg ctggagatcc 129840
   tggtggccgc gcgcccgccg cggagcctgc tctcgcgccg cgcgtggcgg ctggccagcc 129900
   ggacgacagt ttaccgcgcg ccgctccgtg cacgcatcgc ggccatgcgc catcgctcgc 129960
   gactggtgga gcgcgcgctg cgcacgctgc gcggctgcgt gctcccgcgc gaggtgctgg 130020
   agcgcgtgct gcggtgtctg tccacacagg acctgcgggc ctccggactg gccgagtagc 130080
   tttttctgag ataagtgaat aaacatggtg ggattcgatc ggcgccgcca acgccacgcc 130140
   atggacgccg ccgagatgga ggatctcgac atcaacgcgg agtcggcgct gtacgactac 130200
   ttcatcctga acgcggacag agcccgcgtg ggcgaggtgg ttatgcttct cgcacagggc 130260
   gcggaaataa actacgcgga cagcttcgac aagacgccgc tgcacctgta cttgcacacg 130320
   cgacacccgc gctcggacgt gattctggcg ctgatggagg cgggcgcggt cgtggacacg 130380
   ccggagcgct gctgcggcgc gaccgcggcg cacctgtaca tcctcaacgc ggccaaggtc 130440
   gacctgtcgg tgctggaggc catgctgacc tggggcgtgc gccagaacga ccagcactcg 130500
   gagcgggtgc tctcgagctt gttgcgcgag tacgtggtga cccgcgccta ctcggatcag 130560
   accgagccga tcatggactt gctcatcggc atgggcgccg acgtggacat gccggtcggc 130620
   gtgagtcgca cggggctgca cgcctgcctt acgggcctga acgcgaaccc gtgcatgatt 130680
   cgcgcgctgc ttcggcgcgg cgccagcgtg accgcaaaag acacctacga gatgacgccg 130740
   ctggcggtgc tgctgaagtc cgcgagcgcg acgccggagc tcgtgcgcat cctcgtggag 130800
   gcaggctccg acgtgagcgc caccgacttc cgcctcaacg gcatgctgca ccagcacgcg 130860
   cagtccacgc gcccgcgcgc gagcgtcatg cgcgagctca tccggctggg gtgcagccca 130920
   gcggccaaaa acatgtttgg gaacacgccg atgcacatgc tggccatgga aagctcctgc 130980
   cgccgctcac tgatcctccc gctgctggag gcagggcttt ccgtgaacga ggagaacccg 131040
   cactacggca ccgtgcctct gcacgtggcc tcggggtacg acaacacgca gggctgcctc 131100
   aagctcctcc gggatggagg agaccccacc gtcgtgtcgg ccgccggacg cacgccgctc 131160
   tcgaacatgc tcgtcaaacg caaccacgtg gcggtcgccg gcgcgctgtc gacgcacccg 131220
   agcgcggcag tagtcgtgca ggctctcgag caggctctcg agaacgtgct gaacgccggg 131280
   cccagcgagg cctcgcggct cgccgtggcc tttgtggtgg cgcgcgccgg cgcatccgcg 131340
   ctaccggagg ccgtgcgccg tctgcacgag ggctttgtcg ccgactgcga gcgcgaagtc 131400
   gagctgcttt cccgcaccat gctcggcaca ccggccgtga gcgcgctggt cgtgctggtc 131460
   agcaaggagg tctttggcac tgttatctcc tcgcgtgcgc tgcgcgtcgt gcgggaggtc 131520
   cgcgtgtacg caaggccgct ccgcgaggcg ctcataaatc tgcgccacaa atgccgctta 131580
   gtttccagcc ttaaaaggca ggtgggacct tgctcgctgc ccggcgaact ggtggagcgc 131640
   gtgctcgcga ccgtgccact gaccgacttg cgccgctcgt gcggccgccg cgcgcccgag 131700
   taactgcccg tcccgttgct acgcgactcg agactgcccg ctgtttttct ttccccgttt 131760
   cttcttatta ggagttgttg cccgcctcca tgatcctcgc acgcgccggc gggcaacctc 131820
   gcacgcccgc ggcggccgcg ggcgccgccg aggacggcga gcacagtgat cgccggaagc 131880
   gcaagcgcaa gacgcccaac tgcgaagacg ccgacaactc cgacgacgag ctagcgcaga 131940
   cgccgtgcga ccgcgagtgg ccggactgtc gcgcgagctc gatcacgagc tccgactcgg 132000
   tctctctcgg cgacgagatc tacctgcgat acgtggcctc gcaggtggac ttcgcgcaga 132060
   cctgggcccc gccagtgcgg ctgctgcgct ccttcgggaa cttctcgaag gaaacgctca 132120
   accgcatgtc gcggcgcggg tacgtgaacc gctcctactt ccagatggcg cacgcgcgct 132180
   tctcgcccac caacgacgac atgtaccaca tggccacggg cgggtacggc atcgtgttcc 132240
   gcttcgaccg ctacgtggtt aagtacgtct tcgagcaccg caacggcatg tccgagatgg 132300
   acgcctctac ggagtacacg gtgccgcggt tcctgcgcaa taacctcaag ggcgacgagc 132360
   gcgagttcgt ggtctgcgcg ctgcccatgg ggctgaacta ccggctgggc ttcctgcact 132420
   cgctgtaccg gcgcgtgctg cacacgctgc tgctgctcat gcgcgtggag gaaggccagc 132480
   ggccctcggt ggagatgtcc aagaagccgc tgctgcgctg gttcgaggcg cgcaaggaca 132540
   gcgagtcctt cgtgcgcctg atctcgtact tctacccctc ggccgtgcag agcaacgtga 132600
   acctgatcaa caacttccac cacctggtgc acttcttcga gcacgagaag cgcgcgcggt 132660
   acgtgttcga ccgcggggcc gtgatcgtgt tccctctggc gcgcgggtcc gcggactcga 132720
   tctcgccgga ggcggcggcg gcgctgggct tcgcgccgca ctcggagttc ctcaagttcg 132780
   tgttcctgca gatcgcgctg ctgtacctga agatctacga gctcccgggc tgcacgaact 132840
   tcctgcacgt ggacctgaag cccgacaacg tgctcatctt cgacagtgcg cgcgcgctca 132900
   gcgtgaccgc ggccggcgcg actttccgct tcgaggagcc cgtgcgcgcg gcgctgaacg 132960
   acttcgactt cgcgcgcgtg gccaccatcg agaaccgcaa gatcgcgggc agcgtccgcg 133020
   tgccgcagaa ctggtactac gacttccact tcttcgcgca cacgctgctg cgcgcgtacc 133080
   cgcacatcgc cgcggaggac ccgggcttcc acgcgctgct ctcggagctc acggtctcgt 133140
   gctcgcgcgg gacctgcgac cgcttccggc tgcgcgtgtc ctcgccgcac cccatcgagc 133200
   acctcgcgcg gctggtgcgc cgcgacgtct tctcccgctg gataaatgcc gccgcggacg 133260
   cccccgacgc cgccgcactc tcctgagccc acgcccgcgg cgccgggctc gctgtacgac 133320
   gtcttcctcg cgcgcttcct gcgccggctg gccgcgcgcg cggcgccggc ctcggccgcc 133380
   tgcgccgtgc gcgtgggtgc ggtgcgcggc cgcctgcgga actgcgagct agtggtgctg 133440
   aaccgctgcc acgcggacgc ggccggcgcg ctcgcgctag cctccgcggc gctcgccgag 133500
   acgctggcgg agctcccgcg cgcggacaag ctcgccgtcg cgctcgagct gggcgtggac 133560
   cccgagcacc cggagctgac gccggacccc gcctgcgcag gcgagagcgc actcgcacag 133620
   aacatcgaca tccagacgct ggacctgggc gactgcggag accccaaagg ccgccgactg 133680
   cgcgtggcgc tggtgaacag cggccacgcg gccgcgaact gcgcgctcgc gcgcgtggcg 133740
   accgcgctga cgcgccgcgt gcccgcgagc cggcacggcc tcgcggaggg cggcacgccg 133800
   ccgtggacgc tgctgctggc ggtggccgcg gtgacggtgc tcggcgtggt ggcggtttca 133860
   ctgctgcggc gcgcgctgcg ggtacgctac cgcttcgcgc ggccggccgc gctgcgcgcg 133920
   tagccgcgca aaatgtaaat tataacgccc aacttttaag ggtgaggcgc catgaagttg 133980
   ctcgtcggca tactagtagc cgtgtgcttg caccagtatc tgctgaacgc ggacagcaac 134040
   acgaaaggat ggtccgaagt gctgaaaggc agcgagtgca agcctaggcc gattgttgtt 134100
   cctgtaagcg agacgcaccc agagctgact tctcagcggt tcaacccgcc gtgtgtcacg 134160
   ttgatgcgat gcggcgggtg ctgcaacgac gagagcttgg aatgcgtccc cacggaagaa 134220
   gtaaacgtga cgatggaact cctgggggcg tcgggctccg gtagtaacgg gatgcaacgt 134280
   ctgagcttcg tagagcataa gaaatgcgat tgtagaccac gattcacaac cacgccaccg 134340
   acgaccacaa ggccgcccag aagacgccgc tagaactttt tatggaccgc agatccaaac 134400
   gatgatgcga tcaggtcatg cggaagaagg cgccacggag caaagtgaaa aaggaccgcc 134460
   tagcagtcga gaccctcccg ccgcagccgc ggacacccca cacccgcctt ccacccgcca 134520
   gacgccaaca ccgcagccaa caagcatgca cccctcgccg cgcaggctgc tcggcgcgct 134580
   cgcgctggtg gcgctgggct tcctcctcgg cgggctcttc cgccccgcgg cgccgccgct 134640
   gccggccgcc ctcgtggagg cgggccccgt ccgcgcgaac ggctccgcct cggtgacctg 134700
   cctgaccgtc ggcggcgacg ggcggcacat ggcggtggtc gcgcacggcg gcgggacgct 134760
   ctcgccggtg tacccgctcg ccgccggcat gcacgcgacc ttcgcctcgc tgcgcaaggg 134820
   cgcgctgctg ctgaacgtcg cgaccgtgca catctacgac gtgcgcgcgc tcgggccgga 134880
   gttcgagctg acctgcgtcg cggtggcggg cggctacaac gcggcctggg cggccgcgcg 134940
   gcccgcggcc gagtggcgcc gccagctggc gcggatgcac cgctcggagc tgtgaccctc 135000
   tccctcccgg tctcccatcc gtttttgtaa tcggccttag tagattagac cagcatcccg 135060
   cgcccttgtc cgagaacaag taacagtaac cgttacctca ctcgccactc ctcggaataa 135120
   tagaacgaga gaacgagaga acgagttaac cgttgctcac tcgctcactc ggtgtgagag 135180
   aacaagtaac gttgctcact cgctcactcg gtgtgagaga acaagagaac gagtagctgt 135240
   tgctaactca atcacccctc ggagtaagag aacaagagca gtcaactacc cactcagtct 135300
   tggatgagag gcagaggacg agttgacgag ttgaacagtt aatcctcact cactcagagc 135360
   gagagagcga gagagtggag gacgagttaa caagtcaatc ctcactcaga gtgagagagt 135420
   gagagagtgg aggacgagtt aatggttaac agttatcacc actcagagtg agagcggagg 135480
   acgagtcaac cactcgctcg cccactccga gttagagagg gaaccagtgc gagttaacgc 135540
   gcacacgagc gagagaacgt aaactcgctc gcgcgctcgc tcggctaacc gtcggcctct 135600
   cccaaaactc ttcgtaaaac tttcccgatg acagttcacc ctccaaaact ttgtaaaact 135660
   aaactgttcg gaggtcggtc tgctgcctct ctaactctcc gtaaaacgtt tgtaaactgt 135720
   cggaggtcgg tgacccgctc aaccgtccgc gaaaactttt cgcaggcagt gtctgcctct 135780
   ctcggactct ccgcaaacac tttcgcggaa cctcggaggg tggtcgacct ctctccaaac 135840
   tcttgcaaaa ctttttcgcg gaaccgttgg aggccagtcc tccctccaaa ctctttgtaa 135900
   aatcttttcg aggccagtcc tcctctccaa aacgttccgc aaaatctttg ggaggtcggc 135960
   ctctcctctc cagaacgttc cgtaaaactc ttggaggccg cccgcggcac gcgaggcgga 136020
   ggatccgagg tgtcgacctc cctcaaaaac tttgtaaaaa ctttttataa aactttccgc 136080
   ggaacctcgg agagtaggtc gacctctctc aaaactctta taaaactttt ccgcggaacc 136140
   gttggaaggt aggtcgacct ctctcaaaac tcttataaaa cttttccgcg gaaccgttgg 136200
   aaggtaggtc gacctctctc aaaactctta taaaactttt ccgcggaacc gttggaggca 136260
   ggtcggcctc tcaaactctt tgcgagaact cttcgcgaga actcttcgat aactttagga 136320
   ggtcaggtcg acctcccaaa acttttgcga gaactctctg taaaacttta ggaggtcggt 136380
   acctccctca aaacttttta taaaactttt cgcggaacct ccggagacgg gccgccgccg 136440
   cgaccgcggg agcggagagg ccgacctccc gagacgttcc gcgttaccgt cggggtaggc 136500
   gtcctctcga gaacgccaaa agacttcgtg caaaaacttt tcggaggggc gcggagggcg 136560
   ggtcagctcc cgcgaactcc cgcagaacct tttcgcgcga ccgcgaaggc cggccgcctc 136620
   tcccacactc tcaagagctt ttcggaggag aggaagggca ggtcgccccc acctccgacg 136680
   ctttgtaaaa acgtttacgc ggaacctcgg aggcaggtcg cctccctcga aaactcctcg 136740
   cgaaaccttt aaaaactttt tgcgaaaact tttcggagga tgtcggaggg cgggcggctc 136800
   ttccaaacct ccgcagaacc ttttcgcgca accgttggaa gacaggtcgg cctctctcga 136860
   aaacttttaa aactttgtaa acgcgttggc gggaccgtcg cgggagagcg gccgcccgcg 136920
   gcacgcgaga ggaggaaccg ttggaaggca gtcggcctct cccgaaaact ttttataaaa 136980
   acttttccgc ggaaccgttg gaggcaggtc ggcctctctc agagtctaaa aactttttgc 137040
   gggactcgga cggcgcggtc acccgaccac ctgactcctg tctcacccgt actacttgga 137100
   cttctgtttc cctgactccc gactccctga cctcccgact ccctgactcc cgactccctg 137160
   actcccgact ccctgactcc cgactccctg actcccgact ccctgactcc cgactccctg   137220
   actcccgact ccctgactcc cgactccctg actcccgact ccctgactcc cgactccctg   137280
   actcccgact ccctgactcc cgactccctg actcccgact ccctgactcc cgactccctg   137340
   actcccgact ccctgactcc ctgactccag agcgaggtct cgcggctgcg gggtgccgcc   137400
   tccgcggagt cgcgttcccg cggacgcccg tcctcgaaag cattcagcag ttccagcctc   137460
   tgccgtagct cctcccgcag gaactcctgg tccgcgttcg tcgcggcacc gcggctcagc   137520
   cgccgcggga gccggccgcc gcccgcgaag ccgcggatcc                          137560

## Description

### FIELD OF THE INVENTION

The present invention relates to the treatment of coronavirus infections and to preparing subjects for such an infection by administering *Parapoxvirus ovis.* This treatment is to assist the immune system in combatting the virus and to thereby prevent and to ameliorate symptoms of coronavirus disease.

### BACKGROUND OF THE INVENTION

Coronaviruses are enveloped, positive-sense single-stranded RNA viruses, which cause diseases in mammals and birds. In humans, they cause respiratory infections, including the common cold, although some viruses such as SARS and MERS coronaviruses can be lethal. Novel coronaviruses emerge periodically in different areas around the world. SARS-CoV (severe acute respiratory syndrome coronavirus) occurred in 2002 and caused 916 deaths during the epidemic, having a mortality rate of about 12%. MERS-CoV (middle east respiratory syndrome coronavirus) emerged in 2012, with 543 dead reported and a mortality rate of about 39%.

Recently, an outbreak of a coronavirus designated SARS-CoV-2 began in Wuhan, China, and has since spread all over the world. The illness caused by SARS-CoV-2, COVID-19 (coronavirus disease 2019) was declared to be a pandemic by the WHO on March 11, 2020. COVID-19 ranges in severity from asymptomatic or mild to severe, and a significant portion of patients with clinically evident infection develop severe disease. The case fatality rate varies by country and is thought to be between 2% and 3%, but the true mortality rate is uncertain, because the total number of cases is unknown. While SARS-CoV-2 apparently has a lower mortality rate than SARS-CoV and MERS-CoV, it appears to be more infectious and has been able spread around the world, with around 3.66 million confirmed cases and 257,000 confirmed deaths by May 6, 2020 and presumably many more unconfirmed ones. The knowledge of COVID-19 is incomplete and evolving.

The recurrent coronavirus epidemics show that these viruses continue to threaten the world as they emerge spontaneously, spread easily and can have severe consequences on human life, including not only health issues but also dramatic social and economic impacts. Therefore, there is a great medical need for prevention and treatment. However, since coronaviruses are known to mutate and recombine often, they present an ongoing challenge for disease control. Neither vaccines nor antiviral drugs for preventing or treating human coronavirus infections are currently available. A vast number of antivirals are being tested around the world for an effect on coronavirus, with promising candidates failing.

Unexpectedly, the inventors found *Parapoxvirus ovis* (PPVO) to be useful for preparing subjects for and treating coronavirus infections. PPVO causes acute dermal infections in goat and sheep, and while it leads to no serious disease in humans, it induces a complex innate and adaptive immune response in humans and treats infections with some specific viruses such as HBV (WO 2019/048640 A1). However, the immune response induced by PPVO is complex and not necessarily useful for treating viral infections in general. Therefore, PPVO could not have been expected to have utility in preparing subjects for and treating coronavirus infections.

### SUMMARY OF THE INVENTION

In a first aspect, the present invention relates to a *Parapoxvirus ovis* (PPVO) agent for use in (i) preparing a subject for a coronavirus infection or (ii) treating a coronavirus infection in a subject. In a second aspect, the invention relates to a composition or a kit comprising a *Parapoxvirus ovis* (PPVO) agent and a coronavirus agent. In a third aspect, the present invention relates to a pharmaceutical composition or a kit comprising a *Parapoxvirus ovis* (PPVO) agent for the medical use according to the invention, wherein the pharmaceutical composition or the kit comprises a PPVO agent and preferably a further medicament.

### LEGENDS TO THE FIGURES

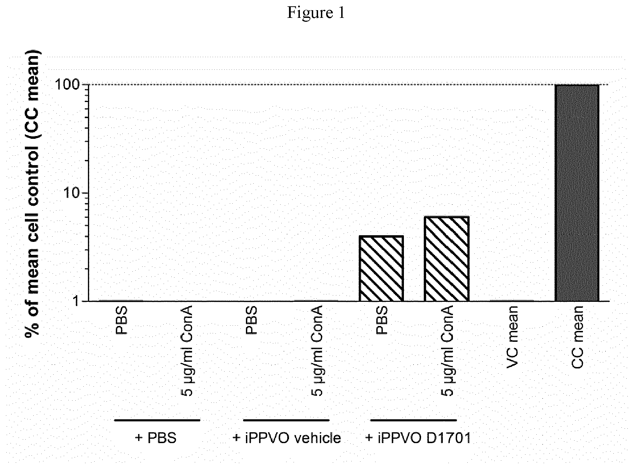

**Figure 1****: Stimulation of inactivated parapoxvirus (iPPVO) induced antiviral activity against SARS-CoV-2 that was enhanced by combinatorial approaches.** Whole-blood cultures were stimulated with iPPVO +/- 5 µg/ml ConA. Where no ConA was added, PBS was added instead. iPPVO vehicle +/- ConA or PBS +/- ConA, respectively, served as a controls. 5 µl/ml ConA was tested as a co-stimulus. Cultures were incubated for 3 days and supernatants were harvested. The supernatant was transferred to Vero-eGFP cells which were 2-4 hours later infected with SARS-CoV-2 to determine the antiviral activity of the respective supernatant samples and incubated for 5 days. eGFP fluorescence as a measure for living cells was analyzed using the ImageJ software. The mean of the cell control to which no virus was added served as positive control (CC mean) and was set to 100 % (see upper dotted line). Cells infected with SARS-CoV2 but not treated gave the highest possible destruction of living cells and served as negative control given as the mean virus control (VC mean). The antiviral activity of the samples were calculated relative to the positive control CC mean and given in %.

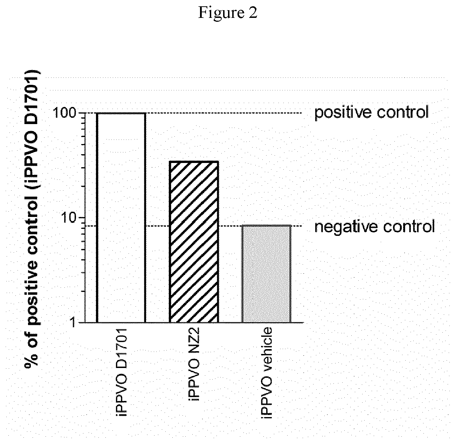

**Figure 2****: iPPVO D1701 and iPPVO NZ2 stimulate antiviral activity against SARS-CoV-2.** Whole-blood cultures were stimulated with iPPVO. iPPVO vehicle served as a negative control. iPPVO D1701 served as positive control. Cultures were incubated for 3 days and supernatants were harvested. The supernatant was transferred to Vero-eGFP cells which were 2-4 hours later infected with SARS-CoV-2 to determine the antiviral activity residing in the respective supernatant samples and incubated for 5 days. eGFP fluorescence as a measure for living cells which have been protected against SARS-CoV-2 due to the antiviral activity of cytokines in the respective supernatant was measured using the ImageJ software. The antiviral activity of the positive control was set to 100 % (see upper dotted line). The antiviral activity of the samples were calculated relative to the positive control and given in %. The lower dotted line marks the value of the negative control.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention, which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments, which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the word "comprise", and variations such as "comprises" and "comprising", are to be understood to imply the inclusion of a stated integer or step or group of integers or steps but not the exclusion of any other integer or step or group of integer or step. In preferred embodiments, "comprise" can mean "consist of'. As used in this specification and the appended claims, the singular forms "a", "an", and "the" include plural referents, unless the content clearly dictates otherwise.

In a first aspect, the present invention relates to a *Parapoxvirus ovis* (PPVO) agent for use in (i) preparing a subject for a coronavirus infection or (ii) treating a coronavirus infection in a subject.

### Parapoxvirus agent

The parapoxvirus is *Parapoxvirus ovis* (PPVO). This includes any PPVO strain, preferably, any strain of the species PPVO as classified by the International Committee on Taxonomy of Viruses (ICTV). Exemplary strains are NZ2, NZ7, NZ10, D1701, OV/20, OV/7, OV/C2, OV/mi-90, OV-Torino, SAOO, Bo29, orf11, Greek orf strain 155, and Greek orf strain 176. Preferred strains are NZ2 and D1701, especially NZ2.

The PPVO agent is, in a preferred embodiment,
(a) PPVO itself or a fragment thereof, or
(b) an agent that comprises genetic information encoding for (a).

In a more preferred embodiment, it is
(i) a live PPVO virion,
(ii) an inactivated PPVO virion,
(iii) a fragment of (i) or (ii),
(iv) a nucleic acid encoding for any of (i) to (iii),
(v) a vector comprising the nucleic acid of (iv), or
(vi) a cell comprising the nucleic acid of (iv) or the vector of (v).

The live PPVO virion may or may not be attenuated. Attenuated PPVO virions are known in the art, e.g. virions lacking a virulence gene such as vegf-e and/or gif. The fragment can be recognized by the immune system and preferably stimulates an immune response (i.e. it is an antigen and preferably an immunogen) and can be any fragment, although preferably it is a fragment that is bound by a human pattern recognition receptor (PRR), e.g. TLR9. In one embodiment, the PPVO fragment is a PPVO protein.

Exemplary fragments are (exemplary coding sequence (nucleotide positions of SEQ ID NO: 3 representing strain NZ2) in parenthesis preceding the fragment): (3 to 539) ORF L1, (781 to 449) ORF L2r, (1933 to 1664) ORF L3r, (3269 to 2790) ORF L4r, (2799 to 3851) ORF L5, (2962 to 3753) ORF L6, (3784 to 3122) ORF L7r, (4341 to 4129) ORF L8r, (4904 to 4428) ORF 1ar, (6517 to 4970) ORF 1r, (8042 to 6684) ORF 2r, (9989 to 8070) ORF 3r, (11195 to 10062) ORF 4r, (11493 to 11227) ORF 5r, (11802 to 12038) ORF 6, (12358 to 12080) ORF 7r, (13980 to 12364) ORF 8r, (14826 to 14053) ORF 9ar, (15080 to 15394) ORF 10, (16838 to 15423) ORF 11r, (19021 to 16847) ORF 12r, (19704 to 19156) ORF 13r, (20314 to 19736) ORF 14r, (20401 to 22101) ORF 15, (22125 to 22940) ORF 6, (23003 to 23866) ORF 17, (26908 to 23873) ORF 18r, (26926 to 27213) ORF 19, (27626 to 27216) ORF 20r, (29754 to 27616) ORF 21r, (32217 to 29800) ORF 22r, (33380 to 32418) ORF 23r, (33602 to 33393 ORF 24r, (34466 to 33612) ORF 25r, (34735 to 34502) ORF 26r, (35905 to 34739) ORF 27r, (37194 to 35905) ORF 28r, (37200 to 39248) ORF 29; 41037 to 39229) ORF 30r, (41374 to 42066) ORF 31, (42336 to 41731) ORF 32r, (42407 to 41997) ORF 33r, (42410 to 43765) ORF 34, (43770 to 43958) ORF 35, (43980 to 44534) ORF 36, (45727 to 44537) ORF 37r, (45760 to 46557) ORF 38, (46567 to 47568) ORF 39, (47572 to 48303) ORF 40, (48352 to 48621) ORF 41, (49887 to 48634) ORF 42r, (49917 to 50793) ORF 43, (50719 to 51102) ORF 44, (51059 to 51511) ORF 44a, (51584 to 52591) ORF 45, (52509 to 53066) ORF 46, (53523 to 53023) ORF 47r, (53607 to 57473) ORF 48, (58070 to 57528) ORF 49r, (57700 to 58662) ORF 50, (59674 to 58673) ORF 51r, (62089 to 59678) ORF 52r, (62198 to 62881) ORF 53, (62909 to 63862) ORF 55, (63858 to 64271) ORF 56, (64309 to 66831) ORF 57, (67266 to 66799) ORF 58r, (67803 to 67273) ORF 58ar, (67915 to 68607) ORF 59, (68624 to 70984) ORF 60, (70994 to 72898) ORF 61, (72938 to 73507) ORF 62, (73540 to 74211) ORF 63, (76120 to 74207) ORF 64r, (76749 to 76186) ORF 65r, (77698 to 76799) ORF 66r, (79343 to 77709) ORF 67r, (79816 to 79367) ORF 68r, (80529 to 79858) ORF 69r, (80774 to 80529 ORF 70r, (82815 to 80788) ORF 71r, (83835 to 82834) ORF 72r, (83874 to 85583) ORF 73, (85535 to 84402) ORF 74r, (88096 to 85574) ORF 75r, (87759 to 88667) ORF 76, (88920 to 88642) ORF 77r, (91652 to 88938) ORF 78r, (91667 to 92674) ORF 79, (93466 to 92681) ORF 80r, (93761 to 93486) ORF 81r, (94060 to 93788) ORF 82r, (94238 to 94080) ORF 83r, (94508 to 94242) ORF 84r, (95571 to 94498) ORF 85r, (96187 to 95600) ORF 86r, (96202 to 97665) ORF 87, (97915 to 97643) ORF 88r, (98251 to 99537) ORF 89, (99537 to 99974) ORF 90, (100001 to 101140) ORF 91, (101168 to 104650 ORF 92, (106354 to 104795) ORF 93r, (107947 to 106400) ORF 94r, (108256 to 107990) ORF 95r, (108719 to 108300) ORF 96r, (109679 to 108738) ORF 97r, (109861 to 109682) ORF 98r, (110830 to 10033) ORF 99r, (110208 to 110417) ORF 100, (110469 to 110651) ORF 100a, (110915 to 111397) ORF 101, (111419 to 111913 ORF 102, (111949 to 112485) ORF 103, (112593 to 113450) ORF 104, (113323 to 112967) ORF I05r, (113526 to 114152) ORF 106, (114199- to 115236) ORF 107, (115353 to 115787 ORF 108, (115859 o 116551) ORF 109, (116729 to 117523) ORF 110, (117572 to 117114) ORF 111r, (117423 to 118085) ORF 12, (118968 to 118375) ORF 114r, (118508 to 119119) ORF' 115, (119588 to 120202) ORF 116, (120314 to 21231) ORF 117, (121380 to 123920) ORF 118, (121288 to 122256) ORF 119, (122350 to 123924) ORF 120, (123962 to 125566) ORF 121, (125193 to 124591) ORF 122r, (125689 to 123935 ORF 123r, (123839 to 123297) ORF 123ar, (125652 to 126170) ORF 124, (126121 to 125699) ORF 125r, (126279 to 127769) ORF 126, (127851 to 128408) ORF 127, (128520 to 130076) ORF 128, (130105 to 131700) ORF 129, (131790 to 133283) ORF 130, (133246 to 133920) ORF 131, (133972 to 134370) ORF 132, (134418 to 134693) ORF 133a, (134402 to 134992) ORF R1, (134853 to 134419) ORF R2r, (135628 to 135897) ORF R3, (136780 to 137112) ORF R4, and (137558 to 137022) ORF R5r. The group of fragments includes homologs of the exemplary fragments listed above, i.e. homologous fragments of another PPVO (e.g. species or strains as described above). It also includes functional variants of the exemplary fragments listed above (the function being as defined for the fragments generally above). Thus, the group of fragments includes variants with at least 80%, 85%, 90%, 95%, 96%, 97%, 98% or preferably at least 99% sequence identity to those fragments encoded by the exemplary coding sequences of SEQ ID NO: 3 listed above.

The inactivated PPVO virion can be obtained by any means known in the art suitable for inactivating enveloped viruses, for example by physical inactivation (e.g. exposure to heat, specifically pasteurization, or UV light) or by chemical inactivation (e.g. by exposure to low pH, to a detergent or to an inactivation agent such as ethyleneimine, binary ethyleneimine, formaldehyde, glutaraldehyde, 2,2'-dithiodipyridine, or beta-propiolactone). In a preferred embodiment, the inactivated PPVO virion is an ethyleneimine- or binary ethyleneimine-inactivated virion, preferably a binary ethyleneimine-inactivated virion.

The PPVO agent may comprise genetic information (DNA or RNA) encoding for a heterologous (i.e. not of parapoxvirus) antigen, and/or an immunomodulator, or in may alternatively not comprise genetic information encoding for a heterologous antigen and/or an immunomodulator. In one embodiment, it does not comprise genetic information encoding for a heterologous antigen, but it optionally comprises genetic information encoding for immunomodulator.

In a preferred embodiment, the PPVO agent is wildtype PPVO. Preferably it is a PPVO virion, more preferably an inactivated PPVO virion (iPPVO).

### Modes of use

The PPVO agent can be used alone or in combination with a further treatment regimen for (i) preparing a subject for a coronavirus infection or (ii) treating a coronavirus infection in a subject.

In a preferred embodiment, both of the preparing and the treating, the further treatment regimen can comprise administering a coronavirus agent.

Specifically for the preparing, the further treatment regimen can comprise administering a further immune stimulatory agent (e.g. an adjuvant, a TLR agonist, a PRR, e.g. a cytosolic PRR such as RIG-I-like receptors including RIG-I, MDA5 and LGP2, or a mitogen, particularly a T cell mitogen or activator such as phytohemagglutinin (PHA), Concanavalin (Con) A, wheat germ agglutinin (WGA), pokeweed mitogen (PWM), a lectin or an anti-CD3 antibody or antigen-binding fragment thereof), or a B cell mitogen or activator such as PWM, lipopolysaccharide (LPS), an anti-CD40 antibody or antigen-binding fragment thereof, or an anti-(B cell antigen receptor) antibody or antigen-binding fragment thereof. The immune stimulatory agent preferably promotes an antiviral cell state. Specifically for the treating, the further treatment regimen can comprise administering one or more further medicaments and/or one or more further therapies (non-medicament/drug treatments) suitable for the treatment of a subject infected by coronavirus or having coronavirus disease. The one or more further medicaments are preferably selected from the group consisting of a further immune stimulatory agent (as exemplified above), an antiviral, an antibiotic, an adjuvant, a glucocorticoid, an antihypertensive drug (e.g. an ACE inhibitor), a hypoglycaemic drug, an anti-shock drug, and a drug suitable for treating one or more symptoms of coronavirus disease and/or to accompany the one or more further therapies (e.g. a pain reliever, a sedative, an anti-fever drug, an antiinflammatory drug such as IL-10, cough medication or an immunosuppressant). The one or more further therapies are preferably selected from the group consisting of oxygen therapy and extra-corporeal organ support (ECOS). Oxygen therapy can be passive (i.e. relying on breathing of the subject) or active (i.e. delivering oxygen to the lungs without relying on breathing of the subject). Passive therapies are non-pressurized oxygen therapy and pressurized oxygen therapy. Active therapies are mechanical ventilation and extra-corporeal membrane oxygenation (ECMO, also a specific form of ECOS). Examples for ECOS include ECMO, continuous renal replacement therapy (CRRT), hemofiltration (HF) and hemoperfusion (HP) e.g. to remove inflammatory cytokines, and left ventricular assistance.

Specific examples of further medicaments for preparing and treating are: Ivermectin, TMPRSS2 inhibitors (e.g. ammonium chloride, serine protease inhibitor camostat mesylate) optionally together with a CatB/L inhibitor (e.g. E-64d), Favipiravir, Pimodivir, Baloxavir optionally together with Marboxil, IL-6 blockers (e.g. sarilumab, tocilizumab), Sarilumab, Zitivekumab, lopinavir optionally together with ritonavir, chloroquine, hydroxychloroquine, Remdesivir, α-Ketoamides (e.g. cyclopentylmethyl, cyclohexylmethyl or α-Ketoamide 13b), Thalidomide, Maraviroc, Tenofovir optionally together with Disoproxil, Dolutegravir, Nelfinavir, Raltegravir, Tipranavir and Type I interferons (IFN-I, specifically IFN-β1). Preferred examples are Remdesivir and Thalidomide.

The PPVO agent can be administered by any route, including but not limited to the intravenous, intramuscular, oral, parenteral, topical, intradermal, or subcutaneous route. In a specific embodiment, it can be administered directly to the respiratory tract, preferably to the lower respiratory tract, more preferably to the lung (pulmonary delivery). As such, it can be administered as an aerosol and/or via inhalation.

The dose of the PPVO agent (in fact of all agents described herein, including a further immune stimulatory agent, a further medicament and a coronavirus agent) is a pharmaceutically effective dose as can be determined by the skilled person. For instance, in case of the agent being a virion (live or inactivated), it can be in the range of from 10⁶ to 10¹⁰ particles. The dose may be administered once or more than once, for example over a period of < 12 weeks, < 6 weeks, < 4 weeks, < 2 weeks, or < 1 week.

### Coronavirus

The coronavirus is, in a preferred embodiment, an alpha-coronavirus or a beta-coronavirus. More preferably, it is a respiratory coronavirus, i.e. a coronavirus that causes a respiratory disease. Examples of alpha-coronaviruses are HCoV-229E and CoV-NL63, and examples of beta-coronaviruses are SARS-CoV, SARS-CoV-2, MERS-CoV, HCoV-HKU1, and HCoV-OC43. It is preferred that the coronavirus is a MERS or a SARS coronavirus, wherein preferably the SARS coronavirus is a virus of the species "*severe acute respiratory syndrome-related coronavirus*" as classified by the International Committee on Taxonomy of Viruses (ICTV). In the most preferred embodiment, it is SARS-CoV-2.

The skilled person appreciates that coronaviruses mutate and that new strains of SARS-CoV-2 are isolated daily, with 461 strains having been isolated by April 6, 2020. Thus, in terms of a sequence definition, a preferred virus of the invention has a genome comprising or consisting of a sequence according to SEQ ID NO: 1 (original strain of the 2019 outbreak "Severe acute respiratory syndrome coronavirus 2 isolate Wuhan-Hu-1", NCBI Reference Sequence NC_045512.2, version of March 30, 2020) or a variant thereof with at least 80%, 85%, 90%, 95%, 96%, 97%, 98% or preferably at least 99% sequence identity. Alternatively or in addition, a preferred virus of the invention has a genome comprising or consisting of a sequence according to SEQ ID NO: 2 (a SARS-CoV-2 consensus sequence of 153 genome assemblies) or a variant thereof with at least 80%, 85%, 90%, 95%, 96%, 97%, 98% or preferably at least 99% sequence identity. It is preferred that the variant is classified as a strain of SARS-CoV-2 by the International Committee on Taxonomy of Viruses (ICTV).

### Coronavirus agent

A coronavirus agent generally is an agent that is suitable for treating a coronavirus infection. It can be (a) the coronavirus itself or a fragment thereof which is recognized by and preferably stimulates an immune response (e.g. an antigen and preferably immunogen), (b) an agent that comprises genetic information encoding for (a), or (c) an agent which interferes with the infection of a cell by the coronavirus or with the replication of the coronavirus within a cell (in a therapeutic setting: is capable upon administration of an effective amount thereof to a subject infected with coronavirus to interfere with the infection of a cell by the coronavirus or with the replication of the coronavirus within a cell). The coronavirus agent is preferably selected from the group consisting of
(i) an inactivated coronavirus,
(ii) an antigenic, preferably immunogenic fragment of coronavirus (e.g. the spike protein or a fragment thereof),
(iii) a nucleic acid encoding for (i) or (ii),
(iv) a vector comprising the nucleic acid of (iii),
(v) a cell comprising the nucleic acid of (iii) or the vector of (iv),
(vi) an antibody or an antigen-binding fragment thereof binding to coronavirus or a fragment thereof, preferably the envelope,
(vii) a nucleotide/nucleoside analogue, preferably coronavirus-specific,
(viii) a coronavirus-specific RNAi molecule,
(ix) a small molecule, an antibody or an antigen-binding fragment thereof, binding to a site of protein-protein interaction (PPI) between a coronavirus protein and a subject protein, and
(x) blood plasma of a subject that has recovered from a coronavirus infection, preferably from coronavirus disease.

A coronavirus can be inactivated as described for PPVO above.

The PPI of (ix) is preferably an interaction of a SARS-CoV-2 protein or homologue thereof of another coronavirus and a subject protein (Uniprot protein ID of each subject protein is <name recited below>_HUMAN) selected from
- SARS-CoV2 E and AP3B1, BRD4, BRD2, CWC27, ZCH18 or CTL2;
- SARS-CoV2 M and MPPB, YIF1A, AT1B1, ACADM, ETFA, STOM, VKGC, VATA, PSMD8, REEP5, MPPA, ANO6, PREP, ZNT9, FAKD5, ZNT7, GCP3, COQ8B, SAAL1, REEP6, INT4, ODC, GCP2, SYTM, RTN4, FA8A1, AASS, AKP8L, AAR2 or BZW2
- SARS-CoV2 N and U3IP2, PABP1, CSK22, CSK2B, G3BP1, PABP4, LARP1, FA98A, SNIP1, RENT1, MOV10, G3BP2, DDX21, RBM28 or RL36;
- SARS-CoV2 Spike and ACE2, GOGA7 or ZDHC5;
- SARS-CoV2 nsp1 and DPOLA, PRI1, PRI2, DPOA2, GT251 or PKP2;
- SARS-CoV2 nsp10 and AP2A2, ALR, ERGI1, AP2M1 or GRPE1;
- SARS-CoV2 nsp11 and TBCA;
- SARS-CoV2 nsp12 and SBNO1, BCKD, AKAP8, MYCB2, SLU7, RIPK1, UBP2L, TYSD1, PDZ11, PRC2B, UBAP2, ZN318, CRTC3, UBP54, Z3H7A, LAR4B, RBM41, HTF4, PPIL3 or PKHA5;
- SARS-CoV2 nsp13 and TBKB1, CTRO, HSBP1, PCNT, FR1OP, KAP2, KAPCA, KAP3, RADI, CENPF, TLE1, TLE3, TLE5, GOGA3, GOGA2, GOGB1, GRAP1, CE350, MYOME, CP135, CEP68, CNTRL, RB6I2, GCC2, CLIP4, NIN, CE112, MIPO1, UBP13, GCC1, JKIP1, CK5P2, AKAP9, GORS1, FYCO1, CA050, CP250, TBK1, HOOK1 or NINL;
- SARS-CoV2 nsp14 and AGAL, IMDH2 or SIR5;
- SARS-CoV2 nsp15 NTF2, ARF6 or RNF41;
- SARS-CoV2 nsp2 and S27A2, IF4E2, NCPR, GDS1, WASC4, FKB15 or GGYF2;
- SARS-CoV2 nsp4 and IDE, TIM10, ALG11, PO210, TIM29, DJC11, T10B or TIM9;
- SARS-CoV2 nsp5 and HDAC2;
- SARS-CoV2 nsp5_C145A and GPX1 or TRM1;
- SARS-CoV2 nsp6 and ATP5L, VAS1, SGMR1 or AT133;
- SARS-CoV2 nsp7 and ADAS, CYB5B, ACSL3, NB5R3, RALA, COMT, RAB5C, RAB7A, RAB8A, RAB2A, RAB10, RAB14, RHOA, RAB1A, GBB1, GBG5, LMAN2, MOGS, TOIP1, MARC1, QSOX2, HS2ST, NDUF2, SCPDL, SCRB1, NAT14, DCAKD, F162A, TIM14, SELS, PGES2 or RAB18;
- SARS-CoV2 nsp8 and MPP10, SRP72, ATE1, NSD2, SRP19, SRP54, RT25, DDX10, LARP7, MEPCE, NGDN, EXOS8, SYNM, NOL10, CCD86, SPCS, EXOS5, EXOS3, AATF, HECD1, RT02, RT05, EXOS2 or RT27;
- SARS-CoV2 nsp9 and T2FB, FBN1, FBN2, NU214, NUP62, DCAF7, IF4H, NUP54, MIB1, NEK9, ZN503, NUP88, NUP58, MAT2B or FBLN5;
- SARS-CoV2 orf10 and PPT1, CUL2, MA7D1, THTPA, ZY11B, TIM8B, RBX1, ELOC or ELOB;
- SARS-CoV2 orf3a and HMOX1, TRI59, AR6P6, VPS39, CLCC1, VPS11, SUN2 or ALG5;
- SARS-CoV2 orf3b and STML2;
- SARS-CoV2 orf6 and NUP98, RAE1L or MTCH1;
- SARS-CoV2 orf7a and HEAT3 or MDN1;
- SARS-CoV2 orf8 and PLOD2, TOR1A, STC2, TPA, ITB1, CISD3, CO6A1, PVR, DNMT1, LYOX, PCSK6, INHBE, NPC2, MFGM, OS9, NPTX1, PLGT2, PLGT3, EROIB, PLD3, FXRD2, CHSS2, PUSL1, EMC1, GGH, ERLEC, I17RA, NGLY1, H6ST2, SDF2, NEUR1, GDF15, TM2D3, ERP44, EDEM3, SIL1, OFUT1, SMOC1, PKHF2, FXL12, UGGG2, CHPF2, ATS1, HYOU1, FKBP7, ADAM9 or FKB10;
- SARS-CoV2 orf9b and NHRF1, CHM2A, CSDE1, TOM70, MARK3, MARK2, DPH5, DCTP1, MARK1, PTBP2 or BAG5; and
- SARS-CoV2 orf9c and UBXN8, GPAA1, WFS1, MRP1, PAR2, SCAP, D19L1, SGMR2, ZNT6, TAPT1, ERMP1, NLRX1, RETR3, PIGO, FACR2, ECSIT, ALG8, TM39B, GHITM, ACAD9, NFIP2, BCS1, CIA30, TMED5, NDUB9 or PIGS.

An antibody or an antigen-binding fragment thereof to a PPI site can be generated by routine methods. For instance, antibodies or antigen-binding fragments thereof to one of the interacting proteins can be generated and then screened for interfering with the interaction.

The small molecule of (ix) is preferably selected from the group consisting of Loratadine, Daunorubicin, Midostaurin, Ponatinib, Silmitasertib, Valproic Acid, Haloperidol, Metformin, Migalastat, S-verapamil, Indomethacin, Ruxolitinib, Mycophenolic acid, Entacapone, Ribavirin, E-52862, Merimepodib, RVX-208, XL413, AC-55541, Apicidin, AZ3451, AZ8838, Bafilomycin A1, CCT 365623, GB110, H-89, JQ1, PB28, PD-144418, RS-PPCC, TMCB, UCPH-101, ZINC1775962367, ZINC4326719, ZINC4511851, ZINC95559591, 4E2RCat, ABBV-744, Camostat, Captopril, CB5083, Chloramphenicol, Chloroquine, PI4K-IIIβ inhibitor Compound 10 (Rutaganira, F. U. et al., J. Med. Chem. 59, 1830-1839 (2016)), Cyclophilin inhibitor Compound 2 (Mackman, R. L. et al., J. Med. Chem. 61, 9473-9499 (2018)), CPI-0610, Dabrafenib, DBeQ, dBET6, IHVR-19029, Linezolid, Lisinopril, Minoxidil, ML240, MZ1, Nafamostat, Pevonedistat, PS3061, Rapamycin (Sirolimus), Sanglifehrin A, Sapanisertib (INK128/M1N128), FK-506 (Tacrolimus), Ternatin 4 (DA3), Tigecycline, Tomivosertib (eFT-508), Verdinexor, WDB002, Azacitidine, Selinexor, and Zotatifin (eFT226).

### Coronavirus infection/disease

A coronavirus infection is defined by the entry of coronavirus into at least one cell of a subject and its replication in the at least one cell. Preferably, the infection is an infection of the respiratory tract, including the upper respiratory tract (nose and nasal passages, paranasal sinuses, the pharynx, and the portion of the larynx above the vocal folds (cords)) and the lower respiratory tract (portion of the larynx below the vocal folds, trachea, bronchi, bronchioles and the lungs including the respiratory bronchioles, alveolar ducts, alveolar sacs and alveoli). More preferably, it is an infection of the lower respiratory tract, most preferably of the lungs (including one or more of respiratory bronchioles, alveolar ducts, alveolar sacs and alveoli). The infection can further be characterized immunologically by the presence of at least one coronavirus-antigen-specific immune factor, preferably selected from the group consisting of B cells, follicular helper T cells (T_{FH} cells), activated CD4⁺ T cells and CD8⁺ T cells (particularly also CD38⁺HLA-DR⁺), IgM antibodies, and IgG antibodies. Alternatively or in addition, it can be characterized immunologically by a coronavirus-specific cytokine profile, preferably in the blood or at the site of infection as specified above (most preferably in the lung). These profiles can be determined by the skilled person without undue burden, e.g. by taking a tissue sample, e.g. from the lung, infecting it with the coronavirus and determining the expression of cytokines. Examples of coronavirus-specific cytokine profiles are (a) for SARS-CoV: upregulation of one or more of, preferably all of IFN-I (e.g. IFN-β), IFN-II (IFN-γ), IFN-III (IFN-λ1, IFN-λ2, and IFN-λ3), IL-1beta, IL-6, IL-12, IL-8, MCP-1, MIP-1alpha, RANTES, CXCL1, CXCL2, CXCL5, and CXCL9, and preferably no upregulation of CXCL10; and (b) for SARS-CoV-2: upregulation of one or more of, preferably all of IL-6, MCP-1, CXCL1, CXCL5, and/or CXLC10, and preferably no upregulation of any IFN and/or of any one or all of IL-1beta, IL-12, IL-8, MIP-1alpha, RANTES, CXCL2, and CXCL9.

The preferred subject is human. While to the inventors' best knowledge coronavirus infects all humans equally, there are risk factors for contracting a coronavirus infection:
(i) Recent (within incubation period, i.e. within the past 27, 24, or 19 days, preferably 14 (CDC, NHC) or 10 (WHO) days) presence in or travel from an area with ongoing spread of coronavirus infection. The area is preferably an 'Affected Area' as determined by the WHO. "Recent" can mean within the incubation period of the coronavirus, preferably within the past 27, 24, or preferably 19 days, more preferably 14 days or most preferably 10 days.
(ii) Contact with a person infected with coronavirus, in particular a person having one or more symptoms of coronavirus disease. "Contact" can mean exposed to respiratory droplets of the person (e.g. within range of respiratory droplets in air or in contact with surfaces touched by person or by respiratory droplets).
(iii) Being in an increased-risk-profession, which usually necessitates being in contact with the general public (contact being defined as above) regularly (e.g. at least daily or at least weekly), e.g. being a medical professional, in particular in a hospital, being a care worker (e.g. for children or the elderly) or retail worker (for instance in a supermarket or pharmacy).

A coronavirus infection may or may not cause symptoms of coronavirus disease in a subject. The terms "coronavirus infection" and "coronavirus disease" are distinguished herein by the presence of at least one coronavirus disease symptom. As long as the infection is not accompanied by at least one symptom of coronavirus disease, it (or the subject) is asymptomatic (includes presymptomatic). The term "coronavirus disease" as used herein requires the presence of a coronavirus infection and at least one symptom of coronavirus disease (also referred to herein as "symptomatic infection").

Coronavirus symptoms include dry cough, fever (≥37.8°C), runny and/or blocked nose, fatigue, breathing difficulty, pneumonia, organ (e.g. heart, lung, liver and/or kidney) failure, itchy throat, headache, joint pain, nausea, diarrhoea, shivering, lymphophenia, loss of smell and/or loss of taste. Preferably, the coronavirus disease is characterized by the presence of two or more, three or more, or four or more symptoms, preferably including one or two or more of dry cough, fever (≥37.8°C), breathing difficulty, loss of smell and/or loss of taste.

The coronavirus disease is preferably a respiratory disease (e.g. SARS or MERS), more preferably Covid-19. The coronavirus disease can take a mild (non-severe) or a severe course. A mild course is characterized by the presence of one or more only mild symptoms (i.e. no severe symptoms) of coronavirus disease. Mild symptoms are selected from the group consisting of dry cough, mild fever (≥37.8°C to <40°C), runny and/or blocked nose, fatigue, itchy throat, headache, joint pain, nausea, diarrhoea, shivering, lymphophenia, loss of smell and loss of taste. Severe symptoms are selected from the group consisting of breathing difficulty in particular acute respiratory distress syndrome, pneumonia, organ (e.g. heart, lung, liver and/or kidney) failure and high fever (≥40°C). A severe course is characterized clinically by one or more of (i) the presence of one or more severe symptoms of coronavirus disease, (ii) need for hospitalization, in particular need for admission to intensive care unit (ICU) and/or need for oxygen therapy (as defined above), in particular for a respirator (also referred to as ventilator), and/or (iii) death of the subject. A need for hospitalization is characterized by one or more of: clinical or radiological evidence of pneumonia, acute respiratory distress syndrome, and/or the presence of two or more symptoms including at least fever and persistent cough (with or without sputum), in particular persistent cough (e.g. of at least 3 days). A need for ICU admission is characterized by severe pneumonia and/or one or more severe complications. Severe pneumonia is characterized by tachypnea (age younger than 2 months: 60 or more breaths per minute; age 2 to 11 months: 50 or more breaths per minute, age 1 to 5 years: 40 or more breaths per minute, age > 5 years: 30 or more breaths per minute), respiratory distress and/or inadequate oxygenation (e.g. SpO₂ of 93% or less for adults, and 90% or less for children). Severe complications are complications that are lethal when untreated and include, for example, septic shock, acute respiratory distress syndrome and organ failure. Alternatively or in addition, a severe course can be characterized immunologically e.g. by the presence or development of a cytokine storm, in particular in the lung. A coronavirus cytokine storm is characterized by hyperinflammation, preferably by an increase (vs. infected but asymptomatic subject, preferably as after the incubation period, or average or mean of a plurality of such subjects) of one or more of IL-2, IL-7, TNF-alpha, MIP-1alpha, MCP-1, G-CSF and/or CXCL10, wherein the increase can be characterized as excessive and/or uncontrolled. In particular, the cytokine storm is a Covid-19 cytokine storm. Furthermore, the cytokine storm is preferably in the respiratory tract as defined for the infection above (including preferred parts thereof).

While the coronavirus disease can take a severe course in any subject regardless of age or pre-existing condition, there are nevertheless risk factors for having a severe course of coronavirus disease, including:
(i) age, e.g. at least 40, 50, 60, 70, or 80 years with risk increasing with age,
(ii) male gender,
(iii) smoking, and
(iii) having one or more comorbidities, preferably selected from the group consisting of cardiovascular disease, diabetes, respiratory disease (in particular chronic), hypertension, AIDS, cancer, liver disease, kidney disease and lung disease. A particular comorbidity as risk factor is a respiratory disease.

### Preparing a subject

The preparing of a subject is meant to prepare a subject for a coronavirus infection, which is afforded by the immunomodulation resulting in the medical use of the invention. It can also be termed "preparatory treatment", "prophylactic treatment" or, with respect to the disease resulting from the invention, "preventative treatment" or "preventive treatment". It is not meant to necessarily prevent a coronavirus infection. As such, the subject is not yet infected by coronavirus.

Thus, the preparing can have one or more immunological effects or (preferably and) it can have one or more clinical effects, both once a subject is infected. These immunological effects are preferably selected from the group consisting of
(I-i) increasing the number of one or more of coronavirus-antigen-specific immune factors (as defined above) in the subject or in a part of the subject,
(I-ii) inhibiting (i.e. reducing or stopping) coronavirus replication in the subject or in a part of the subject,
(I-iii) reducing the coronavirus load in the subject or in a part of the subject, and
(I-iv) preventing or ameliorating a coronavirus-specific cytokine profile or a cytokine storm (as defined above) in the subject or in a part of the subject.
Therein, the part of the subject is preferably the respiratory tract (upper or preferably lower respiratory tract, more preferably the lung). (I-ii) is preferred over (I-i), and (I-iii) over (I-ii). Further (I-ii) may involve (I-i), and (I-iii) may involve (I-i) and/or (I-ii). (I-iv) is generally desired.

The clinical effects are preferably selected from the group consisting of
(C-i) reducing contagiousness,
(C-ii) delaying the onset of coronavirus disease,
(C-iii) reducing the degree of one or more prospective symptoms of coronavirus disease, preferably of one or more prospective severe symptoms of coronavirus disease,
(C-iv) preventing one or more symptoms of coronavirus disease, preferably one or more severe symptoms of coronavirus disease,
(C-v) preventing a severe course of coronavirus disease, and
(C-vi) preventing coronavirus disease.
Therein, (C-ii) is preferred over (C-i), (C-iii) over (C-ii), (C-iv) over (C-iii), (C-v) over (C-iv), and (C-vi) over (C-v).

Prospective symptoms are symptoms that are not yet present but can or will arise. Thus, the reduction of the degree of prospective symptoms refers to a prophylactic treatment of symptoms.

Accordingly, the PPVO agent can be for use in one or more of (I-i) to (I-iv) and/or one or more of (C-i) to (C-vi) in a subject not yet infected by coronavirus. In other words, the PPVO agent is capable upon administration of an effective amount thereof to achieve one or more of (I-i) to (I-iv) and/or one or more of (C-i) to (C-vi).

In a preferred embodiment, the subject to be prepared is characterized by one or more risk factors for contracting a coronavirus infection and/or by one or more risk factors for having a severe course of coronavirus disease.

In another embodiment, the subject to be prepared is selected by a method according to the corresponding further aspect of the invention described below.

### Treating a subject

The treating of a coronavirus infection (i.e. of a subject that is infected) is not meant necessarily to treat or prevent coronavirus disease, although it is preferred that it does. As such, the treating can be of an asymptomatic infection (i.e. of an infected subject not having symptoms) or of coronavirus disease (i.e. of an infected subject having one or more symptoms).

The treating can have one or more immunological effects or (preferably and) it can have one or more clinical effects. These immunological effects are preferably as defined for the preparing above, for either an asymptomatic infection or for the disease. The clinical effects for an asymptomatic infection are preferably selected from the group consisting of (C-i) to (C-vi) as defined for the preparing above. The clinical effects for a symptomatic infection (i.e. on coronavirus disease) are preferably selected from the group consisting of
(C-i), (C-vi), both as defined for the preparing above,
(C-vii) ameliorating one or more (or ideally all) symptoms of coronavirus disease,
(C-viii) preventing, one or more (or ideally all) further symptoms of coronavirus disease, and
(C-ix) ameliorating, preferably preventing, a severe course of coronavirus disease.
Therein, "further symptoms" are those not yet characterizing the symptomatic infection.

Accordingly, the PPVO agent can be for use in one or more of (I-i) to (I-iv) and/or one or more of (C-i) to (C-ix) in a subject infected by coronavirus. In other words, the PPVO agent is capable upon administration of an effective amount thereof to achieve one or more of (I-i) to (I-iv) and/or one or more of (C-i) to (C-ix). In a preferred embodiment, the PPVO agent is for use in preventing or treating coronavirus disease.

As indicated above, the subject to be treated may be asymptomatic. Alternatively, the subject to be treated may have coronavirus disease. The subject having coronavirus disease may not have any severe symptoms, e.g. it has only one or more mild symptoms. In another embodiment, it may have a severe course of coronavirus disease (excluding (iii) death; preferably (i) one or more severe symptoms). In a preferred embodiment, the subject to be treated (e.g. any of the afore-mentioned subjects except those already having a severe course of coronavirus disease) is characterized by one or more risk factors for having a severe course of coronavirus disease.

In a further preferred embodiment, the subject is characterized by at least one of:
(i) no cytokine storm in the lung, and preferably no cytokine storm at all,
(ii) age up to 75, 70 or preferably 65 years, more preferably 18 to 65 years, and/or
(iii) HScore of 169 or less, preferably 150 or less, 130 or less, or 110 or less,
Preferably, the subject is characterized by (ii) and/or (iii), (ii) and HScore of 169 or less, or (iii) and age 18 to 65 years old.

The HScore generates a probability for the presence of secondary haemophagocytic lymphohistoicytosis (sHLH), a hyperinflammatory syndrome with a cytokine storm profile similar to that of coronavirus disease (Pehta et al., The Lancet Vol 395 March 28, 2020). An HScore of greater than 169 is 93% sensitive and 86% specific for sHLH. The HScore is calculated using the following criteria: temperature (<38.4°C: 0 points, 38.4-39.4°C: 33 points, >39.4°C: 49 points), organomegaly (none: 0 points, hepatomegaly or splenomegaly: 23 points, hepatomegaly and splenomegaly: 38 points), number of cytopenias^{∗} (one lineage; 0 points, two lineages: 24 points, three linages: 34 points), triglycerides (<1.5 mmol/l: 0 points, 1.5-4.0 mmol/l: 44 points, >4.0 mmol/l: 64 points), fibrinogen (>2.5 g/L: 0 points, ≤2.5 g/L: 30 points), ferritin (<2000 ng/ml: 0 points, 2000-60000 ng/ml: 35 points, >6000 ng/ml: 50 points), serum aspartate aminotransferase (<30 IU/L: 0 points, ≥30 IU/L: 19 points), haemophagocytosis on bone marrow aspirate (no: 0 points, yes: 35 points), and known immunosuppression† (no: 0 points, yes: 18 points). ^{∗}Defined as either haemoglobin concentration of 9.2 g/dL or less (≤5.71 mmol/L), a white blood cell count of 5000 white blood cells per mm³ or less, or platelet count of 110000 platelets per mm³ or less, or all of these criteria combined. †HIV positive or receiving long-term immunosuppressive therapy (e.g. one or more of glucocorticoids, cyclosporine and/or azathioprine).

In another embodiment, the subject to be treated is selected by a method according to the corresponding further aspect of the invention described below.

### Further medical use

In a further medical use aspect, the invention relates to a PPVO agent for use as a medicament promoting an antiviral (preferably anticoronaviral) cell state in a subject (i) having a coronavirus infection or (ii) characterized by one or more risk factors for contracting a coronavirus infection and/or by one or more risk factors for having a severe course of coronavirus disease.

In one embodiment, the one or more risk factors for contracting a coronavirus infection are selected from factors (i) and (ii) as defined above.

In another embodiment, the one or more risk factors for having a severe course of coronavirus disease comprise at least (ii) and (iii), or (ii) and (iv) as defined above, or comprise at least a comorbidity selected from respiratory disease (in particular chronic) and lung disease. In addition, the age of the subject is preferably at least 60, at least 70, or more preferably at least 80 years.

The phrase "promoting an antiviral cell state" refers to the promotion of the transcription of cellular antiviral genes coding for host defence proteins, e.g. as promoted by IFN-α and/or β. Preferably it inhibits (i.e. prevents or at least reduces) virus replication in the cell and/or induces apoptosis (to prevent further virus replication in the cell), and more preferably it reduces the amount of infectious virions released by an infected cells compared to a cell in which the antiviral cell state was not promoted by the medicament. Thereby, it stops or at least slows down virus propagation. "Promoting" includes an induction (e.g. for cells not yet in an antiviral state) and a reinforcement (i.e. potentiation) (e.g. for cells already in an antiviral state). Although not limited thereto, the former is preferred for a subject not (yet) having a coronavirus infection, e.g. characterized by one or more risk factors as specified above, and the latter is preferred for a subject having a coronavirus infection.

### Methods of treatment

Further disclosed are the following methods, which are to be interpreted as references to the agents and compositions of the invention for use in a method of preparing a subject for infection or treating an infection:
A method of administering a PPVO agent to a subject (i) having a coronavirus infection or (ii) characterized by one or more risk factors for contracting a coronavirus infection and/or by one or more risk factors for having a severe course of coronavirus disease.

A method of promoting an antiviral (preferably anticoronaviral) cell state in a subject (i) having a coronavirus infection or (ii) characterized by one or more risk factors for contracting a coronavirus infection and/or by one or more risk factors for having a severe course of coronavirus disease, the method comprising administering a PPVO agent to the subject.

A method of (i) preparing a subject for a coronavirus infection or (ii) treating a coronavirus infection in a subject, the method comprising administering a PPVO to the subject.

A method of treating coronavirus disease in a subject, the method comprising administering a PPVO agent to the subject.

Definitions and embodiments described below, in particular under the header 'Definitions and further embodiments of the invention' apply to the first aspect of the invention.

In a second aspect, the invention relates to a composition or a kit comprising a PPVO agent and a coronavirus agent.

In a preferred embodiment, the composition or kit further comprises one or more pharmaceutically acceptable diluents, carriers, and/or preservatives; and/or it is a pharmaceutical composition or kit. Pharmaceutically acceptable diluents, carriers, and/or preservatives are described below and may in addition thereto include an (e.g. aqueous) medium suitable for structurally maintaining a parapoxvirus, in particular an inactivated parapoxvirus, or lyophilisate of this medium. Specifically, the composition or kit may be for any of the medical uses of the invention described herein.

The composition can be formulated for any route of administration as defined above. Also, it can be formulated for simultaneous or separate administration or for simultaneous or separate release of the PPVO agent and the coronavirus agent.

In a preferred embodiment, the kit comprises a composition of the second aspect.

In another embodiment, the kit comprises a leaflet with instructions for use of the PPVO agent and the coronavirus agent, or of the composition of the second aspect, preferably according to a medical use described herein.

Definitions given and embodiments described with respect to the first aspect apply also to the second aspect, in as far as they are applicable. Also, definitions and embodiments described below, in particular under the header 'Definitions and further embodiments of the invention' apply to the composition or kit of the second aspect.

In a third aspect, the invention relates to a pharmaceutical composition or kit for any of the medical uses of the invention described herein, wherein the pharmaceutical composition or kit comprises a PPVO agent and preferably a further medicament. The pharmaceutical composition usually comprises one or more pharmaceutically acceptable diluents, carriers, and/or preservatives.

In a preferred embodiment, the kit comprises a composition of the third aspect.

In another embodiment, the kit comprises a leaflet with instructions for use of the PPVO agent and the further medicament, or of the composition of the third aspect, preferably according to a medical use described herein.

Definitions given and embodiments described with respect to the first and second aspect (e.g. formulation) apply also to the third aspect, in as far as they are applicable. Also, definitions and embodiments described below, in particular under the header 'Definitions and further embodiments of the invention' apply to the composition or kit of the third aspect.

In a further aspect, the invention relates to a method of selecting a subject for any of the medical uses of the invention described herein, wherein the method comprises the steps of (i) determining the presence or absence of a cytokine storm in the subject, and (ii) selecting the subject if a cytokine storm is absent.

In one embodiment, step (i) comprises determining the HScore of the subject, and step (ii) comprises selecting the subject if the HScore is 169 or less.

In another embodiment, step (i) comprises detecting in a biological sample of the subject one or more signs of hyperinflammation, and step (ii) comprises selecting the subject if no sign of hyperinflammation is detectable. Hyperinflammation therein is an increased level, e.g. excessive and/or uncontrolled, (vs. infected but asymptomatic subject, preferably as after the incubation period, or average or mean of a plurality of such subjects) of one or more of IL-2, IL-7, TNF-alpha, MIP-1alpha, MCP-1, G-CSF and/or CXCL10; or of one or more of IL-6, ferritin, platelet count and/or erythrocyte sedimentation rate (ESR). The biological sample is preferably selected from the group consisting of blood or a blood-derived sample (e.g. plasma or serum), sputum, saliva, and a sample derived from the lung (e.g. by bronchoscopy including bronchial lavage, bronchial alveolar lavage, bronchial brushing, and bronchial abrasion).

The method of the fourth aspect is preferably an *ex vivo* method.

Definitions given and embodiments described with respect to the first, second and third aspect apply also to the fourth aspect, in as far as they are applicable. Also, definitions and embodiments described below, in particular under the header 'Definitions and further embodiments of the invention' apply to the method of the fourth aspect.

### Definitions and further embodiments of the invention

The specification uses a variety of terms and phrases, which have certain meanings as defined below. Preferred meanings are to be construed as preferred embodiments of the aspects of the invention described herein. As such, they and also further embodiments described in the following can be combined with any embodiment of the aspects of the invention and in particular any preferred embodiment of the aspects of the invention described above.

The term "identity" or "identical" in the context of polynucleotide sequences refers to the number of residues in the two sequences that are identical when aligned for maximum correspondence. Specifically, the percent sequence identity of two sequences is the number of exact matches between two aligned sequences divided by the length of the shorter sequence and multiplied by 100. Alignment tools that can be used to align two sequences are well known to the person skilled in the art and can, for example, be obtained on the World Wide Web, e.g. Needle (EMBOSS) (https://www.ebi.ac.uk/Tools/psa/emboss_needle/), MUSCLE (http://www.ebi.ac.uk/Tools/msa/muscle/), MAFFT (http://www.ebi.ac.uk/Tools/msa/ mafft/) or WATER (http://www.ebi.ac.uk/Tools/psa/ emboss_water/). The alignments between two sequences may be carried out using default parameters settings, e.g. for Needle preferably MATRIX: BLOSUM62, Gap Open: 10.0, Gap Extend: 0.5, for MAFFT preferably: Matrix: Blosum62, Gap Open 1.53, Gap Extend 0.123, for WATER polynucleotides preferably: MATRIX: DNAFULL, Gap Open: 10.0, Gap Extend 0.5 and for WATER polypeptides preferably MATRIX: BLOSUM62, Gap Open: 10.0, Gap Extend: 0.5. Those skilled in the art understand that it may be necessary to introduce gaps in either sequence to produce a satisfactory alignment. The "best sequence alignment" is defined as the alignment that produces the largest number of aligned identical residues while having a minimal number of gaps. Preferably, it is a global alignment, which includes every residue in every sequence in the alignment.

The term "variant" refers generally to a modified version of the polynucleotide, e.g. a mutation, so one or more nucleotides of the polynucleotide may be mutated. Generally, the variant is functional, meaning e.g. with regard to a virus that the virus is capable of infecting a host cell. The functionality is generally that described for the polynucleotide the variant is from. A "mutation" can be a nucleotide substitution, deletion and/or insertion ("and" may apply if there is more than one mutation). Preferably, it is a substitution.

The term "antigen" refers to any substance, e.g. protein or peptide, that is capable of being bound by an antibody, a B cell receptor and/or a T cell receptor. An antigen comprises at least one epitope preferably comprising at least 8 amino acids and more preferably comprising between 8 and 17 amino acids. The epitope can be a T cell and/or a B cell epitope. A T cell epitope is an epitope that can be presented on the surface of an antigen-presenting cell, where it is bound to an MHC molecule. In humans, professional antigen-presenting cells are specialized to present MHC class II peptides, whereas most nucleated somatic cells present MHC class I peptides. T cell epitopes presented by MHC class I molecules are typically peptides between 8 and 11 amino acids in length, whereas MHC class II molecules present longer peptides, e.g. 13-17 amino acids in length. A B cell epitope is an epitope that is recognised as three-dimensional structures on the surface of native antigens by B cells. Epitopes can be predicted with *in silico* tools, e.g. the online B- or T-cell prediction tools of the IEDB Analysis Resource.

The term "immunogen" refers to an antigen that is capable of inducing an immune response.

"Pattern recognition receptor" or "PRR", as used herein, refers to a germline-encoded host sensor which detects a pathogen-associated molecular pattern (PAMP), which is specific for a pathogen, and/or a damage-associated molecular pattern (DAMP), which is specific for components of a host cell that are released during cell damage or death. A PRR can be a membrane-bound PRR (cell-surface PRR) or a cytosolic PRR

An "adjuvant" is a substance that accelerates, prolongs and/or enhances the quality and/or strength of an immune response to an antigen/immunogen, in comparison to the administration of the antigen alone, thus, reducing the quantity of antigen/immunogen necessary, and/or the frequency of injection necessary in order to generate an adequate immune response to the antigen/immunogen of interest. Examples of adjuvants that may be used in the context of the composition according to the present invention are gel-like precipitates of aluminum hydroxide (alum); AlPO₄; alhydrogel; bacterial products from the outer membrane of Gram-negative bacteria, in particular monophosphoryl lipid A (MPLA), lipopolysaccharides (LPS), muramyl dipeptides and derivatives thereof; Freund's incomplete adjuvant; liposomes, in particular neutral liposomes, liposomes containing the composition and optionally cytokines; non-ionic block copolymers; ISCOMATRIX adjuvant (Drane et al., 2007); unmethylated DNA comprising CpG dinucleotides (CpG motif), in particular CpG ODN with a phosphorothioate (PTO) backbone (CpG PTO ODN) or phosphodiester (PO) backbone (CpG PO ODN); synthetic lipopeptide derivatives, in particular Pam₃Cys; lipoarabinomannan; peptidoglycan; zymosan; heat shock proteins (HSP), in particular HSP 70; dsRNA and synthetic derivatives thereof, in particular Poly I:C; polycationic peptides, in particular poly-L-arginine; taxol; fibronectin; flagellin; imidazoquinoline; cytokines with adjuvant activity, in particular GM-CSF, interleukin-(IL-)2, IL-6, IL-7, IL-18, type I and II interferons, in particular IFN-γ, TNF-α; 25-dihydroxyvitamin D3 (calcitriol); and synthetic oligopeptides, in particular MHC-II-presented peptides. Non-ionic block polymers containing polyoxyethylene (POE) and polyoxypropylene (POP), such as POE-POP-POE block copolymers may be used as an adjuvant (Newman *et al.,* 1998). This type of adjuvant is particularly useful for compositions comprising nucleic acids as active ingredient.

The term "recombinant" refers in particular to a virus that is modified to comprise a heterologous polynucleotide sequence in its genome.

The term "immunomodulator" refers to a drug used to regulate or normalize the immune system by inducing, enhancing, suppressing and/or weakening an immune response in a subject ("and" meaning that some parts of the immune system are selectively induced or enhanced and others are selectively suppressed or weakened). Examples are cytokines, non-cytokine agonists or antagonists of cytokine receptors (e.g. antibodies or small compounds), antibodies or small compounds binding (preferably neutralizing) cytokines, and soluble cytokine receptors (e.g. for trapping cytokines). It does not include an antigen.

The term "vector" as used herein includes any vectors known to the skilled person including plasmid vectors, cosmid vectors, phage vectors such as lambda phage, viral vectors such as adenovirus (Ad) vectors), adeno-associated virus (AAV) vectors, alphavirus vectors (e.g., Venezuelan equine encephalitis virus (VEE), sindbis virus (SIN), semliki forest virus (SFV), and VEE-SIN chimeras), herpes virus vectors, measles virus vectors, pox virus vectors (e.g., vaccinia virus, modified vaccinia virus Ankara (MVA), NYVAC (derived from the Copenhagen strain of vaccinia), and avipox vectors: canarypox (ALVAC) and fowlpox (FPV) vectors), and vesicular stomatitis virus vectors, or virus like particles. As used herein, the term "virus-like particle" or "VLP" refers to a non-replicating, empty viral shell. VLPs are generally composed of one or more viral proteins, such as, but not limited to those proteins referred to as capsid, coat, shell, surface and/or envelope proteins. They contain functional viral proteins responsible for cell penetration by the virus, which ensures efficient cell entry. Methods for producing particular VLPs are known in the art.

The term "mitogen" refers to an agent, e.g. a peptide or protein that induces a cell to begin cell division. The term "cell activator" refers to an agent, e.g. a peptide or protein that binds and cross-links cell receptors, e.g. T cell or B cell receptors.

The term "nucleoside analogue" refers to a nucleoside containing a nucleic acid analogue and a sugar. The term "nucleotide analogue" refers to a nucleotide containing a nucleic acid analogue, a sugar and a phosphate group with 1 to 3 phosphates. The term "nucleic acid analogue" refers to a compound that is structurally similar to RNA and DNA in that it has one or more of phosphate backbone, pentose sugar and/or the nucleobase altered such that it has different base pairing and/or base stacking properties. Examples include peptide nucleic acid (PNA), Morpholino and locked nucleic acid (LNA), as well as glycol nucleic acid (GNA) and threose nucleic acid (TNA).

The term "RNAi molecule" refers to a molecule capable of neutralizing mRNA molecules in a target-sequence-specific manner. Examples include dsRNA, miRNA and siRNA.

Particular preferred pharmaceutical forms for the administration of the agents according to the invention are forms suitable for injectable use and include sterile aqueous solutions or dispersions and sterile powders for the extemporaneous preparation of sterile injectable solutions or dispersion. Typically, such a solution or dispersion will include a solvent or dispersion medium, containing, for example, water-buffered aqueous solutions, e.g. biocompatible buffers, ethanol, polyol, such as glycerol, propylene glycol, polyethylene glycol, suitable mixtures thereof, surfactants or vegetable oils. Infusion or injection solutions can be accomplished by any number of art-recognized techniques including but not limited to addition of preservatives like anti-bacterial or anti-fungal agents, e.g. parabene, chlorobutanol, phenol, sorbic acid or thiomersal. Further, isotonic agents, such as sugars or salts, in particular sodium chloride may be incorporated in infusion or injection solutions.

Preferred diluents of the present invention are water, physiological acceptable buffers, physiological acceptable buffer salt solutions or salt solutions. Excipients which can be used with the various pharmaceutical forms of the pharmaceutical according to the invention can be chosen from the following non-limiting list:
a) binders such as lactose, mannitol, crystalline sorbitol, dibasic phosphates, calcium phosphates, sugars, microcrystalline cellulose, carboxymethyl cellulose, hydroxyethyl cellulose, polyvinyl pyrrolidone and the like,
b) lubricants such as magnesium stearate, talc, calcium stearate, zinc stearate, stearic acid, hydrogenated vegetable oil, leucine, glycerids and sodium stearyl fumarates,
c) disintegrants such as starches, croscaramellose, sodium methyl cellulose, agar, bentonite, alginic acid, carboxymethyl cellulose, polyvinyl pyrrolidone and the like.

The term "pharmaceutically acceptable carrier" refers to any substrate which serves to improve the selectivity, effectiveness, and/or safety of drug administration. Such carriers can be used to control the release of a drug into systemic circulation. This can be accomplished either by slow release of the drug over a long period of time (typically diffusion) or by triggered release at the drug's target by some stimulus, such as changes in pH, application of heat, and activation by light. Carriers can also be used to improve the pharmacokinetic properties, specifically the bioavailability, of many drugs with poor water solubility and/or membrane permeability. A wide variety of drug carrier systems have been developed and studied. Examples include liposomes, polymeric micelles, microspheres, nanoparticles, nanofibers, protein-drug conjugates, erythrocytes, virosomes and dendrimers. Different methods of attaching the drug to the carrier can be used, including adsorption, integration into the bulk structure, encapsulation, and covalent bonding.

The invention is described by way of the following examples which are to be construed as merely illustrative and not limitative of the scope of the invention.

### EXAMPLE 1

### Aim

Testing the antiviral activity of inactivated parapoxvirus *orf* (iPPVO) virions against coronavirus. PPVO virions stimulate the innate as well as the adaptive arm of the immune system.

### Approach

In whole blood, APCs (antigen-presenting cells) take up the iPPVO virion, which leads to their activation and the release of cytokines. This in turn activates T cells as well as NK cells which also start to proliferate and release cytokines. After 3 days of incubation the cells are spun down and the supernatant containing soluble factors like cytokines are harvested. These supernatants are assayed for their activity on coronavirus.

### Test substances

- iPPVO of strain D1701 (available from Pfizer); 230 IFN units reconstituted in 2 ml water
- iPPVO vehicle (PPVO buffer only, i.e. without virus), negative control
- PBS, negative control
- Concavalin (Con) A, co-stimulus, stock solution 1 mg/ml PBS = 10X (used in well at 5 µg/ml)

### Further materials

- Whole blood of a healthy donor: 9 ml whole blood freshly collected in a Sarstedt Monovette, Li-Heparin
- Medium for whole blood incubation: RPMI (500 ml) supplemented with 1% GlutaMAX^{™} and 0.25 ml Heparin-Sodium (5000 U)
- Cells of coronavirus assay: VeroE6-EGFP propagated in growth medium prepared by supplementing DMEM with 10% v/v heat-inactivated FCS and 5 mL sodium bicarbonate 7.5%, and cultured in T150 bottle and split 1/4 twice a week. Pen-strep added directly to the T150 bottle at a 1/100 dilution.
- Coronavirus assay medium: prepared by supplementing DMEM with 2% v/v heat-inactivated FCS and 5 mL sodium bicarbonate 7.5%.

### Preparation of samples

Per well in a 96 well round-bottom plate: 120 µl cell medium for whole blood incubation + 20 µl ConA or PBS + 20 µl iPPVO, iPPVO vehicle or PBS + 40 µl whole blood.

The plate was incubated at 37 °C, 5 % CO₂ for 72 hrs and then centrifuged for 5 min at 230 g. The supernatant was aliquoted to new 96 well plates (plate layout maintained). Sample plates were frozen at -80 °C until further use.

### Coronavirus assay

Dilution of the samples (test substance as prepared above): 100 µL assay medium were added to wells of a Greiner Bio One 655090 plate and 15 µL were removed again from wells (except from wells for controls of this assay: virus control (VC) controlling for virus replication and effect on cells, and cell control (CC) for controlling cell viability without virus). 15 µL from wells of sample plate was added to wells of which 15 µL medium was removed.

Preparation of the cell suspension: A confluent culture (monolayer) of VeroE6-EGFP in T150 bottle was washed with DPBS and 10 mL trypsine 0.25%trypsine/EDTA was added. The bottle was incubated for 1 minute at room temperature, and trypsine/EDTA was removed except for 2 mL. The bottle was then incubated for 15 minutes at 37°C. After incubation, the cells were resuspended in 10 mL assay medium, passed through a Cell Strainer (FALCON CAT NO 352350) and counted using coulter (3 samples of 10 µL in 10 mL were counted). 3000 cells were then resuspended in 50 µL assay medium and 50 µL of the cell suspension were seeded to each well of the Greiner plate.

Assay: Plates with cell suspension as prepared above were incubated overnight (37°C / 5%CO₂), after which SARS-CoV-2 (strain BetaCoV/Belgium/GHB-03021/2020, stock titer 4.8 x 10⁷ TCID50/ml) was added to all wells (except the cell control wells) at a final dilution of 1/200,0000 in 200 µl/well (3000 cells/well, resulting in an MOI of 0.016 TCID₅₀/cell). The plates were incubated at 37°C / 5% CO₂ for 5 days, after which GFP signal was measured using whole-well fluorescence, HCI (High Content Imaging) and the ImageJ software. Reduced EGFP expression is a marker for virus-induced cytopathic activity. See also Ivens et al. (Journal of Virological Methods 129, 2005, 56-63).

### Results

The results are shown in Fig. 1. Cells not infected with coronavirus (CC mean) were viable and expressed eGFP. Cells infected with coronavirus (VC mean) died and no eGFP expression was detectable. Results for PBS and iPPVO vehicle alone were similar to VC mean, i.e. PBS and iPPVO vehicle alone had no effect on cell viability, i.e. against coronavirus, as expected. Also 5 µg/ml ConA alone (in PBS) did not retain eGFP expression, and it also did not affect eGFP expression when given together with iPPVO vehicle as shown by the comparison to ConA alone in PBS. iPPVO D1701 alone (in PBS) also had a clear positive effect on cell viability, and eGFP expression was further increased when iPPVO D1701 was used in combination with 5 µg/ml ConA (which by itself showed no effect on eGFP expression at this concentration). Accordingly, iPPVO and ConA act synergistically.

These results show the efficacy of parapoxvirus in promoting an antiviral cell state which is protective and inhibits coronavirus replication and propagation and thereby the infection of further cells. This effect can be improved by adding an immune stimulatory agent such as ConA, which acts synergistically, i.e. the efficacy of iPPVO is enhanced in a combinatorial approach.

### EXAMPLE 2

### Aim

Determining whether the proof-of-principle shown in Example 1 applies to parapoxvirus in general by testing the antiviral activity of a further parapoxvirus strain against coronavirus.

### Approach

See Example 1.

### Test substances

- iPPVO of strain NZ2 (chemically inactivated by binary ethyleneimine, BEI), internal designation AIC649, 1×10⁹ lyophilized virions reconstituted in 1 ml water
- iPPVO of strain D1701, see Example 1
- iPPVO vehicle, negative control, see Example 1

### Further materials, preparation of samples, coronavirus assay

See Example 1. iPPVO and iPPVO vehicle were used alone. iPPVO D1701 shown to increase cell viability in Example 1 was used as a positive control in Example 2.

### Results

The results are shown in Fig. 2. iPPVO D1701, which in Example 1 was shown to be effective, was used as a positive control and as a 100% benchmark. Both PPVO strains D1701 and NZ2 are active against coronavirus as shown by the comparison to the negative control. Accordingly, the promotion of an antiviral cell state which is effective against coronavirus is a hallmark of parapoxvirus in general and not only of the strain tested in Example 1.

### EXAMPLE 3

### Aim

Investigating the timing of parapoxvirus addition, specifically determining how the antiviral activity of parapoxvirus shown in Examples 1 and 2 is modulated by adding the supernatants at different time points to the cells of the coronavirus assay.

### Approach

See Examples 1 and 2, but with different time points for adding the supernatants of the sample plate to the cells of the coronavirus assay (before or after the infection of the VeroE6-EGFP cells with SARS-CoV-2).

### Test substances

- iPPVO of strain NZ2, see Example 2
- iPPVO of strain D1701, see Example 1
- iPPVO vehicle, negative control, see Example 1
- ConA, see Example 1

### Further materials, preparation of samples, coronavirus assay

See Examples 1 and 2. Whole blood was incubated with iPPVO or iPPVO vehicle (alone or in combination with ConA). Supernatants were generated and added at different timepoints before or after the infection of the VeroE6-EGFP cells with the coronavirus (SARS-CoV-2).

### EXAMPLE 4

### Aim

Testing further combinatorial approaches, specifically confirming that the strength of the antiviral activity of parapoxvirus shown in Examples 1, 2, and 3 can be enhanced in combinatorial approaches using further combination partners, e.g. an immune stimulatory agent or an antiviral as described herein. This includes investigating the timing of parapoxvirus and combination partner addition (parapoxvirus to blood samples and combination partner to blood sample or directly to cells of coronavirus assay), i.e. relative to each other, and also the timing of adding the coronavirus relative to parapoxvirus and combination partner. It also includes testing the influence of the concentrations of parapoxvirus and combination partner.

### Approach

See Examples 1, 2, and 3, but with different time points for adding the supernatants (of blood treated with parapoxvirus and/or combination partner, at the same or different time points) of the sample plate to the cells of the coronavirus assay (before or after the infection of the VeroE6-EGFP cells with SARS-CoV-2), and with different time points for adding the combination partner (if not already added to the blood sample) to the cells of the coronavirus assay (before or after the supernatant addition and/or the SARS-CoV-2 addition).

### Test substances

- iPPVO of strain NZ2, see Example 2
- iPPVO of strain D 1701, see Examples 1
- iPPVO vehicle, negative control, see Examples 1
- ConA, see Example 1 (combination partner control)
- Thalidomide (combination partner)
- Remdesivir (combination partner)

### Further materials, preparation of samples, coronavirus assay

See Examples 1 and 2. Whole blood was incubated with iPPVO or iPPVO vehicle (alone or in combination with one or more combination partners). Supernatants were added at different timepoints before or after the infection of the VeroE6-EGFP cells with the coronavirus (SARS-CoV-2). One or more combination partners not used for incubation of the blood samples were added at different time points to the VeroE6-EGFP cells (before both the supernatant and the coronavirus, after both or between the two). Different concentrations of the combination partners were used.

## Claims

1. A *Parapoxvirus ovis* (PPVO) agent for use in (i) preparing a subject for a coronavirus infection or (ii) treating a coronavirus infection in a subject.

2. The PPVO agent for use according to claim 1, wherein the PPVO agent is:
(a) PPVO itself or a fragment thereof, or
(b) an agent that comprises genetic information encoding for (a).

3. The PPVO agent of claim 2, wherein the PPVO agent is an inactivated PPVO virion.

4. The PPVO agent for use according to any one of claims 1 to 3, wherein the coronavirus is a SARS coronavirus.

5. The PPVO agent for use according to any one of claims 1 to 4, wherein the coronavirus is SARS-CoV-2 or a variant thereof with at least 80% sequence identity to SEQ ID NO: 1 or 2.

6. The PPVO agent for use according to any one of claims 1 to 5, wherein the subject to be prepared is **characterized by** one or more risk factors for contracting a coronavirus infection and/or by one or more risk factors for having a severe course of coronavirus disease.

7. The PPVO agent for use according to any one of claims 1 to 5, wherein the subject to be treated is asymptomatic.

8. The PPVO agent for use according to any one of claims 1 to 5, wherein the subject to be treated has coronavirus disease, preferably Covid-19.

9. The PPVO agent for use according claim 8, wherein the subject has either no severe symptoms, or has a severe course of coronavirus disease.

10. The PPVO agent for use according to any one of claims 1 to 9, wherein the use comprises the administration of a further treatment regimen for (i) or (ii).

11. The PPVO agent for use according claim 10, wherein the further treatment regimen for (i) comprises administering a coronavirus agent and/or an immune stimulatory agent, and the further treatment regimen for (ii) comprises administering a coronavirus agent, one or more further medicaments and/or one or more further non-drug treatments.

12. The PPVO agent for use according claim 11, wherein
- the coronavirus agent is selected from the group consisting of:
(i) an inactivated coronavirus,
(ii) an antigenic, preferably immunogenic fragment of a coronavirus,
(iii) a nucleic acid encoding for (i) or (ii),
(iv) a vector comprising the nucleic acid of (iii),
(v) a cell comprising the nucleic acid of (iii) or the vector of (iv),
(vi) an antibody or an antigen-binding fragment thereof binding to a coronavirus or a fragment thereof, preferably the envelope,
(vii) a nucleotide/nucleoside analogue, preferably coronavirus-specific,
(viii) a coronavirus-specific RNAi molecule,
(ix) a small molecule, an antibody or an antigen-binding fragment thereof, binding to a site of protein-protein interaction (PPI) between a coronavirus protein and a subject protein, and
(x) blood plasma of a subject that has recovered from a coronavirus infection, preferably from coronavirus disease;
- the further medicament is selected from the group consisting of an immune stimulatory agent, an antiviral, an antibiotic, an adjuvant, a glucocorticoid, an antihypertensive drug, a hypoglycaemic drug, an anti-shock drug, and a drug suitable for treating one or more symptoms of coronavirus disease; and/or
- the one or more further therapies are preferably selected from the group consisting of oxygen therapy and extra-corporeal organ support (ECOS).

13. The PPVO agent for use according to claim 12, wherein the small molecule is selected from the group consisting of Loratadine, Daunorubicin, Midostaurin, Ponatinib, Silmitasertib, Valproic Acid, Haloperidol, Metformin, Migalastat, S-verapamil, Indomethacin, Ruxolitinib, Mycophenolic acid, Entacapone, Ribavirin, E-52862, Merimepodib, RVX-208, XL413, AC-55541, Apicidin, AZ3451, AZ8838, Bafilomycin A1, CCT 365623, GB110, H-89, JQ1, PB28, PD-144418, RS-PPCC, TMCB, UCPH-101, ZINC1775962367, ZINC4326719, ZINC4511851, ZINC95559591, 4E2RCat, ABBV-744, Camostat, Captopril, CB5083, Chloramphenicol, Chloroquine, PI4K-IIIβ inhibitor Compound 10, Cyclophilin inhibitor Compound 2, CPI-0610, Dabrafenib, DBeQ, dBET6, IHVR-19029, Linezolid, Lisinopril, Minoxidil, ML240, MZ1, Nafamostat, Pevonedistat, PS3061, Rapamycin (Sirolimus), Sanglifehrin A, Sapanisertib (INK128/MlN128), FK-506 (Tacrolimus), Ternatin 4 (DA3), Tigecycline, Tomivosertib (eFT-508), Verdinexor, WDB002, Azacitidine, Selinexor, and Zotatifin (eFT226).

14. A composition or a kit comprising a PPVO agent, preferably as defined in claim 2 or 3, and a coronavirus agent, preferably as defined in claim 12.

15. A pharmaceutical composition or kit for use in (i) preparing a subject for a coronavirus infection or (ii) treating a coronavirus infection in a subject, wherein the pharmaceutical composition or kit comprises a PPVO agent, preferably as defined in claim 2 or 3, and preferably a further medicament, more preferably as defined in claim 12.

## Patentansprüche

1. Ein *Parapoxvirus ovis* (PPVO) Agens zur Verwendung bei (i) der Vorbereitung eines Subjekts auf eine Coronavirus-Infektion oder (ii) der Behandlung einer Coronavirus-Infektion bei einem Subjekt.

2. Das PPVO Agens zur Verwendung gemäß Anspruch 1, wobei das PPVO Agens ist:
(a) PPVO selbst oder ein Fragment davon, oder
(b) ein Agens, das genetische Information umfasst, die für (a) kodiert.

3. Das PPVO Agens nach Anspruch 2, wobei das PPVO Agens ein inaktiviertes PPVO Virion ist.

4. Das PPVO Agens zur Verwendung nach einem der Ansprüche 1 bis 3, wobei das Coronavirus ein SARS-Coronavirus ist.

5. Das PPVO Agens zur Verwendung nach einem der Ansprüche 1 bis 4, wobei das Coronavirus SARS-CoV-2 oder eine Variante davon mit mindestens 80 % Sequenzidentität zu SEQ ID NO: 1 oder 2 ist.

6. Das PPVO Agens zur Verwendung nach einem der Ansprüche 1 bis 5, wobei das vorzubereitende Subjekt durch einen oder mehrere Risikofaktoren für eine Coronavirus-Infektion und/oder durch einen oder mehrere Risikofaktoren für einen schweren Verlauf einer Coronavirus-Erkrankung gekennzeichnet ist.

7. Das PPVO Agens zur Verwendung nach einem der Ansprüche 1 bis 5, wobei das zu behandelnde Subjekt asymptomatisch ist.

8. Das PPVO Agens zur Verwendung nach einem der Ansprüche 1 bis 5, wobei das zu behandelnde Subjekt eine Coronavirus-Erkrankung, vorzugsweise Covid-19, hat.

9. Das PPVO Agens zur Verwendung nach Anspruch 8, wobei das Subjekt entweder keine schweren Symptome hat, oder einen schweren Verlauf der Coronavirus-Erkrankung aufweist.

10. Das PPVO Agens zur Verwendung nach einem der Ansprüche 1 bis 9, wobei die Verwendung die Verabreichung eines weiteren Behandlungsschemas für (i) oder (ii) umfasst.

11. Das PPVO Agens zur Verwendung nach Anspruch 10, wobei das weitere Behandlungsschema für (i) die Verabreichung eines Coronavirus-Agens und/oder eines immunstimulierenden Agens umfasst, und das weitere Behandlungsschema für (ii) die Verabreichung eines Coronavirus-Agens, eines oder mehrerer weiterer Medikamente und/oder einer oder mehrerer weiterer nicht-medikamentöser Behandlungen umfasst.

12. Das PPVO Agens zur Verwendung gemäß Anspruch 11, wobei
- das Coronavirus-Agens ausgewählt ist aus der Gruppe bestehend aus:
(i) einem inaktivierten Coronavirus,
(ii) ein antigenes, vorzugsweise immunogenes Fragment eines Coronavirus,
(iii) einer Nukleinsäure, die für (i) oder (ii) kodiert,
(iv) einen Vektor, der die Nukleinsäure von (iii) enthält,
(v) eine Zelle, die die Nukleinsäure von (iii) oder den Vektor von (iv) enthält,
(vi) ein Antikörper oder ein Antigen-bindendes Fragment davon, der/das an ein Coronavirus oder ein Fragment davon bindet, vorzugsweise die Hülle,
(vii) ein Nukleotid/Nukleosid-Analogon, vorzugsweise Coronavirus-spezifisch,
(viii) ein Coronavirus-spezifisches RNAi-Molekül,
(ix) ein kleines Molekül, ein Antikörper oder ein Antigen-bindendes Fragment davon, das an eine Stelle der Protein-Protein-Interaktion (PPI) zwischen einem Coronavirus-Protein und einem Subjekt-Protein bindet, und
(x) Blutplasma einer Person, die sich von einer Coronavirus-Infektion, vorzugsweise von einer Coronavirus-Erkrankung, erholt hat;
- das weitere Medikament ausgewählt ist aus der Gruppe bestehend aus einem immunstimulierenden Agens, einem antiviralen Agens, einem Antibiotikum, einem Adjuvans, einem Glukokortikoid, einem blutdrucksenkenden Medikament, einem blutzuckersenkenden Medikament, einem Anti-Schock Medikament und einem Medikament, das zur Behandlung eines oder mehrerer Symptome einer Coronavirus-Erkrankung geeignet ist; und/oder
- die eine oder mehrere weitere Therapien vorzugsweise aus der Gruppe ausgewählt werden, die aus Sauerstofftherapie und extrakorporaler Organunterstützung (ECOS) besteht.

13. Das PPVO Agens zur Verwendung nach Anspruch 12, wobei das kleine Molekül ausgewählt ist aus der Gruppe bestehend Loratadin, Daunorubicin, Midostaurin, Ponatinib, Silmitasertib, Valproinsäure, Haloperidol, Metformin, Migalastat, S-verapamil, Indomethacin, Ruxolitinib, Mycophenolsäure, Entacapone, Ribavirin, E-52862, Merimepodib, RVX-208, XL413, AC-55541, Apicidin, AZ3451, AZ8838, Bafilomycin A1, CCT 365623, GB110, H-89, JQ1, PB28, PD-144418, RS-PPCC, TMCB, UCPH-101, ZINC1775962367, ZINC4326719, ZINC4511851, ZINC95559591, 4E2RCat, ABBV-744, Camostat, Captopril, CB5083, Chloramphenicol, Chloroquine, PI4K-IIIß Hemmer Compound 10, Cyclophilin Hemmer Compound 2, CPI-0610, Dabrafenib, DBeQ, dBET6, IHVR-19029, Linezolid, Lisinopril, Minoxidil, ML240, MZ1, Nafamostat, Pevonedistat, PS3061, Rapamycin (Sirolimus), Sanglifehrin A, Sapanisertib (INK128/MIN128), FK-506 (Tacrolimus), Ternatin 4 (DA3), Tigecycline, Tomivosertib (eFT-508), Verdinexor, WDB002, Azacitidine, Selinexor, und Zotatifin (eFT226).

14. Eine Zusammensetzung oder ein Kit, umfassend ein PPVO Agens, vorzugsweise wie in Anspruch 2 oder 3 definiert, und ein Coronavirus Agens, vorzugsweise wie in Anspruch 12 definiert.

15. Eine Pharmazeutische Zusammensetzung oder ein Kit zur Verwendung bei (i) der Vorbereitung eines Subjekts auf eine Coronavirus-Infektion oder (ii) der Behandlung einer Coronavirus-Infektion bei einem Subjekt, wobei die pharmazeutische Zusammensetzung oder das Kit ein PPVO Agens, vorzugsweise wie in Anspruch 2 oder 3 definiert, und vorzugsweise ein weiteres Medikament, besonders bevorzugt wie in Anspruch 12 definiert, umfasst.

## Revendications

1. Agent *Parapoxvirus ovis* (PPVO) pour son utilisation dans (i) la préparation d'un sujet pour une infection à coronavirus ou (ii) le traitement d'une infection à coronavirus chez un sujet.

2. Agent PPVO pour son utilisation selon la revendication 1, dans lequel l'agent PPVO est :
(a) le PPVO lui-même ou un fragment de celui-ci, ou
(b) un agent qui comprend des informations génétiques codant pour (a).

3. Agent PPVO selon la revendication 2, dans lequel l'agent PPVO est un virion de PPVO inactivé.

4. Agent PPVO pour son utilisation selon l'une quelconque des revendications 1 à 3, dans lequel le coronavirus est un coronavirus SARS.

5. Agent PPVO pour son utilisation selon l'une quelconque des revendications 1 à 4, dans lequel le coronavirus est le SARS-CoV-2 ou un variant de celui-ci présentant une identité de séquence d'au moins 80 % avec SEQ ID NO : 1 ou 2.

6. Agent PPVO pour son utilisation selon l'une quelconque des revendications 1 à 5, dans lequel le sujet à préparer est **caractérisé par** un ou plusieurs facteurs de risque de contracter une infection à coronavirus et/ou par un ou plusieurs facteurs de risques d'avoir une évolution grave d'une maladie à coronavirus.

7. Agent PPVO pour son utilisation selon l'une quelconque des revendications 1 à 5, dans lequel le sujet à traiter est asymptomatique.

8. Agent PPVO pour son utilisation selon l'une quelconque des revendications 1 à 5, dans lequel le sujet à traiter présente une maladie à coronavirus, de préférence la Covid-19.

9. Agent PPVO pour son utilisation selon la revendication 8, dans lequel le sujet soit ne présente pas de symptôme grave, soit présente une évolution grave de la maladie à coronavirus.

10. Agent PPVO pour son utilisation selon l'une quelconque des revendications 1 à 9, dans lequel l'utilisation comprend l'administration d'un autre régime thérapeutique pour (i) ou (ii).

11. Agent PPVO pour son utilisation selon la revendication 10, dans lequel le régime posologique supplémentaire pour (i) comprend l'administration d'un agent coronavirus et/ou d'un agent stimulant immunitaire, et le régime thérapeutique supplémentaire pour (ii) comprend l'administration d'un agent coronavirus, d'un ou plusieurs médicaments supplémentaires et/ou d'un ou plusieurs traitements non médicamenteux.

12. Agent PPVO pour son utilisation selon la revendication 11, dans lequel
- l'agent coronavirus est sélectionné dans le groupe constitué par :
(i) un coronavirus inactivé,
(ii) un fragment antigénique, de préférence immunogène, d'un coronavirus,
(iii) un acide nucléique codant pour (i) ou (ii),
(iv) un vecteur comprenant l'acide nucléique de (iii),
(v) une cellule comprenant l'acide nucléique de (iii) ou le vecteur de (iv),
(vi) un anticorps ou un fragment de liaison à un antigène de celui-ci se liant à un coronavirus ou un fragment de celui-ci, de préférence l'enveloppe,
(vii) un analogue nucléotidique/nucléosidique, de préférence spécifique d'un coronavirus,
(viii) une molécule d'ARNi spécifique d'un coronavirus,
(ix) une petite molécule, un anticorps ou un fragment de liaison à l'antigène de celui-ci, se liant à un site d'interaction protéine-protéine (PPI) entre une protéine de coronavirus et une protéine du sujet, et
(x) du plasma sanguin d'un sujet qui a guéri d'une infection à coronavirus, de préférence d'une maladie à coronavirus ;
- le médicament supplémentaire est sélectionné dans le groupe constitué par un agent stimulant immunitaire, un antiviral, un antibiotique, un adjuvant, un glucocorticoïde, un médicament anti-hypertenseur, un médicament hypoglycémique, un médicament anti-choc, et un médicament approprié pour le traitement d'un ou plusieurs symptômes d'une maladie à coronavirus ; et/ou
- les une ou plusieurs thérapies sont de préférence sélectionnées dans le groupe constitué par une oxygénothérapie et un support d'organe extracorporel (ECOS).

13. Agent PPVO pour son utilisation selon la revendication 12, dans lequel la petite molécule est sélectionnée dans le groupe constitué par la loratadine, la daunorubicine, la midostaurine, le ponatinib, le silmitasertib, l'acide valproïque, l'halopéridol, la metformine, le migalastat, le S-vérapamil, l'indométhacine, le ruxolitinib, l'acide mycophénolique, l'entacapone, la ribavirine, E-52862, le mérimépodib, RVX-208, XL413, AC-55541, l'apicidine, AZ3451, AZ8838, la bafilomycine A1, CCT 365623, GB110, H-89, JQ1, PB28, PD-144418, RS-PPCC, TMCB, UCPH-101, ZINC1775962367, ZINC4326719, ZINC4511851, ZINC95559591, 4E2RCat, ABBV-744, le camostat, le captopril, CB5083, le chloramphénicol, la chloroquine, un composé 10 inhibiteur de PI4K-IIIβ, un composé 2 inhibiteur de la cyclophiline, CPI-0610, le dabrafenib, DBeQ, dBET6, IHVR-19029, le linézolid, le lisinopril, le minoxidil, ML240, MZ1, le nafamostat, le pévonedistat, PS3061, la rapamycine (sirolimus), la sangliféhrine A, le sapanisertib (INK128/M1N128), FK-506 (tacrolimus), la ternatine 4 (DA3), la tigécycline, le tomivosertib (eFT-508), le verdinexor, WDB002, l'azacitidine, le sélinexor, et la zotatifine (eFT226).

14. Composition ou kit comprenant un agent PPVO, de préférence selon la revendication 2 ou 3, et un agent coronavirus, de préférence selon la revendication 12.

15. Composition pharmaceutique ou kit pour son utilisation dans (i) la préparation d'un sujet pour une infection à coronavirus ou (ii) le traitement d'une infection à coronavirus chez un sujet, dans lequel la composition pharmaceutique ou le kit comprend un agent PPVO, de préférence tel que défini dans la revendication 2 ou 3, et de préférence un médicament supplémentaire, de manière davantage préférée tel que défini dans la revendication 12.
